(19) Europäisches Patentamt
European Patent Office
Office européen des brevets

(11) EP 4 722 213 A1

(12) **EUROPEAN PATENT APPLICATION**
published in accordance with Art. 153(4) EPC

(43) Date of publication:
**08.04.2026 Bulletin 2026/15**

(21) Application number: **24814523.7**

(22) Date of filing: **30.05.2024**

(51) International Patent Classification (IPC):
***C07D 471/04*** *(2006.01)* ***C07D 237/00*** *(2006.01)*
***A61K 31/5025*** *(2006.01)* ***A61P 35/00*** *(2006.01)*

(52) Cooperative Patent Classification (CPC):
**A61K 31/5025; A61P 35/00; C07D 237/00; C07D 471/04**

(86) International application number:
**PCT/CN2024/096254**

(87) International publication number:
**WO 2024/245326 (05.12.2024 Gazette 2024/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.05.2023 CN 202310632762**

(71) Applicants:
- **Sichuan Huiyu Pharmaceutical Co., Ltd.**
  **Neijiang, Sichuan 641000 (CN)**
- **Sichuan Huiyu Seacross Pharmaceutical Technology**
  **Co., Ltd.**
  **Chengdu, Sichuan 610200 (CN)**

(72) Inventors:
- **CHEN, Shoujun**
  **Neijiang, Sichuan 641000 (CN)**
- **QIANG, Xiaoming**
  **Neijiang, Sichuan 641000 (CN)**
- **NING, Dezheng**
  **Neijiang, Sichuan 641000 (CN)**
- **DING, Zhao**
  **Neijiang, Sichuan 641000 (CN)**
- **YU, Xingchi**
  **Neijiang, Sichuan 641000 (CN)**

(74) Representative: **Elzaburu S.L.P.**
**Paseo de la Castellana 259C**
**Torre de Cristal, planta 28**
**28046 Madrid (ES)**

# (54) NITROGEN-CONTAINING FUNCTIONAL GROUP-SUBSTITUTED PYRIDAZINOPYRIDONE DERIVATIVE AND USE THEREOF

(57) The present invention relates to the technical field of medicines, and in particular to a nitrogen-containing functional group-substituted pyridazinopyridone derivative as an SOS1 protein inhibitor and a use thereof. A provided compound has an obvious inhibitory effect on the activity of an SOS1 protein, can be used as an SOS1 protein inhibitor, has good druggability, can be used for preparing a drug for treating cancer and pathogenic rash that are mediated by an SOS1 protein and other diseases, and has a wide application prospect.



Processed by Luminess, 75001 PARIS (FR)

## Description

### TECHNICAL FIELD

The present disclosure relates to the technical field of medicines, and particularly relates to a nitrogen-containing functional group-substituted pyridazinopyridone derivative as an SOS1 protein inhibitor and use thereof.

### BACKGROUND

SOS1 (son of sevenless homolog 1) protein is a regulatory protein widely expressed in cells. As a type of guanine nucleotide exchange factor for Ras or Rac1 proteins, it plays an important regulatory role in intracellular Ras or Rac1 signal transduction pathways (Baltanás, F.C.; Zarich, N.; Rojas-Cabañeros, J.M.; Santos, E. Biochim. Biophys. Acta. Rev. Cancer. 2020,1874,188445). The role of the SOS1 protein in the Ras signal transduction pathway is to promote Ras to release GDP and bind to GTP, thereby converting the Ras protein from an inactive state to an active state.

Currently, the known RAS family has three genes: KRAS, NRAS, and HRAS. Mutations of RAS enzymes are closely associated with tumorigenesis, with mutations detected in approximately 25% of all tumors (de Castro Carpeño, J.; Belda-Iniesta C.; Transl Lung Cancer Res. 2013,2(2),142-51.). In different types of tumors, the types of RAS mutations also vary. RAS mutations (KRAS, NRAS, HRAS) are present in 90% of pancreatic cancers, 45% of colon cancers, and 35% of lung cancers. Non-small cell lung cancer (NSCLC) accounts for 80% of all lung cancer cases (Jemal, B.; et al, CA Cancer J. Clin. 2011, 61(2), 69-90). The Ras proto-oncogene is the most common mutated gene in NSCLC (Prior, L. et al, Cancer Res. 2012, 72(10), 2457-2467; Li, L. et al, J Exp Clin Cancer Res. 2018, 37(1), 178), wherein, the v-Ki-ras2 Kirsten rat sarcoma viral oncogene (Kras) accounts for 90% of RAS mutations in lung adenocarcinoma (Hunter, J.C.; et al, Mol. Cancer Res. 2015,13(9),1325-35).

As the "pacemaker" for Kras, SOS1 plays a crucial regulatory role in many intracellular signal transduction pathways. Studies have shown that inhibiting SOS1 activity effectively suppresses the proliferation of cancer cells driven by all major Kras gene mutations (Kessler, D.; Gerlach, D.; Kraut, N.; McConnell, D.B. Curr. Opin. Chem. Biol. 2021, 62,109-118). In addition, a clinical study involving 62 ovarian cancer patients showed that the expressions of RAS mutations and SOS1 mutations are significantly increased in ovarian cancer tissues, and both are associated with shortened PFS (progression-free survival) in terms of prognosis, suggesting that targeted therapy against RAS and SOS1 holds potential value in ovarian cancer patients. Meanwhile, SOS1 gene mutations have also been identified in many other types of cancer cells, such as embryonal rhabdomyosarcoma, Sertoli cell testicular tumors, cutaneous granular cell tumors (Denayer, et al., Genes Chromosomes Cancer, 2010, 49(3):242-52) and lung adenocarcinoma (Cancer Genome Atlas Research Network, Nature, 2014, 511(7511), 543-50). In addition, studies have found that the SOS1 gene is highly expressed in both bladder cancer (Watanabe, et al., IUBMB Life., 2000, 49(4), 317-20) and prostate cancer (Timofeeva, et al., Int. J. Oncol., 2009, 35(4):751-60). In the development of chronic granulocytic leukemia, BCR-ABL activates GRB2 through phosphorylation, recruits SOS1, and thereby continuously activates the Ras/MAPK signaling pathway, leading to the malignant proliferation of hematopoietic stem cells. Thus, the SOS1 protein is also a potential new target for the treatment of chronic granulocytic leukemia. Beyond cancer, studies have shown that hereditary SOS1 gene mutations are also closely associated with several pathogenic skin rash diseases, such as Noonan syndrome (NS), cardiofaciocutaneous syndrome (CFC), and hereditary gingival fibromatosis type I (Pierre, et al., Biochem.Pharmacol., 2011, 82(9):1049-56).

SOS1 inhibition is mechanistically quite similar to SHP2 inhibition (Nichols, R.J.; et al. Nat Cell Biol. 2018, 20(9),1064-1073), suggesting that SOS1 inhibition can also enhance the efficacy of $KRAS^{G12C}$ and MEK inhibitors. Preliminary data show that significant synergistic effects exist between SOS1 and MEK inhibitions in multiple PDX (patient-derived xenograft) models of G12 and G13 KRAS mutations (Hofmann, M.H.; et al, Cancer Discov. 2020,10.1158/2159-8290.CD-20-0142.). In addition to inhibiting the feedback activation of $Kras^{wt}$, since $KRAS^{G12C}$ allosteric inhibitors can only bind to $KRAS^{GDP}$, inhibiting SOS1 has the potential advantage of directly enhancing the efficacy of $KRAS^{G12C}$ inhibitors by increasing the amount of mutant $KRAS^{G12C}$ (Hillig, R.C.; et al, Proc. Nat. Acad. Sci. U S A. 2019,116(7),2551-2560). Although further research is still needed, as a strategy of combination therapy, inhibiting SOS1 clinically holds significant application potential.

Currently, there is still no medicament targeting the SOS1 target approved for marketing in the world, and all the compounds under investigation are in the early clinical or preclinical research stage. Although a small number of pharmaceutical companies or research institutions have conducted relevant research on SOS1 inhibitors and related patents have been published, for example, Boehringer Ingelheim disclosed a class of benzylamine-substituted quinazoline derivatives as SOS1 inhibitors (US20190358230A1), and Bayer Pharmaceuticals disclosed a class of 2-methyl-azaquinazoline compounds as SOS1 inhibitors (WO2019201848A1), the SOS1 inhibitors developed so far are far from meeting clinical needs. Thus, the development of new SOS1 inhibitors still holds extremely broad clinical application prospects.

## SUMMARY

The main technical problem addressed by the present disclosure is to provide a class of nitrogen-containing functional group-substituted pyridazinopyridone derivatives that exhibit potent inhibitory effects on SOS1.

In order to solve the above technical problem, the present disclosure provides a compound represented by Formula I, or a tautomer, stereoisomer, solvate, metabolite, isotopically labeled compound, pharmaceutically acceptable salt or co-crystal thereof:

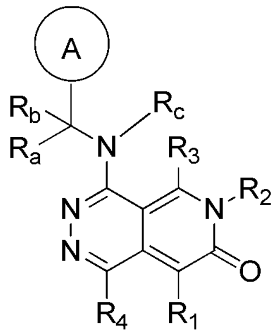

Formula I

wherein:

ring A is selected from aryl, heteroaryl, the aryl, heteroaryl are optionally substituted with a substituent selected from halogen, alkyl, alkoxy, -OH, -CN, $-NR_5R_6$, $-SF_5$, $-SO_2R_{10}$, the alkyl, alkoxy are further optionally substituted with a substituent selected from halogen, $-NR_5R_6$, -OH, wherein, $R_5$, $R_6$ are independently selected from H, alkyl, $R_{10}$ is alkyl and optionally substituted with halogen; or, two substituents on the aryl or heteroaryl are connected to form an aliphatic carbocyclic ring, the aliphatic carbocyclic ring is optionally substituted with halogen;

$R_a$, $R_b$, $R_c$ are independently selected from H, alkyl;

$R_1$, $R_3$ are independently selected from H, halogen, alkyl, alkoxy, -OH, -CN, $-NR_7R_8$, the alkyl, alkoxy are optionally substituted with a substituent selected from halogen, $-NR_7R_8$, wherein $R_7$, $R_8$ are independently selected from H, alkyl;

$R_2$ is selected from H, alkyl, cycloalkyl, heterocycloalkyl, cycloalkenyl, heterocycloalkenyl, the cycloalkyl, heterocycloalkyl, cycloalkenyl, heterocycloalkenyl are optionally substituted with a substituent selected from halogen, alkyl, alkoxy,

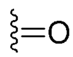 ,

$-C(=O)R_9$, $R_9$ is selected from alkyl, cycloalkyl, heterocycloalkyl;

$R_4$ is $-NR_{11}R_{12}$, $R_{11}$, $R_{12}$ are independently selected from H, alkyl, cycloalkyl, heterocycloalkyl, wherein, when $R_{11}$ and/or $R_{12}$ are alkyl, the alkyl is optionally substituted with a substituent selected from -OH, alkenyl, halogen, $-NR_{14}R_{15}$, cycloalkyl, $R_{14}$, $R_{15}$ are independently selected from H, alkyl; when $R_{11}$ and/or $R_{12}$ are selected from cycloalkyl, heterocycloalkyl, the cycloalkyl, heterocycloalkyl are optionally substituted with alkyl; or,

$R_{11}$, $R_{12}$ and the nitrogen atom to which they are attached collectively form a heterocycloalkyl, the heterocycloalkyl is optionally substituted with a substituent selected from halogen, alkyl, cycloalkyl, - $C(=O)R_{13}$,

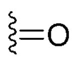 ,

-OH, the alkyl is further optionally substituted with a substituent selected from -OH, cycloalkyl, wherein, $R_{13}$ is alkyl; or, $R_{11}$, $R_{12}$ and the nitrogen atom to which they are attached collectively form a heteroaryl.

Further, ring A is selected from aryl, heteroaryl, the aryl, heteroaryl are optionally substituted with a substituent selected from halogen, alkyl, alkoxy, -OH, -CN, $-NR_5R_6$, $-SF_5$, $-SO_2R_{10}$, the alkyl, alkoxy are further optionally substituted with a substituent selected from halogen, $-NR_5R_6$, wherein, $R_5$, $R_6$ are independently selected from H, alkyl, $R_{10}$ is alkyl and optionally substituted with halogen.

Further, $R_1$, $R_3$ are independently selected from H, alkyl, alkoxy, -OH, -CN, -$NR_7R_8$, the alkyl, alkoxy are optionally substituted with a substituent selected from halogen, -$NR_7R_8$, wherein $R_7$, $R_8$ are independently selected from H, alkyl.

Further, $R_4$ is -$NR_{11}R_{12}$, wherein $R_{11}$, $R_{12}$ are independently selected from H, alkyl, cycloalkyl, the alkyl is optionally substituted with a substituent selected from -OH, alkenyl, the cycloalkyl is optionally substituted with alkyl; or, $R_{11}$, $R_{12}$ and the nitrogen atom to which they are attached collectively form a heterocycloalkyl, the heterocycloalkyl is optionally substituted with a substituent selected from halogen, alkyl, cycloalkyl, -C(=O)$R_{13}$,

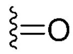,

$R_{13}$ is alkyl; or, $R_{11}$, $R_{12}$ and the nitrogen atom to which they are attached collectively form a heteroaryl.

Alternatively, the present disclosure provides the compound represented by Formula I, or tautomer, stereoisomer, solvate, metabolite, isotopically labeled compound, pharmaceutically acceptable salt or co-crystal thereof:

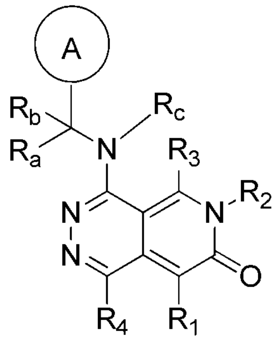

Formula I

wherein:

ring A is selected from aryl, heteroaryl, the aryl, heteroaryl are optionally substituted with a substituent selected from halogen, alkyl, alkoxy, -OH, -CN, -$NR_5R_6$, -$SF_5$, -$SO_2R_{10}$, the alkyl, alkoxy are further optionally substituted with a substituent selected from halogen, -$NR_5R_6$, wherein, $R_5$, $R_6$ are independently selected from H, alkyl, $R_{10}$ is alkyl and optionally substituted with halogen;

$R_a$, $R_b$, $R_c$ are independently selected from H, alkyl;

$R_1$, $R_3$ are independently selected from H, alkyl, alkoxy, -OH, -CN, -$NR_7R_8$, the alkyl, alkoxy are optionally substituted with a substituent selected from halogen, -$NR_7R_8$, wherein $R_7$, $R_8$ are independently selected from H, alkyl;

$R_2$ is selected from H, alkyl, cycloalkyl, heterocycloalkyl, cycloalkenyl, heterocycloalkenyl, the cycloalkyl, heterocycloalkyl, cycloalkenyl, heterocycloalkenyl are optionally substituted with a substituent selected from halogen, alkyl, alkoxy,

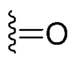,

-C(=O)$R_9$, $R_9$ is selected from alkyl, cycloalkyl, heterocycloalkyl;

$R_4$ is -$NR_{11}R_{12}$, wherein $R_{11}$, $R_{12}$ are independently selected from H, alkyl, cycloalkyl, the alkyl is optionally substituted with a substituent selected from -OH, alkenyl, the cycloalkyl is optionally substituted with alkyl; or, $R_{11}$, $R_{12}$ and the nitrogen atom to which they are attached collectively form a heterocycloalkyl, the heterocycloalkyl is optionally substituted with a substituent selected from halogen, alkyl, cycloalkyl, - C(=O)$R_{13}$,

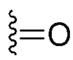,

$R_{13}$ is alkyl; or, $R_{11}$, $R_{12}$ and the nitrogen atom to which they are attached collectively form a heteroaryl.

Further, ring A is selected from 6~12 membered aryl, 5~12 membered heteroaryl, the heteroaryl contains

heteroatoms selected from N, O, S, the substituents of the above groups are as defined in any one of the above technical solutions.

Preferably, ring A is phenyl, the substituents thereof are as defined in any one of the above technical solutions.

Further, the substituent contained in ring A is selected from halogen, C1~C6 alkyl, C1~C6 alkoxy, -OH, -CN, $-NR_5R_6$, $-SF_5$, $-SO_2R_{10}$, the alkyl, alkoxy are further optionally substituted with a substituent selected from halogen, $-NR_5R_6$, -OH, wherein, $R_5$, $R_6$ are independently selected from H, C1~C6 alkyl, $R_{10}$ is C1~C6 alkyl and optionally substituted with halogen; or, two substituents on ring A are connected to form a 5~6 membered aliphatic carbocyclic ring, the aliphatic carbocyclic ring is optionally substituted with halogen.

Preferably, the substituent contained in ring A is selected from halogen, C1~C6 alkyl, -CN, $-NR_5R_6$, - $SF_5$, $-SO_2R_{10}$, the alkyl is further optionally substituted with a substituent selected from halogen, -OH, wherein, $R_5$, $R_6$ are independently selected from H, C1~C6 alkyl, $R_{10}$ is C1~C6 alkyl and optionally substituted with halogen; or, two substituents on ring A are connected to form a 5~6 membered aliphatic carbocyclic ring, the aliphatic carbocyclic ring is optionally substituted with halogen.

Preferably, the substituent contained in ring A is selected from halogen, C1~C6 alkyl, C1~C6 alkoxy, - OH, -CN, $-NR_5R_6$, $-SF_5$, $-SO_2R_{10}$, the alkyl, alkoxy are further optionally substituted with a substituent selected from halogen, $-NR_5R_6$, wherein, $R_5$, $R_6$ are independently selected from H, C1~C6 alkyl, $R_{10}$ is C1~C6 alkyl and optionally substituted with halogen.

Preferably, the substituent contained in ring A is selected from halogen, C1~C6 alkyl, -CN, $-NR_5R_6$, the alkyl is further optionally substituted with halogen, $R_5$, $R_6$ are independently selected from H, C1~C6 alkyl.

Preferably, the substituent contained in ring A is selected from -F, $-CH_3$, $-CHF_2$, $-CF_3$, -CN, $-NH_2$, $-SF_5$, $-SO_2CHF_2$, $-CF_2CH_2OH$; or, two substituents on ring A are connected to form

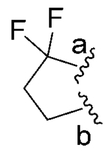,

wherein the two ends of a and b are respectively connected to two adjacent ring carbon atoms on ring A.

Preferably, the substituent contained in ring A is selected from -F, $-CH_3$, $-CHF_2$, $-CF_3$, -CN, $-NH_2$.

Further, ring A is selected from:

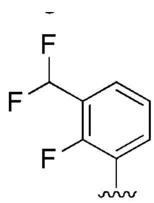, 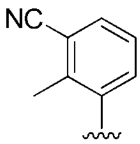, 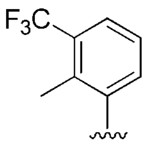, 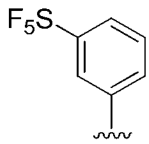,

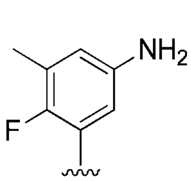, 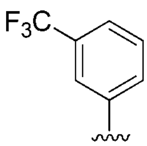, 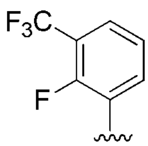, 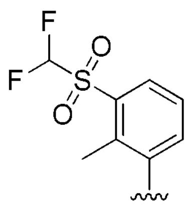, 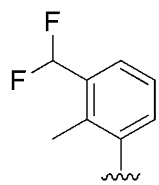, 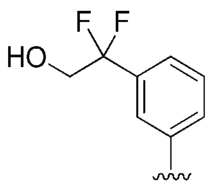,

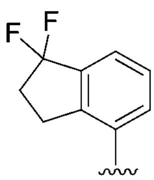.

Preferably, ring A is selected from:

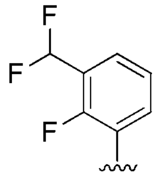, 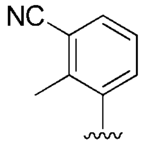, 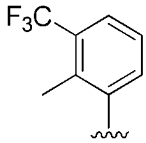.

Further, $R_a$, $R_b$, $R_c$ are independently selected from H, C1-C6 alkyl.
Preferably, $R_a$, $R_b$, $R_c$ are independently selected from H, methyl.
Preferably, $R_a$ is methyl, $R_b$ is H, $R_c$ is H.
Further, the structure of the compound is represented by Formula II:

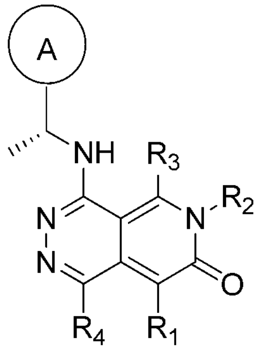

Formula II

wherein each group is as defined in any one of the above technical solutions.
Further, $R_1$, $R_3$ are independently selected from H, halogen, C1~C6 alkyl, C1~C6 alkoxy, -OH, -CN, - $NR_7R_8$, the alkyl, alkoxy are optionally substituted with a substituent selected from halogen, -$NR_7R_8$, wherein $R_7$, $R_8$ are independently selected from H, C1~C6 alkyl.
Further, $R_1$, $R_3$ are independently selected from H, C1~C6 alkyl, C1~C6 alkoxy, -OH, -CN, -$NR_7R_8$, the C1~C6 alkyl, C1~C6 alkoxy are optionally substituted with a substituent selected from halogen, -$NR_7R_8$, wherein $R_7$, $R_8$ are independently selected from H, C1~C6 alkyl.
Preferably, both $R_1$, $R_3$ are H.
Further, $R_2$ is selected from H, C1~C6 alkyl, 3~10 membered cycloalkyl, 3~10 membered heterocycloalkyl, 3~10 membered cycloalkenyl, 3~10 membered heterocycloalkenyl, the cycloalkyl, heterocycloalkyl, cycloalkenyl, heterocycloalkenyl are optionally substituted with a substituent selected from halogen, C1~C6 alkyl, C1~C6 alkoxy,

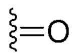,

-C(=O)$R_9$, $R_9$ is selected from C1~C6 alkyl, 3~10 membered cycloalkyl, 3~10 membered heterocycloalkyl, the heterocycloalkyl, heterocycloalkenyl contain heteroatoms selected from N, O, S.
Preferably, $R_2$ is selected from H, C1~C6 alkyl, 3~10 membered cycloalkyl, 3~10 membered heterocycloalkyl, 3~10 membered heterocycloalkenyl, the cycloalkyl, heterocycloalkyl, heterocycloalkenyl are optionally substituted with a substituent selected from C1~C6 alkyl, C1~C6 alkoxy, -C(=O)$R_9$, $R_9$ is selected from C1~C6 alkyl, 3~10 membered heterocycloalkyl, the heterocycloalkyl, heterocycloalkenyl contain 1~2 heteroatoms selected from N, O, S.
Preferably, $R_2$ is selected from H, C1~C6 alkyl, 3 membered cycloalkyl, 5~6 membered heterocycloalkyl, 6 membered heterocycloalkenyl, the cycloalkyl, heterocycloalkyl, heterocycloalkenyl are optionally substituted with a substituent selected from C1~C6 alkyl, C1~C6 alkoxy, -C(=O)$R_9$, $R_9$ is selected from C1~C6 alkyl, 6 membered heterocycloalkyl, the heterocycloalkyl, heterocycloalkenyl contain 1~2 heteroatoms selected from N, O.
Preferably, $R_2$ is selected from C1~C6 alkyl, 3~8 membered cycloalkyl, 5~8 membered heterocycloalkyl, the cycloalkyl, heterocycloalkyl are optionally substituted with a substituent selected from halogen, C1~C6 alkyl, the heterocycloalkyl contains 1~2 heteroatoms selected from N, O.
Preferably, $R_2$ is C1~C6 alkyl; or,
**[0035]** $R_2$ is selected from cyclopropyl, cyclobutyl, tetrahydrofuranyl, piperidinyl, piperazinyl, morpholinyl, 3-azabicyclo [3.3.0]octy, the above groups are optionally substituted with a substituent selected from halogen, C1~C6 alkyl.
Preferably, $R_2$ is cyclopropyl, the cyclopropyl is optionally substituted with C1~C6 alkyl.

Preferably, $R_2$ is selected from: methyl, ethyl,

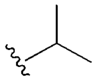, 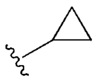, 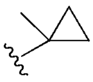, 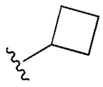, 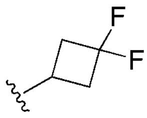,

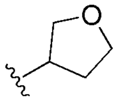, 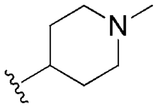, 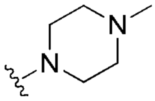, 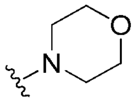, 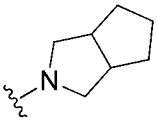.

Preferably, $R_2$ is selected from:

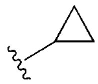, 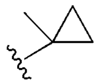.

Further, $R_4$ is $-NR_{11}R_{12}$, $R_{11}$, $R_{12}$ are independently selected from **H,** C1~C6 alkyl, 3~10 membered cycloalkyl, 3~10 membered heterocycloalkyl, the heterocycloalkyl contains heteroatoms selected from N, O, S; wherein, when $R_{11}$ and/or $R_{12}$ are C1~C6 alkyl, the alkyl is optionally substituted with a substituent selected from halogen, $-NR_{14}R_{15}$, 3~10 membered cycloalkyl, $R_{14}$, $R_{15}$ are independently selected from H, C1~C6 alkyl; when $R_{11}$ and/or $R_{12}$ are selected from 3~10 membered cycloalkyl, 3~10 membered heterocycloalkyl, the cycloalkyl, heterocycloalkyl are optionally substituted with C1~C6 alkyl.

Preferably, $R_4$ is $-NR_{11}R_{12}$, $R_{11}$, $R_{12}$ are independently selected from H, C1~C6 alkyl, 3~8 membered cycloalkyl, 4~8 membered heterocycloalkyl, the heterocycloalkyl contains 1~2 heteroatoms selected from N, O; wherein, when $R_{11}$ and/or $R_{12}$ are C1~C6 alkyl, the alkyl is optionally substituted with a substituent selected from halogen, $-NR_{14}R_{15}$, 3~8 membered cycloalkyl, $R_{14}$, $R_{15}$ are independently selected from H, C1~C6 alkyl; when $R_{11}$ and/or $R_{12}$ are selected from 3~8 membered cycloalkyl, 4~8 membered heterocycloalkyl, the cycloalkyl, heterocycloalkyl are optionally substituted with C1~C6 alkyl.

Preferably, $R_4$ is $-NR_{11}R_{12}$, wherein $R_{11}$, $R_{12}$ are independently selected from H, C1~C6 alkyl, 3~10 membered cycloalkyl, the C1~C6 alkyl is optionally substituted with a substituent selected from -OH, C2~C6 alkenyl, the 3~10 membered cycloalkyl is optionally substituted with C1~C6 alkyl.

Preferably, $R_4$ is $-NR_{11}R_{12}$, wherein $R_{11}$, $R_{12}$ are independently selected from H, C1-C6 alkyl, 3~5 membered cycloalkyl, the C1~C6 alkyl is optionally substituted with a substituent selected from -OH, C2~C6 alkenyl, the 3~5 membered cycloalkyl is optionally substituted with C1~C6 alkyl.

Preferably, $R_4$ is $-NR_{11}R_{12}$, $R_{11}$, $R_{12}$ are independently selected from H, C1~C6 alkyl, cyclopropyl, bicyclo[1.1.1]pentyl, oxacyclobutyl, tetrahydropyrrolyl, tetrahydrofuranyl, piperidinyl, piperazinyl, morpholinyl, 1-azabicyclo[2.2.2]octyl; wherein, when $R_{11}$ and/or $R_{12}$ are C1~C6 alkyl, the alkyl is optionally substituted with a substituent selected from halogen, $-NR_{14}R_{15}$, 3 membered cycloalkyl, $R_{14}$, $R_{15}$ are independently selected from H, C1~C6 alkyl; when $R_{11}$ and/or $R_{12}$ are selected from cyclopropyl, bicyclo[1.1.1]pentyl, oxacyclobutyl, tetrahydropyrrolyl, tetrahydrofuranyl, piperidinyl, piperazinyl, morpholinyl, 1-azabicyclo[2.2.2]octyl, the above groups are optionally substituted with C1~C6 alkyl.

Preferably, $R_4$ is $-NR_{11}R_{12}$, wherein $R_{11}$, $R_{12}$ are independently selected from H, methyl, ethyl, propyl, cyclopropyl, the cyclopropyl is optionally substituted with methyl.

Preferably, $R_4$ is selected from:

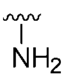, 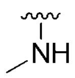, 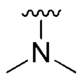, 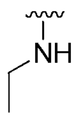, 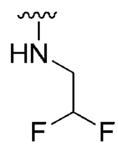, 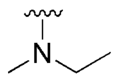,

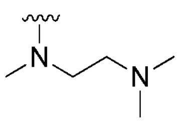, 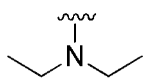, 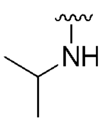, 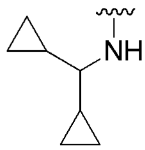, 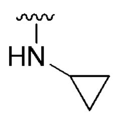, 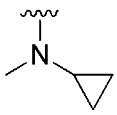, 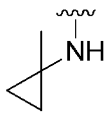, 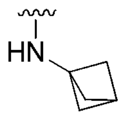,

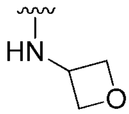, 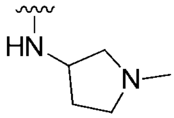, 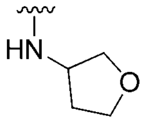, 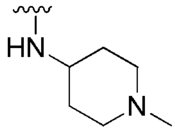, 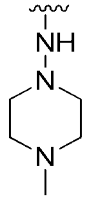, 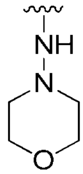, 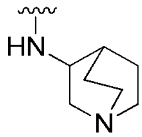.

Preferably, $R_4$ is selected from:

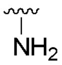 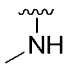 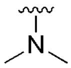 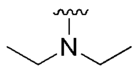 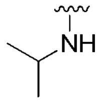 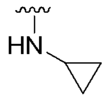 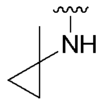

.

Preferably, $R_4$ is

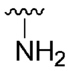.

Further, $R_4$ is $-NR_{11}R_{12}$, $R_{11}$, $R_{12}$ and the nitrogen atom to which they are attached collectively form a 3~10 membered heterocycloalkyl, the heterocycloalkyl contains heteroatoms selected from N, O, S, the heterocycloalkyl is optionally substituted with a substituent selected from halogen, C1~C6 alkyl, 3~10 membered cycloalkyl, $-C(=O)R_{13}$,

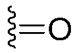,

-OH, the alkyl is further optionally substituted with a substituent selected from -OH, 3~10 membered cycloalkyl, wherein, $R_{13}$ is C1~C6 alkyl.

Preferably, $R_4$ is $-NR_{11}R_{12}$, wherein $R_{11}$, $R_{12}$ and the nitrogen atom to which they are attached collectively form a 3~10 membered heterocycloalkyl, the heterocycloalkyl contains heteroatoms selected from N, O, S, the heterocycloalkyl is optionally substituted with a substituent selected from halogen, C1~C6 alkyl, 3~10 membered cycloalkyl, $-C(=O)R_{13}$,

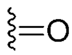,

$R_{13}$ is C1~C6 alkyl.

Preferably, $R_4$ is $-NR_{11}R_{12}$, $R_{11}$, $R_{12}$ and the nitrogen atom to which they are attached collectively form a 4~10 membered heterocycloalkyl, the heterocycloalkyl contains 1~2 heteroatoms selected from N, O, S, the heterocycloalkyl is optionally substituted with a substituent selected from halogen, C1~C6 alkyl, 3~5 membered cycloalkyl, $-C(=O)R_{13}$,

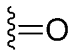,

-OH, the alkyl is further optionally substituted with a substituent selected from -OH, 3~5 membered cycloalkyl, wherein, $R_{13}$ is C1~C6 alkyl.

Preferably, $R_4$ is $-NR_{11}R_{12}$, wherein $R_{11}$, $R_{12}$ and the nitrogen atom to which they are attached collectively form a 4~6 membered heterocycloalkyl, the heterocycloalkyl contains 1~2 heteroatoms selected from N, O, S, the heterocycloalkyl is optionally substituted with a substituent selected from halogen, C1~C6 alkyl, $-C(=O)R_{13}$,

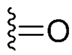,

$R_{13}$ is C1~C6 alkyl.

Preferably, $R_4$ is $-NR_{11}R_{12}$, $R_{11}$, $R_{12}$ and the nitrogen atom to which they are attached collectively form

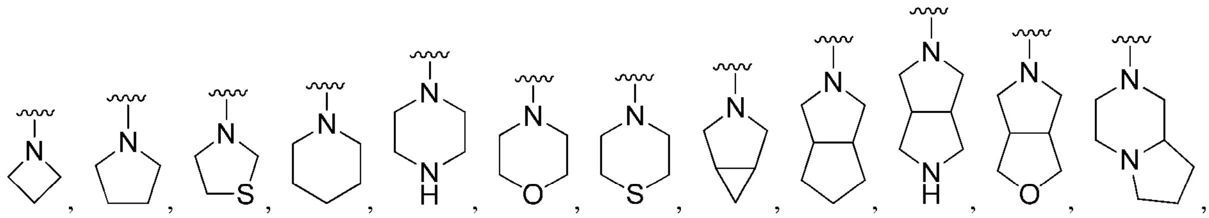

, , , , , , , , , , , ,

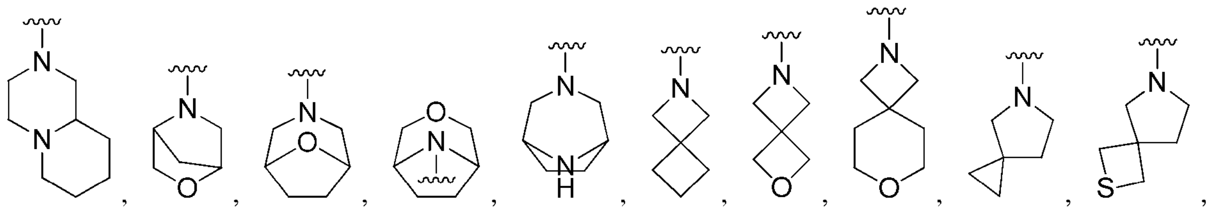

, , , , , , , , , , ,

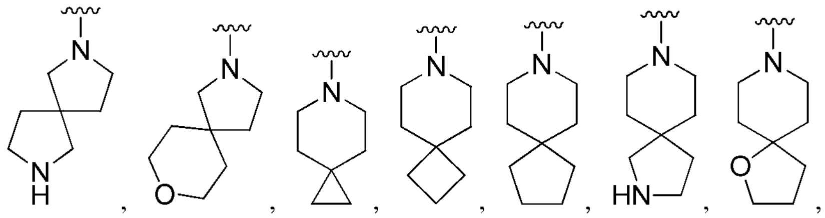

, , , , , , ,

the above groups are optionally substituted with a substituent selected from halogen, C1~C6 alkyl, 3~5 membered cycloalkyl, -C(=O)$R_{13}$,

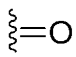

,

-OH, the alkyl is further optionally substituted with a substituent selected from -OH, 3~5 membered cycloalkyl, wherein, $R_{13}$ is C1~C6 alkyl.

Preferably, $R_4$ is -$NR_{11}R_{12}$, wherein $R_{11}$, $R_{12}$ and the nitrogen atom to which they are attached collectively form azetidinyl, tetrahydropyrrolyl, tetrahydrothiazolyl, piperidinyl, piperazinyl, morpholinyl, thiomorpholinyl, 3-azabicyclo [3.1.0] hexyl, the above groups are optionally substituted with a substituent selected from fluorine, methyl, -C(=O)$R_{13}$,

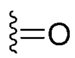

,

$R_{13}$ is methyl.

Preferably, $R_4$ is -$NR_{11}R_{12}$, wherein $R_{11}$, $R_{12}$ and the nitrogen atom to which they are attached collectively form azetidinyl, piperazinyl, the above groups are optionally substituted with a substituent selected from fluorine, methyl, -C(=O)$R_{13}$,

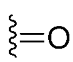

, $R_{13}$ is methyl.

Preferably, $R_4$ is selected from:

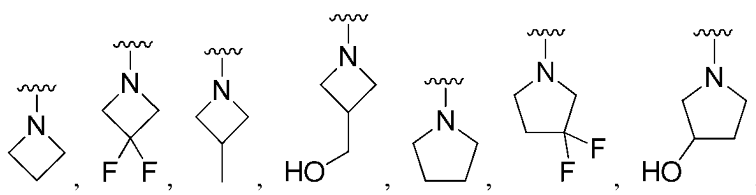

, , , , , , ,

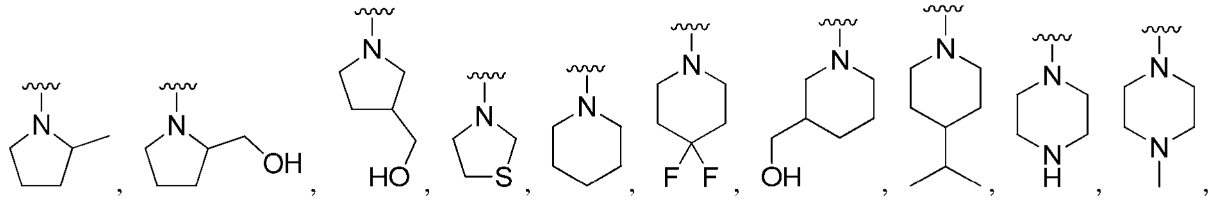
, , , , , , , , , ,

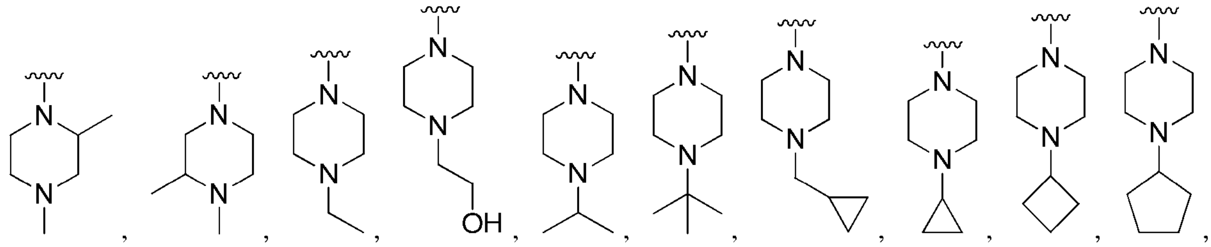
, , , , , , , , , , ,

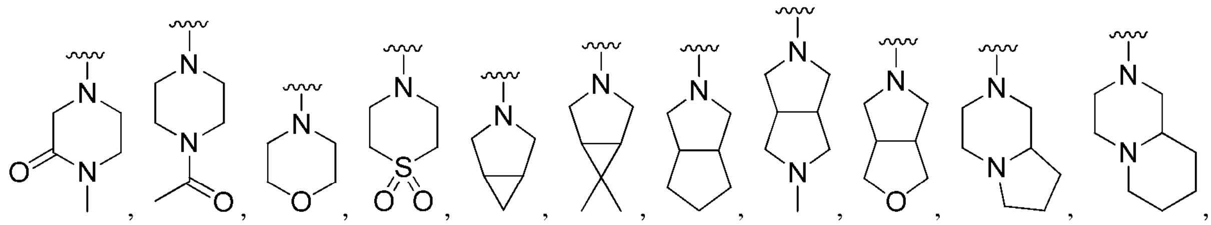
, , , , , , , , , , , ,

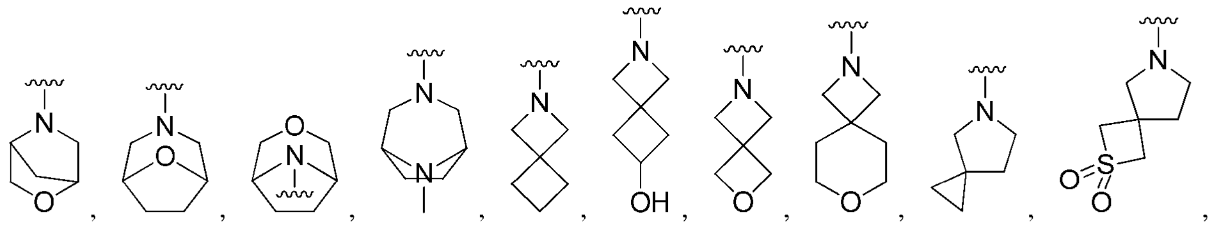
, , , , , , , , , , ,

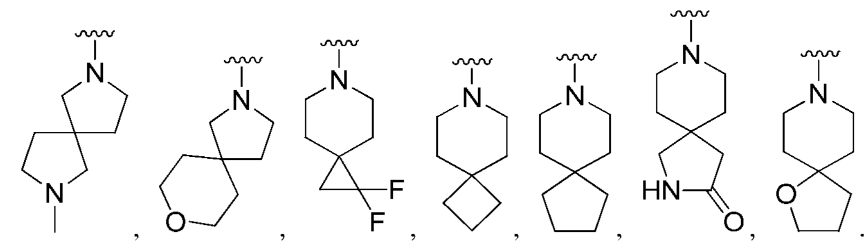
, , , , , , .

Preferably, the configuration of $R_4$ is selected from at least one of the following:

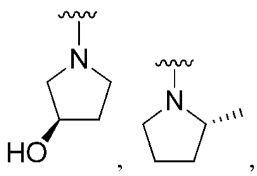
, ,

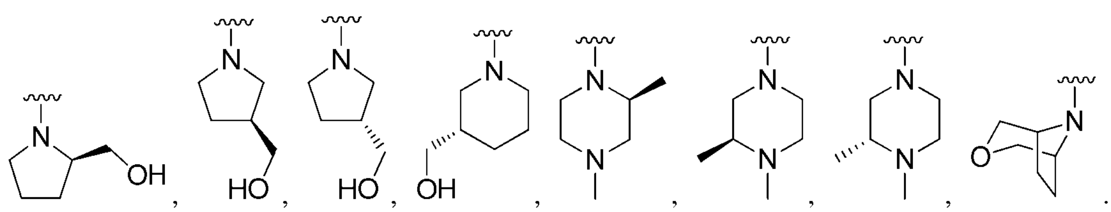
, , , , , , , .

Preferably, $R_4$ is selected from:

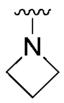 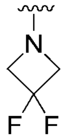 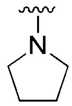 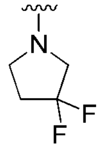 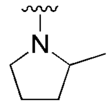 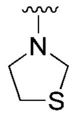 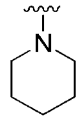

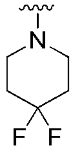 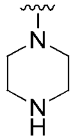 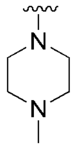 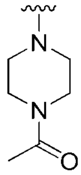 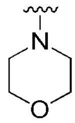 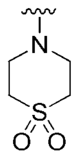 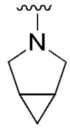 .

Preferably,

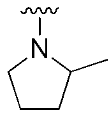 is 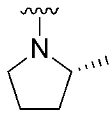 .

Further, $R_4$ is $-NR_{11}R_{12}$, wherein $R_{11}$, $R_{12}$ and the nitrogen atom to which they are attached collectively form a 5~10 membered heteroaryl, the heteroaryl contains heteroatoms selected from N, O, S.

Preferably, $R_4$ is $-NR_{11}R_{12}$, wherein $R_{11}$, $R_{12}$ and the nitrogen atom to which they are attached collectively form a 5 membered heteroaryl, the heteroaryl contains 1~3 nitrogen heteroatoms.

Preferably, $R_4$ is $-NR_{11}R_{12}$, wherein $R_{11}$, $R_{12}$ and the nitrogen atom to which they are attached collectively form pyrazolyl, imidazolyl, 1,2,3-triazolyl, 1,2,4-triazolyl.

Preferably, $R_4$ is selected from:

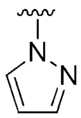 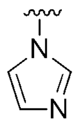 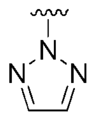 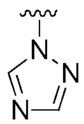 .

Further, the compound is selected from one of the following:

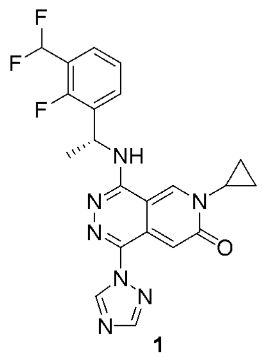
**1**

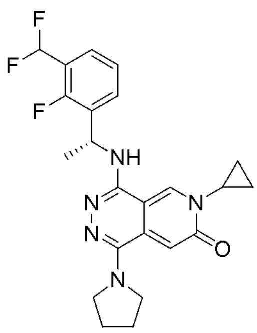
**4**

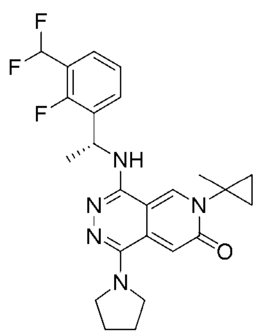
**5**

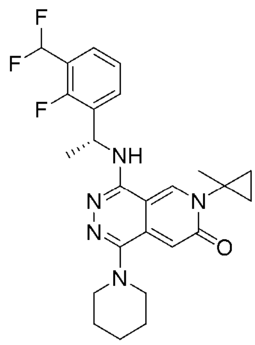
**6**

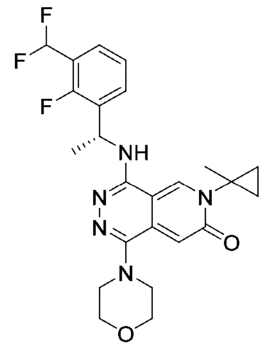

**7**

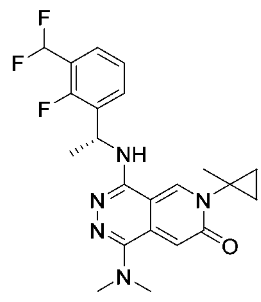

**8**

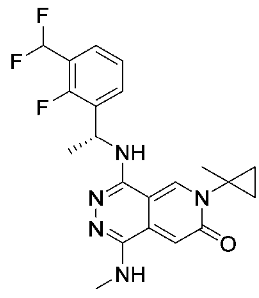

**9**

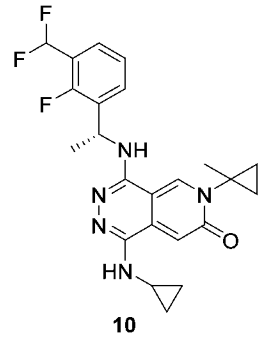

**10**

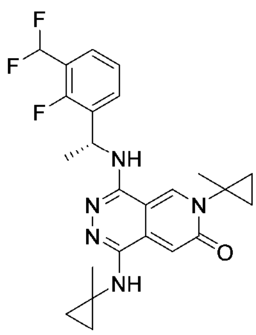

**11**

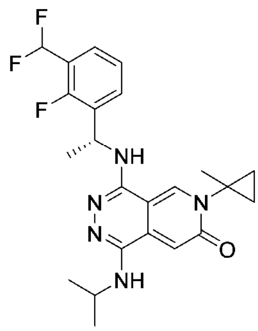

**12**

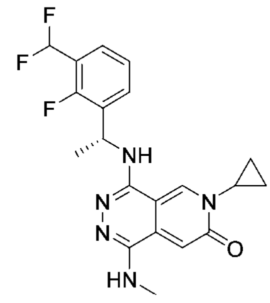

**13**

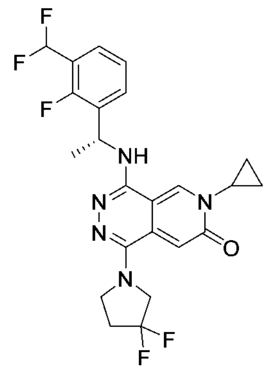

**14**

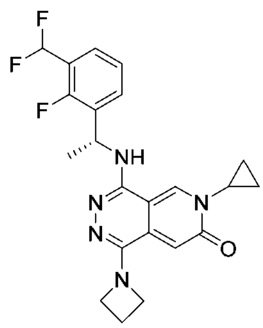

**15**

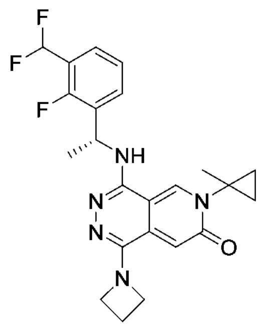

**16**

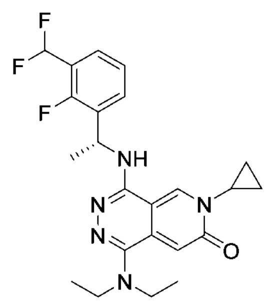

**17**

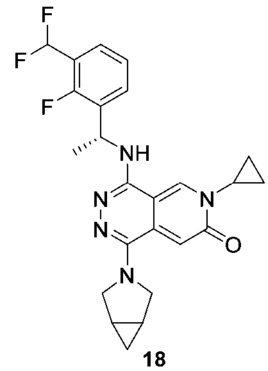

**18**

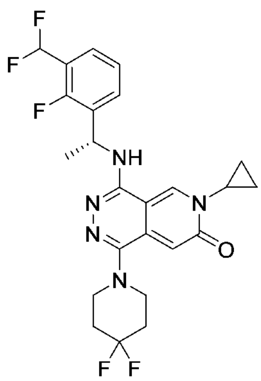

**19**

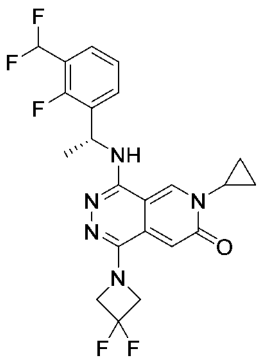

**20**

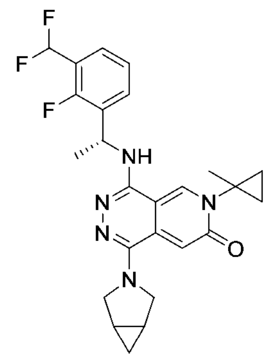

**21**

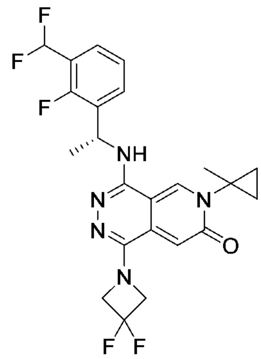

**22**

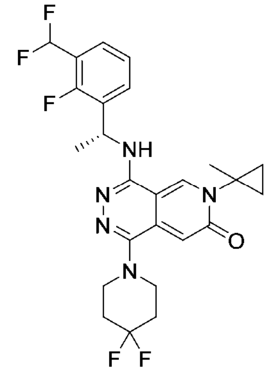
23

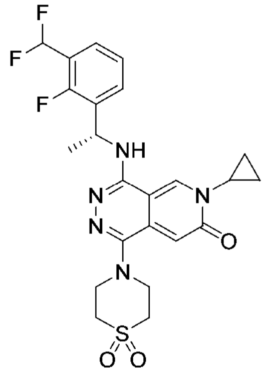
24

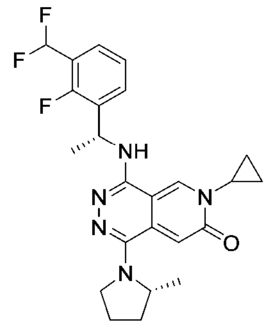
25

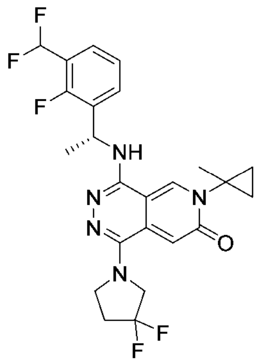
26

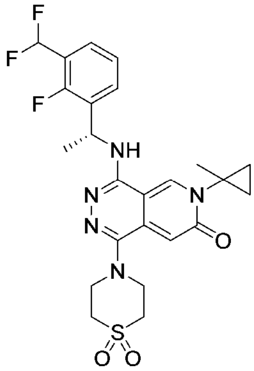
27

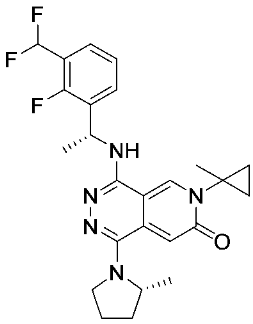
28

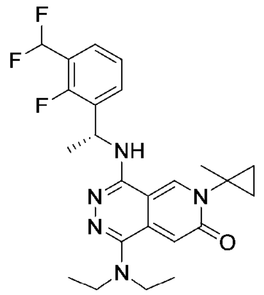
29

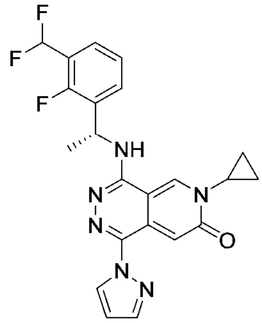
30

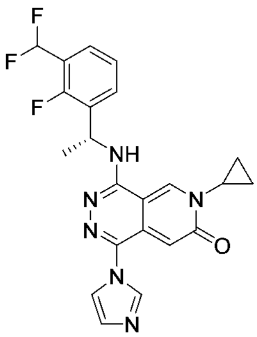
31

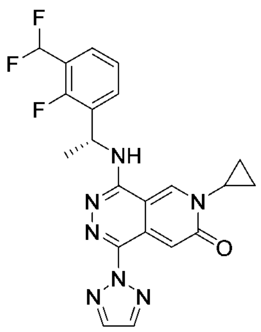
32

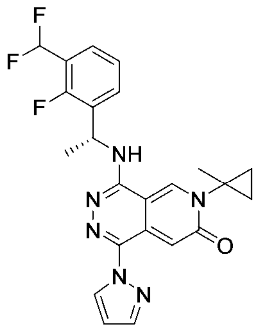
33

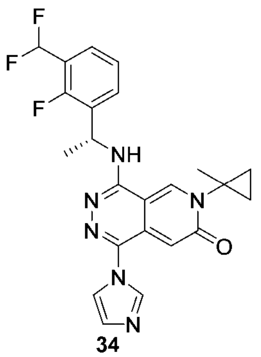
34

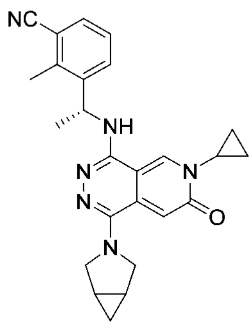
35

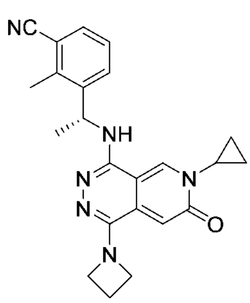
36

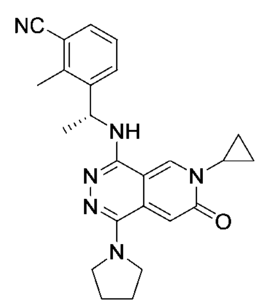
37

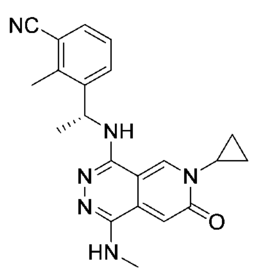
38

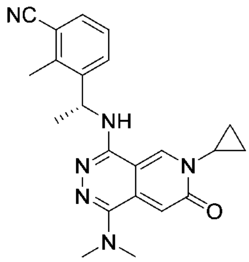
39

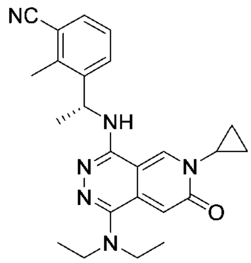
40

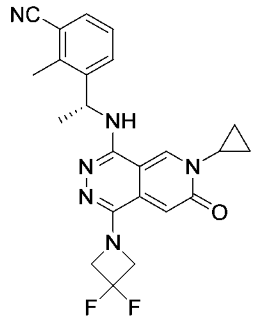
41

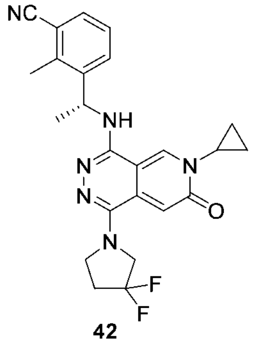
42

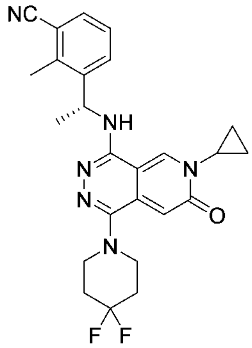
43

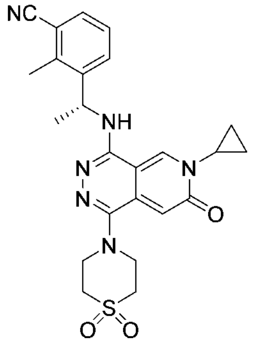
44

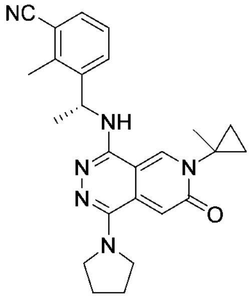
45

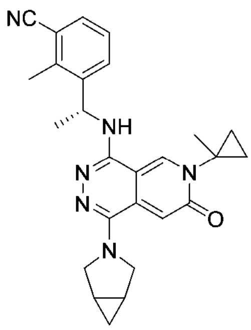
46

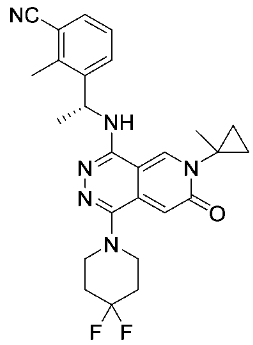
47

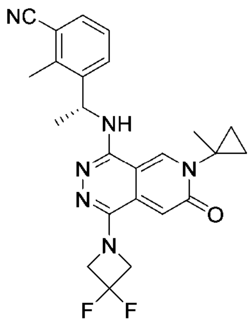
48

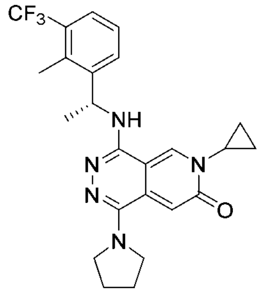
49

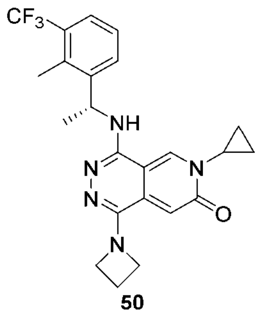
50

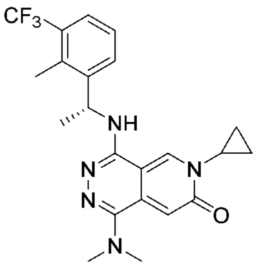
51

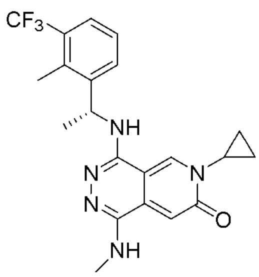
52

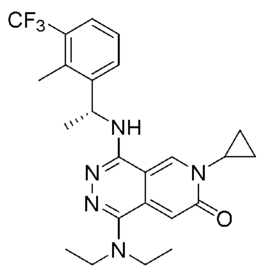
53

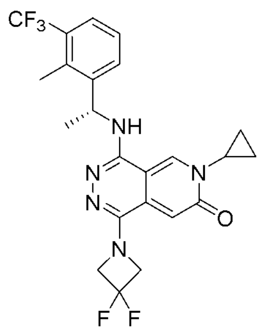
54

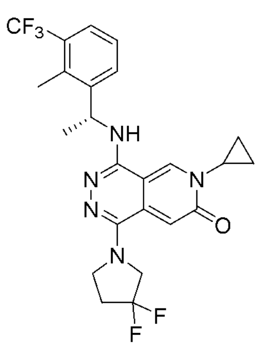

**55**

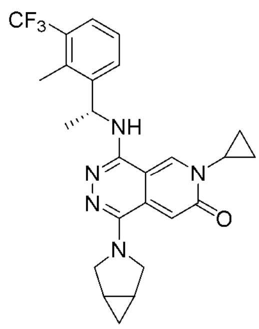

**56**

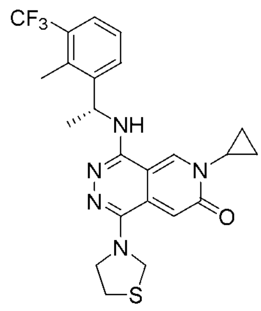

**57**

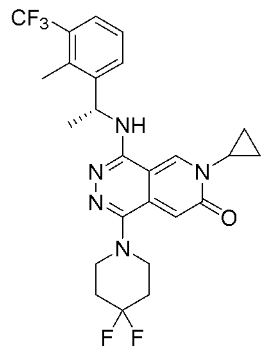

**58**

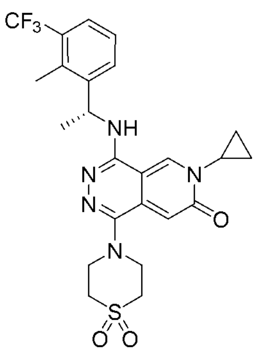

**59**

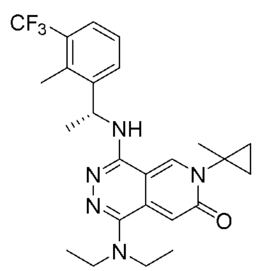

**60**

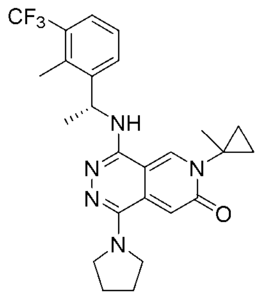

**61**

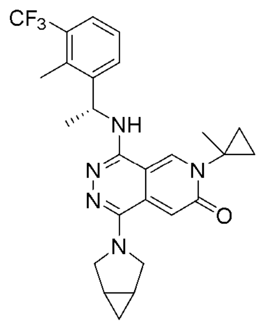

**62**

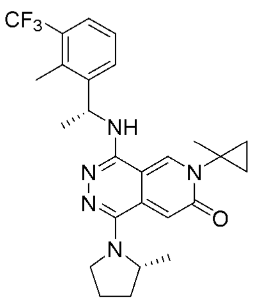

**63**

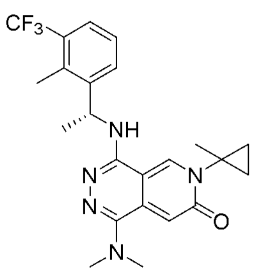

**64**

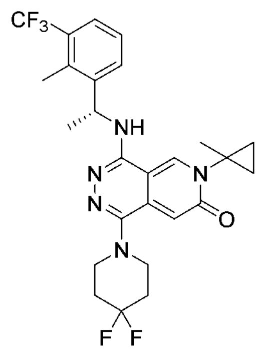

**65**

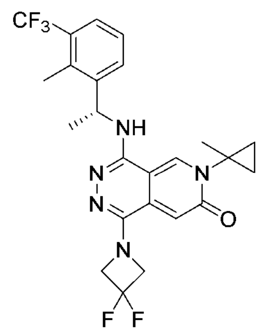

**66**

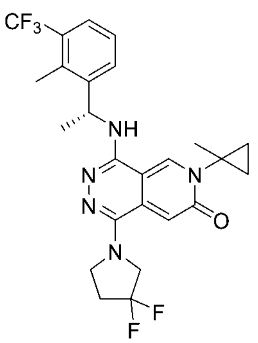

**67**

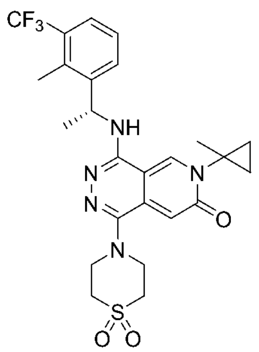

**68**

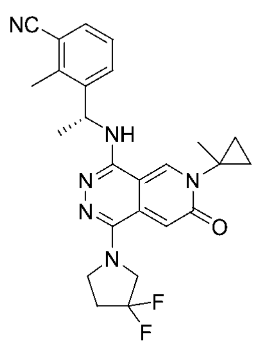

**69**

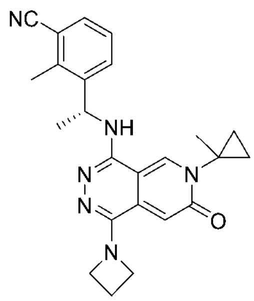

**70**

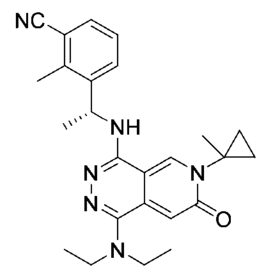

**71**

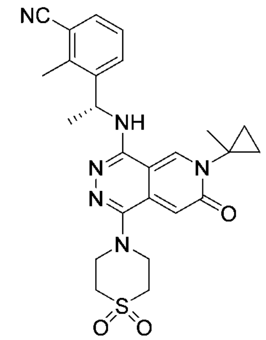

**72**

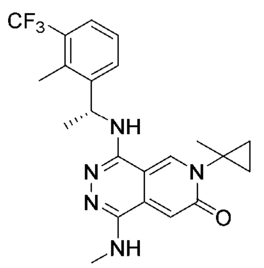

**73**

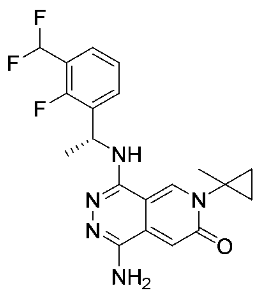

**74**

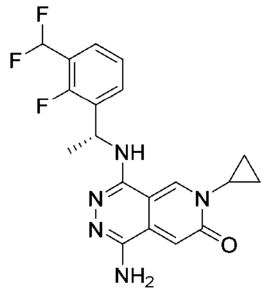

**75**

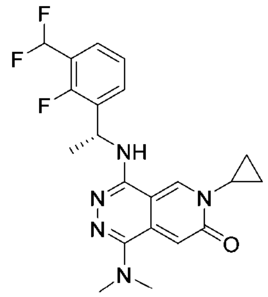

**76**

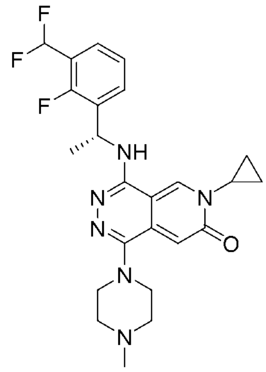

**77**

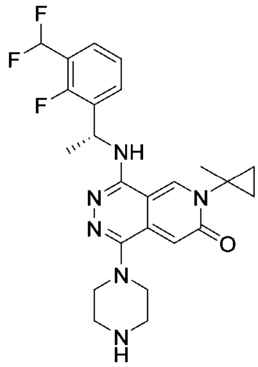

**78**

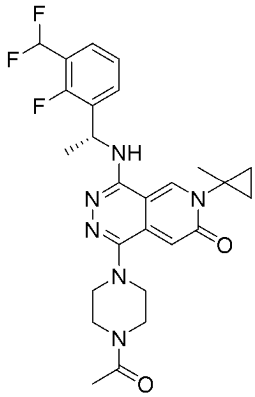

**79**

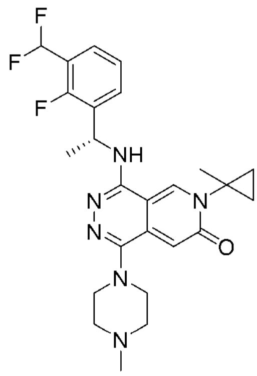

**80**

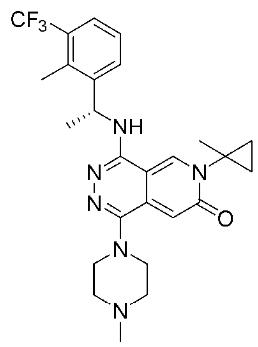

**81**

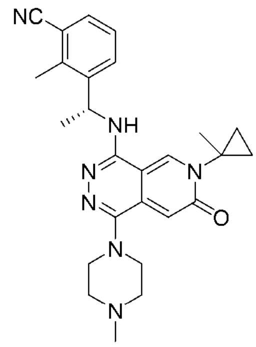

**82**

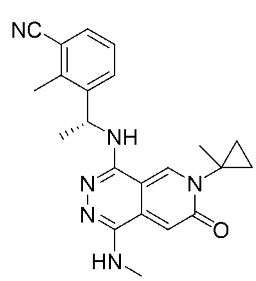

**83**

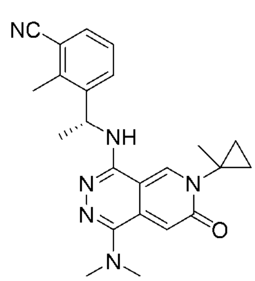

**84**

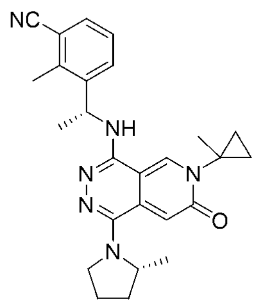

**85**

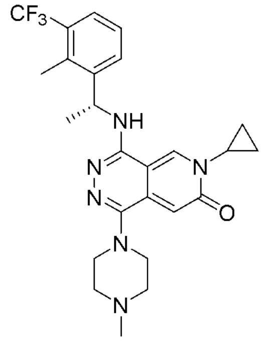

**86**

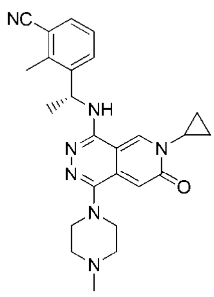
87

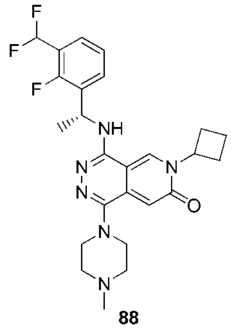
88

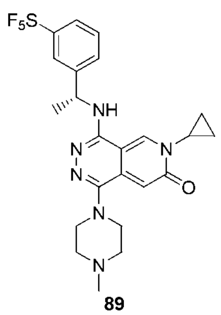
89

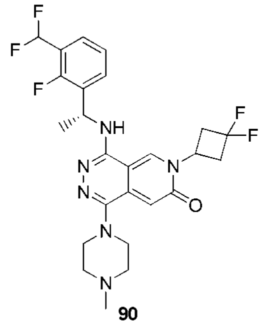
90

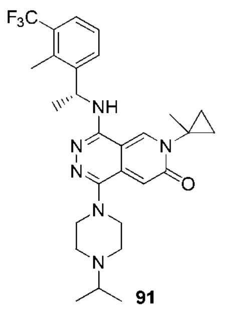
91

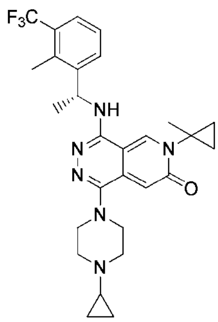
92

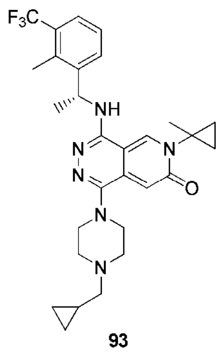
93

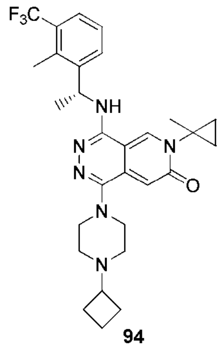
94

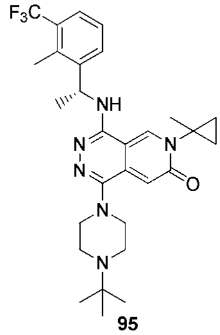
95

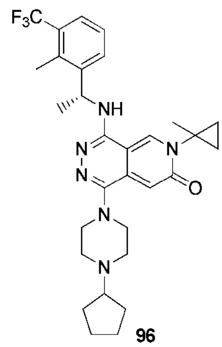
96

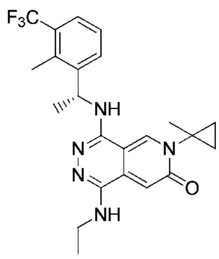
97

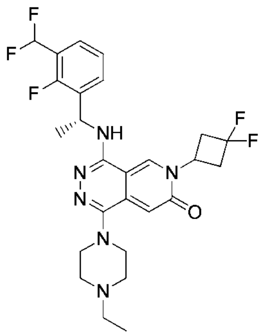
98

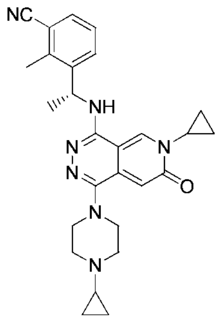
99

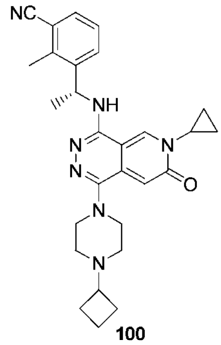
100

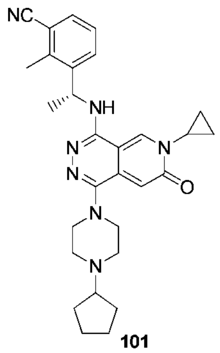
101

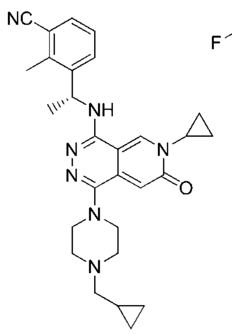
102

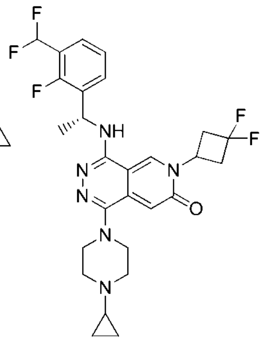
103

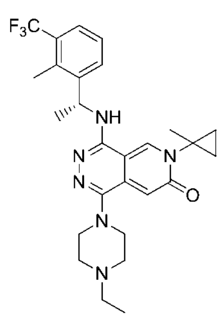
104

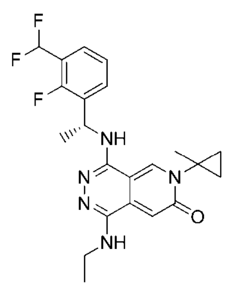
105

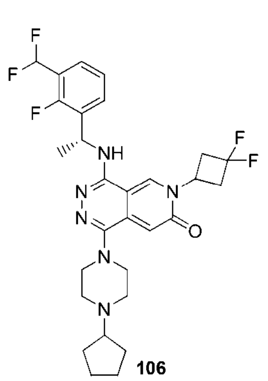
106

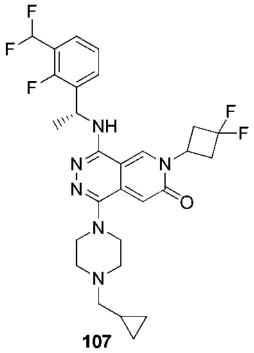

**107**

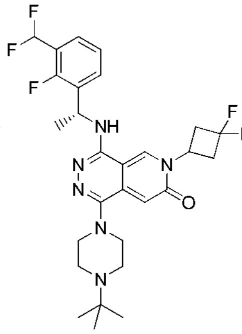

**108**

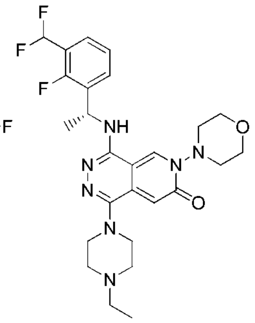

**109**

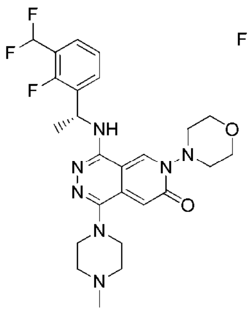

**110**

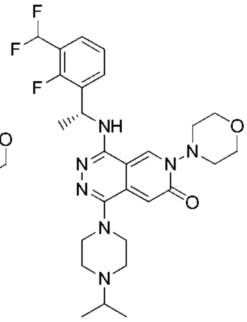

**111**

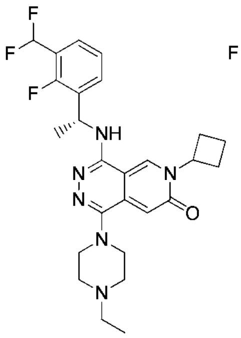

**112**

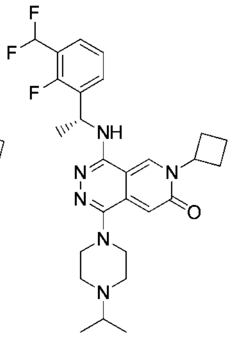

**113**

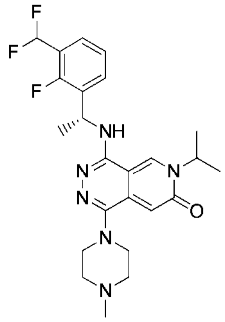

**114**

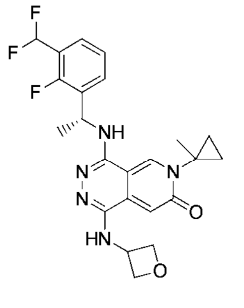

**115**

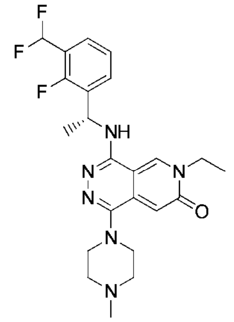

**116**

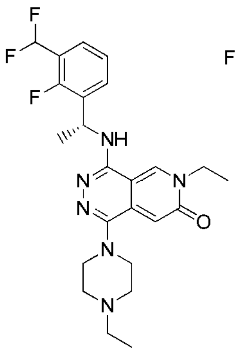

**117**

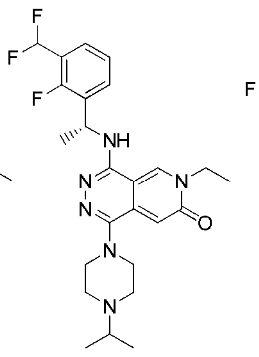

**118**

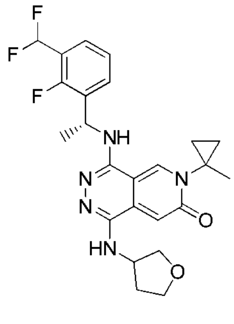

**119**

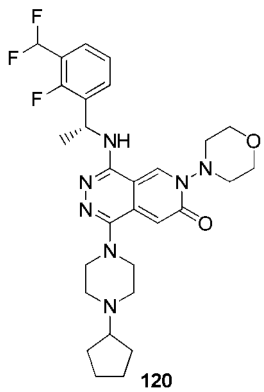

**120**

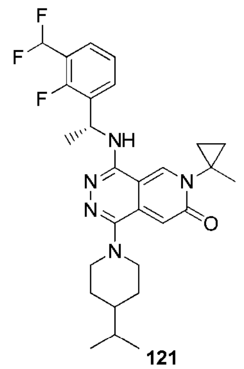

**121**

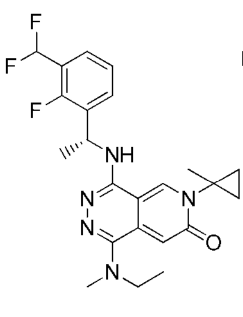

**122**

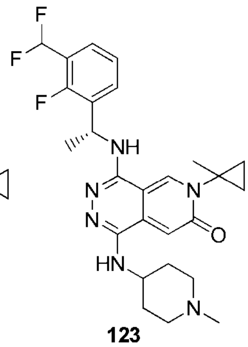

**123**

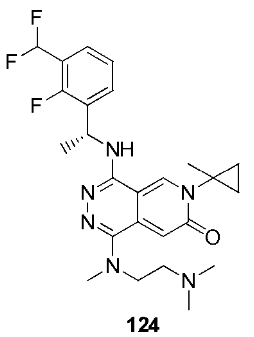

**124**

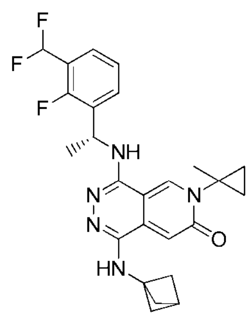

**125**

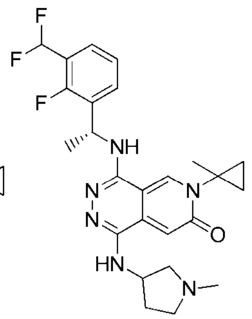

**126**

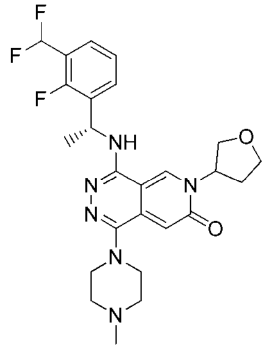
127

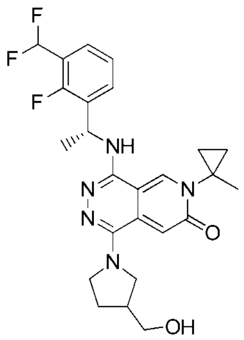
128

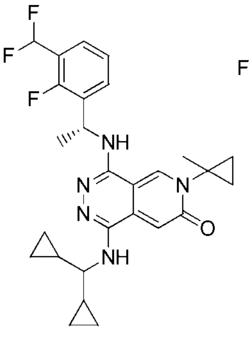
129

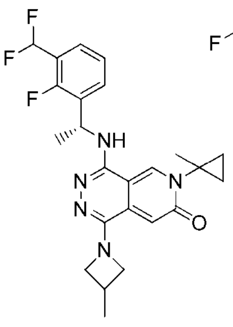
130

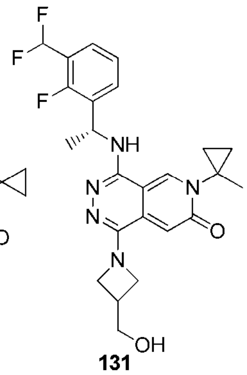
131

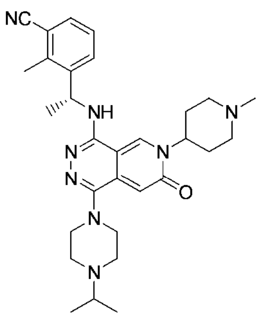
132

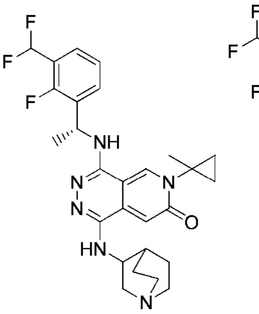
133

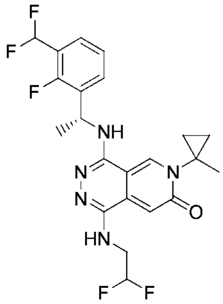
134

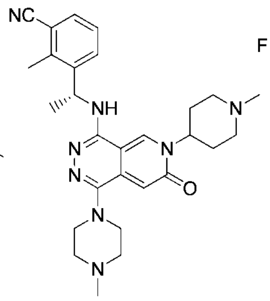
135

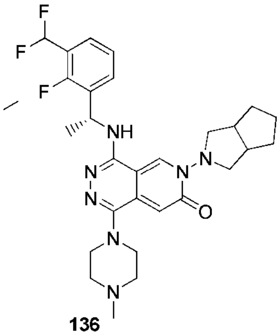
136

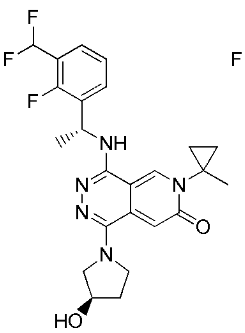
137

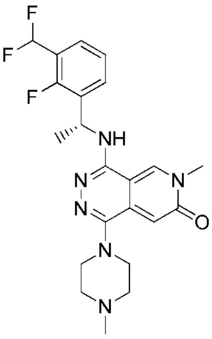
138

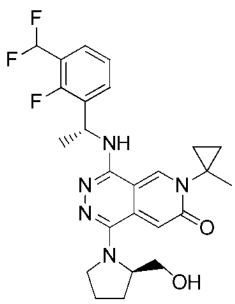
139

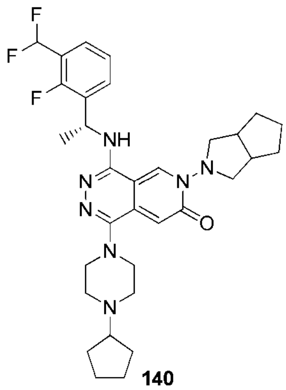
140

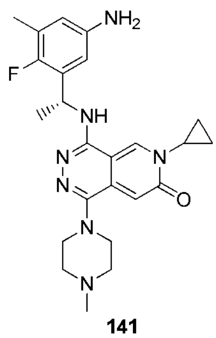
141

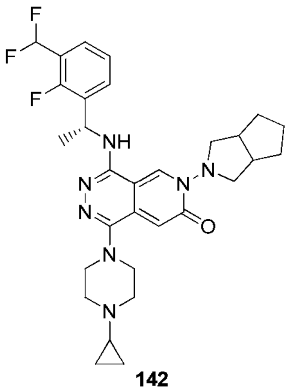
142

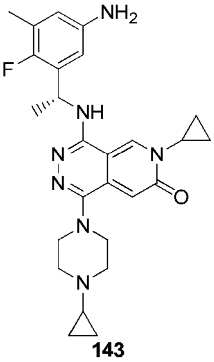
143

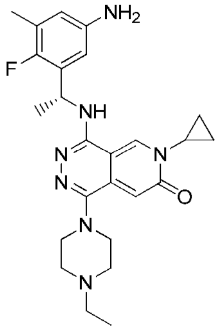
144

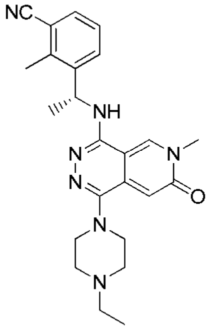
145

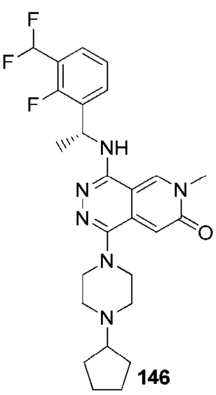
146

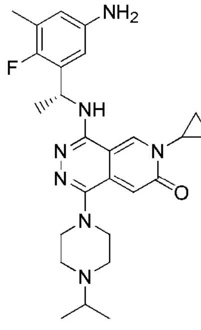
147

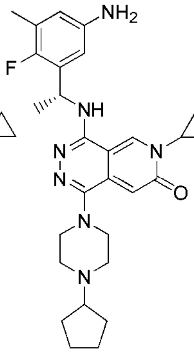
148

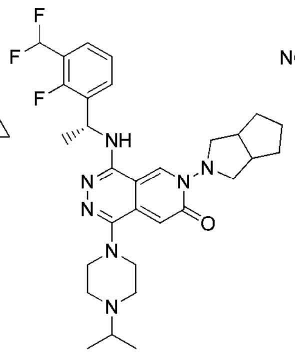
149

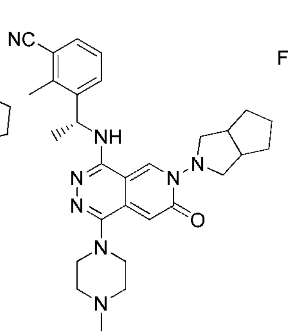
150

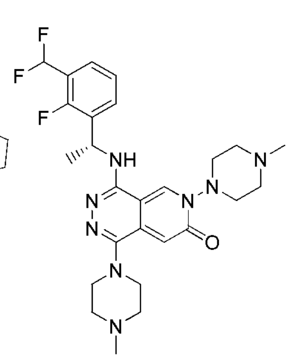
151

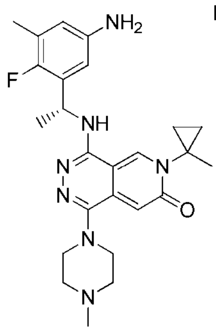
152

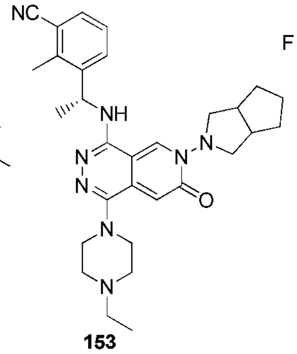
153

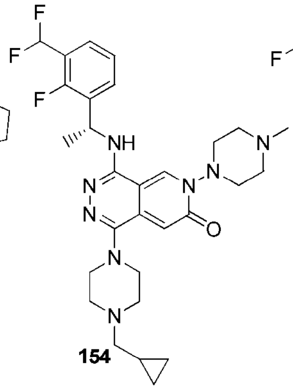
154

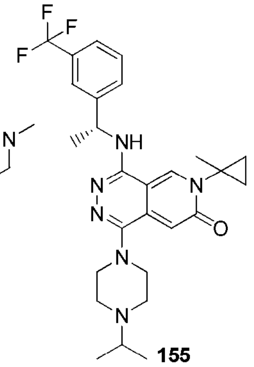
155

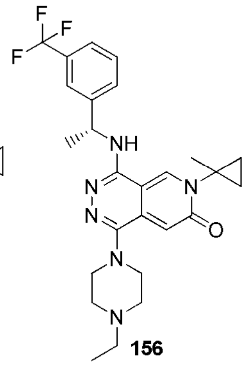
156

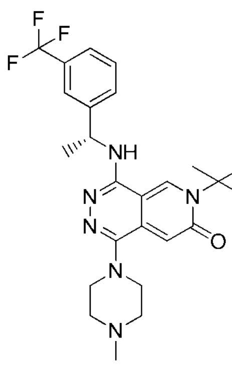
157

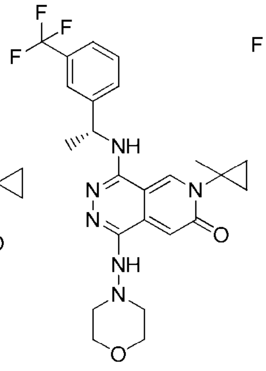
158

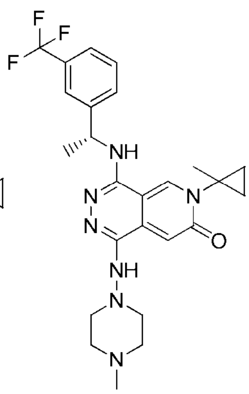
159

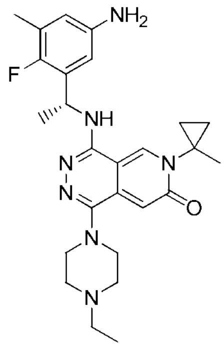
160

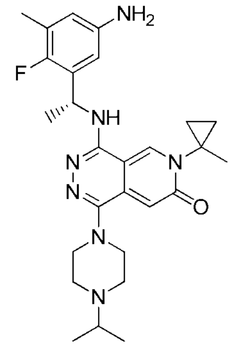
161

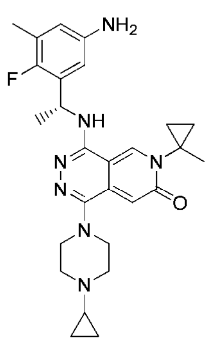
162

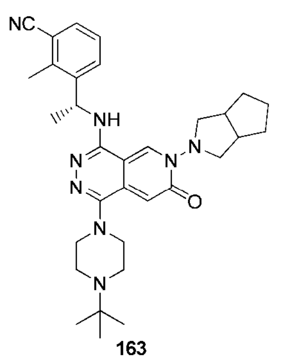
163

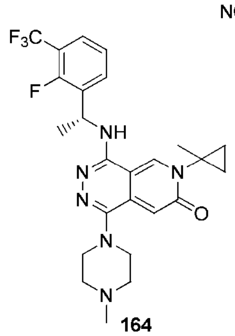
164

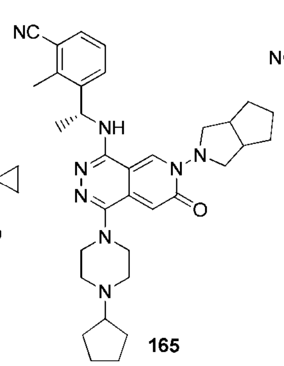
165

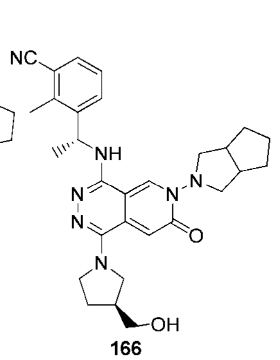
166

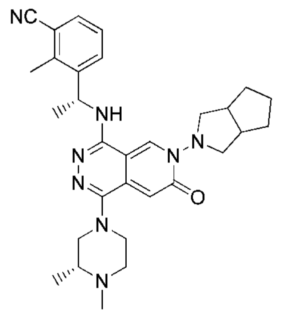
167

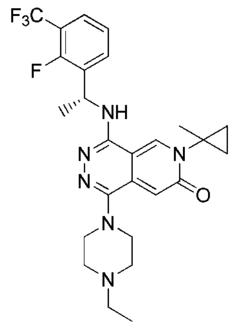
168

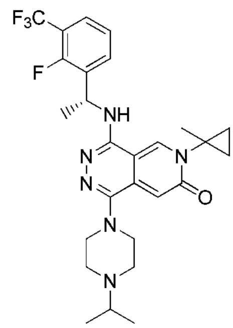
169

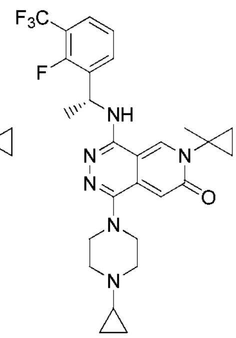
170

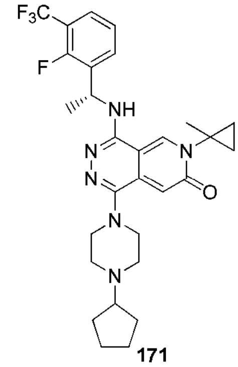
171

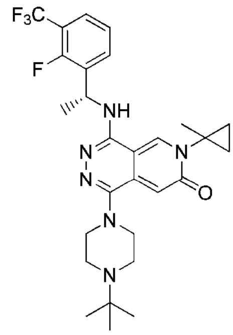
172

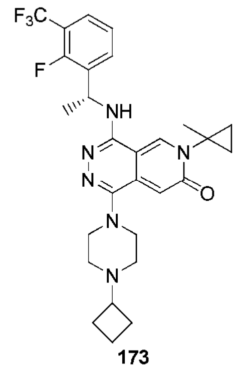
173

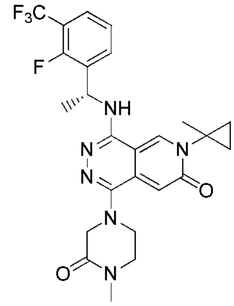
174

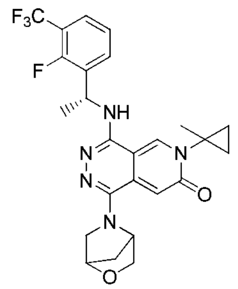
175

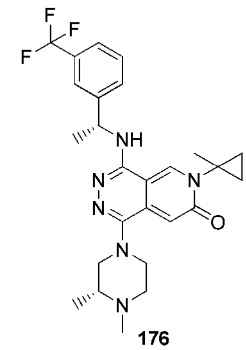
176

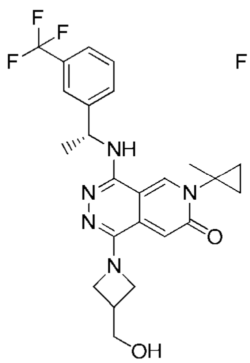
177

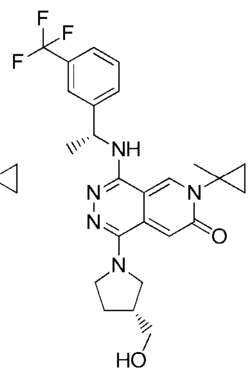
178

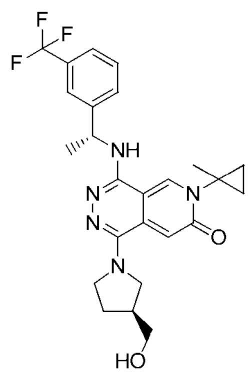
179

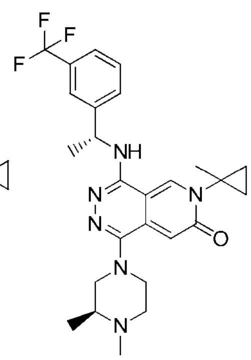
180

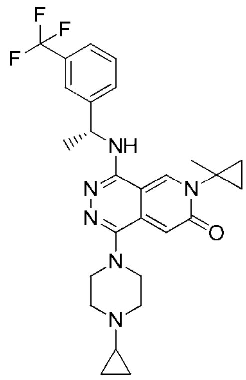
181

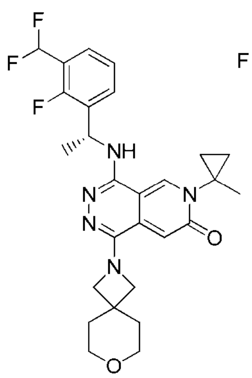
182

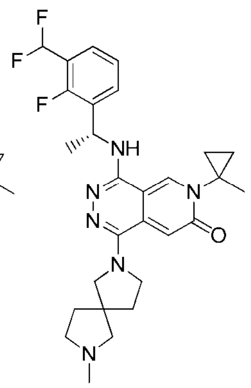
183

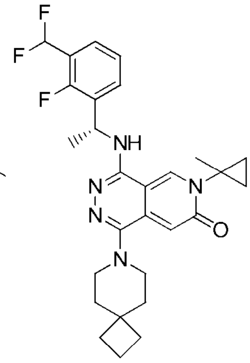
184

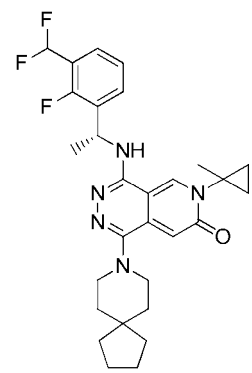
185

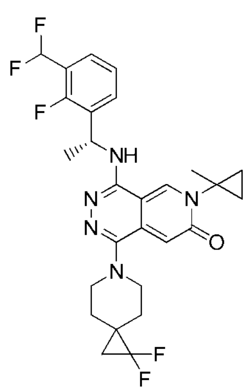
186

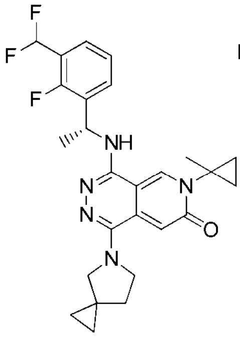
187

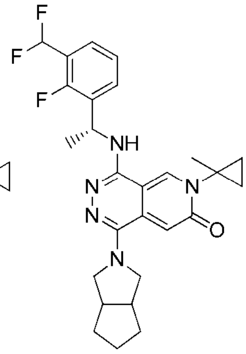
188

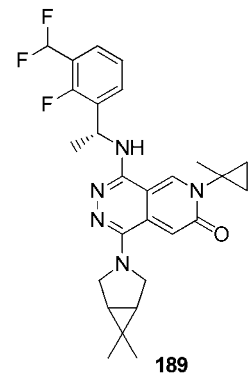
189

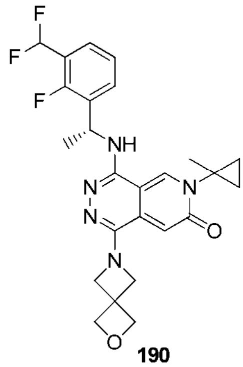
190

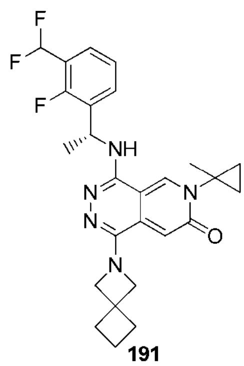
191

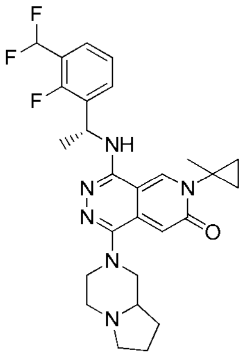
192

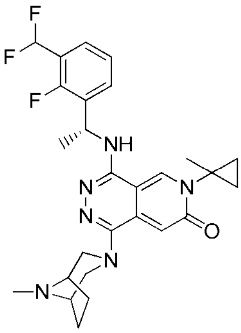
193

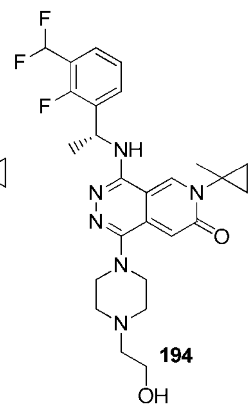
194

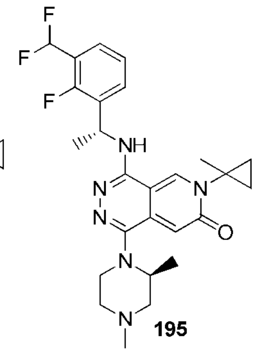
195

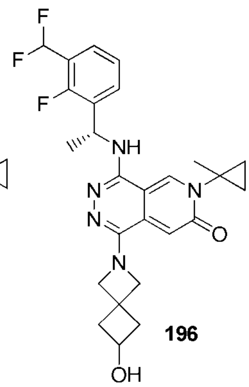
196

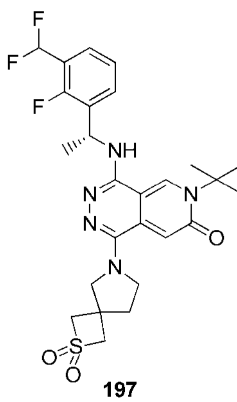
197

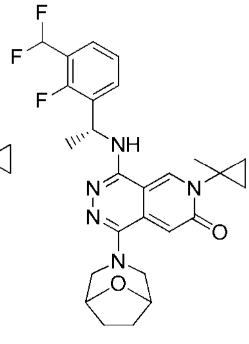
198

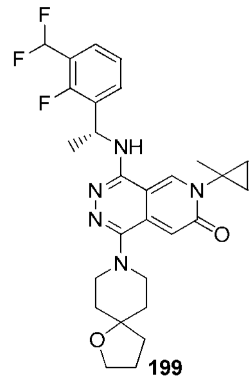
199

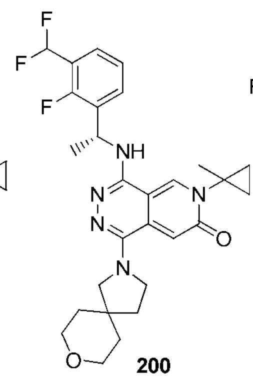
200

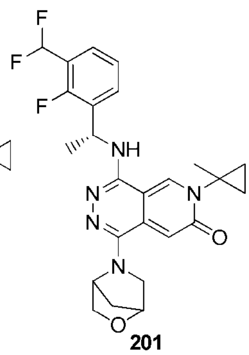
201

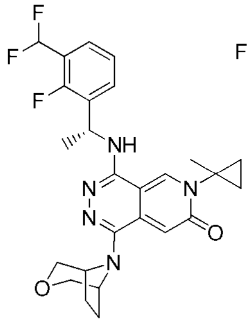
202

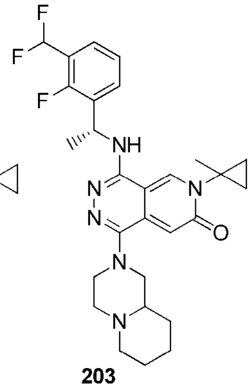
203

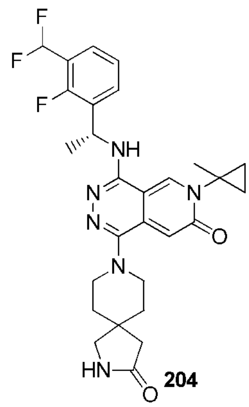
204

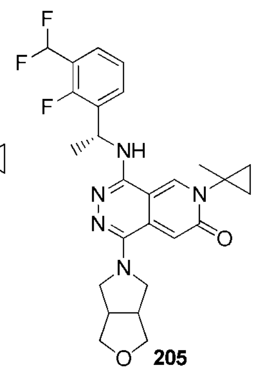
205

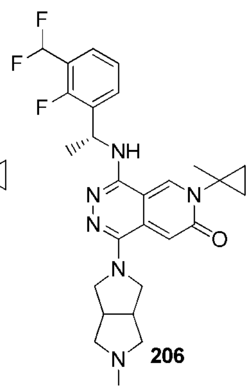
206

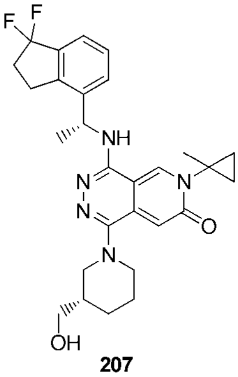
207

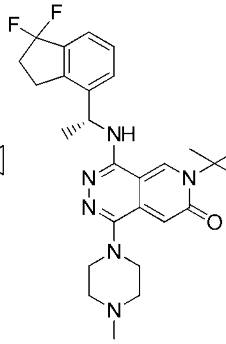
208

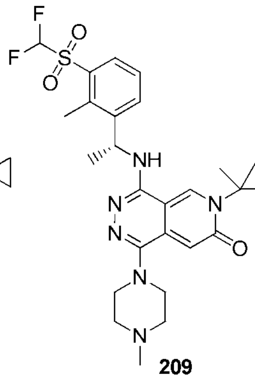
209

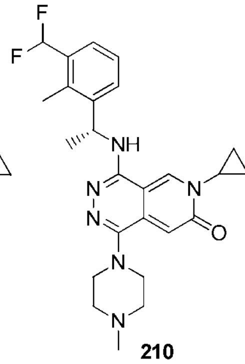
210

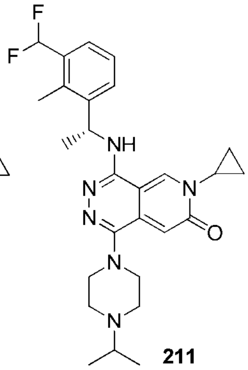
211

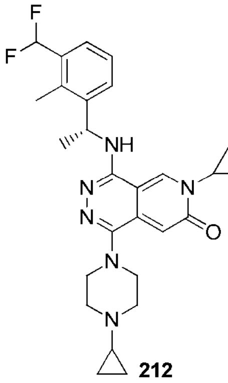
212

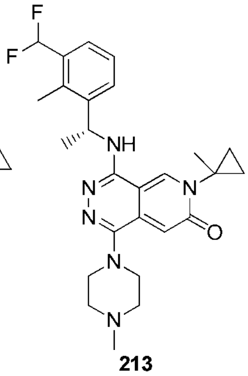
213

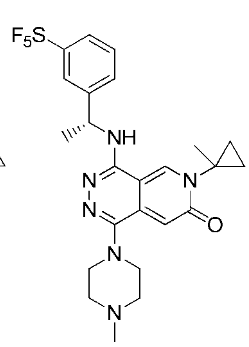
214

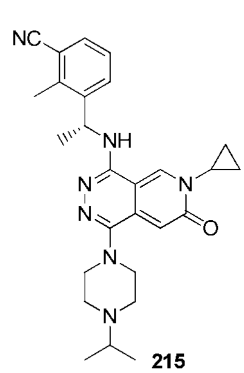
215

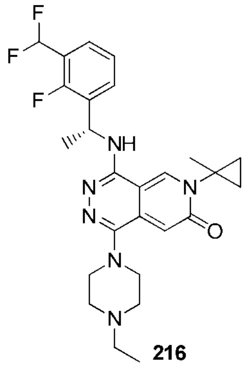
216

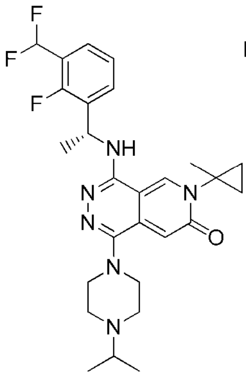
217

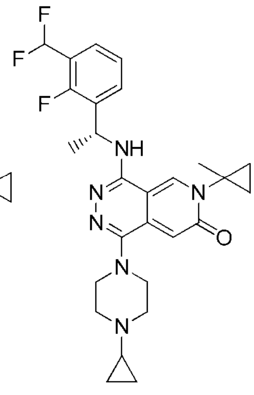
218

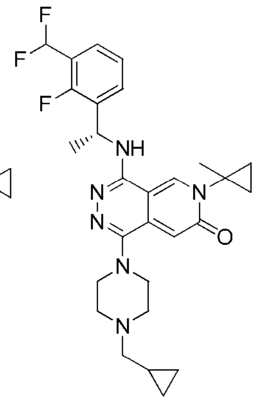
219

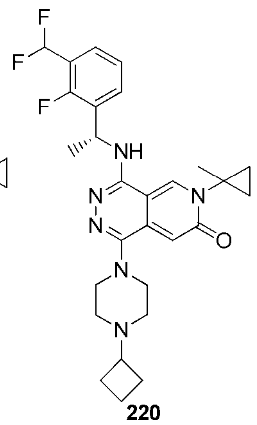
220

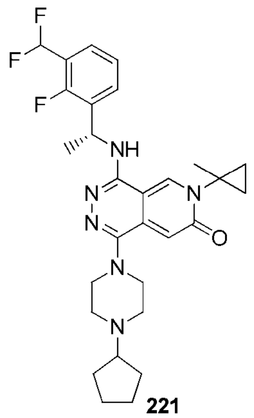
221

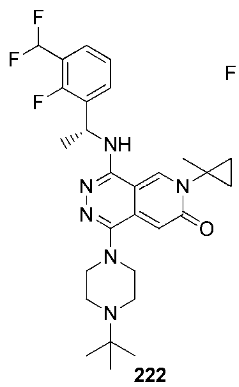
222

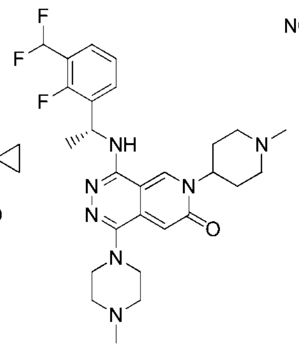
223

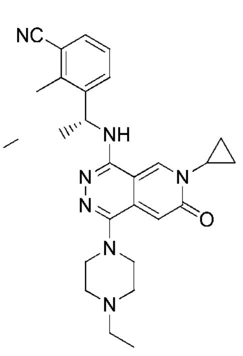
224

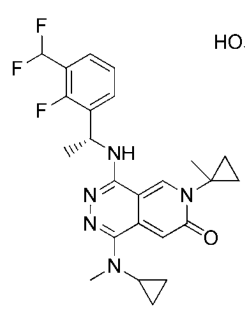
225

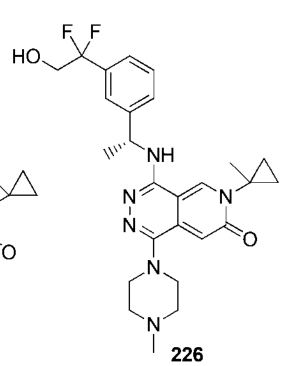
226

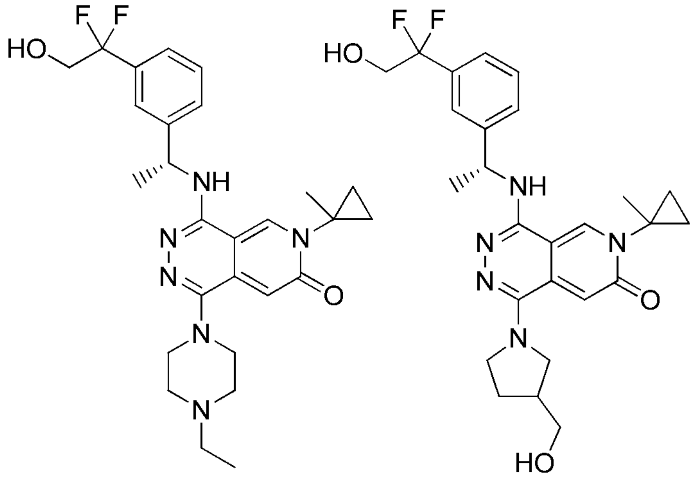

227 228

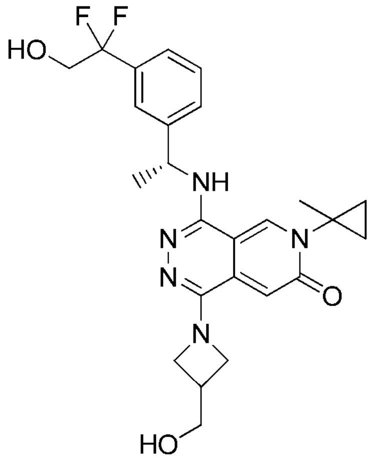

229

The present disclosure provides a pharmaceutical composition, which comprises the compound, or tautomer, stereoisomer, solvate, metabolite, isotopically labeled compound, pharmaceutically acceptable salt or co-crystal thereof, and a pharmaceutically acceptable excipient or an accessory ingredient.

The present disclosure provides a use of the compound, or tautomer, stereoisomer, solvate, metabolite, isotopically labeled compound, pharmaceutically acceptable salt or co-crystal thereof, or the pharmaceutical composition in the manufacture of an SOS1 inhibitor.

The present disclosure provides a use of the compound, or tautomer, stereoisomer, solvate, metabolite, isotopically labeled compound, pharmaceutically acceptable salt or co-crystal thereof, or the pharmaceutical composition in the manufacture of a medicament for treating a disease mediated by SOS1.

Further, the disease is selected from: cancer, pathogenic skin rash disease.

Further, the cancer is selected from: non-small cell lung cancer, lung cancer, pancreatic cancer, ovarian cancer, bladder cancer, prostate cancer, chronic granulocytic leukemia, colorectal cancer, brain cancer, liver cancer, kidney cancer, gastric cancer, and breast cancer;

the pathogenic skin rash disease is selected from: Noonan syndrome, cardiofaciocutaneous syndrome, hereditary gingival fibromatosis type I.

The present disclosure provides a use of the compound, or tautomer, stereoisomer, solvate, metabolite, isotopically labeled compound, pharmaceutically acceptable salt or co-crystal thereof, or the pharmaceutical composition in the manufacture of a medicament for treating a disease causing overexpression of an SOS1 protein.

The present disclosure provides a use of the compound, or tautomer, stereoisomer, solvate, metabolite, isotopically labeled compound, pharmaceutically acceptable salt or co-crystal thereof, or the pharmaceutical composition in the manufacture of a medicament for treating a disease caused by overexpression of an SOS1 protein.

The present disclosure provides a method for inhibiting SOS1, comprising administering an effective amount of the compound, or tautomer, stereoisomer, solvate, metabolite, isotopically labeled compound, pharmaceutically acceptable salt or co-crystal thereof, or the pharmaceutical composition.

The present disclosure provides a method for treating a disease mediated by SOS1, comprising administering a therapeutically effective amount of the compound, or tautomer, stereoisomer, solvate, metabolite, isotopically labeled compound, pharmaceutically acceptable salt or co-crystal thereof, or the pharmaceutical composition.

The present disclosure provides a method for treating a disease causing overexpression of an SOS1 protein, comprising administering a therapeutically effective amount of the compound, or tautomer, stereoisomer, solvate, metabolite, isotopically labeled compound, pharmaceutically acceptable salt or co-crystal thereof, or the pharmaceutical composition.

The present disclosure provides a method for treating a disease caused by overexpression of an SOS1 protein, comprising administering a therapeutically effective amount of the compound, or tautomer, stereoisomer, solvate, metabolite, isotopically labeled compound, pharmaceutically acceptable salt or co-crystal thereof, or the pharmaceutical composition.

## Definition:

The "tautomer" of the present disclosure refers to functional group isomers formed by the movement of an atom between two positions in a molecule, particularly when there are labile hydrogen atoms in the molecule, such as ketone

and enol tautomers.

The "stereoisomer" of the present disclosure refers to isomers formed by the same order of connection of atoms or atomic groups in a molecule but different spatial arrangements, including cis-trans isomers, optical isomers, and conformational isomers. The stereoisomers of the present disclosure also include forms of mixtures of two or more stereoisomers, such as mixtures of enantiomers and/or diastereomers in any proportion.

The "isotopically labeled compound" of the present disclosure refers to one or more atoms in a molecule are replaced by atoms with different atomic masses or mass numbers. Examples of isotopes that can be incorporated into the compounds of the present disclosure include isotopes of hydrogen, carbon, nitrogen, oxygen, phosphorus, sulfur, fluorine, chlorine, and iodine, such as but not limited to $^{2}H$, $^{3}H$, $^{11}C$, $^{13}C$, $^{14}C$, $^{13}N$, $^{15}N$, $^{15}O$, $^{17}O$, $^{18}O$, $^{35}S$, $^{18}F$, $^{36}Cl$, $^{123}I$ and $^{125}I$, respectively. Certain isotopically labeled compounds of the present disclosure can be used in medicament and/or substrate tissue distribution studies. The radioactive isotopes tritium i.e., $^{3}H$ and carbon-14 i.e., $^{14}C$ are particularly useful for this purpose due to their ease of incorporation and convenient detection methods. For example, the compounds of the present disclosure can be enriched with the specified isotope at 1%, 2%, 5%, 10%, 25%, 50%, 75%, 90%, 95%, or 99%. In addition, compounds of the present disclosure substituted with heavier isotopes such as deuterium $^{2}H$ can provide certain therapeutic advantages.

The "pharmaceutically acceptable salt" of the present disclosure refers to salts formed by the compounds of the present disclosure with acids or bases that are suitable for use as a medicament. The above acids and bases are Lewis acids and bases in a broad sense. Acids suitable for forming salts include inorganic acids, organic acids, and acidic amino acids.

The "solvate" of the present disclosure refers to associates formed by one or more solvent molecules and the compounds of the present disclosure that are suitable for use as a medicament. Solvents that form solvates include water and organic solvents.

In the present disclosure, "optionally substituted with..." refers to being capable of being substituted with one or more (including two) of the specified substituents, or can be unsubstituted.

In the present disclosure, "ring" refers to any covalently closed structure, including, for example, carbocycles (such as aryl or cycloalkyl), heterocycles (such as heteroaryl or heterocycloalkyl), aromatic groups (such as aryl or heteroaryl), and non-aromatic groups (such as cycloalkyl or heterocycloalkyl). The "ring" of the present disclosure can be monocyclic or can be polycyclic (including bicyclic), and can be a fused ring, spiro ring, or bridged ring.

In the present disclosure, "membered" refers to the number of ring atoms constituting the ring skeleton.

The term "halogen" includes F, Cl, Br, or I.

The term "alkyl" refers to a straight or branched chain saturated aliphatic hydrocarbon group. "C1~C6 alkyl" refers to a straight or branched chain saturated aliphatic hydrocarbon group having 1, 2, 3, 4, 5, or 6 carbon atoms, examples of which include, but are not limited to, methyl, ethyl, n-propyl, isopropyl, n-butyl, sec-butyl, tert-butyl, n-pentyl, n-hexyl, etc. Unless otherwise specified, the alkyl group is optionally substituted with one or more suitable substituents.

The term "alkoxy" refers to the "alkyl" group as defined above that is attached via an oxygen atom, i.e., an "alkoxy" group can be defined as -OR, wherein R is alkyl as defined above. Examples of "C1~C6 alkoxy" include, but are not limited to, methoxy, ethoxy, n-propoxy, isopropoxy, n-butoxy, sec-butoxy, tert-butoxy, n-pentyloxy, n-hexyloxy, etc. Unless otherwise specified, the alkoxy group is optionally substituted with one or more suitable substituents.

The term "alkenyl" refers to a straight or branched chain aliphatic hydrocarbon group containing at least one carbon-carbon double bond (i.e., C=C). The double bond can exist as either the E or Z isomer. The double bond can be located at any possible position along the hydrocarbon chain. Unless otherwise specified, the alkenyl group is optionally substituted with one or more suitable substituents.

The term "aliphatic carbocyclic ring" refers to a non-aromatic carbocyclic system having three or more ring carbon atoms and contains no heteroatoms in the ring skeleton. "5~6 membered aliphatic carbocyclic ring" refers to a non-aromatic carbocyclic ring having 5 to 6 ring carbon atoms. Unless otherwise specified, the aliphatic carbocyclic ring is optionally substituted with one or more suitable substituents.

The term "cycloalkyl" refers to a saturated carbocyclic hydrocarbon group having a single ring or multiple rings. "3~10 membered cycloalkyl" refers to a saturated monocyclic or polycyclic hydrocarbon ring having 3~10 ring carbon atoms. Examples of which include, but are not limited to, cyclopropyl, cyclobutyl, bicyclo[1.1.1]pentyl, etc. The structure of cyclopropane is

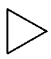.

The structure of cyclobutane is

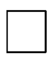.

The structure of bicyclo[1.1.1]pentane is

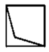.

Unless otherwise specified, the cycloalkyl group is optionally substituted with one or more suitable substituents.

The term "heterocycloalkyl" refers to a saturated cyclic group having a single ring or multiple rings, and wherein at least one ring atom is a heteroatom and the remaining ring atoms are carbon. The heteroatom is preferably N, O, S. "3~10 membered heterocycloalkyl" refers to a saturated monocyclic or polycyclic group having 3 to 10 ring atoms, and wherein at least one ring atom is a heteroatom and the remaining ring atoms are carbon. Examples of 3~10 membered heterocycloalkyl include, but are not limited to:

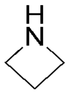,

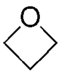, 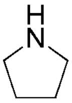, 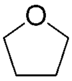, 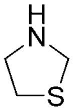, 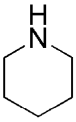, 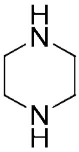, 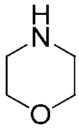, 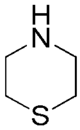, 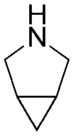, 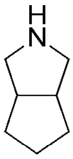, 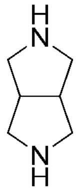, 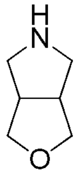,

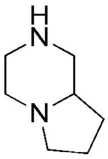, 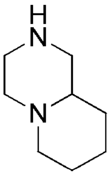, 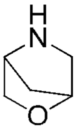, 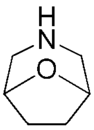, 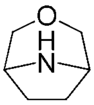, 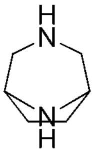, 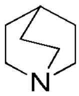, 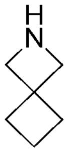, 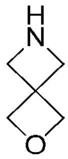, 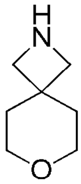, 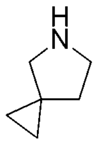,

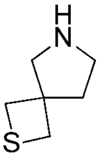, 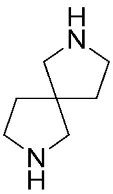, 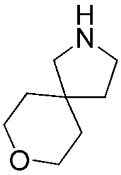, 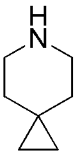, 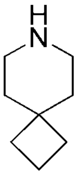, 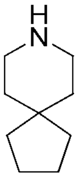, 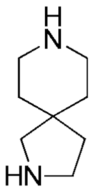, 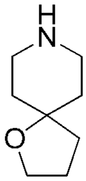.

Unless otherwise specified, the heterocycloalkyl is optionally substituted with one or more suitable substituents.

The term "cycloalkenyl" refers to a non-aromatic monocyclic or polycyclic carbocyclic hydrocarbon group containing at least one carbon-carbon double bond (i.e., C=C). "3~10 membered cycloalkenyl" refers to an unsaturated non-aromatic monocyclic or polycyclic carbocyclic hydrocarbon group having 3~10 ring carbon atoms. Unless otherwise specified, the cycloalkenyl is optionally substituted with one or more suitable substituents.

The term "heterocycloalkenyl" refers to a non-aromatic monocyclic or polycyclic group containing at least one double bond, and wherein at least one ring atom is a heteroatom and the remaining ring atoms are carbon. The heteroatom includes, but is not limited to, N, O, S, P, Si, etc., preferably N, O, S. Unless otherwise specified, the heterocycloalkenyl is optionally substituted with one or more suitable substituents.

The term "aryl" refers to an all-carbon monocyclic or polycyclic aromatic group having a conjugated π-electron system, including monocyclic aryl (such as 6 membered phenyl), fused-ring aryl (with a shared ring edge between aromatic rings, such as 10 membered naphthyl), and biaryl (an aryl-aryl structure formed by a single bond connection, such as 12 membered biphenyl). Unless otherwise specified, the aryl is optionally substituted with one or more suitable substituents.

The term "heteroaryl" refers to a monocyclic or polycyclic aromatic group with a conjugated π-electron system, and wherein at least one ring atom is a heteroatom and the remaining ring atoms are carbon. The heteroatom preferably is N, O, S. The heteroaryl of the present disclosure includes monocyclic heteroaryl (such as pyrazolyl, imidazolyl, 1,2,3-triazolyl, and 1,2,4-triazolyl of 5 membered rings), fused heteroaryl (with a shared ring edge between heteroaromatic rings,

or between an aromatic ring and a heteroaromatic ring). Examples of 5~10 membered heteroaryl include, but are not limited to:

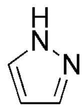, 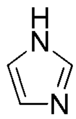, 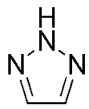,

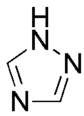.

Unless otherwise specified, the heteroaryl is optionally substituted with one or more suitable substituents.

The pharmaceutically acceptable excipient of the present disclosure is the general term for all additional materials in a medicament except the active pharmaceutical ingredient. Excipients should possess the following properties: (1) No toxic or harmful effects on the human body and almost no side effects; (2) Chemically stable, not easily affected by temperature, pH, storage time, etc.; (3) No incompatibility with the main medicament, and no impact on the efficacy of the main medicament or quality inspection; (4) No interaction with packaging materials. Excipients in the present disclosure include, but are not limited to, fillers (diluents), lubricants (glidants or anti-adherents), dispersants, wetting agents, binders, regulators, solubilizers, antioxidants, bacteriostats, emulsifiers, disintegrants, etc. There is no particular restriction on the administration mode of the compound or pharmaceutical composition of the present disclosure, and representative administration modes include (but are not limited to): oral administration, parenteral administration (intravenous, intramuscular, or subcutaneous), and topical administration.

The beneficial effects of the present disclosure are: the present disclosure provides a series of compounds with obvious inhibitory effects on the SOS1 protein, offering new therapeutic regimens for diseases targeting SOS1, such as cancer and pathogenic skin rash diseases, etc. Compared with existing SOS1 inhibitors, the compounds of the present disclosure show significant improvements in aspects such as activity and druggability, etc. In particular, they exhibit better metabolic stability in liver microsomes, weaker inhibitory effects on liver drug-metabolizing enzymes, and higher oral bioavailability, with excellent druggable properties. They can be used in the manufacture of medicaments for treating related diseases and have broad application prospects.

## DETAILED EMBODIMENTS

The technical solutions of the present disclosure are clearly and completely described below. Obviously, the described Examples are part of the Examples of the present disclosure, rather than all the Examples. Based on the Examples in the present disclosure, all other Examples obtained by those of ordinary skill in the art without making creative efforts shall fall within the protection scope of the present disclosure.

The structures of the compounds of the present disclosure were determined by nuclear magnetic resonance (NMR) and/or liquid chromatography-mass spectrometry (LC-MS). The chemical shifts ($\delta$) of NMR were given in parts per million (ppm). NMR measurements were performed using an AVANCE NEO 400 MHz Bruker instrument. The deuterated solvents were deuterated dimethyl sulfoxide (DMSO-$d_6$), deuterated chloroform ($CDCl_3$), and deuterated methanol ($CD_3OD$), and tetramethylsilane (TMS) was used as the internal standard. MS measurements were conducted using an ISQ-EC Thermo Fisher LC-MS instrument. Prep-HPLC was a GX-281 Gilson chromatograph, the separation methods were: (method 1) Sun Fire Prep C18 OBDTM 5$\mu$m, 30×150 mm Column, 0.04% aqueous HCl solution/acetonitrile; (method 2) Sun Fire Prep C18 OBDTM 5$\mu$m, 30×150 mm Column, 0.06% aqueous formic acid solution/acetonitrile; (method 3) Xbridge Prep C18 OBDTM 5$\mu$m, 30×150 mm Column, 10 mM aqueous $NH_4HCO_3$ solution/acetonitrile; (method 4) SunFire Prep C18 OBDTM 5$\mu$m, 30×150 mm Column, 0.02% $CF_3COOH$ aqueous trifluoroacetic acid/acetonitrile.

The starting materials in the Examples of the present disclosure are known and either commercially available or synthesizable according to methods known in the art.

Unless otherwise specified, the solvents used in the present disclosure are commercially available.

Unless otherwise specified, the reaction temperature is room temperature, i.e., 20°C-30°C.

The chemical abbreviations involved in the present disclosure have the following meanings:

DMF: *N*,*N*-dimethylformamide
DMSO: dimethyl sulfoxide
DIPEA: *N*,*N*-diisopropylethylamine

DMF-DMA: *N*,*N*-dimethylformamide dimethyl acetal
Prep-HPLC: preparative high-performance liquid chromatography

**Example 1**

**Preparation of (*R*)-6-cyclopropyl-4-((1-(3-(difluoromethyl)-2-fluorophenyl)ethyl)amino)-1-(1*H*-1,2,4-triazol-1-yl) pyrido[3,4-d]pyridazin-7(6*H*)-one**

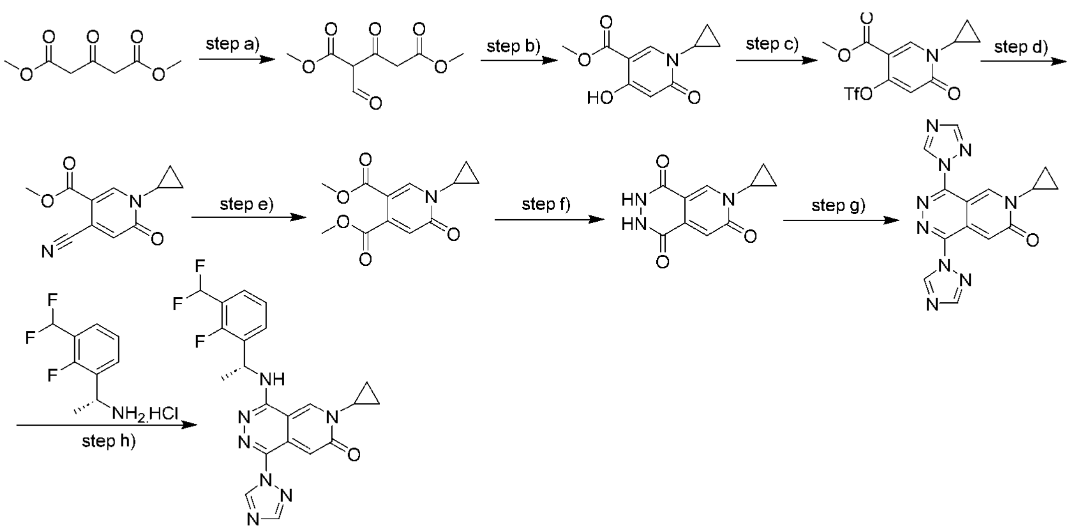


step a): Preparation of dimethyl 2-formyl-3-oxopentanedioate

Dimethyl 3-oxopentanedioate (5.0 g, 28.711 mmol) and 1-methyltetrahydrofuran (100 mL) were added to a reaction flask. Under an ice-water bath, DMF-DMA (4.1 g, 34.454 mmol) was added dropwise. After the addition was completed, the mixture was warmed to room temperature and reacted for 2 h. Hydrochloric acid (100 mL, 4 N) was added to the reaction solution, and the mixture was continued to react at room temperature for 1 h. After the reaction was completed, the reaction solution was extracted with ethyl acetate (100 mL). The organic phases were combined, washed with saturated brine (100 mL), dried over anhydrous sodium sulfate, and filtered. The filtrate was concentrated under reduced pressure to obtain dimethyl 2-formyl-3-oxopentanedioate, yield 99.9%; ESI-MS(m/z): 203.2 $[M+H]^+$.

step b): Preparation of methyl 1-cyclopropyl-4-hydroxy-6-oxo-1,6-dihydropyridine-3-carboxylate

Dimethyl 2-formyl-3-oxopentanedioate (5.6 g, 27.716 mmol), cyclopropylamine (1.9 g, 33.259 mmol), sodium methoxide (3.5 mg, 60.975 mmol), and methanol (50 mL) were added to a reaction flask. The mixture was warmed to 80°C and reacted for 2 h. After the reaction was completed, the mixture was cooled to room temperature and quenched by adding saturated aqueous ammonium chloride (50 mL). The reaction solution was adjusted to pH 2-3 with 4N hydrochloric acid, extracted with dichloromethane (20 mL × 3). The organic phases were combined, washed with saturated brine (50 mL), dried over anhydrous sodium sulfate, and filtered. The filtrate was concentrated under reduced pressure to obtain methyl 1-cyclopropyl-4-hydroxy-6-oxo-1,6-dihydropyridine-3-carboxylate, which was used directly in the next step without purification, yield 82.2%; ESI-MS (m/z): 210.0 $[M+H]^+$.

step c): Preparation of methyl 1-cyclopropyl-6-oxo-4-(((trifluoromethyl)sulfonyl)oxy)-1,6-dihydropyridine-3- carboxylate

Methyl 1-cyclopropyl-4-hydroxy-6-oxo-1,6-dihydropyridine-3-carboxylate (4.8 g, 22.967 mmol) was dissolved in dichloromethane (50 mL). Pyridine (3.6 g, 45.934 mmol) was added under an ice bath, and trifluoromethanesulfonic anhydride (9.7 g, 34.450 mmol) was slowly added dropwise. After the addition was completed, the mixture was warmed to room temperature and reacted for 20 min. The reaction was quenched by adding saturated aqueous ammonium chloride (50 mL) and extracted with dichloromethane (20 mL). The organic phases were combined, washed with saturated brine (30 mL), dried over anhydrous sodium sulfate, filtered, and the filtrate was concentrated under reduced pressure. The residue was purified by silica gel column chromatography (eluent: petroleum ether/ethyl acetate = 3/1) to obtain methyl 1-cyclopropyl-6-oxo-4-(((trifluoromethyl)sulfonyl)oxy)-1,6-dihydropyridine-3-carboxylate, yield 60.5%; ESI-MS (m/z): 342.0 $[M+H]^+$.

step d): Preparation of methyl 4-cyano-1-cyclopropyl-6-oxo-1,6-dihydropyridine-3-carboxylate

At room temperature, methyl 1-cyclopropyl-6-oxo-4-(((trifluoromethyl)sulfonyl)oxy)-1,6-dihydropyridine-3- carboxylate (4.1 g, 12.113 mmol), zinc cyanide (2.2 g, 24.226 mmol), tetrakis(triphenylphosphine)palladium (280 mg, 0.242 mmol), and DMF (20 mL) were added to a reaction flask. The mixture was purged with nitrogen three times, warmed to 80°C and reacted for 1 h, then cooled and filtrated. The filtrate was poured into water (30 mL) and extracted with ethyl acetate (50 mL × 2). The organic phases were combined, washed with saturated brine (50 mL × 3), dried over anhydrous sodium sulfate, filtered, and the filtrate was concentrated. The residue was purified by silica gel column chromatography (eluent: petroleum ether/ethyl acetate = 1/1) to obtain methyl 4-cyano-1-cyclopropyl-6-oxo-1,6-dihydropyridine-3- carboxylate, yield 78.2%; ESI-MS (m/z): 219.0 $[M+H]^+$.

step e): Dimethyl 1-cyclopropyl-6-oxo-1,6-dihydropyridine-3,4-dicarboxylate

At room temperature, methyl 4-cyano-1-cyclopropyl-6-oxo-1,6-dihydropyridine-3-carboxylate (2.1 g, 9.633 mmol) and 6 N hydrochloric acid (10 mL) were added to a reaction flask. The mixture was warmed to reflux for 5 hours, methanol (40 mL) was added, then reflux was continued overnight. After the reaction was completed, the mixture was cooled to room temperature and concentrated under reduced pressure. Water (30 mL) and ethyl acetate (50 mL) were added to the residue for extraction. The organic phases were combined, washed with saturated sodium bicarbonate (50 mL), dried over anhydrous sodium sulfate, filtered. The filtrate was concentrated to obtain dimethyl 1-cyclopropyl-6-oxo-1,6-dihydropyridine-3,4-dicarboxylate, which was used directly in the next step without purification; yield 75.1%; ESI-MS (m/z): 252.0 $[M+H]^+$.

step f): 6-Cyclopropyl-2,3-dihydropyrido[3,4-*d*]pyridazine-1,4,7(6*H*)-trione

At room temperature, dimethyl 1-cyclopropyl-6-oxo-1,6-dihydropyridine-3,4-dicarboxylate (1.6 g, 6.375 mmol) and hydrazine hydrate (10 mL, 80%) were added to a reaction flask. The mixture was warmed to 100°C and reacted overnight. After the reaction was completed, the mixture was cooled to room temperature, acetonitrile (100 mL) was added, and a large amount of solid precipitated. The mixture was filtered, and filter cake was washed with acetonitrile (10 mL), collected, and dried under reduced pressure to obtain 6-cyclopropyl-2,3-dihydropyrido[3,4-*d*]pyridazine-1,4,7(6*H*)-trione; yield 68.3%; ESI-MS (m/z): 220.0 $[M+H]^+$.

step g): Preparation of 6-cyclopropyl-1,4-di(1*H*-1,2,4-triazol-1-yl)pyrido[3,4-*d*]pyridazin-7(6H)-one

6-Cyclopropyl-2,3-dihydropyrido[3,4-*d*]pyridazine-1,4,7(6*H*)-trione(3.0 g, 13.686 mmol), triethylamine (33.2 g, 328.096 mmol), and acetonitrile (90 mL) were added to a reaction flask. A solution of phosphorus oxychloride (12.6 g, 82.176 mmol) in acetonitrile (20 mL) was added dropwise. After the addition was completed, the mixture was stirred at room temperature for 5 min. 1,2,4-Triazole (22.7 g, 328.652 mmol) was added, and the mixture was stirred to react at room temperature for 15 min. After the reaction was completed, the mixture was concentrated under reduced pressure, poured into ice water (80 mL), and stirred at room temperature for 10 min, and filtered. The filter cake was washed with water (20 mL × 3) and dried under vacuum to obtain 6-cyclopropyl-1,4-di(1*H*-1,2,4-triazol-1-yl)pyrido[3,4-*d*]pyridazin-7(6*H*)-one; yield 71.2%; ESI-MS (m/z): 322.2 $[M+H]^+$.

step h): Preparation of (*R*)-6-cyclopropyl-4-((1-(3-(difluoromethyl)-2-fluorophenyl)ethyl)amino)-1-(1*H*-1,2,4-triazol-1-yl) pyrido[3,4-*d*]pyridazin-7(6*H*)-one

6-Cyclopropyl-1,4-di(1*H*-1,2,4-triazol-1-yl)pyrido[3,4-*d*]pyridazin-7(6*H*)-one (160 mg, 0.498 mmol), (*R*)-1-(3-(difluoromethyl)-2-fluorophenyl)ethan-1-amine hydrochloride (110 mg, 0.488 mmol), and 1,4-dioxane (8 mL) were added to a reaction flask. The mixture was warmed to reflux and stirred to react for 2 h. After the reaction was completed, the mixture was concentrated under reduced pressure. The residue was purified by silica gel column chromatography (eluent: dichloromethane/methanol = 96/4), and the resulting crude product was further purified by Prep-HPLC (separation method 3) to obtain (*R*)-6-cyclopropyl-4-((1-(3-(difluoromethyl)-2-fluorophenyl)ethyl)amino)-1- (1*H*-1,2,4-triazol-1-yl) pyrido[3,4-*d*]pyridazin-7(6*H*)-one, yield 6.1%; $^1$H NMR (400 MHz, DMSO-$d_6$) δ 9.29 (s, 1H), 9.06 (s, 1H), 8.68 (s, 1H), 8.34 (s, 1H), 7.69 (t, *J* = 7.2 Hz, 1H), 7.53 (t, *J* = 6.8 Hz, 1H), 7.39-7.12(m, 2H), 6.67 (s, 1H), 5.77-5.69 (m, 1H), 3.71-3.63 (m, 1H), 1.64 (d, *J* = 7.2 Hz, 3H), 1.22-1.14 (m, 4H); ESI-MS(m/z): 442.0 $[M+H]^+$.

## Example 2

**Preparation of (*R*)-1-chloro-6-cyclopropyl-4-((1-(3-(difluoromethyl)-2-fluorophenyl)ethyl)amino)pyrido[3,4-*d*]pyridazin-7(6*H*)-one**

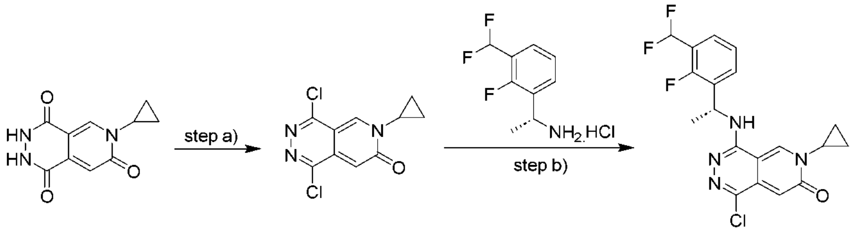


step a): Preparation of 1,4-dichloro-6-cyclopropylpyrido[3,4-*d*]pyridazin-7(6*H*)-one

6-Cyclopropyl-2,3-dihydropyrido[3,4-*d*]pyridazine-1,4,7(6*H*)-trione(8.8 g, 68.493 mmol) and acetonitrile (500 mL) were added to a reaction flask. Under stirring at room temperature, DIPEA (26.5 g, 205.479 mmol) and phosphorus oxychloride (31.5 g, 205.479 mmol) were added. The mixture was warmed to 100°C under nitrogen protection and reacted for 3 h. After the reaction was completed, the mixture was cooled to room temperature. The reaction solution was slowly poured into a mixture of ice and sodium bicarbonate for quenching, and the mixture was extracted with dichloromethane (100 mL × 3). The organic phases were combined, dried over anhydrous sodium sulfate, filtered, and the filtrate was concentrated under reduced pressure. The residue was purified by silica gel column chromatography (eluent: dichloromethane/methanol = 49/1) to obtain 1,4-dichloro-6-cyclopropylpyrido[3,4-d]pyridazin-7(6*H*)-one; yield 5.6%; ESI-MS (m/z): 256.0 $[M+H]^+$.

step b): Preparation of (*R*)-1-chloro-6-cyclopropyl-4-((1-(3-(difluoromethyl)-2-fluorophenyl)ethyl)amino)pyrido[3,4-*d*]pyridazin-7(6*H*)-one

1,4-Dichloro-6-cyclopropylpyrido[3,4-*d*]pyridazin-7(6*H*)-one (500 mg, 1.953 mmol), (*R*)-1-(3-(difluoromethyl)-2-fluorophenyl)ethan-1-amine hydrochloride (439 mg, 1.953 mmol), and 1,4-dioxane (10 mL) were added to a microwave reaction flask. The mixture was warmed to 120°C in a microwave reactor and reacted for 2 h. After the reaction was completed, the mixture was cooled to room temperature and concentrated under reduced pressure. The residue was purified by Prep-HPLC (separation method 3) to obtain (*R*)-1-chloro-6-cyclopropyl-4-((1-(3-(difluoromethyl)-2-fluorophenyl)ethyl)amino)pyrido[3,4-*d*]pyridazin-7(6*H*)-one, yield 26.6%; $^1$H NMR (400 MHz, DMSO-$d_6$) δ 9.14 (s, 1H), 8.14 (d, *J* = 6.8 Hz, 1H), 7.63 (t, *J* = 7.6 Hz, 1H), 7.50 (t, *J* = 7.2 Hz, 1H), 7.28 (t, *J* = 7.6 Hz, 1H), 7.24 (t, *J* = 56.0 Hz, 1H), 6.51 (s, 1H), 5.66-5.59 (m, 1H), 3.66-3.60 (m, 1H), 1.58 (d, *J* = 7.2 Hz, 3H), 1.20-1.09 (m, 4H); ESI-MS (m/z): 409.0 $[M+H]^+$.

According to the preparation method of **Example 2,** with the corresponding raw materials, the compound in the following Example was prepared.

| Number | Chemical Name | Structure | $^1$H NMR and MS |
|---|---|---|---|
| **Example 3** | (*R*)-1-chloro-4-((1-(3-(difluoromethyl)-2-fluorophenyl)ethyl)a mino)-6-(1-methylcyclopropyl)p yrido [3,4-*d*]pyridazin-7(6*H*)-one | 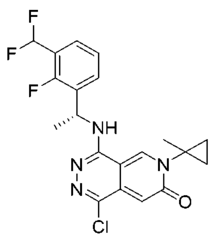 | $^1$H NMR (400 MHz, DMSO-$d_6$) δ 9.39 (s, 1H), 8.17 (d, *J* = 6.4 Hz, 1H), 7.64 (t, *J* = 7.2 Hz, 1H), 7.51 (t, *J* = 6.8 Hz, 1H), 7.38-7.11 (m, 2H), 6.49 (s, 1H), 5.68-5.55 (m, 1H), 1.58 (d, *J* = 7.2 Hz, 3H), 1.55 (s, 3H), 1.22-1.04 (m, 4H); ESI-MS(m/z): 423.0 $[M+H]^+$. |

## Example 4

**Preparation of (*R*)-6-cyclopropyl-4-((1-(3-(difluoromethyl)-2-fluorophenyl)ethyl)amino)-1-(pyrrolidin-1-yl) pyrido[3,4-*d*]pyridazin-7(6*H*)-one**

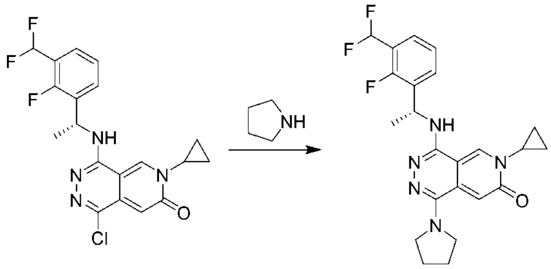

(*R*)-1-Chloro-6-cyclopropyl-4-((1-(3-(difluoromethyl)-2-fluorophenyl)ethyl)amino)pyrido[3,4-*d*]pyridazin-7(6*H*)-one (150 mg, 0.368 mmol), pyrrolidine (52 mg, 0.735 mmol), and 1,4-dioxane (2 mL) were added to a microwave reaction flask. The mixture was warmed to 100°C under microwave irradiation for 1 h. After the reaction was completed, the mixture was concentrated under reduced pressure. The residue was purified by Prep-HPLC (separation method 3) to obtain (*R*)-6-cyclopropyl-4-((1-(3-(difluoromethyl)-2-fluorophenyl)ethyl)amino)-1-(pyrrolidin-1-yl)pyrido[3,4-*d*]pyridazin-7(6*H*)-one, yield 27.6%; $^1$H NMR (400 MHz, DMSO-$d_6$) δ 8.87 (s, 1H), 7.60 (t, *J* = 7.6 Hz, 1H), 7.47 (t, *J* = 7.2 Hz, 1H), 7.27-7.25 (m, 2H), 7.24 (t, *J* = 56.0 Hz, 1H), 6.65 (s, 1H), 5.55-5.48 (m, 1H), 3.66-3.59 (m, 1H), 3.43-3.35 (m, 4H), 1.87-1.77 (m, 4H), 1.52 (d, *J* = 7.2 Hz, 3H), 1.19-1.09 (m, 4H); ESI-MS (m/z): 444.0 [M+H]$^+$.

According to the preparation method of **Example 4,** with the corresponding raw materials, the compounds in the following Examples were prepared.

| Number | Chemical Name | Structure | $^1$H NMR and MS |
|---|---|---|---|
| **Example 5** | (*R*)-4-((1-(3-(difluoromethyl)-2-fluorophenyl)ethyl) amino)-6-(1-methylcyclopropyl)-1-(pyrrolidin-1-yl)pyrido [3,4-*d*]pyridazin-7(6*H*)-one | 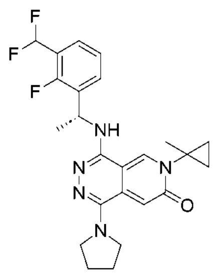 | $^1$H NMR (400 MHz, DMSO-$d_6$) δ 9.11 (s, 1H), 7.61 (t, *J* = 7.6 Hz, 1H), 7.47 (t, *J* = 7.2 Hz, 1H), 7.37-7.10 (m, 3H), 6.62 (s, 1H), 5.55-5.45 (m, 1H), 3.41-3.33 (m, 4H), 1.86-1.75 (m, 4H), 1.57-1.49 (m, 6H), 1.21-1.05 (m, 4H); ESI-MS(m/z): 458.0 [M+H]$^+$. |
| **Example 6** | (*R*)-4-((1-(3-(difluoromethyl)-2-fluorophenyl)ethyl) amino)-6-(1-methylcyclopropyl)-1-(piperidin-1-yl)pyrido[3,4-d]pyridazin-7(6*H*)-one | 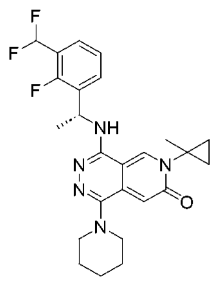 | $^1$H NMR (400 MHz, DMSO-$d_6$) δ 9.21 (s, 1H), 7.62 (t, *J* = 7.6 Hz, 1H), 7.53 (d, *J* = 6.8 Hz, 1H), 7.48 (t, *J* = 7.2 Hz, 1H), 7.27 (t, *J* = 7.6 Hz, 1H), 7.24 (t, *J* = 56.0 Hz, 1H), 6.38 (s, 1H), 5.59-5.52 (m, 1H), 2.94 (t, *J* = 5.2 Hz, 4H), 1.66-1.61 (m, 4H), 1.57-1.51 (m, 8H), 1.23-1.13 (m, 2H), 1.10-1.05 (m, 2H); ESI-MS (m/z): 472.0 [M+H]$^+$. |
| **Example 7** | (*R*)-4-((1-(3-(difluoromethyl)-2-fluorophenyl)ethyl) amino)-6-(1-methylcyclopropyl)-1-morpholinopyrido[3 ,4-*d*] pyridazin-7(6*H*)-one | 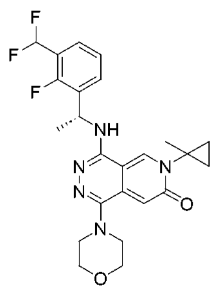 | $^1$H NMR (400 MHz, DMSO-$d_6$) δ 9.24 (s, 1H), 7.66-7.58 (m, 2H), 7.48 (t, *J* = 7.2 Hz, 1H), 7.27 (t, *J* = 7.6 Hz, 1H), 7.24 (t, *J* = 52.0 Hz, 1H), 6.46 (s, 1H), 5.61-5.54 (m, 1H), 3.73 (t, *J* = 4.4 Hz, 4H), 2.98 (t, *J* = 4.4 Hz, 4H), 1.58-1.53 (m, 6H), 1.20-1.13 (m, 2H), 1.10-1.07 (m, 2H); ESI-MS (m/z): 474.0 [M+H]$^+$. |

(continued)

| Number | Chemical Name | Structure | $^1$H NMR and MS |
|---|---|---|---|
| **Example 8** | (*R*)-4-((1-(3-(difluoromethyl)-2-fluorophenyl)ethyl) amino)-1-(dimethylamino)-6-(1-methylcyclopropyl) pyrido [3,4-*d*]pyridazin-7(6*H*)-one | 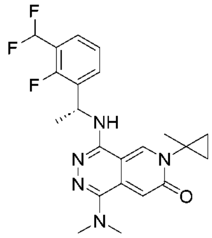 | $^1$H NMR (400 MHz, DMSO-$d_6$) δ 9.20 (s, 1H), 7.62 (t, *J* = 7.2 Hz, 1H), 7.54-7.44 (m, 2H), 7.38-7.11 (m, 2H), 6.46 (s, 1H), 5.60-5.49 (m, 1H), 2.69 (s, 6H), 1.58-1.49 (m, 6H), 1.21-1.05 (m, 4H); ESI-MS(m/z): 432.0 [M+H]$^+$. |
| **Example 9** | (*R*)-4-((1-(3-(difluoromethyl)-2-fluorophenyl)ethyl) amino)-1-(methylamino)-6-(1-methylcyclopropyl) pyrido [3,4-*d*]pyridazin-7(6*H*)-one formate | 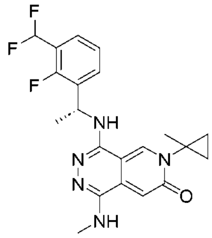 | $^1$H NMR (400 MHz, DMSO-*d*6) δ 12.69 (s, 1H), 9.08 (s, 1H), 8.14 (s, 1H), 7.60 (t, *J* = 7.2 Hz, 1H), 7.47 (t, *J* = 6.8 Hz, 1H), 7.37-7.10 (m, 3H), 7.09-6.75 (m, 2H), 5.49-5.37 (m, 1H), 2.75 (s, 3H), 1.55-1.51 (m, 6H), 1.19-1.05 (m, 4H); ESI-MS(m/z): 418.0 [M+H]$^+$. |
| **Example 10** | (*R*)-1-(cyclopropylamino) -4-((1-(3-(difluoromethyl)-2-fluorophenyl)ethyl) amino)-6-(1-methylcyclopropyl) pyrido [3,4-*d*]pyridazin-7(6*H*)-one | 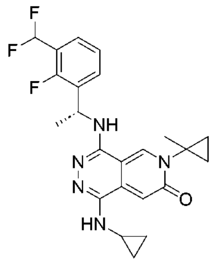 | $^1$H NMR (400 MHz, DMSO-$d_6$) δ 9.04 (s, 1H), 7.60 (t, *J* = 7.6 Hz, 1H), 7.47 (t, *J* = 7.2 Hz, 1H), 7.26 (t, *J* = 7.6 Hz, 1H), 7.47 (t, *J* = 56.0 Hz, 1H), 7.02 (d, *J* = 6.8 Hz, 1H), 6.81 (s, 1H), 6.51 (s, 1H), 5.52-5.42 (m, 1H), 2.69-2.60 (m, 1H), 1.53 (s, 3H), 1.51 (s, 3H), 1.19-1.14 (m, 2H), 1.09-1.05 (m, 2H), 0.62-0.57 (m, 2H), 0.42-0.38 (m, 2H); ESI-MS (m/z): 444.0 [M+H]$^+$. |
| **Example 11** | (*R*)-4-((1-(3-(difluoromethyl)-2-fluorophenyl)ethyl) amino)-6-(1-methylcyclopropyl)-1-((1-methylcyclopropyl) amino)pyrido[3,4-*d*]pyridazin-7(6*H*)-one | 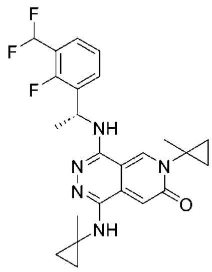 | $^1$H NMR (400 MHz, DMSO-$d_6$) δ 9.03 (s, 1H), 7.63 (t, *J* = 7.6 Hz, 1H), 7.48 (t, *J* = 6.8 Hz, 1H), 7.38-7.11 (m, 2H), 7.01-6.92 (m, 1H), 6.81 (s, 1H), 6.57 (s, 1H), 5.57-5.46 (m, 1H), 1.56-1.48 (m, 6H), 1.31 (s, 3H), 1.16-1.03 (m, 4H), 0.65-0.49 (m, 4H); ESI-MS(m/z): 458.0 [M+H]$^+$. |
| **Example 12** | (*R*)-4-((1-(3-(difluoromethyl)-2-fluorophenyl)ethyl) amino)-1-(isopropylamino)-6-(1-methylcyclopropyl) pyrido [3,4-*d*]pyridazin-7(6*H*)-one formate | 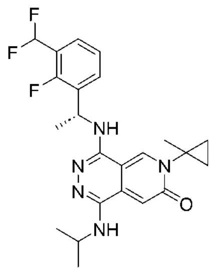 | the resulting crude product was purified by Prep-HPLC (separation method 2) to obtain (*R*)-4-((1-(3-(difluoromethyl)-2-fluorophenyl)ethyl)amino)-1-(isopropylamino)-6-(1-methylcyclopropyl)pyrido [3,4-*d*]pyridazin-7(6*H*)-one formate, yield 16.9%; $^1$H NMR (400 MHz, DMSO-$d_6$) δ 12.66 (s, 1H), 9.12 (s, 1H), 8.14 (s, 1H), 7.61 (t, *J* = 7.2 Hz, 1H), 7.56-7.43 (m, 2H), 7.37-6.95 (m, 4H), 5.45-5.36 (m, 1H), 4.09-3.96 (m, 1H), 1.57-1.49 (m, 6H), 1.23-1.05 (m, 10H); ESI-MS(m/z): 446.0 [M+H]$^+$. |

(continued)

| Number | Chemical Name | Structure | $^1$H NMR and MS |
|---|---|---|---|
| **Example 13** | (*R*)-6-cyclopropyl-4-((1-(3-(difluoromethyl)-2-fluorophenyl)ethyl) amino)-1-(methylamino)pyrid o[3,4-*d*]pyridazin-7(6*H*)-one | 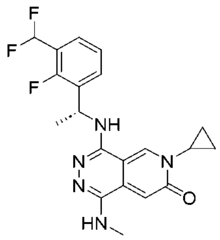 | $^1$H NMR (400 MHz, DMSO-$d_6$) δ 8.82 (s, 1H), 7.58 (t, *J* = 7.6 Hz, 1H), 7.46 (t, *J* = 7.2 Hz, 1H), 7.27-7.21 (m, 1H), 7.23 (t, *J* = 54.4 Hz, 1H), 7.00 (s, 1H), 6.81 (s, 1H), 6.48 (s, 1H), 5.46 (p, *J* = 6.8 Hz, 1H), 3.64-3.56 (m, 1H), 2.74 (d, *J* = 4.4 Hz, 3H), 1.51 (d, *J* = 7.2 Hz, 3H), 1.18-1.09 (m, 4H); ESI-MS (m/z): 404.2 [M+H]$^+$. |
| **Example 14** | (*R*)-6-cyclopropyl-4-((1-(3-(difluoromethyl)-2-fluorophenyl)ethyl) amino)-1-(3,3-difluoropyrrolidin-1-yl)pyrido[3,4-*d*]pyridazin-7(6*H*)-one | 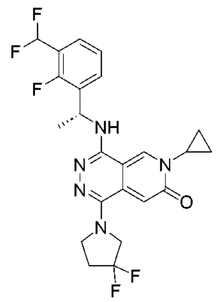 | $^1$H NMR (400 MHz, DMSO-$d_6$) δ 13.03 (s, 1H), 9.17 (s, 1H), 7.65 (t, *J* = 7.6 Hz, 1H), 7.53 (t, *J* = 6.8 Hz, 1H), 7.30 (t, *J* = 7.6 Hz, 1H), 7.25 (t, *J* = 54.0 Hz, 1H), 6.71 (s, 1H), 5.54-5.47 (m, 1H), 3.85-3.75 (m, 2H), 3.66-3.57 (m, 3H), 2.46-2.32 (m, 2H), 1.61 (d, *J* = 6.8 Hz, 3H), 1.22-1.14 (m, 4H); ESI-MS(m/z): 480.0 [M+H]$^+$. |
| **Example 15** | (*R*)-1-(azetidin-1-yl)-6-cyclopropyl-4-((1-(3-(difluoromethyl)-2-fluorophenyl)ethyl) amino)pyrido[3,4-*d*]pyridazin-7(6*H*)-one | 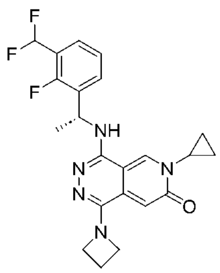 | $^1$H NMR (400 MHz, DMSO-*d6*) δ 8.66 (s, 1H), 8.35 (s, 1H), 7.65 (t, *J* = 7.2 Hz, 1H), 7.49 (t, *J* = 6.8 Hz, 1H), 7.36-7.09 (m, 2H), 7.05 (s, 1H), 5.24-5.12 (m, 1H), 3.76-3.70 (m, 1H), 3.58-3.49 (m, 4H), 1.92-1.82 (m, 2H), 1.52 (d, *J* = 7.2 Hz, 3H), 1.16-1.05 (m, 4H); ESI-MS(m/z): 430.0 [M+H]$^+$. |
| **Example 16** | (*R*)-1-(azetidin-1-yl)-4-((1-(3-(difluoromethyl)-2-fluorophenyl)ethyl) amino)-6-(1-methylcyclopropyl) pyrido [3,4-*d*]pyridazin-7(6*H*)-one formate | 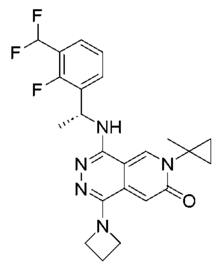 | the resulting crude product was purified by Prep-HPLC (separation method 2) to obtain (*R*)-1-(azetidin-1-yl)-4-((1-(3-(difluoromethyl)-2-fluorophenyl)ethyl)amino)-6-(1-methylcyclopropyl)pyrido [3,4-*d*]pyridazin-7(6*H*)-one formate, yield 16.9%; $^1$H NMR (400 MHz, DMSO-$d_6$) δ 9.14 (s, 1H), 8.13 (s, 1H), 7.60 (t, *J* = 7.2 Hz, 1H), 7.47 (t, *J* = 6.8 Hz, 1H), 7.38-7.10 (m, 3H), 6.38 (s, 1H), 5.53-5.43 (m, 1H), 4.03 (t, *J* = 6.8 Hz, 4H), 2.24-2.17 (m, 2H), 1.57-1.50 (m, 6H), 1.21-1.04 (m, 4H); ESI-MS(m/z): 444.0 [M+H]$^+$. |
| **Example 17** | (*R*)-6-cyclopropyl-1-(diethylamino)-4-((1-(3-(difluoromethyl)-2-fluorophenyl)ethyl) amino)pyrido[3,4-*d*]pyridazin-7(6*H*)-one | 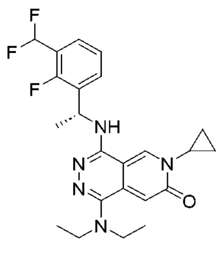 | $^1$H NMR (400 MHz, DMSO-*d6*) δ 8.98 (s, 1H), 7.63 (t, *J* = 7.2 Hz, 1H), 7.55 (d, *J* = *6.8 Hz,* 1H), 7.49 (t, *J* = 7.2 Hz, 1H), 7.39-7.12 (m, 2H), 6.45 (s, 1H), 5.65-5.55 (m, 1H), 3.65-3.57 (m, 1H), 3.08 (q, *J* = 6.8 Hz, 4H), 1.54 (d, *J* = 7.2 Hz, 3H), 1.19-1.08 (m, 4H), 0.98 (t, *J* = 6.8 Hz, 6H); ESI-MS(m/z): 446.0 [M+H]$^+$. |

(continued)

| Number | Chemical Name | Structure | $^1$H NMR and MS |
|---|---|---|---|
| **Example 18** | 1-(3-azabicyclo[3.1.0]he xan-3-yl)-6-cyclopropyl-4-(((*R*)-1-(3-(difluoromethyl)-2-fluorophenyl)ethyl) amino)pyrido[3,4-*d*]pyridazin-7(6*H*)-one | 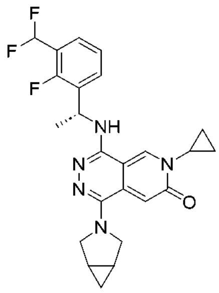 | $^1$H NMR (400 MHz, DMSO-*d*6) δ 8.91 (s, 1H), 7.59 (t, *J* = 7.6 Hz, 1H), 7.47 (t, *J* = 6.8 Hz, 1H), 7.42-7.10 (m, 3H), 6.50 (s, 1H), 5.59-5.47 (m, 1H), 3.65-3.46 (m, 3H), 3.30-3.18 (m, 2H), 1.58-1.48 (m, 5H), 1.19-1.07 (m, 4H), 0.58-0.47 (m, 2H); ESI-MS(m/z): 456.0 [M+H]$^+$. |
| **Example 19** | (*R*)-6-cyclopropyl-4-((1-(3-(difluoromethyl)-2-fluorophenyl)ethyl) amino)-1-(4,4-difluoropiperidin-1-yl)pyrido [3,4-*d*]pyridazin-7(6*H*)-one | 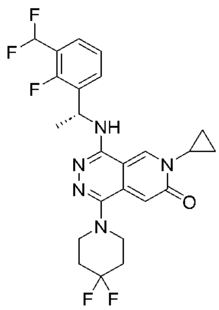 | $^1$H NMR (400 MHz, DMSO-*d*6) δ 9.02 (s, 1H), 7.67-7.59 (m, 2H), 7.53-7.45 (m, 1H), 7.39-7.11 (m, 2H), 6.50 (s, 1H), 5.64-5.53 (m, 1H), 3.67-3.59 (m, 1H), 3.16-3.07 (m, 4H), 2.19-2.08 (m, 4H), 1.54 (d, *J* = 7.2 Hz, 3H), 1.21-1.08 (m, 4H); ESI-MS(m/z): 494.0 [M+H]$^+$. |
| **Example 20** | (*R*)-6-cyclopropyl-1-(3,3-difluoroazetidin-1-yl)-4-((1-(3-(difluoromethyl)-2-fluorophenyl)ethyl) amino)pyrido [3,4-*d*]pyridazin-7(6*H*)-one | 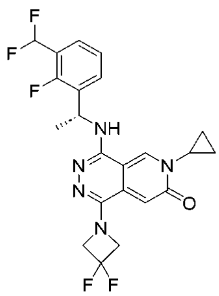 | $^1$H NMR (400 MHz, DMSO-*d*6) δ 8.98 (s, 1H), 7.61 (t, *J* = 7.2 Hz, 1H), 7.53-7.45 (m, 2H), 7.38-7.11 (m, 2H), 6.42 (s, 1H), 5.59-5.49 (m, 1H), 4.57-4.42 (m, 4H), 3.69-3.55 (m, 1H), 1.54 (d, *J* = 7.2 Hz, 3H), 1.20-1.10 (m, 4H); ESI-MS(m/z): 466.0 [M+H]$^+$. |
| **Example 21** | 1-(3-azabicyclo[3.1.0]he xan-3-yl)-4-(((*R*)-1-(3-(difluoromethyl)-2-fluorophenyl)ethyl) amino)-6-(1-methylcyclopropyl) pyrido[3,4-*d*]pyridazin-7(6*H*)-one | 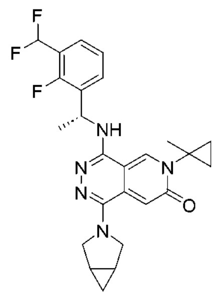 | $^1$H NMR (400 MHz, DMSO-$d_6$) δ 9.15 (s, 1H), 7.61 (t, *J* = 7.6 Hz, 1H), 7.50-7.45 (m, 1H), 7.41-7.10 (m, 3H), 6.49 (s, 1H), 5.57-5.46 (m, 1H), 3.64-3.47 (m, 2H), 3.29-3.21 (m, 2H), 1.58-1.50 (m, 8H), 1.19-1.05 (m, 4H), 0.54-0.47 (m, 2H); ESI-MS(m/z): 470.0 [M+H]$^+$. |
| **Example 22** | (*R*)-1-(3,3-difluoroazetidin-1-yl)-4-((1-(3-(difluoromethyl)-2-fluorophenyl)ethyl) amino)-6-(1-methylcyclopropyl) pyrido[3,4-*d*]pyridazin-7(6*H*)-one | 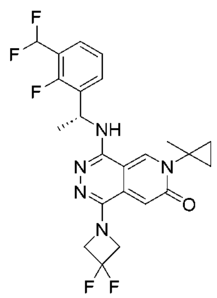 | $^1$H NMR (400 MHz, DMSO-$d_6$) δ 9.21 (s, 1H), 7.61 (t, *J* = 7.2 Hz, 1H), 7.51-7.44 (m, 2H), 7.37-7.10 (m, 2H), 6.38 (s, 1H), 5.59-5.48(m, 1H), 4.52-4.36 (m, 4H), 1.58-1.49 (m, 6H), 1.20-1.05 (m, 4H); ESI-MS(m/z): 480.0 [M+H]$^+$. |

(continued)

| Number | Chemical Name | Structure | $^1$H NMR and MS |
|---|---|---|---|
| **Example 23** | (*R*)-4-((1-(3-(difluoromethyl)-2-fluorophenyl)ethyl) amino)-1-(4,4-difluoropiperidin-1-yl)-6-(1-methylcyclopropyl) pyrido[3,4-*d*]pyridazin-7(6*H*)-one | 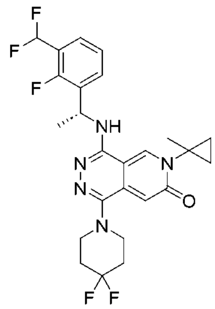 | $^1$H NMR (400 MHz, DMSO-d6) δ 9.32(s, 1H), 7.72-7.65(m, 2H), 7.59-7.52(m, 1H), 7.45-7.18(m, 2H), 6.53(s, 1H), 5.68-5.57(m, 1H), 3.21-3.13(m, 4H), 2.23-2.11(m, 4H), 1.63-1.54(m, 6H), 1.26-1.10(m, 4H); ESI-MS(m/z): 508.0 $[M+H]^+$. |
| **Example 24** | (*R*)-6-cyclopropyl-4-((1-(3-(difluoromethyl)-2-fluorophenyl)ethyl) amino)-1-(1,1-dioxidothiomorphol ino)pyrido[3,4-*d*]pyridazin-7(6*H*)-one | 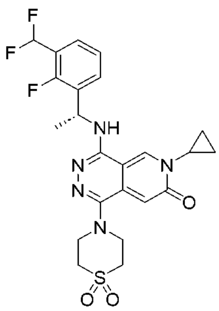 | $^1$H NMR (400 MHz, DMSO-$d_6$) δ 9.03 (s, 1H), 7.69 (d, *J* = 6.8 Hz, 1H), 7.62 (t, *J* = 7.2 Hz, 1H), 7.49 (t, *J* = 7.2 Hz, 1H), 7.27 (t, *J* = 7.8 Hz, 1H), 7.24 (t, *J* = 54.4 Hz, 1H), 6.56 (s, 1H), 5.61 (p, *J* = 7.2 Hz, 1H), 3.66-3.60 (m, 1H), 3.52-3.41 (m, 4H), 3.31-3.21 (m, 4H), 1.54 (d, *J* = 7.2 Hz, 3H), 1.20-1.08 (m, 4H); ESI-MS (m/z): 508.2 $[M+H]^+$. |
| **Example 25** | 6-cyclopropyl-4-(((*R*)-1-(3-(difluoromethyl)-2-fluorophenyl)ethyl) amino)-1-((*R*)-2-methylpyrrolidin-1-yl)pyrido[3,4-*d*]pyridazin-7(6*H*)-one | 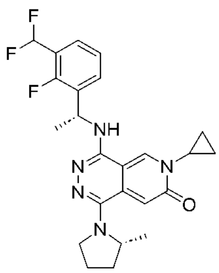 | $^1$H NMR (400 MHz, DMSO-$d_6$) δ 8.91 (s, 1H), 7.63 (t, *J* = 7.6 Hz, 1H), 7.48 (t, *J* = 7.2 Hz, 1H), 7.35 (d, *J* = 6.8 Hz, 1H), 7.26 (t, *J* = 7.6 Hz, 1H), 7.24 (t, *J* = 56.0 Hz, 1H), 6.55 (s, 1H), 5.57-5.50 (m, 1H), 4.18-4.07 (m, 1H), 3.72-3.57 (m, 2H), 3.14-3.04 (m, 1H), 2.13-2.02 (m, 1H), 1.91-1.81 (m, 1H), 1.73-1.62 (m, 1H), 1.56-1.47 (m, 4H), 1.19-1.13 (m, 3H), 1.11-1.04 (m, 1H), 0.95 (d, *J* = 6.0 Hz, 3H); ESI-MS (m/z): 458.0 $[M+H]^+$. |
| **Example 26** | (*R*)-4-((1-(3-(difluoromethyl)-2-fluorophenyl)ethyl) amino)-1-(3,3-difluoropyrrolidin-1-yl)-6-(1-methylcyclopropyl) pyrido[3,4-*d*]pyridazin-7(6*H*)-one | 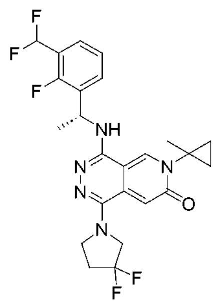 | $^1$H NMR (400 MHz, DMSO-$d_6$) δ 9.21 (s, 1H), 7.62 (t, *J* = 7.6 Hz, 1H), 7.55-7.45 (m, 2H), 7.27 (t, *J* = 7.6 Hz, 1H), 7.24 (t, *J* = 56.0 Hz, 1H), 6.56 (s, 1H), 5.59-5.49 (m, 1H), 3.69 (t, *J* = 13.2 Hz, 2H), 3.56-3.45 (m, 2H), 2.47-2.36 (m, 2H), 1.57-1.53 (m, 6H), 1.24-1.13 (m, 2H), 1.12-1.06 (m, 2H); ESI-MS (m/z): 494.0 $[M+H]^+$. |
| **Example 27** | (*R*)-4-((1-(3-(difluoromethyl)-2-fluorophenyl)ethyl) amino)-1-(1,1-dioxidothiomorphol ino)-6-(1-methylcyclopropyl) pyrido[3,4-*d*]pyridazin-7(6*H*)-one | 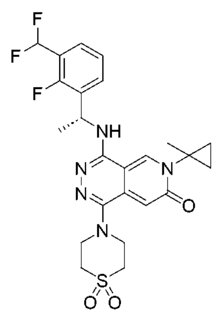 | $^1$H NMR (400 MHz, DMSO-$d_6$) δ 9.27 (s, 1H), 7.68 (d, *J* = 6.4 Hz, 1H), 7.64 (t, *J* = 7.6 Hz, 1H), 7.49 (t, *J* = 7.2 Hz, 1H), 7.28 (t, *J* = 7.6 Hz, 1H), 7.24 (t, *J* = 56.0 Hz, 1H), 6.53 (s, 1H), 5.65-5.55 (m, 1H), 3.50-3.44 (m, 4H), 3.30-3.25 (m, 4H), 1.58-1.53 (m, 6H), 1.21-1.12 (m, 2H), 1.11-1.06 (m, 2H); ESI-MS (m/z): 522.0 $[M+H]^+$. |

(continued)

| Number | Chemical Name | Structure | $^1$H NMR and MS |
|---|---|---|---|
| **Example 28** | 4-(((*R*)-1-(3-(difluoromethyl)-2-fluorophenyl)ethyl) amino)-6-(1-methylcyclopropyl)-1-((*R*)-2-methylpyrrolidin-1-yl)pyrido[3,4-*d*]pyridazin-7(6*H*)-one | 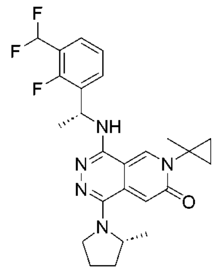 | $^1$H NMR (400 MHz, DMSO-$d_6$) δ 9.16 (s, 1H), 7.64 (t, *J* = 7.6 Hz, 1H), 7.48 (t, *J* = 7.2 Hz, 1H), 7.34 (s, 1H), 7.27 (t, *J*= 7.6 Hz, 1H), 7.24 (t, *J* = 56.0 Hz, 1H), 6.53 (s, 1H), 5.56-5.47 (m, 1H), 4.16-4.06 (m, 1H), 3.71-3.62 (m, 1H), 3.14-3.04 (m, 1H), 2.12-2.04 (m, 1H), 1.91-1.82 (m, 1H), 1.74-1.62 (m, 1H), 1.54 (m, 6H), 1.52-1.44 (m, 1H), 1.22-1.13 (m, 2H), 1.10-1.05 (m, 2H), 0.95 (d, *J* = 6.0 Hz, 3H); ESI-MS (m/z): 472.0 [M+H]$^+$. |
| **Example 29** | (*R*)-1-(diethylamino)-4-(((*R*)1-(3-(difluoromethyl)-2-fluorophenyl)ethyl) amino)-6-(1-methylcyclopropyl) pyrido [3,4-*d*]pyridazin-7(6*H*)-one | 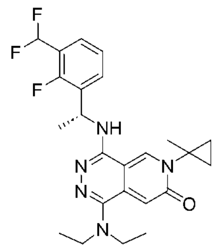 | $^1$H NMR (400 MHz, DMSO-$d_6$) δ 9.21 (s, 1H), 7.64 (t, *J* = 7.6 Hz, 1H), 7.57-7.45 (m, 2H), 7.28 (t, *J* = 7.6 Hz, 1H), 7.24 *(t, J* = 56.0 Hz, 1H), 6.42 (s, 1H), 5.62-5.54 (m, 1H), 3.07 (q, *J* = 7.2 Hz, 4H), 1.55 (d, *J*= 5.2 Hz, 6H), 1.23-1.13 (m, 2H), 1.11-1.05 (m, 2H), 0.97 (t, *J* = 7.2 Hz, 6H); ESI-MS (m/z): 460.0 [M+H]$^+$. |

**Example 30**

**Preparation of (*R*)-6-cyclopropyl-4-((1-(3-(difluoromethyl)-2-fluorophenyl)ethyl)amino)-1-(1*H*-pyrazol-1-yl) pyrido[3,4-*d*]pyridazin-7(6*H*)-one**

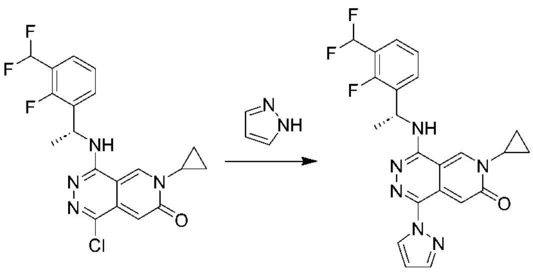

(*R*)-1-Chloro-6-cyclopropyl-4-((1-(3-(difluoromethyl)-2-fluorophenyl)ethyl)amino)pyrido[3,4-d]pyridazin-7(6*H*)-one (40 mg, 0.098 mmol), pyrazole (20 mg, 0.293 mmol), and 1,4-dioxane (2 mL) were added to a microwave reaction flask. The mixture was warmed to 100°C under microwave irradiation for 1 h. After the reaction was completed, the mixture was cooled to room temperature, and the reaction solution was concentrated under reduced pressure. The residue was purified by Prep-HPLC (separation method 3) to obtain (*R*)-6-cyclopropyl-4-((1-(3-(difluoromethyl)-2-fluorophenyl)ethyl)amino)-1- (1*H*-pyrazol-1-yl)pyrido[3,4-*d*]pyridazin-7(6*H*)-one, yield 23.6%; $^1$H NMR (400 MHz, DMSO-$d_6$) δ 9.17 (s, 1H), 8.25 (s, 1H), 8.20 (d, *J* = 6.8 Hz, 1H), 7.83 (s, 1H), 7.67 (t, *J* = 7.6 Hz, 1H), 7.51 (t, *J* = 7.2 Hz, 1H), 7.29 (t, *J* = 7.6 Hz, 1H), 7.25 (t, *J* = 56.0 Hz, 1H), 6.97 (s, 1H), 6.52 (t, *J* = 2.0 Hz, 1H), 5.77-5.68 (m, 1H), 3.69-3.62 (m, 1H), 1.60 (d, *J* = 7.2 Hz, 3H), 1.22-1.11 (m, 4H); ESI-MS (m/z): 441.0 [M+H]$^+$.

According to the preparation method of **Example 30,** with the corresponding raw materials, the compounds in the following Examples were prepared.

| Number | Chemical Name | Structure | $^1$H NMR and MS |
|---|---|---|---|
| **Example 31** | (*R*)-6-cyclopropyl-4-((1-(3-(difluoromethyl)-2-fluorophenyl) ethyl) amino)-1-(1*H*-imidazol-1-yl)pyrido[3,4-*d*]pyridazin-7(6*H*)-one | 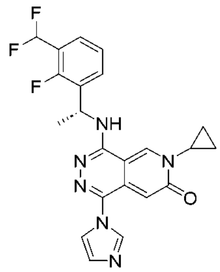 | $^1$H NMR (400 MHz, DMSO-$d_6$) δ 9.21 (s, 1H), 8.28 (d, *J* = 6.8 Hz, 1H), 7.96 (s, 1H), 7.67 (t, *J* = 7.6 Hz, 1H), 7.54-7.49 (m, 2H), 7.30 (t, *J* = 7.6 Hz, 1H), 7.25 (t, *J* = 52.0 Hz, 1H), 7.12 (s, 1H), 6.03 (s, 1H), 5.78-5.68 (m, 1H), 3.69-3.61 (m, 1H), 1.61 (d, *J* = 7.2 Hz, 3H), 1.21-1.12 (m, 4H); ESI-MS (m/z): 441.0 [M+H]$^+$. |
| **Example 32** | (*R*)-6-cyclopropyl-4-((1-(3-(difluoromethyl)-2-fluorophenyl) ethyl) amino)-1-(2*H*-1,2,3-triazol-2-yl)pyrido[3,4-*d*]pyridazin-7(6*H*)-one | 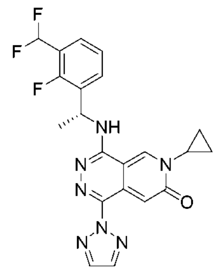 | $^1$H NMR (400 MHz, DMSO-$d_6$) δ 9.24 (s, 1H), 8.48-8.42 (m, 1H), 8.17 (s, 1H), 7.98 (s, 1H), 7.68 (t, *J* = 7.6 Hz, 1H), 7.52 (t, *J* = 7.2 Hz, 1H), 7.31 (t, *J* = 7.6 Hz, 1H), 7.25 (t, *J* = 56.0 Hz, 1H), 6.15 (s, 1H), 5.83-5.73 (m, 1H), 3.69-3.61 (m, 1H), 1.63 (d, *J* = 7.2 Hz, 3H), 1.21-1.14 (m, 4H); ESI-MS (m/z): 442.0 [M+H]$^+$. |
| **Example 33** | (*R*)-4-((1-(3-(difluoromethyl)-2-fluorophenyl)ethyl) amino)-6-(1-methylcyclopropyl)-1-(1*H*-pyrazol-1-yl)pyrido [3,4-*d*]pyridazin-7(6*H*)-one | 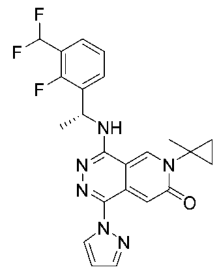 | $^1$H NMR (400 MHz, DMSO-$d_6$) δ 9.41 (s, 1H), 8.23 (d, *J* = 2.4 Hz, 1H), 8.20 (d, *J* = 6.8 Hz, 1H), 7.82 (s, 1H), 7.68 (t, *J* = 7.6 Hz, 1H), 7.51 (t, *J* = 7.2 Hz, 1H), 7.30 (t, *J* = 7.6 Hz, 1H), 7.25 (t, *J* = 52.0 Hz, 1H), 6.94 (s, 1H), 6.51 (t, *J* = 2.4 Hz, 1H), 5.75-5.66 (m, 1H), 1.61 (d, *J* = 7.2 Hz, 3H), 1.56 (s, 3H), 1.25-1.15 (m, 2H), 1.14-1.07 (m, 2H); ESI-MS (m/z): 455.0 [M+H]$^+$. |
| **Example 34** | (*R*)-4-((1-(3-(difluoromethyl)-2-fluorophenyl)ethyl) amino)-1-(1*H*-imidazol-1-yl)-6-(1-methylcyclopropyl) pyrido [3,4-*d*]pyridazin-7(6*H*)-one | 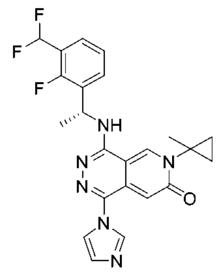 | $^1$H NMR (400 MHz, DMSO-$d_6$) δ 9.45 (s, 1H), 8.29 (d, *J* = 6.8 Hz, 1H), 7.96 (s, 1H), 7.69 (t, *J* = 7.6 Hz, 1H), 7.54-7.49 (m, 2H), 7.31 (t, *J* = 7.6 Hz, 1H), 7.25 (t, *J* = 52.0 Hz, 1H), 7.12 (s, 1H), 6.01 (s, 1H), 5.76-5.68 (m, 1H), 1.61 (d, *J* = 7.2 Hz, 3H), 1.56 (s, 3H), 1.24-1.15 (m, 2H), 1.13-1.08 (m, 2H); ESI-MS (m/z): 455.0 [M+H]$^+$. |

**Example 35**

**Preparation of 3-((1*R*)-1-((1-(3-azabicyclo[3.1.0]hexan-3-yl)-6-cyclopropyl-7-oxo-6,7-dihydropyrido[3,4-*d*] pyridazin-4-yl)amino)ethyl)-2-methylbenzonitrile**

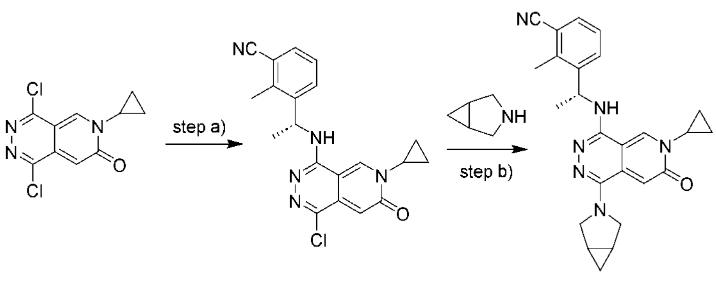

step a): Preparation of (*R*)-3-(1-((1-chloro-6-cyclopropyl-7-oxo-6,7-dihydropyrido[3,4-d]pyridazin-4-yl)amino)ethyl)- 2-methylbenzonitrile

1,4-Dichloro-6-cyclopropylpyrido[3,4-*d*]pyridazin-7(6*H*)-one (230 mg, 0.898 mmol), (*R*)-3-(1-aminoethyl)-2-methylbenzonitrile hydrochloride (194 mg, 0.988 mmol), *N*,*N*-diisopropylethylamine (290 mg, 2.245 mmol), and DMSO (10 mL) were added to a reaction flask. The mixture was warmed to 120°C under nitrogen protection and reacted for 1 h. After the reaction was completed, the mixture was cooled to room temperature. Ethyl acetate (100 mL) was added. The mixture was washed with saturated brine (30 mL × 3), dried over anhydrous sodium sulfate, filtered, and the filtrate was concentrated under reduced pressure. The residue was purified by silica gel column chromatography (eluent: petroleum ether/ethyl acetate = 1/3) to obtain (*R*)-3-(1-((1-chloro-6-cyclopropyl-7-oxo-6,7-dihydropyrido[3,4-*d*]pyridazin-4-yl)amino)ethyl)-2-methylbenzonitrile, yield 76.1%; ESI-MS (m/z): 380.1 $[M+H]^+$.

step b): Preparation of 3-((1*R*)-1-((1-(3-azabicyclo[3.1.0]hexan-3-yl)-6-cyclopropyl-7-oxo-6,7-dihydropyrido[3,4-*d*] pyridazin-4-yl)amino)ethyl)-2-methylbenzonitrile.

(*R*)-3-(1-((1-Chloro-6-cyclopropyl-7-oxo-6,7-dihydropyrido[3,4-*d*]pyridazin-4-yl)amino)ethyl)-2-methylbenzonitrile (50 mg, 0.132 mmol), 3-azabicyclo[3.1.0]hexane (47 mg, 0.396 mmol), and 1,4-dioxane (2 mL) were added to a microwave reaction flask. The mixture was warmed to 120°C in a microwave reactor and reacted for 1 h. After the reaction was completed, the mixture was concentrated under reduced pressure. The residue was purified by Prep-HPLC (separation method 3) to obtain 3-((1*R*)-1-((1-(3-azabicyclo[3.1.0]hexan-3-yl)-6-cyclopropyl-7-oxo-6,7-dihydropyrido [3,4-d] pyridazin-4-yl)amino)ethyl)-2-methylbenzonitrile, yield 27.6%; $^1$H NMR (400 MHz, DMSO-$d_6$) δ 8.89 (s, 1H), 7.72 (d, *J* = 7.6 Hz, 1H), 7.62 (d, *J*= 7.6 Hz, 1H), 7.42 (d, *J* = 6.4 Hz, 1H), 7.34 (t, *J* = 7.6 Hz, 1H), 6.48 (s, 1H), 5.43-5.38 (m, 1H), 3.64-3.54 (m, 2H), 3.49 (d, *J* = 10.0 Hz, 1H), 3.30-3.22 (m, 2H), 2.60 (s, 3H), 1.52-1.50 (m, 2H), 1.46 (d, *J* = 7.2 Hz, 3H), 1.17-1.13 (m, 2H), 1.12-1.07 (m, 2H), 0.55-0.48 (m, 2H); ESI-MS (m/z): 427.0 $[M+H]^+$.

According to the preparation method of **Example 35,** with the corresponding raw materials, the compounds in the following Examples were prepared.

| Number | Chemical Name | Structure | $^1$H NMR and MS |
|---|---|---|---|
| **Example 36** | (*R*)-3-(1-((1-(azetidin-1-yl)-6-cyclopropyl-7-oxo-6,7-dihydropyrido [3,4-*d*]pyridazin-4-yl)amino) ethyl)-2-methylbenzonitrile | 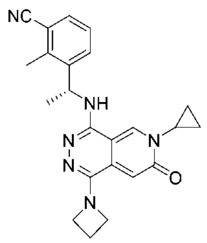 | $^1$H NMR (400 MHz, DMSO-$d_6$) δ 8.41 (s, 1H), 7.73 (d, *J* = 7.6 Hz, 1H), 7.60 (d, *J* = 7.2 Hz, 1H), 7.35 (t, *J* = 7.6 Hz, 1H), 6.87 (s, 1H), 6.76 (d, *J* = 6.4 Hz, 1H), 5.03 - 4.97 (m, 1H), 3.62-3.56 (m, 1H), 3.37-3.31 (m, 4H), 2.60 (s, 3H), 1.83-1.72 (m, 2H), 1.41 (d, *J* = 6.8 Hz, 3H), 1.13-1.08 (m, 2H), 1.05-0.98 (m, 2H); ESI-MS (m/z): 401.0 $[M+H]^+$. |
| **Example 37** | (*R*)-3-(1-((6-cyclopropyl-7-oxo-1-(pyrrolidin-1-yl)-6,7-dihydropyrido[3,4-*d*]pyridazin-4-yl)amino) ethyl)-2-methylbenzonitrile | 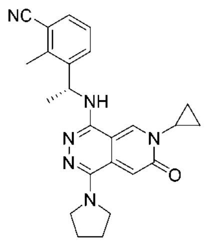 | $^1$H NMR (400 MHz, DMSO-$d_6$) δ 8.84 (s, 1H), 7.72 (d, *J* = 7.6 Hz, 1H), 7.61 (d, *J*= 7.6 Hz, 1H), 7.34 (t, *J* = 7.6 Hz, 1H), 7.26 (d, *J* = 6.8 Hz, 1H), 6.63 (s, 1H), 5.43-5.37 (m, 1H), 3.63-3.57 (m, 1H), 3.37 (d, *J* = 6.8 Hz, 4H), 2.60 (s, 3H), 1.86-1.77 (m, 4H), 1.46 *(d, J= 7.2* Hz, 3H), 1.17-1.07 (m, 4H); ESI-MS (m/z): 415.0 $[M+H]^+$. |
| **Example 38** | (*R*)-3-(1-((6-cyclopropyl-1-(methylamino)-7-oxo-6,7-dihydropyrido [3,4-*d*]pyridazin-4-yl)amino) ethyl)-2-methylbenzonitrile | 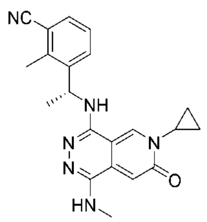 | $^1$H NMR (400 MHz, DMSO-$d_6$) δ 8.80 (s, 1H), 7.72 (d, *J* = 7.6 Hz, 1H), 7.61 (d, *J*= 7.6 Hz, 1H), 7.32 (t, *J* = 7.6 Hz, 1H), 7.05 (s, 1H), 6.80 (s, 1H), 6.52 (s, 1H), 5.37-5.30 (m, 1H), 3.61-3.55 (m, 1H), 2.75 (s, 3H), 2.60 (s, 3H), 1.44 (d, *J* = 6.8 Hz, 3H), 1.16-1.06 (m, 4H); ESI-MS (m/z): 375.0 $[M+H]^+$. |

(continued)

| Number | Chemical Name | Structure | $^1$H NMR and MS |
|---|---|---|---|
| **Example 39** | (*R*)-3-(1-((6-cyclopropyl-1-(dimethylamino)-7-oxo-6,7-dihydropyrido[3,4-*d*]pyridazin-4-yl)amino) ethyl)-2-methylbenzonitrile | 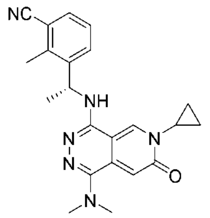 | $^1$H NMR (400 MHz, DMSO-$d_6$) δ 8.94 (s, 1H), 7.73 (d, *J* = 7.6 Hz, 1H), 7.63 (d, *J*= 7.6 Hz, 1H), 7.53 (d, *J*= 7.2 Hz, 1H), 7.34 (t, *J* = 7.6 Hz, 1H), 6.48 (s, 1H), 5.47 - 5.40 (m, 1H), 3.63-3.57 (m, 1H), 2.70 (s, 6H), 2.61 (s, 3H), 1.47 (d, *J* = 6.8 Hz, 3H), 1.18-1.09 (m, 4H); ESI-MS (m/z): 389.0 $[M+H]^+$. |
| **Example 40** | (*R*)-3-(1-((6-cyclopropyl-1-(diethylamino)-7-oxo-6,7-dihydropyrido [3,4-*d*]pyridazin-4-yl)amino) ethyl)-2-methylbenzonitrile | 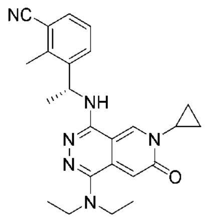 | $^1$H NMR (400 MHz, DMSO-$d_6$) δ 8.95 (s, 1H), 7.74 (d, *J* = 7.6 Hz, 1H), 7.63 (d, *J*= 7.6 Hz, 1H), 7.55 (d, *J* = 6.8 Hz, 1H), 7.35 (t, *J* = 7.6 Hz, 1H), 6.43 (s, 1H), 5.51-5.45 (m, 1H), 3.62-3.56 (m, 1H), 3.07 (q, *J* = 7.2 Hz, 4H), 2.61 (s, 3H), 1.47 (d, *J* = 6.8 Hz, 3H), 1.17-1.07 (m, 4H), 0.97 (t, *J* = 7.2 Hz, 6H); ESI-MS (m/z): 417.0 $[M+H]^+$. |
| **Example 41** | (*R*)-3-(1-((6-cyclopropyl-1-(3,3-difluoroazetidin-1-yl)-7-oxo-6,7-dihydropyrido[3,4-*d*]pyridazin-4-yl)amino)ethyl)-2-methylbenzonitrile | 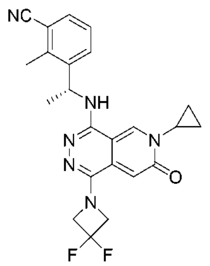 | $^1$H NMR (400 MHz, DMSO-$d_6$) δ 12.98 (s, 1H), 9.47 (s, 1H), 7.80 (d, *J* = 7.6 Hz, 1H), 7.74 (d, *J* = 7.6 Hz, 1H), 7.41 (t, *J* = 8.0 Hz, 1H), 6.62 (s, 1H), 5.37-5.31 (m, 1H), 4.67-4.61 (m, 4H), 3.65-3.59 (m, 1H), 2.57 (s, 3H), 1.61 (d, *J* = 6.4 Hz, 3H), 1.18-1.16 (m, 4H); ESI-MS (m/z): 437.2 $[M+H]^+$. |
| **Example 42** | (*R*)-3-(1-((6-cyclopropyl-1-(3,3-difluoropyrrolidin-1-yl)-7-oxo-6,7-dihydropyrido[3,4-*d*]pyridazin-4-yl) amino)ethyl)-2-methylbenzonitrile | 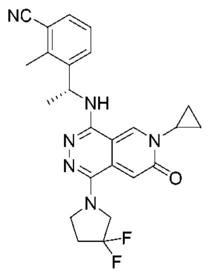 | $^1$H NMR (400 MHz, DMSO-$d_6$) δ 8.95 (s, 1H), 7.73 (d, *J* = 7.6 Hz, 1H), 7.63 (d, *J* = 7.6 Hz, 1H), 7.55 (s, 1H), 7.35 (t, *J* = 8.0 Hz, 1H), 6.58 (s, 1H), 5.47-5.40 (m, 1H), 3.73-3.66 (m, 2H), 3.64-3.58 (m, 1H), 3.56-3.46 (m, 2H), 2.61 (s, 3H), 2.48-2.37 (m, 2H), 1.48 (d, *J* = 6.8 Hz, 3H), 1.19-1.08 (m, 4H); ESI-MS (m/z): 451.2 $[M+H]^+$. |
| **Example 43** | (*R*)-3-(1-((6-cyclopropyl-1-(4,4-difluoropiperidin-1-yl)-7-oxo-6,7-dihydropyrido[3,4-*d*]pyridazin-4-yl) amino)ethyl)-2-methylbenzonitrile | 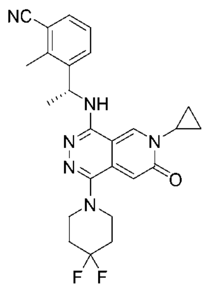 | $^1$H NMR (400 MHz, DMSO-$d_6$) δ 8.99 (s, 1H), 7.75-7.72 (m, 1H), 7.67-7.62 (m, 2H), 7.35 (t, *J* = 8.0 Hz, 1H), 6.49 (s, 1H), 5.50-5.43 (m, 1H), 3.65-3.59 (m, 1H), 3.13-3.10 (m, 4H), 2.61 (s, 3H), 2.17-2.07 (m, 4H), 1.48 (d, *J* = 7.2 Hz, 3H), 1.19-1.07 (m, 4H); ESI-MS (m/z): 465.2 $[M+H]^+$. |
| **Example 44** | (*R*)-3-(1-((6-cyclopropyl-1-(1,1-dioxidothiomorphol ino)-7-oxo-6,7-dihydropyrido[3,4-*d*]pyridazin-4-yl) amino)ethyl)-2-methylbenzonitrile | 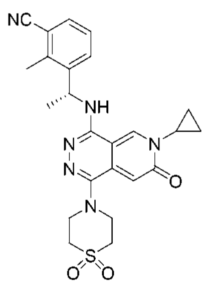 | $^1$H NMR (400 MHz, DMSO-$d_6$) δ 9.01 (s, 1H), 7.75-7.70 (m, 2H), 7.64 (d, *J* = 7.6 Hz, 1H), 7.36 (t, *J* = 8.0 Hz, 1H), 6.56 (s, 1H), 5.52-5.45 (m, 1H), 3.65-3.58 (m, 1H), 3.48-3.46 (m, 4H), 3.30-3.27 (m, 4H), 2.61 (s, 3H), 1.49 (d, *J* = 6.8 Hz, 3H), 1.19-1.07 (m, 4H); ESI-MS (m/z): 479.2 $[M+H]^+$. |

(continued)

| Number | Chemical Name | Structure | $^1$H NMR and MS |
|---|---|---|---|
| **Example 45** | (*R*)-2-methyl-3-(1-((6-(1-methylcyclopropyl)-7-oxo-1-(pyrrolidin-1-yl)-6,7-dihydropyrido[3,4-*d*]pyridazin-4-yl)amino)ethyl)benz onitrile | 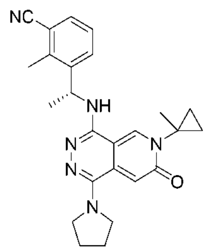 | $^1$H NMR (400 MHz, DMSO-$d_6$) δ 9.09 (s, 1H), 7.74 (d, *J* = 8.0 Hz, 1H), 7.62 (d, *J* = 6.8 Hz, 1H), 7.37-7.29 (m, 2H), 6.63 (s, 1H), 5.41-5.34 (m, 1H), 3.41-3.37 (m, 4H), 2.61 (s, 3H), 1.84-1.80 (m, 4H), 1.54 (s, 3H), 1.47 (d, *J* = 7.2 Hz, 3H), 1.19-1.05 (m, 4H); ESI-MS (m/z): 429.2 $[M+H]^+$. |
| **Example 46** | 3-((1*R*)-1-((1-(3-azabicyclo[3.1.0] he xan-3-yl)-6-(1-methylcyclopropyl)-7-oxo-6,7-dihydropyrido[3,4-*d*] pyridazin-4-yl)amino)ethyl)-2-methylbenzonitrile | 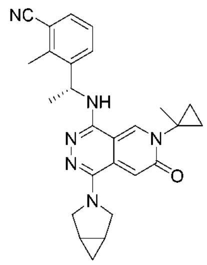 | $^1$H NMR (400 MHz, DMSO-$d_6$) δ 9.13 (s, 1H), 7.73 (d, *J* = 8.0 Hz, 1H), 7.63 (d, *J* = 7.6 Hz, 1H), 7.43 (s, 1H), 7.34 (t, *J* = 8.0 Hz, 1H), 6.46 (s, 1H), 5.43-5.36 (m, 1H), 3.57-3.48 (m, 2H), 3.29-3.24 (m, 2H), 2.60 (s, 3H), 1.53-1.50 (m, 5H), 1.47 (d, *J* = 6.8 Hz, 3H), 1.19-1.04 (m, 4H), 0.53-0.48 (m, 2H); ESI-MS (m/z): 441.2 $[M+H]^+$. |
| **Example 47** | (*R*)-3-(1-((1-(4,4-difluoropiperidin-1-yl)-6-(1-methylcyclopropyl)-7-oxo-6,7-dihydropyrido[3,4-*d*]pyridazin-4-yl)amino)ethyl)-2-methylbenzonitrile | 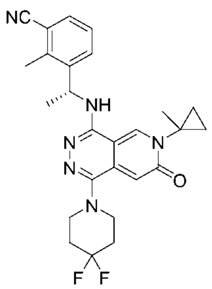 | $^1$H NMR (400 MHz, DMSO-$d_6$) δ 9.23 (s, 1H), 7.75 (d, *J* = 8.0 Hz, 1H), 7.69-7.60 (m, 2H), 7.36 (t, *J* = 8.0 Hz, 1H), 6.46 (s, 1H), 5.49-5.42 (m, 1H), 3.12-3.10 (m, 4H), 2.61 (s, 3H), 2.16-2.06 (m, 4H), 1.54 (s, 3H), 1.49 (d, *J* = 6.8 Hz, 3H), 1.19-1.07 (m, 4H); ESI-MS (m/z): 479.2 $[M+H]^+$. |
| **Example 48** | (*R*)-3-(1-((1-(3,3-difluoroazetidin-1-yl)-6-(1-methylcyclopropyl)-7-oxo-6,7-dihydropyrido[3,4-*d*]pyridazin-4-yl)amino)ethyl)-2-methylbenzonitrile | 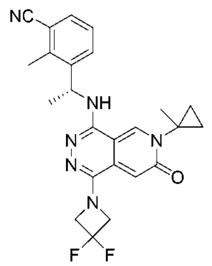 | $^1$H NMR (400 MHz, DMSO-$d_6$) δ 9.18 (s, 1H), 7.74 (d, *J* = 8.0 Hz, 1H), 7.62 (d, *J*= 7.2 Hz, 1H), 7.50 (d, *J* = 6.4 Hz, 1H), 7.35 (t, *J* = 7.6 Hz, 1H), 6.37 (s, 1H), 5.45-5.38 (m, 1H), 4.50-4.43 (m, 4H), 2.61 (s, 3H), 1.54 (s, 3H), 1.48 (d, *J* = 7.2 Hz, 3H), 1.17-1.07 (m, 4H); ESI-MS (m/z): 451.2 $[M+H]^+$. |

**Example 49**

**Preparation of (*R*)-6-cyclopropyl-4-((1-(2-methyl-3-(trifluoromethyl)phenyl)ethyl)amino)-1-(pyrrolidin-1-yl) pyrido[3,4-*d*]pyridazin-7(6*H*)-one**

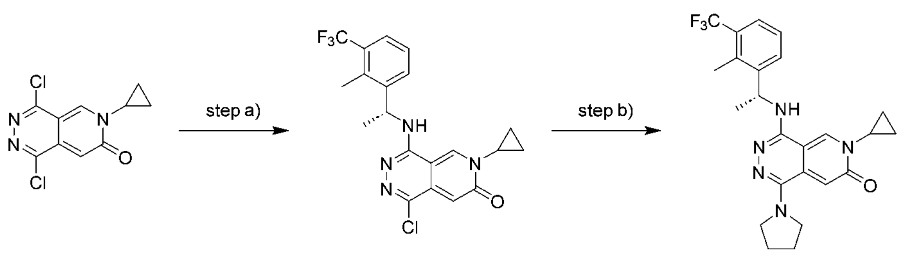

step a): Preparation of (*R*)-1-chloro-6-cyclopropyl-4-((1-(2-methyl-3-(trifluoromethyl)phenyl)ethyl)amino) pyrido[3,4-*d*] pyridazin-7(6*H*)-one

1,4-Dichloro-6-cyclopropylpyrido[3,4-*d*]pyridazin-7(6*H*)-one (320 mg, 1.250 mmol), (*R*)-1-(2-methyl-3-(trifluoromethyl)phenyl)ethan-1-amine hydrochloride (329 mg, 1.375 mmol), *N*,*N*-diisopropylethylamine (485 mg, 3.750 mmol), and dimethyl sulfoxide (8 mL) were added to a reaction flask. The mixture was warmed to 120°C and reacted for 1 h. After the reaction was completed, the mixture was cooled to room temperature. The reaction was quenched by adding saturated aqueous ammonium chloride (50 mL), and extracted with ethyl acetate (30 mL × 3). The organic phases were combined, washed with saturated brine (60 mL), dried over anhydrous sodium sulfate, filtered, and the filtrate was concentrated under reduced pressure. The residue was purified by silica gel column chromatography (eluent: petroleum ether/ethyl acetate = 1:4) to obtain (*R*)-1-chloro-6-cyclopropyl-4-((1-(2-methyl-3-(trifluoromethyl)phenyl) ethyl)amino)pyrido[3,4-*d*]pyridazin-7(6*H*)-one; yield: 59.0%; ESI-MS (m/z): 423.1 $[M+H]^+$.

step b): Preparation of (*R*)-6-cyclopropyl-4-((1-(2-methyl-3-(trifluoromethyl)phenyl)ethyl)amino)-1-(pyrrolidin-1-yl) pyrido[3,4-d]pyridazin-7(6*H*)-one

(*R*)-1-Chloro-6-cyclopropyl-4-((1-(2-methyl-3-(trifluoromethyl)phenyl)ethyl)amino)pyrido[3,4-*d*]pyridazin-7(6*H*)-one (50 mg, 0.118 mmol), pyrrolidine (42 mg, 0.591 mmol), and 1,4-dioxane (2 mL) were added to a microwave tube. The mixture was warmed to 120°C under microwave irradiation for 1 h. After the reaction was completed, the mixture was cooled to room temperature and concentrated under reduced pressure. The residue was purified by Prep-HPLC (separation method 3) to obtain (*R*)-6-cyclopropyl-4-((1-(2-methyl-3-(trifluoromethyl)phenyl)ethyl)amino)-1-(pyrrolidin-1-yl) pyrido[3,4-d]pyridazin-7(6*H*)-one, yield 40.7%; $^1$H NMR (400 MHz, DMSO-$d_6$) δ 8.86 (s, 1H), 7.73 (d, *J* = 7.6 Hz, 1H), 7.53 (d, *J* = 7.6 Hz, 1H), 7.34 (t, *J* = 7.6 Hz, 1H), 7.28 (d, *J* = 6.8 Hz, 1H), 6.63 (s, 1H), 5.50 (p, *J* = 6.8 Hz, 1H), 3.64-3.57 (m, 1H), 3.41-3.34 (m, 4H), 2.51 (s, 3H), 1.85-1.77 (m, 4H), 1.47 (d, *J* = 6.9 Hz, 3H), 1.17-1.09 (m, 4H); ESI-MS (m/z): 458.2 $[M+H]^+$.

According to the preparation method of **Example 49,** with the corresponding raw materials, the compounds in the following Examples were prepared.

| Number | Chemical Name | Structure | $^1$H NMR and MS |
|---|---|---|---|
| **Exampl e 50** | (*R*)-1-(azetidin-1-yl)-6-cyclopropyl-4-((1-(2-methyl-3-(trifluoromethyl)phe nyl)ethyl)amino)pyr ido[3,4-*d*]pyridazin-7(6*H*)-one formate | 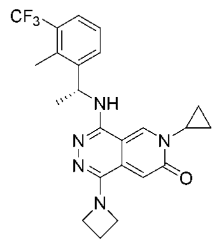 | The resulting crude product was purified by Prep-HPLC (separation method 2) to obtain (*R*)-1-(azetidin-1-yl)-6-cyclopropyl-4-((1-(2-methyl-3-(trifluoromethyl)phenyl)ethyl)amino )pyrido[3,4-*d*]pyridazin-7(6*H*)-one formate; $^1$H NMR (400 MHz, DMSO-$d_6$) δ 9.84 (s, 1H), 8.96 (s, 1H), 8.22 (s, 1H), 7.89 (s, 1H), 7.79 (d, *J* = 7.6 Hz, 1H), 7.55 (d, *J* = 7.6 Hz, 1H), 7.37 (t, *J* = 7.6 Hz, 1H), 7.22 (s, 1H), 5.21 (p, *J* = 6.8 Hz, 1H), 3.97-3.87 (m, 1H), 3.70-3.55 (m, 2H), 3.45-3.37 (m, 2H), 2.54 (s, 3H), 2.01 (p, *J* = 6.0 Hz, 2H), 1.50 (d, *J* = 6.8 Hz, 3H), 1.20-1.09 (m, 4H); ESI-MS (m/z): 444.2 [M+H]$^+$. |
| **Exampl e 51** | (*R*)-6-cyclopropyl-1-(dimethylamino)-4-((1-(2-methyl-3-(trifluoromethyl)phe nyl)ethyl)amino)pyr ido[3,4-*d*]pyridazin-7(6*H*)-one | 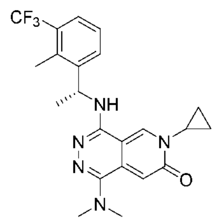 | $^1$H NMR (400 MHz, DMSO-$d_6$) δ 8.96 (s, 1H), 7.73 (d, *J* = 7.6 Hz, 1H), 7.54 (d, *J* = 7.6 Hz, 2H), 7.35 (t, *J* = 7.6 Hz, 1H), 6.49 (s, 1H), 5.55 (p, *J* = 6.8 Hz, 1H), 3.66-3.56 (m, 1H), 2.71 (s, 6H), 2.51 (s, 3H), 1.48 (d, *J* = 6.8 Hz, 3H), 1.19-1.08 (m, 4H); ESI-MS (m/z): 432.2 [M+H]$^+$. |
| **Exampl e 52** | (*R*)-6-cyclopropyl-4-((1-(2-methyl-3-(trifluoromethyl)phe nyl)ethyl)amino)-1-(methylamino)pyrido[3,4-*d*]pyridazin-7(6*H*)-one | 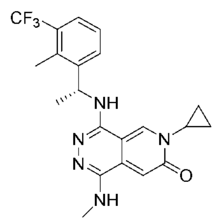 | $^1$H NMR (400 MHz, DMSO-$d_6$) δ 8.82 (s, 1H), 7.72 (d, *J* = 7.6 Hz, 1H), 7.52 (d, *J* = 7.6 Hz, 1H), 7.32 (t, *J* = 7.6 Hz, 1H), 7.06 (s, 1H), 6.81 (s, 1H), 6.52 (s, 1H), 5.44 (p, *J* = 6.8 Hz, 1H), 3.64-3.53 (m, 1H), 2.75 (s, 3H), 2.51 (s, 3H), 1.45 (d, *J* = 6.8 Hz, 3H), 1.17-1.07 (m, 4H); ESI-MS (m/z): 418.2 [M+H]$^+$. |
| **Exampl e 53** | (*R*)-6-cyclopropyl-1-(diethylamino)-4-((1-(2-methyl-3-(trifluoromethyl)phe nyl)ethyl)amino)pyr ido[3,4-*d*]pyridazin-7(6*H*)-one | 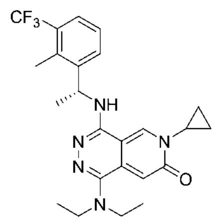 | $^1$H NMR (400 MHz, DMSO-$d_6$) δ 8.97 (s, 1H), 7.75 (d, *J* = 7.6 Hz, 1H), 7.55 (d, *J* = 7.6 Hz, 2H), 7.35 (t, *J* = 7.6 Hz, 1H), 6.44 (s, 1H), 5.59 (p, *J* = 6.8 Hz, 1H), 3.64-3.56 (m, 1H), 3.07 (q, *J* = 7.2 Hz, 4H), 2.52 (s, 3H), 1.48 (d, *J* = 6.8 Hz, 3H), 1.18-1.08 (m, 4H), 0.98 (t, *J* = 7.2 Hz, 6H); ESI-MS (m/z): 460.2 [M+H]$^+$. |

(continued)

| Number | Chemical Name | Structure | $^1$H NMR and MS |
|---|---|---|---|
| **Example 54** | (*R*)-6-cyclopropyl-1-(3,3-difluoroazetidin-1-yl)-4-((1-(2-methyl-3-(trifluoromethyl)phe nyl)ethyl)amino) pyr ido[3,4-d]pyridazin-7(6*H*)-one | 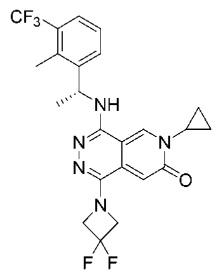 | $^1$H NMR (400 MHz, DMSO-$d_6$) δ 8.96 (s, 1H), 7.73 (d, *J* = 7.6 Hz, 1H), 7.55-7.48 (m, 2H), 7.34 (t, *J* = 7.6 Hz, 1H), 6.40 (s, 1H), 5.53 (p, *J* = 6.8 Hz, 1H), 4.47 (t, *J* = 12.8 Hz, 4H), 3.64-3.56 (m, 1H), 2.51 (s, 3H), 1.48 (d, *J* = 6.8 Hz, 3H), 1.19-1.08 (m, 4H); ESI-MS (m/z): 480.2 $[M+H]^+$. |
| **Example 55** | (*R*)-6-cyclopropyl-1-(3,3-difluoropyrrolidin-1-yl)-4-((1-(2-methyl-3-(trifluoromethyl)phe nyl) ethyl)amino)pyr ido[3,4-*d*]pyridazin-7(6*H*)-one | 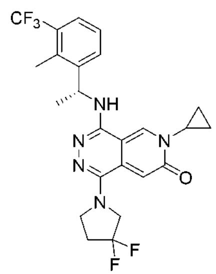 | $^1$H NMR (400 MHz, DMSO-$d_6$) δ 8.97 (s, 1H), 7.73 (d, *J* = 7.6 Hz, 1H), 7.54 (d, *J* = 7.6 Hz, 2H), 7.35 (t, *J* = 7.6 Hz, 1H), 6.58 (s, 1H), 5.54 (p, *J* = 6.8 Hz, 1H), 3.76-3.65 (m, 2H), 3.64-3.58 (m, 1H), 3.55-3.44 (m, 2H), 2.51 (s, 3H), 2.46-2.35 (m, 2H), 1.48 (d, *J* = 6.8 Hz, 3H), 1.19-1.08 (m, 4H); ESI-MS (m/z): 494.2 $[M+H]^+$. |
| **Example 56** | 1-(3-azabicyclo[3.1.0]he xan-3-yl)-6-cyclopropyl-4-(((*R*)-1-(2-methyl-3-(trifluoromethyl)phe nyl) ethyl)amino)pyr ido[3,4-*d*]pyridazin-7(6*H*)-one | 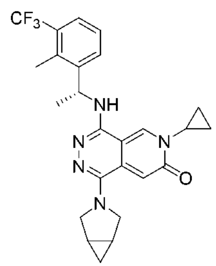 | $^1$H NMR (400 MHz, DMSO-$d_6$) δ 8.92 (s, 1H), 7.72 (d, *J* = 7.6 Hz, 1H), 7.54 (d, *J* = 7.6 Hz, 1H), 7.44 (s, 1H), 7.34 (t, *J* = 7.6 Hz, 1H), 6.49 (s, 1H), 5.52 (p, *J* = 6.8 Hz, 1H), 3.65-3.54 (m, 2H), 3.49 (d, *J* = 10.0 Hz, 1H), 3.31-3.22 (m, 2H), 2.49 (s, 3H), 1.56-1.48 (m, 2H), 1.47 (d, *J* = 6.8 Hz, 3H), 1.19-1.06 (m, 4H), 0.57-0.46 (m, 2H); ESI-MS (m/z): 470.2 $[M+H]^+$. |
| **Example 57** | (*R*)-6-cyclopropyl-4-((1-(2-methyl-3-(trifluoromethyl)phe nyl) ethyl)amino)-1-(thiazolidin-3-yl)pyrido[3,4-*d*]pyridazin-7(6*H*)-one | 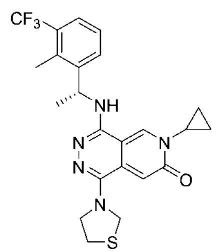 | $^1$H NMR (400 MHz, DMSO-$d_6$) δ 9.00 (s, 1H), 7.74 (d, *J* = 7.6 Hz, 1H), 7.63 (d, *J* = 6.8 Hz, 1H), 7.55 (d, *J* = 8.0 Hz, 1H), 7.36 (t, *J* = 8.0 Hz, 1H), 6.59 (s, 1H), 5.61-5.54 (m, 1H), 4.55-4.46 (m, 2H), 3.72-3.65 (m, 1H), 3.65-3.60 (m, 1H), 3.59-3.53 (m, 1H), 3.01-2.92 (m, 2H), 2.51 (s, 3H), 1.49 (d, *J* = 6.8 Hz, 3H), 1.19-1.09 (m, 4H); ESI-MS (m/z): 476.2 $[M+H]^+$. |

(continued)

| Number | Chemical Name | Structure | $^1$H NMR and MS |
|---|---|---|---|
| **Example 58** | (*R*)-6-cyclopropyl-1-(4,4-difluoropiperidin-1-yl)-4-((1-(2-methyl-3-(trifluoromethyl)phe nyl)ethyl)amino) pyr ido[3,4-*d*]pyridazin-7(6*H*)-one | 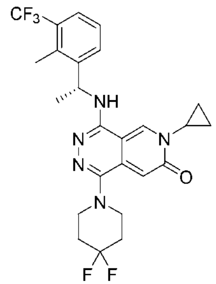 | $^1$H NMR (400 MHz, DMSO-$d_6$) δ 9.00 (s, 1H), 7.73 (d, *J* = 8.0 Hz, 1H), 7.65 (d, *J* = 6.8 Hz, 1H), 7.54 (d, *J* = 8.0 Hz, 1H), 7.35 (t, *J* = 8.0 Hz, 1H), 6.48 (s, 1H), 5.57 (p, *J* = 6.8 Hz, 1H), 3.67-3.57 (m, 1H), 3.16-3.05 (m, 4H), 2.51 (s, 3H), 2.18-2.06 (m, 4H), 1.48 (d, *J* = 6.4 Hz, 3H), 1.19-1.07 (m, 4H); ESI-MS (m/z): 508.2 $[M+H]^+$. |
| **Example 59** | (*R*)-6-cyclopropyl-1-(1,1-dioxidothiomorphol ino)-4-((1-(2-methyl-3-(trifluoromethyl)phe nyl) ethyl)amino)pyr ido[3,4-*d*]pyridazin-7(6*H*)-one | 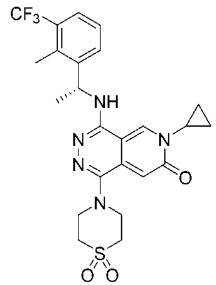 | $^1$H NMR (400 MHz, DMSO-$d_6$) δ 9.02 (s, 1H), 7.74 (d, *J* = 7.6 Hz, 1H), 7.71 (d, *J* = 6.8 Hz, 1H), 7.55 (d, *J* = 7.6 Hz, 1H), 7.36 (t, *J* = 7.6 Hz, 1H), 6.55 (s, 1H), 5.60 (p, *J* = 6.8 Hz, 1H), 3.65-3.59 (m, 1H), 3.51-3.42 (m, 4H), 3.31-3.23 (m, 4H), 2.52 (s, 3H), 1.49 (d, *J* = 6.8 Hz, 3H), 1.20-1.08 (m, 4H); ESI-MS (m/z): 522.2 $[M+H]^+$. |
| **Example 60** | *(R)-1*-(diethylamino)-4-((1-(2-methyl-3-(trifluoromethyl)phe nyl) ethyl)amino)-6-(1-methylcyclopropyl) pyrido[3,4-*d*]pyridazin-7(6*H*)-one | 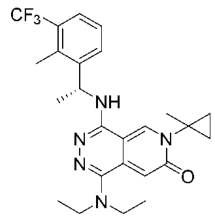 | $^1$H NMR (400 MHz, DMSO-$d_6$) δ 9.29 (s, 1H), 7.77 (d, *J* = 7.8 Hz, 1H), 7.58 (d, *J* = 7.8 Hz, 1H), 7.38 (t, *J* = 7.8 Hz, 1H), 6.46 (s, 1H), 5.61-5.49 (m, 1H), 3.17-3.03 (m, 4H), 2.52 (s, 3H), 1.57-1.49 (m, 6H), 1.21-1.06 (m, 4H), 1.00 (t, *J* = 7.2 Hz, 6H); ESI-MS (m/z): 474.2 $[M+H]^+$. |
| **Example 61** | (*R*)-4-((1-(2-methyl-3-(trifluoromethyl)phe nyl)ethyl)amino)-6-(1-methylcyclopropyl)-1-(pyrrolidin-1-yl)pyrido[3,4-*d*]pyridazin-7(6*H*)-one | 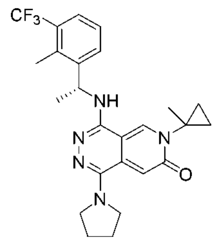 | $^1$H NMR (400 MHz, DMSO-$d_6$) δ 9.11 (s, 1H), 7.74 (d, *J* = 7.6 Hz, 1H), 7.53 (d, *J* = 7.6 Hz, 1H), 7.34 (t, *J* = 7.6 Hz, 1H), 7.29 (s, 1H), 6.62 (s, 1H), 5.49 (p, *J* = 6.8 Hz, 1H), 3.42-3.35 (m, 4H), 2.50 (s, 3H), 1.85-1.78 (m, 4H), 1.54 (s, 3H), 1.48 (d, *J* = 6.8 Hz, 3H), 1.20-1.12 (m, 2H), 1.11-1.04 (m, 2H); ESI-MS (m/z): 472.2 $[M+H]^+$. |

(continued)

| Number | Chemical Name | Structure | $^1$H NMR and MS |
|---|---|---|---|
| **Example 62** | 1-(3-azabicyclo[3.1.0]he xan-3-yl)-4-(((*R*)-1-(2-methyl-3-(trifluoromethyl)phe nyl)ethyl)amino)-6-(1-methylcyclopropyl) pyrido[3,4-*d*] pyridazin-7(6*H*)-one | 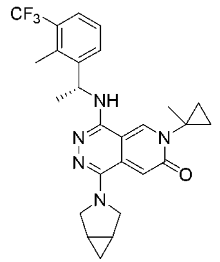 | $^1$H NMR (400 MHz, DMSO-$d_6$) δ 9.15 (s, 1H), 7.73 (d, *J* = 7.6 Hz, 1H), 7.54 (d, *J* = 7.6 Hz, 1H), 7.43 (s, 1H), 7.34 (t, *J* = 7.6 Hz, 1H), 6.46 (s, 1H), 5.50 (p, *J* = 6.8 Hz, 1H), 3.55 (d, *J* = 10.0 Hz, 1H), 3.50 (d, *J* = 10.0 Hz, 1H), 3.29-3.24 (m, 2H), 2.49 (s, 3H), 1.55-1.50 (m, 5H), 1.48 (d, *J* = 6.8 Hz, 3H), 1.19-1.12 (m, 2H), 1.09-1.02 (m, 2H), 0.55-0.48 (m, 2H); ESI-MS (m/z): 484.2 $[M+H]^+$. |
| **Example 63** | 4-(((*R*)-1-(2-methyl-3-(trifluoromethyl)phe nyl)ethyl)amino)-6-(1-methylcyclopropyl)-1-((*R*)-2-methylpyrrolidin-1-yl)pyrido[3,4-*d*] pyridazin-7(6*H*)-one | 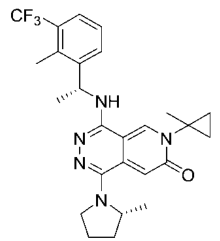 | $^1$H NMR (400 MHz, DMSO-$d_6$) δ 9.14 (s, 1H), 7.77 (d, *J* = 7.8 Hz, 1H), 7.54 (d, *J* = 6.8 Hz, 1H), 7.35 (t, *J* = 7.2 Hz, 2H), 6.51 (s, 1H), 5.51 (p, *J* = 6.8 Hz, 1H), 4.18-4.07 (m, 1H), 3.70-3.60 (m, 1H), 3.10-3.01 (m, 1H), 2.51 (s, 3H), 2.12 -2.03 (m, 1H), 1.91-1.82 (m, 1H), 1.73-1.63 (m, 1H), 1.54 (s, 3H), 1.52-1.44 (m, 4H), 1.18-1.03 (m, 4H), 0.94 (d, *J* = 6.0 Hz, 3H); ESI-MS (m/z): 486.2 $[M+H]^+$. |
| **Example 64** | *(R)-1*-(dimethylamino)-4-((1-(2-methyl-3-(trifluoromethyl)phe nyl) ethyl)amino)-6-(1-methylcyclopropyl) pyrido[3,4-*d*]pyridazin-7(6*H*)-one | 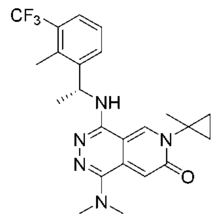 | $^1$H NMR (400 MHz, DMSO-$d_6$) δ 9.19 (s, 1H), 7.74 (d, *J* = 8.0 Hz, 1H), 7.59-7.46 (m, 2H), 7.35 (t, *J* = 8.0 Hz, 1H), 6.45 (s, 1H), 5.53 (p, *J* = 7.6 Hz, 1H), 2.69 (s, 6H), 2.49 (s, 3H), 1.54 (s, 3H), 1.48 (d, *J* = 7.2 Hz, 3H), 1.20-1.03 (m, 4H); ESI-MS (m/z): 446.2 $[M+H]^+$. |
| **Example 65** | (*R*)-1-(4,4-difluoropiperidin-1-yl)-4-((1-(2-methyl-3-(trifluoromethyl)phe nyl)ethyl)amino)-6-(1-methylcyclopropyl) pyrido[3,4-*d*] pyridazin-7(6*H*)-one | 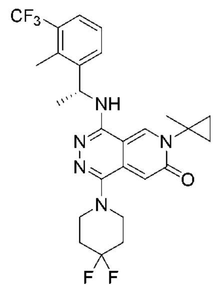 | $^1$H NMR (400 MHz, DMSO-$d_6$) δ 9.25 (s, 1H), 7.74 (d, *J* = 7.6 Hz, 1H), 7.65 (s, 1H), 7.55 (d, *J* = 7.6 Hz, 1H), 7.35 (t, *J* = 7.6 Hz, 1H), 6.46 (s, 1H), 5.57 (p, *J* = 6.8 Hz, 1H), 3.16-3.05 (m, 4H), 2.51 (s, 3H), 2.17-2.05 (m, 4H), 1.54 (s, 3H), 1.49 (d, *J* = 6.9 Hz, 3H), 1.20-1.04 (m, 4H); ESI-MS (m/z): 522.2 $[M+H]^+$. |

(continued)

| Number | Chemical Name | Structure | $^1$H NMR and MS |
|---|---|---|---|
| **Example 66** | *(R)-1-(3,3*-difluoroazetidin-1-yl)-4-((1-(2-methyl-3-(trifluoromethyl)phe nyl)ethyl)amino)-6-(1-methylcyclopropyl) pyrido[3,4-*d*] pyridazin-7(6*H*)-one | 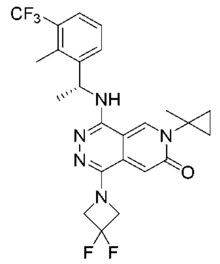 | $^1$H NMR (400 MHz, DMSO-$d_6$) δ 9.21 (s, 1H), 7.74 (d, *J* = 7.6 Hz, 1H), 7.54 (d, *J* = 7.6 Hz, 1H), 7.49 (s, 1H), 7.35 (t, *J* = 7.6 Hz, 1H), 6.37 (s, 1H), 5.52 (p, *J* = 6.8 Hz, 1H), 4.46 (t, *J* = 12.8 Hz, 4H), 2.52 (s, 3H), 1.54 (s, 3H), 1.49 (d, *J* = 6.8 Hz, 3H), 1.20-1.04 (m, 4H); ESI-MS (m/z): 494.2 $[M+H]^+$ |
| **Example 67** | *(R)-1-(3,3*-difluoropyrrolidin-1-yl)-4-((1-(2-methyl-3-(trifluoromethyl)phe nyl)ethyl)amino)-6-(1-methylcyclopropyl) pyrido[3,4-*d*] pyridazin-7(6*H*)-one | 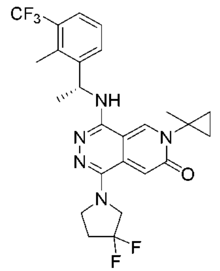 | $^1$H NMR (400 MHz, DMSO-$d_6$) δ 9.21 (s, 1H), 7.74 (d, *J* = 7.6 Hz, 1H), 7.55 (s, 1H), 7.53 (s, 1H), 7.35 (t, *J* = 7.6 Hz, 1H), 6.54 (s, 1H), 5.53 (p, *J* = 6.8 Hz, 1H), 3.68 (t, *J* = 13.2 Hz, 2H), 3.49 (t, *J* = 7.2 Hz, 2H), 2.51 (s, 3H), 2.47-2.36 (m, 2H), 1.55 (s, 3H), 1.49 (d, *J* = 7.2 Hz, 3H), 1.21-1.04 (m, 4H); ESI-MS (m/z): 508.2 $[M+H]^+$. |
| **Example 68** | (*R*)-1-(1,1-dioxidothiomorphol ino)-4-((1-(2-methyl-3-(trifluoromethyl)phe nyl)ethyl)amino)-6-(1-methylcyclopropyl) pyrido[3,4-*d*] pyridazin-7(6*H*)-one | 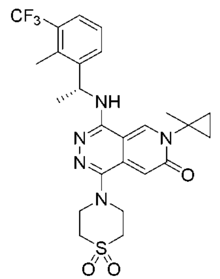 | $^1$H NMR (400 MHz, DMSO-$d_6$) δ 9.26 (s, 1H), 7.75 (d, *J* = 7.8 Hz, 1H), 7.70 (d, *J* = 6.8 Hz, 1H), 7.55 (d, *J* = 7.8 Hz, 1H), 7.36 (t, *J* = 7.8 Hz, 1H), 6.52 (s, 1H), 5.58 (p, *J* = 6.8 Hz, 1H), 3.51-3.42 (m, 4H), 3.30-3.23 (m, 4H), 2.52 (s, 3H), 1.54 (s, 3H), 1.50 (d, *J* = 6.8 Hz, 3H), 1.18-1.05 (m, 4H); ESI-MS (m/z): 536.2 $[M+H]^+$. |
| **Example 69** | (*R*)-3-(1-((1-(3,3-difluoropyrrolidin-1-yl)-6-(1-methylcyclopropyl)-7-oxo-6,7-dihydropyrido[3,4-*d*]pyridazin-4-yl)amino)ethyl)-2-methylbenzonitrile | 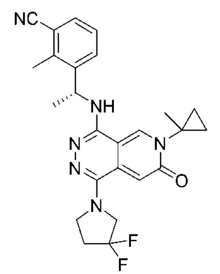 | $^1$H NMR (400 MHz, DMSO-$d_6$) δ 9.19 (s, 1H), 7.75 (d, *J* = 7.6 Hz, 1H), 7.64 (d, *J* = 7.6 Hz, 1H), 7.55 (s, 1H), 7.36 (t, *J* = 8.0 Hz, 1H), 6.55 (s, 1H), 5.47-5.40 (m, 1H), 3.72-3.66 (m, 2H), 3.55-3.47 (m, 2H), 2.61 (s, 3H), 2.47-2.37 (m, 2H), 1.55 (s, 3H), 1.49 (d, *J* = 6.8 Hz, 3H), 1.18-1.07 (m, 4H); ESI-MS (m/z): 465.2 $[M+H]^+$. |

(continued)

| Number | Chemical Name | Structure | $^1$H NMR and MS |
| --- | --- | --- | --- |
| **Example 70** | (*R*)-3-(1-((1-(azetidin-1-yl)-6-(1-methylcyclopropyl)-7-oxo-6,7-dihydropyrido[3,4-*d*]pyridazin-4-yl)amino)ethyl)-2-methylbenzonitrile | 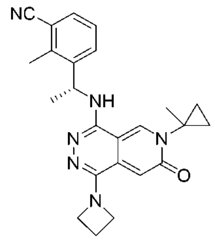 | $^1$H NMR (400 MHz, DMSO-$d_6$) δ 9.10 (s, 1H), 7.73 (d, J= 7.6 Hz, 1H), 7.62 (d, *J* = 6.8 Hz, 1H), 7.34 (t, *J* = 8.0 Hz, 1H), 7.28 (d, *J* = 6.4 Hz, 1H), 6.35 (s, 1H), 5.41-5.34 (m, 1H), 4.03-4.00 (m, 4H), 2.61 (s, 3H), 2.26-2.18 (m, 2H), 1.53 (s, 3H), 1.47 (d, *J* = 6.8 Hz, 3H), 1.19-1.05 (m, 4H); ESI-MS (m/z): 415.2 $[M+H]^+$. |
| **Example 71** | (*R*)-3-(1-((1-(diethylamino)-6-(1-methylcyclopropyl)-7-oxo-6,7-dihydropyrido[3,4-*d*]pyridazin-4-yl)amino)ethyl)-2-methylbenzonitrile | 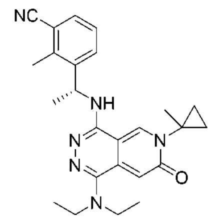 | $^1$H NMR (400 MHz, DMSO-$d_6$) δ 9.19 (s, 1H), 7.76 (d, *J* = 8.0 Hz, 1H), 7.64 (d, *J* = 7.6 Hz, 1H), 7.55 (s, 1H), 7.36 (t, *J* = 8.0 Hz, 1H), 6.42 (s, 1H), 5.51-5.44 (m, 1H), 3.09-3.04 (m, 4H), 2.62 (s, 3H), 1.54 (s, 3H), 1.49 (d, *J* = 7.2 Hz, 3H), 1.20-1.05 (m, 4H), 0.99-0.95 (m, 6H); ESI-MS (m/z): 431.2 $[M+H]^+$. |
| **Example 72** | (*R*)-3-(1-((1-(1,1-dioxidothiomorphol ino)-6-(1-methylcyclopropyl)-7-oxo-6,7-dihydropyrido[3,4-*d*] pyridazin-4-yl)amino)ethyl)-2-methylbenzonitrile | 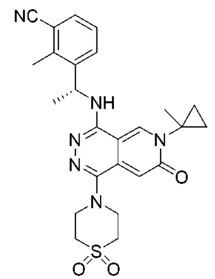 | $^1$H NMR (400 MHz, DMSO-$d_6$) δ 9.24 (s, 1H), 7.76-7.69 (m, 2H), 7.64 (d, *J* = 7.6 Hz, 1H), 7.36 (t, *J* = 8.0 Hz, 1H), 6.52 (s, 1H), 5.51-5.44 (m, 1H), 3.47-3.45 (m, 4H), 3.29-3.26 (m, 4H), 2.61 (s, 3H), 1.54 (s, 3H), 1.49 (d, *J* = 6.8 Hz, 3H), 1.19-1.05 (m, 4H); ESI-MS (m/z): 493.2 $[M+H]^+$. |
| **Example 73** | (*R*)-4-((1-(2-methyl-3-(trifluoromethyl)phe nyl)ethyl)amino)-1-(methylamino)-6-(1-methylcyclopropyl) pyrido[3,4-*d*]pyridazin-7(6*H*)-one | 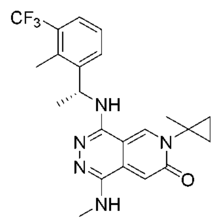 | $^1$H NMR (400 MHz, DMSO-$d_6$) δ 9.04 (s, 1H), 7.73 (d, *J* = 7.8 Hz, 1H), 7.52 (d, *J* = 7.8 Hz, 1H), 7.33 (t, *J* = 7.8 Hz, 1H), 7.00 (s, 1H), 6.77 (s, 1H), 6.43 (s, 1H), 5.43 (p, *J* = 6.8 Hz, 1H), 2.74 (d, *J* = 4.0 Hz, 3H), 2.50 (s, 3H), 1.53 (s, 3H), 1.46 (d, *J* = 6.8 Hz, 3H), 1.18-1.02 (m, 4H); ESI-MS (m/z): 432.2 $[M+H]^+$. |

**Example 74**

**Preparation of (*R*)-1-amino-4-((1-(3-(difluoromethyl)-2-fluorophenyl)ethyl)amino)-6-(1-methylcyclopropyl) pyrido[3,4-*d*]pyridazin-7(6*H*)-one formate**

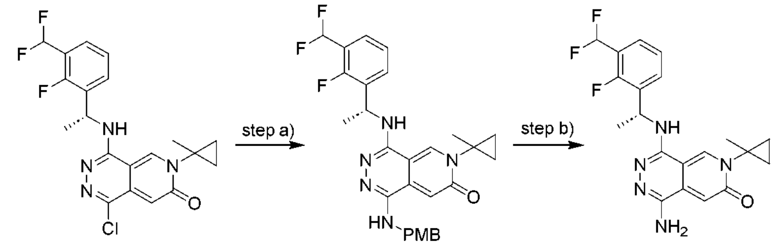


step a): Preparation of (*R*)-4-((1-(3-(difluoromethyl)-2-fluorophenyl)ethyl)amino)-1-((4-methoxybenzyl)amino)-6-(1-methylcyclopropyl)pyrido[3,4-*d*]pyridazin-7(6*H*)-one

(*R*)-1-Chloro-4-((1-(3-(difluoromethyl)-2-fluorophenyl)ethyl)amino)-6-(1-methylcyclopropyl)pyrido[3,4-*d*]pyridazin-7(6*H*)-one (300 mg, 0.710 mmol), 4-methoxybenzylamine (320 mg, 3.500 mmol) and 1,4-dioxane (3 mL) were added to a reaction flask. The mixture was warmed to 115°C and reacted for 30 min. After the reaction was completed, the mixture was cooled to room temperature. The residue was concentrated under reduced pressure to obtain (*R*)-4-((1-(3-(difluoromethyl)-2-fluorophenyl)ethyl)amino)-1-((4-methoxybenzyl)amino)-6-(1-methylcyclopropyl)pyrido[3,4-*d*]pyridazin-7(6*H*)-one, yield 93.1%; ESI-MS(m/z): 524.0 $[M+H]^+$.

step b): Preparation of (*R*)-1-amino-4-((1-(3-(difluoromethyl)-2-fluorophenyl)ethyl)amino)-6-(1-methylcyclopropyl) pyrido[3,4-*d*]pyridazin-7(6*H*)-one formate

(*R*)-4-((1-(3-(Difluoromethyl)-2-fluorophenyl)ethyl)amino)-1-((4-methoxybenzyl)amino)-6-(1-methylcyclopropyl)pyrido[3,4-*d*]pyridazin-7(6*H*)-one (260 mg, 0.497 mmol) and trifluoroacetic acid (5 mL) were added to a reaction flask. The mixture was warmed to 85°C and stirred to reacted for 20 min. After the reaction was completed, the mixture was concentrated under reduced pressure. The residue was purified by Prep-HPLC (separation method 2) to obtain (R)-1-amino-4-((1-(3-(difluoromethyl)-2-fluorophenyl)ethyl)amino)-6-(1-methylcyclopropyl)pyrido[3,4-*d*]pyridazin-7(6*H*)-one formate, yield 6.1%; $^1$H NMR (400 MHz, DMSO-$d_6$) δ 9.05 (s, 1H), 8.18 (s, 1H), 7.60 (t, *J* = 7.6 Hz, 1H), 7.46 (t, *J* = 7.2 Hz, 1H), 7.36-7.09 (m, 2H), 7.06-6.98 (m, 1H), 6.82 (s, 1H), 5.97-5.81 (m, 2H), 5.49-5.40 (m, 1H), 1.56-1.48 (m, 6H), 1.21-1.03 (m, 4H); ESI-MS(m/z): 404.0 $[M+H]^+$.

According to the preparation method of **Example 74,** with the corresponding raw materials, the compound in the following Example was prepared.

| Number | Chemical Name | Structure | $^1$H NMR and MS |
|---|---|---|---|
| **Example 75** | (*R*)-1-amino-6-cyclopropyl-4-((1-(3-(difluoromethyl)-2-fluorophenyl)ethyl) amino)pyrido[3,4-*d*]pyridazin-7(6*H*)-one | 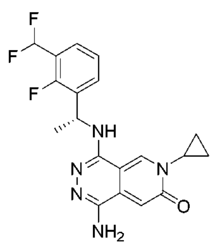 | The crude product was purified by Prep-HPLC (separation method 3) to obtain (*R*)-1-amino-6-cyclopropyl-4-((1-(3-(difluoromethyl)-2-fluorophenyl)ethyl)amino)pyrido [3,4-d]pyridazin-7(6*H*)-one; $^1$H NMR (400 MHz, DMSO-$d_6$) δ 8.84 (s, 1H), 7.59 (t, *J* = 7.6 Hz, 1H), 7.46 (t, *J* = 7.2 Hz, 1H), 7.25 (t, *J* = 7.6 Hz, 1H), 7.09 (t, *J* = 53.6 Hz, 1H), 7.14-7.09 (m, 1H), 6.87 (s, 1H), 6.02 (s, 2H), 5.46 (p, *J* = 6.8 Hz, 1H), 3.63-3.57 (m, 1H), 1.51 (d, *J* = 7.2 Hz, 3H), 1.18-1.07 (m, 4H); ESI-MS (m/z): 390.2 $[M+H]^+$. |

**Example 76**

**Preparation of (*R*)-6-cyclopropyl-4-((1-(3-(difluoromethyl)-2-fluorophenyl)ethyl)amino)-1-(dimethylamino) pyrido[3,4-*d*]pyridazin-7(6*H*)-one**

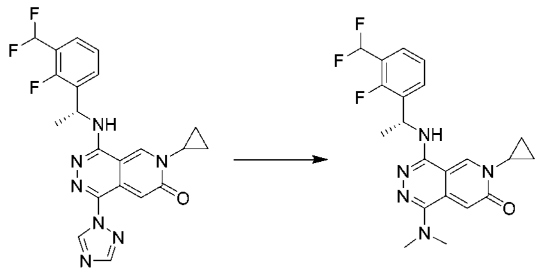

(*R*)-6-Cyclopropyl-4-((1-(3-(difluoromethyl)-2-fluorophenyl)ethyl)amino)-1-(1*H*-1,2,4-triazol-1-yl)pyrido[3,4-*d*] pyridazin-7(6*H*)-one (100 mg, 0.227 mmol), dimethylamine hydrochloride (190 mg, 2.330 mmol) and 1,4-dioxane (5 mL) were added to a microwave reaction flask. The mixture was warmed to 110°C under microwave irradiation for 4 h. After the reaction was completed, the mixture was concentrated under reduced pressure. The residue was purified by Prep-HPLC (separation method 3) to obtain (R)-6-cyclopropyl-4-((1-(3-(difluoromethyl)-2-fluorophenyl)ethyl)amino)-1- (dimethylamino)pyrido[3,4-d]pyridazin-7(6*H*)-one, yield 6.2%; $^1$H NMR (400 MHz, DMSO-$d_6$) δ 8.96 (s, 1H), 7.61 (t, *J* = 7.2 Hz, 1H), 7.54-7.44 (m, 2H), 7.38-7.10 (m, 2H), 6.49 (s, 1H), 5.61-5.49 (m, 1H), 3.67-3.56 (m, 1H), 2.70 (s, 6H), 1.53 (d, *J* = 7.2 Hz, 3H), 1.18-1.09 (m, 4H)); ESI-MS(m/z): 418.0 [M+H]$^+$.

According to the preparation method of **Example 76,** with the corresponding raw materials, the compound in the following Example was prepared.

| Number | Chemical Name | Structure | $^1$H NMR and MS |
|---|---|---|---|
| **Example 77** | (*R*)-6-cyclopropyl-4-((1-(3-(difluoromethyl)-2-fluorophenyl)ethyl) amino)-1-(4-methylpiperazin-1-yl)pyrido[3,4-*d*]pyridazin-7(6*H*)-one | 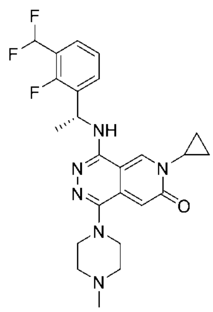 | $^1$H NMR (400 MHz, DMSO-$d_6$) δ 8.99 (s, 1H), 7.68-7.55 (m, 2H), 7.48 (t, *J* = 6.8 Hz, 1H), 7.38-7.10 (m, 2H), 6.43 (s, 1H), 5.62-5.53 (m, 1H), 3.68-3.56 (m, 1H), 3.12-2.92 (m, 4H), 2.63-2.51 (m, 4H), 2.28 (s, 3H), 1.54 (d, *J* = 7.2 Hz, 3H), 1.19-1.08 (m, 4H); ESI-MS(m/z): 473.0 [M+H]$^+$. |

**Example 78**

**Preparation of (*R*)-4-((1-(3-(difluoromethyl)-2-fluorophenyl)ethyl)amino)-6-(1-methylcyclopropyl)-1- (piperazin-1-yl)pyrido[3,4-*d*]pyridazin-7(6*H*)-one**

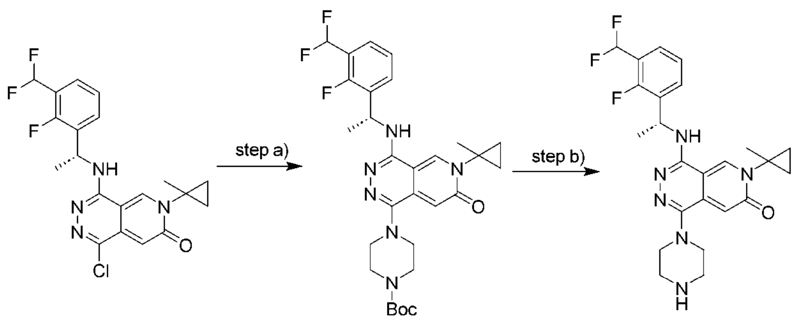

step a): Preparation of tert-butyl (*R*)-4-(4-((1-(3-(difluoromethyl)-2-fluorophenyl)ethyl)amino)-6-(1-methylcyclopropyl) -7-oxo-6,7-dihydropyrido[3,4-*d*]pyridazin-1-yl)piperazine-1-carboxylate

The preparation was carried out by referring to **Example 4,** to obtain tert-butyl (*R*)-4-(4-((1-(3-(difluoromethyl)-2-fluorophenyl)ethyl)amino)-6-(1-methylcyclopropyl)-7-oxo-6,7-dihydropyrido[3,4-*d*]pyridazin-1-yl)piperazine-1-carboxylate, yield 97.1%; ESI-MS(m/z): 573.3 $[M+H]^+$.

step b): Preparation of (*R*)-4-((1-(3-(difluoromethyl)-2-fluorophenyl)ethyl)amino)-6-(1-methylcyclopropyl)-1- (piperazin-1-yl)pyrido[3,4-*d*]pyridazin-7(6*H*)-one

Tert-butyl (*R*)-4-(4-((1-(3-(difluoromethyl)-2-fluorophenyl)ethyl)amino)-6-(1-methylcyclopropyl)-7-oxo-6,7- dihydropyrido[3,4-*d*]pyridazin-1-yl)piperazine-1-carboxylate (230 mg, 0.402 mmol), 1,4-dioxane (2 mL) and a solution of hydrogen chloride in 1,4-dioxane (8 mL, 4 M) were added to a reaction flask. The mixture was stirred to react at room temperature for 1 h. After the reaction was completed, the mixture was concentrated under reduced pressure. The residue was purified by Prep-HPLC (separation method 3) to obtain (*R*)-4-((1-(3-(difluoromethyl)-2-fluorophenyl)ethyl)amino) -6-(1-methylcyclopropyl)-1-(piperazin-1-yl)pyrido[3,4-*d*]pyridazin-7(6*H*)-one, yield 22.0%; $^1$H NMR (400 MHz, DMSO-$d_6$) δ 9.22 (s, 1H), 7.62 (t, *J* = 7.2 Hz, 1H), 7.55 (d, *J* = 6.8 Hz, 1H), 7.48 (t, *J* = 6.8 Hz, 1H), 7.38-7.11 (m, 2H), 6.41 (s, 1H), 5.61-5.51 (m, 1H), 2.95-2.86 (m, 4H), 2.86-2.80 (m, 4H), 1.58-1.49 (m, 6H), 1.20-1.04 (m, 4H); ESI-MS(m/z): 473.0 $[M+H]^+$.

**Example 79**

**Preparation of (*R*)-1-(4-acetylpiperazin-1-yl)-4-((1-(3-(difluoromethyl)-2-fluorophenyl)ethyl)amino)-6- (1-methylcyclopropyl)pyrido[3,4-*d*]pyridazin-7(6*H*)-one**

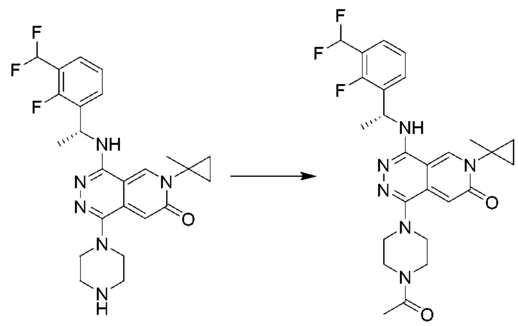

(*R*)-4-((1-(3-(Difluoromethyl)-2-fluorophenyl)ethyl)amino)-6-(1-methylcyclopropyl)-1-(piperazin-1-yl)pyrido [3,4-*d*]pyridazin-7(6*H*)-one (142 mg, 0.279 mmol), triethylamine (120 mg, 1.186 mmol), and dichloromethane (10 mL) were added to a reaction flask. A solution of acetyl chloride (22 mg, 0.280 mmol) in dichloromethane (1 mL) was added dropwise, and the mixture was reacted at room temperature for 30 min. The reaction was quenched with saturated aqueous sodium bicarbonate solution (30 mL), extracted with dichloromethane (30 mL × 2). The organic phases were combined, washed with saturated brine (10 mL), dried over anhydrous sodium sulfate, filtered, and the filtrate was concentrated under reduced pressure. The residue was purified by Prep-HPLC (separation method 3) to obtain (R)-1-(4-acetylpiperazin-1-yl)-4-((1-(3-(difluoromethyl)-2-fluorophenyl)ethyl)amino)-6-(1-methylcyclopropyl)pyrido[3,4-d]pyridazin-7(6*H*)-one, yield 14.8%; $^1$H NMR (400 MHz, DMSO-$d_6$) δ 9.28 (s, 1H), 7.71-7.43 (m, 3H), 7.38-7.11(m, 2H), 6.51 (s, 1H), 5.61-5.47 (m, 1H), 3.63-3.55 (m, 4H), 3.08-2.90 (m, 4H), 2.02 (s, 3H), 1.61-1.50 (m, 6H), 1.21-1.06 (m, 4H); ESI-MS(m/z): 515.0 $[M+H]^+$.

**Example 80**

**Preparation of (*R*)-4-((1-(3-(difluoromethyl)-2-fluorophenyl)ethyl)amino)-6-(1-methylcyclopropyl)-1- (4-methylpiperazin-1-yl)pyrido[3,4-*d*]pyridazin-7(6*H*)-one formate**

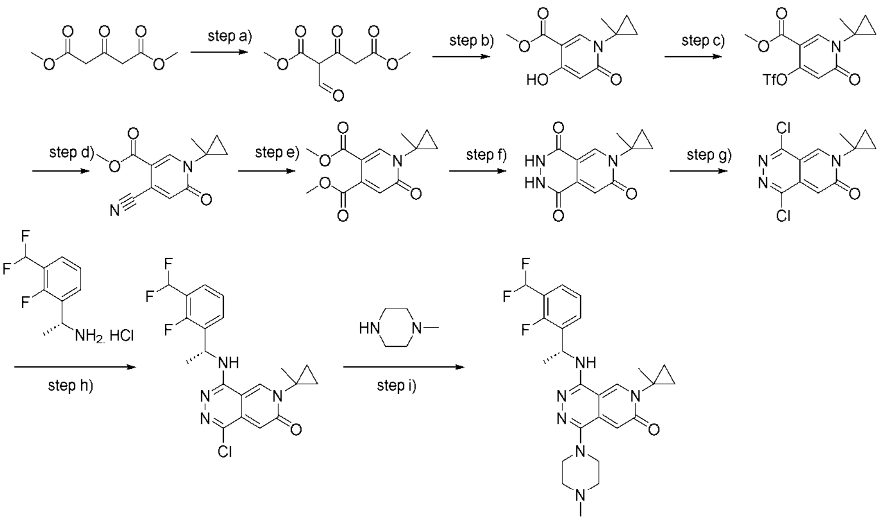


steps a-f): Preparation of 6-(1-methylcyclopropyl)-2,3-dihydropyrido[3,4-*d*]pyridazine-1,4,7(6*H*)-trione

The preparation was carried out by referring to steps a-f of **Example 1** to obtain 6-(1-methylcyclopropyl)-2,3-dihydropyrido[3,4-*d*]pyridazine-1,4,7(6*H*)-trione; ESI-MS(m/z): 234.1 $[M+H]^+$.

step g): Preparation of 1,4-dichloro-6-(1-methylcyclopropyl)pyrido[3,4-*d*]pyridazin-7(6*H*)-one

6-(1-Methylcyclopropyl)-2,3-dihydropyrido[3,4-*d*]pyridazine-1,4,7(6*H*)-trione (300 mg, 1.286 mmol) and acetonitrile (30 mL) were added to a reaction flask. Under stirring at room temperature, DIPEA (499 mg, 3.858 mmol) and phosphorus oxychloride (592 mg, 3.858 mmol) were added. The mixture was warmed to 100°C under nitrogen protection and reacted for 3 h. After the reaction was completed, the mixture was cooled to room temperature. The reaction solution was slowly poured into a mixture of ice and sodium bicarbonate for quenching, extracted with dichloromethane (30 mL × 3). The organic phases were combined, dried over anhydrous sodium sulfate, filtered, and the filtrate was concentrated under reduced pressure. The residue was purified by silica gel column chromatography (eluent: dichloromethane/methanol = 50/1) to obtain 1,4-dichloro-6-(1-methylcyclopropyl)pyrido[3,4-*d*]pyridazin-7(6*H)*-one, yield 22.5%; ESI-MS(m/z): 270.0 $[M+H]^+$.

step h): Preparation of (*R*)-1-chloro-4-((1-(3-(difluoromethyl)-2-fluorophenyl)ethyl)amino)-6-(1-methylcyclopropyl) pyrido[3,4-*d*]pyridazin-7(6*H*)-one

1,4-Dichloro-6-(1-methylcyclopropyl)pyrido[3,4-*d*]pyridazin-7(6*H*)-one (75 mg, 0.278 mmol), (*R*)-1-(3-(difluoromethyl)-2-fluorophenyl)ethan-1-amine hydrochloride (63 mg, 0.278 mmol), and 1,4-dioxane (2 mL) were added to a microwave reaction flask. The mixture was warmed to 120°C in a microwave reactor and reacted for 2 h. After the reaction was completed, the mixture was cooled to room temperature and concentrated under reduced pressure. The residue was purified by silica gel column chromatography (eluent: dichloromethane/methanol = 30/1) to obtain (*R*)-1-chloro-4-((1-(3-(difluoromethyl)-2-fluorophenyl)ethyl)amino)-6-(1-methylcyclopropyl)pyrido[3,4-*d*]pyridazin-7(6*H*)-one, yield 73.9%; ESI-MS(m/z): 423.2 $[M+H]^+$.

step i): Preparation of (*R*)-4-((1-(3-(difluoromethyl)-2-fluorophenyl)ethyl)amino)-6-(1-methylcyclopropyl)-1- (4-methylpiperazin-1-yl)pyrido[3,4-*d*]pyridazin-7(6*H*)-one formate

(*R*)-1-Chloro-4-((1-(3-(difluoromethyl)-2-fluorophenyl)ethyl)amino)-6-(1-methylcyclopropyl)pyrido[3,4-*d*]pyridazin-7(6*H*)-one(50 mg, 0.118 mmol), 4-methylpiperazine(36 mg, 0.359 mmol) and 1,4-dioxane (1 mL) were added to a reaction flask. The mixture was warmed to 110°C and reacted for 30 min. After the reaction was completed, the mixture was cooled to room temperature and concentrated under reduced pressure. The residue was purified by Prep-HPLC (separation method 2) to obtain (*R*)-4-((1-(3-(difluoromethyl)-2-fluorophenyl)ethyl)amino)-6-(1-methylcyclopropyl)-1-(4-methylpiperazin-1-yl) pyrido[3,4-*d*]pyridazin-7(6*H*)-one formate, yield 36.2%; $^1$H NMR (400 MHz, DMSO-$d_6$) δ 9.22 (s, 1H), 8.16 (s, 1H), 7.62 (t, *J* = 7.2 Hz, 1H), 7.57 (d, *J* = 6.8 Hz, 1H), 7.48 (t, *J* = 6.8 Hz, 1H), 7.38-7.11 (m, 2H), 6.39 (s, 1H), 5.62-5.51 (m, 1H), 3.05-2.95 (m, 4H), 2.50-2.41(m, 4H), 2.23 (s, 3H), 1.59-1.49 (m, 6H), 1.20-1.03(m, 4H); ESI-MS(m/z): 487.0 $[M+H]^+$.

**Example 81**

**Preparation of (*R*)-4-((1-(2-methyl-3-(trifluoromethyl)phenyl)ethyl)amino)-6-(1-methylcyclopropyl)-1- (4-methylpiperazin-1-yl)pyrido[3,4-*d*]pyridazin-7(6*H*)-one**

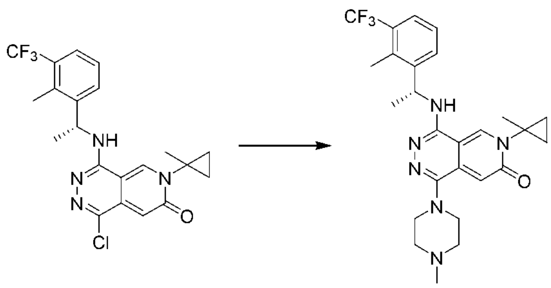

The preparation was carried out by referring to **Example 80,** the resulting crude product was purified by Prep-HPLC (separation method 3) to obtain (R)-4-((1-(2-methyl-3-(trifluoromethyl)phenyl)ethyl)amino)-6-(1-methylcyclopropyl)-1- (4-methylpiperazin-1-yl)pyrido[3,4-*d*]pyridazin-7(6*H*)-one, yield 37.3%; $^1$H NMR (400 MHz, DMSO-$d_6$) δ 9.22 (s, 1H), 7.74 (d, $J$ = 7.8 Hz, 1H), 7.58 (d, $J$ = 6.8 Hz, 1H), 7.54 (d, $J$ = 7.8 Hz, 1H), 7.35 (t, $J$ = 7.8 Hz, 1H), 6.39 (s, 1H), 5.55 (p, $J$ = 6.8 Hz, 1H), 3.29-3.26 (m, 4H), 3.12-2.87 (m, 4H), 2.51 (s, 3H) 2.26 (s, 3H), 1.54 (s, 3H), 1.49 (d, $J$ = 6.9 Hz, 3H), 1.20-1.04 (m, 4H); ESI-MS (m/z): 501.2 [M+H]$^+$.

**Example 82**

**Preparation of (*R*)-2-methyl-3-(1-((6-(1-methylcyclopropyl)-1-(4-methylpiperazin-1-yl)-7-oxo-6,7-dihydropyrido [3,4-*d*]pyridazin-4-yl)amino)ethyl)benzonitrile**

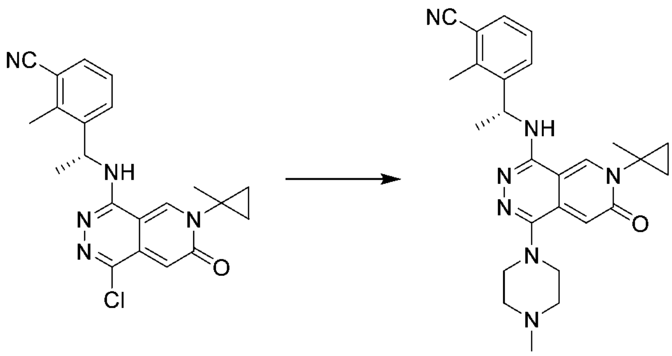

The preparation was carried out by referring to **Example 80,** the resulting crude product was purified by Prep-HPLC (separation method 3) to obtain (*R*)-2-methyl-3-(1-((6-(1-methylcyclopropyl)-1-(4-methylpiperazin-1-yl)-7-oxo-6,7-dihydropyrido[3,4-d]pyridazin-4-yl)amino)ethyl)benzonitrile, yield 41.0%; $^1$H NMR (400 MHz, DMSO-$d_6$) δ 9.20 (s, 1H), 7.74 (d, $J$ = 8.0 Hz, 1H), 7.63 (d, $J$ = 7.2 Hz, 1H), 7.58 (d, $J$ = 6.4 Hz, 1H), 7.36 (t, $J$ = 7.6 Hz, 1H), 6.39 (s, 1H), 5.49-5.42 (m, 1H), 3.00-2.97 (m, 4H), 2.62 (s, 3H), 2.46 (s, 4H), 2.22 (s, 3H), 1.54 (s, 3H), 1.49 (d, $J$ = 7.2 Hz, 3H), 1.19-1.04 (m, 4H); ESI-MS (m/z): 458.3 [M+H]$^+$.

**Example 83**

**Preparation of (*R*)-2-methyl-3-(1-((1-(methylamino)-6-(1-methylcyclopropyl)-7-oxo-6,7-dihydropyrido[3,4-*d*] pyridazin-4-yl)amino)ethyl)benzonitrile**

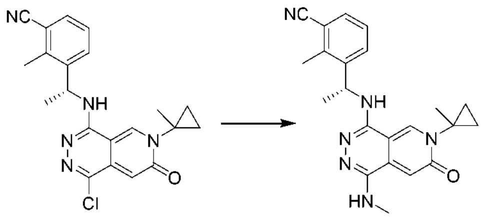

The preparation was carried out by referring to Example 80, the resulting crude product was purified by Prep-HPLC (separation method 3) to obtain (R)-2-methyl-3-(1-((1-(methylamino)-6-(1-methylcyclopropyl)-7-oxo-6,7-dihydropyrido [3,4-d]pyridazin-4-yl)amino)ethyl)benzonitrile, yield 37.2%; $^1$H NMR (400 MHz, DMSO-$d_6$) δ 9.01 (s, 1H), 7.73 (d, *J* = 7.6 Hz, 1H), 7.61 (d, *J* = 7.2 Hz, 1H), 7.34 (t, *J* = 7.6 Hz, 1H), 6.98 (d, *J* = 6.4 Hz, 1H), 6.76 (s, 1H), 6.41-6.38 (m, 1H), 5.38-5.31 (m, 1H), 2.75 (d, *J* = 4.4 Hz, 3H), 2.61 (s, 3H), 1.53 (s, 3H), 1.46 (d, *J* = 6.8 Hz, 3H), 1.17-1.03 (m, 4H); ESI-MS (m/z): 389.2 $[M+H]^+$.

**Example 84**

**Preparation of (*R*)-3-(1-((1-(dimethylamino)-6-(1-methylcyclopropyl)-7-oxo-6,7-dihydropyrido[3,4-*d*] pyridazin-4-yl)amino)ethyl)-2-methylbenzonitrile**

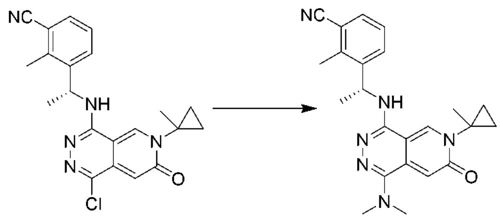

The preparation was carried out by referring to **Example 80,** the resulting crude product was purified by Prep-HPLC (separation method 3) to obtain (*R*)-3-(1-((1-(dimethylamino)-6-(1-methylcyclopropyl)-7-oxo-6,7-dihydropyrido [3,4-*d*] pyridazin-4-yl)amino)ethyl)-2-methylbenzonitrile, yield 39.7%; $^1$H NMR (400 MHz, DMSO-$d_6$) δ 9.17 (s, 1H), 7.75 (d, *J* = 7.6 Hz, 1H), 7.63 (d, *J* = 7.2 Hz, 1H), 7.50 (d, *J* = 6.4 Hz, 1H), 7.36 (t, *J* = 8.0 Hz, 1H), 6.46 (s, 1H), 5.48-5.41 (m, 1H), 2.70 (s, 6H), 2.62 (s, 3H), 1.55 (s, 3H), 1.49 (d, *J* = 6.8 Hz, 3H), 1.18-1.06 (m, 4H); ESI-MS (m/z): 403.2 $[M+H]^+$.

**Example 85**

**Preparation of 2-methyl-3-((*R*)-1-((6-(1-methylcyclopropyl)-1-((*R*)-2-methylpyrrolidin-1-yl)-7-oxo-6,7-dihydropyrido[3,4-*d*]pyridazin-4-yl)amino)ethyl)benzonitrile**

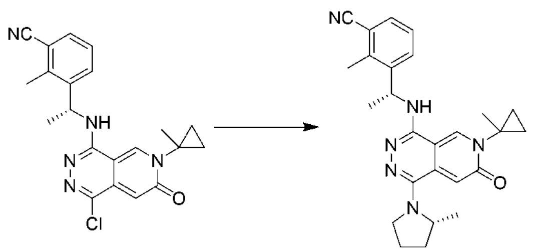

The preparation was carried out by referring to **Example 80,** the resulting crude product was purified by Prep-HPLC (separation method 3) to obtain 2-methyl-3-((*R*)-1-((6-(1-methylcyclopropyl)-1-((*R*)-2-methylpyrrolidin-1-yl)-7-oxo-6,7- dihydropyrido[3,4-d]pyridazin-4-yl)amino)ethyl)benzonitrile, yield 29.0%; $^1$H NMR (400 MHz, DMSO-$d_6$) δ 9.12 (s, 1H), 7.77 (d, *J* = 8.0 Hz, 1H), 7.63 (d, *J* = 7.2 Hz, 1H), 7.38-7.33 (m, 2H), 6.52 (s, 1H), 5.46-5.39 (m, 1H), 4.17-4.09 (m, 1H), 3.69-3.63 (m, 1H), 3.09-3.04 (m, 1H), 2.61 (s, 3H), 2.12-2.05 (m, 1H), 1.90-1.83 (m, 1H), 1.72-1.63 (m, 1H), 1.54-1.47 (m, 7H), 1.20-1.06 (m, 4H), 0.95 (d, *J* = 4.8 Hz, 3H); ESI-MS (m/z): 443.2 $[M+H]^+$.

**Example 86**

**Preparation of (*R*)-6-cyclopropyl-4-((1-(2-methyl-3-(trifluoromethyl)phenyl)ethyl)amino)-1-(4-methylpiperazin-1-yl)pyrido[3,4-*d*]pyridazin-7(6*H*)-one**

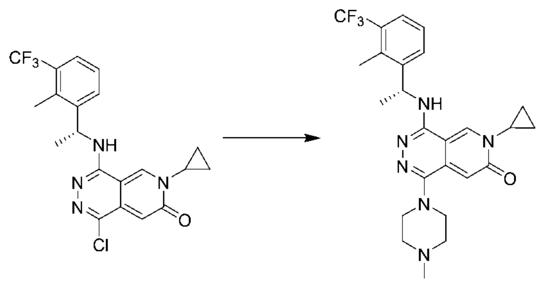

The preparation was carried out by referring to **Example 80,** the resulting crude product was purified by Prep-HPLC (separation method 3) to obtain (R)-6-cyclopropyl-4-((1-(2-methyl-3-(trifluoromethyl)phenyl)ethyl)amino)-1- (4-methylpiperazin-1-yl)pyrido[3,4-*d*]pyridazin-7(6*H*)-one, yield 17.5%; $^1$H NMR (400 MHz, DMSO-$d_6$) δ 8.98 (s, 1H), 7.73 (d, *J* = 7.6 Hz, 1H), 7.60-7.52 (m, 2H), 7.35 (t, *J* = 8.0 Hz, 1H), 6.41 (s, 1H), 5.61-5.52 (m, 1H), 3.65-3.58 (m, 1H), 3.02-2.94 (m, 4H), 2.49-2.43 (m, 7H), 2.22 (s, 3H), 1.52-1.46 (m, 3H), 1.18-1.08 (m, 4H); ESI-MS (m/z): 487.0 [M+H]$^+$.

**Example 87**

**Preparation of (*R*)-3-(1-((6-cyclopropyl-1-(4-methylpiperazin-1-yl)-7-oxo-6,7-dihydropyrido[3,4-*d*]pyridazin-4-yl) amino)ethyl)-2-methylbenzonitrile**

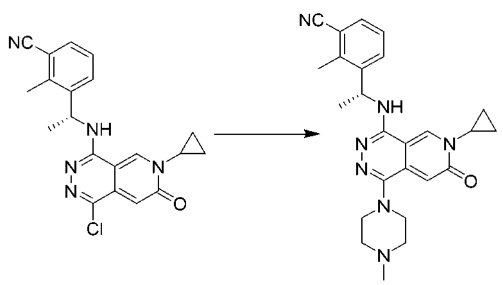

The preparation was carried out by referring to **Example 80,** the resulting crude product was purified by Prep-HPLC (separation method 3) to obtain (*R*)-3-(1-((6-cyclopropyl-1-(4-methylpiperazin-1-yl)-7-oxo-6,7-dihydropyrido [3,4-*d*] pyridazin-4-yl)amino)ethyl)-2-methylbenzonitrile, yield 44.7%; $^1$H NMR (400 MHz, DMSO-$d_6$) δ 8.97 (s, 1H), 7.73 (d, *J* = 8.0 Hz, 1H), 7.63 (d, *J* = 7.6 Hz, 1H), 7.60 (s, *J* = 6.8 Hz, 1H), 7.35 (t, *J* = 8.0 Hz, 1H), 6.41 (s, 1H), 5.50-5.43 (m, 1H), 3.64-3.59 (m, 1H), 2.99 (s, 4H), 2.62 (s, 4H), 2.47 (s, 3H), 2.23 (s, 3H), 1.48 (d, *J* = 7.2 Hz, 3H), 1.17-1.09 (m, 4H); ESI-MS (m/z): 444.2 [M+H]$^+$.

**Example 88**

**Preparation of (*R*)-6-cyclobutyl-4-((1-(3-(difluoromethyl)-2-fluorophenyl)ethyl)amino)-1-(4-methylpiperazin -1-yl)pyrido[3,4-*d*]pyridazin-7(6*H*)-one**

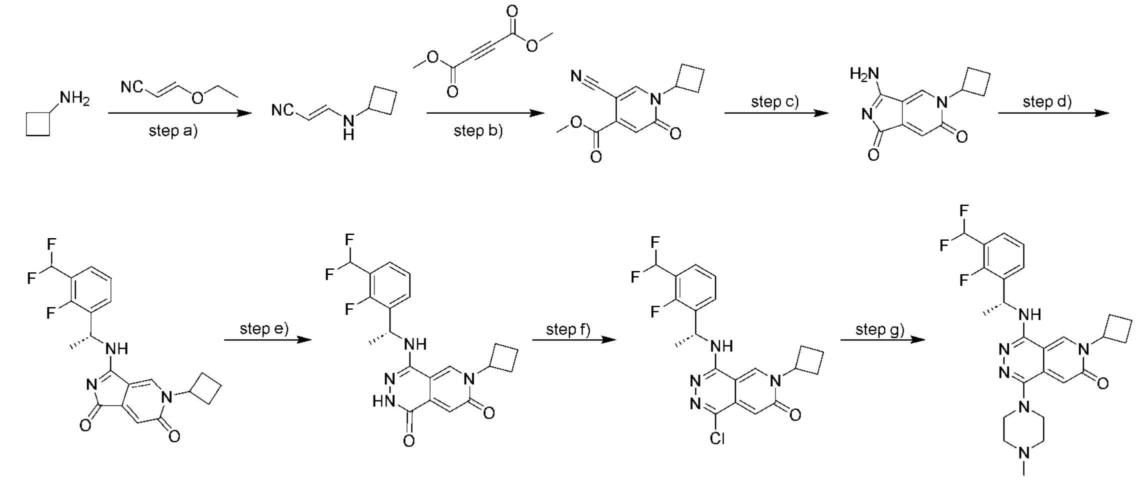


step a): Preparation of (*E*)-3-(cyclobutylamino)acrylonitrile

Cyclobutanamine (2.3 g, 32.394 mmol) and (*E*)-3-ethoxyacrylonitrile (3.5 g, 35.634 mmol) were added to a sealed tube, and the mixture was heated to 80°C and stirred to react for 20 h. After the reaction was completed, the mixture was cooled to room temperature to obtain (*E*)-3-(cyclobutylamino)acrylonitrile (3.8 g, crude product) as a brown liquid, which was used directly in the next step without purification; ESI-MS (m/z): 123.0 $[M+H]^+$.

step b): Preparation of methyl 5-cyano-1-cyclobutyl-2-oxo-1,2-dihydropyridine-4-carboxylate

(*E*)-3-(Cyclobutylamino)acrylonitrile (3.8 g, crude product) was dissolved in dichloromethane (50 mL), then DBU (4.9 g, 32.394 mmol) was added. Dimethyl but-2-ynedioate (6.9 g, 48.591 mmol) was slowly added dropwise at 0°C. After the addition was completed, the mixture was maintained at 0°C and continuted to stir to react for 0.5 h. The reaction solution was concentrated under reduced pressure. The residue was purified by silica gel column chromatography (eluent: petroleum ether/ethyl acetate=1/2) to obtain methyl 5-cyano-1-cyclobutyl-2-oxo-1,2-dihydropyridine-4-carboxylate, yield 31.9%; ESI-MS (m/z): 233.0 $[M+H]^+$.

step c): Preparation of 3-amino-5-cyclobutyl-1*H*-pyrrolo[3,4-c]pyridine-1,6(5*H*)-dione

Methyl 5-cyano-1-cyclobutyl-2-oxo-1,2-dihydropyridine-4-carboxylate (2.4 g, 10.345 mmol) and a ammonia methanol solution (25 mL) were added to a sealed tube. The mixture was stirred to react at room temperature overnight. The reaction solution was filtered, and the filter cake was dried to obtain 3-amino-5-cyclobutyl-1*H*-pyrrolo[3,4-*c*]pyridine-1,6(5*H*)-dione (yield 97.9%); ESI-MS (m/z): 218.0 $[M+H]^+$.

step d): Preparation of (*R*)-5-cyclobutyl-3-((1-(3-(difluoromethyl)-2-fluorophenyl)ethyl)amino)-1*H*-pyrrolo[3,4-*c*] pyridine-1,6(5*H*)-dione

(*R*)-5-cyclobutyl-3-((1-(3-(difluoromethyl)-2-fluorophenyl)ethyl)amino)-1*H*-pyrrolo[3,4-*c*]pyridine-1,6(5*H*)-dione (800 mg, 3.687 mmol), (*R*)-1-(3-(difluoromethyl)-2-fluorophenyl)ethan-1-amine hydrochloride (912 mg, 4.055 mmol) and ethanol (30 mL) were added to a reaction flask. The mixture was warmed to 80°C and reacted for 16 h. After the reaction was completed, the mixture was cooled to room temperature and concentrated under reduced pressure. The residue was purified by silica gel column chromatography (eluent: dichloromethane/methanol=97/3) to obtain (*R*)-5-cyclobutyl-3-((1-(3-(difluoromethyl)-2-fluorophenyl)ethyl)amino)-1*H*-pyrrolo[3,4-*c*]pyridine-1,6(5*H*)-dione, yield 56.5%; ESI-MS (m/z): 390.0 $[M+H]^+$.

step e): Preparation of (*R*)-5-cyclobutyl-3-((1-(3-(difluoromethyl)-2-fluorophenyl)ethyl)amino)-1*H*-pyrrolo[3,4-*c*] pyridine-1,6(5*H*)-dione

(*R*)-5-cyclobutyl-3-((1-(3-(difluoromethyl)-2-fluorophenyl)ethyl)amino)-1*H*-pyrrolo[3,4-*c*]pyridine-1,6(5*H*)-dione (810 mg, 2.082 mmol) and ethanol (20 mL) were added to a reaction flask. Hydrazine hydrate (260 mg, 4.165 mmol, 80%) was added dropwise at 0°C. After the addition was completed, the reaction was maintained at 0°C for another 2 h. The reaction solution was concentrated under reduced pressure, and the residue was purified by silica gel

column chromatography (eluent: dichloromethane/methanol = 97/3) to obtain (*R*)-5-cyclobutyl-3-((1-(3-(difluoromethyl)-2- fluorophenyl)ethyl)amino)-1*H*-pyrrolo[3,4-*c*]pyridine-1,6(5*H*)-dione, with a yield of 38.0%; ESI-MS (m/z): 405.2 $[M+H]^+$.

step f): (*R*)-1-chloro-6-cyclobutyl-4-((1-(3-(difluoromethyl)-2-fluorophenyl)ethyl)amino)pyrido[3,4-*d*]pyridazin-7(6*H*) -one

(*R*)-5-Cyclobutyl-3-((1-(3-(difluoromethyl)-2-fluorophenyl)ethyl)amino)-1*H*-pyrrolo[3,4-*c*]pyridine-1,6(5*H*)-dione (320 mg, 0.792 mmol), DIPEA (153 mg, 1.188 mmol), phosphorus oxychloride (364 mg, 2.376 mmol) and acetonitrile (50 mL) were added to a reaction flask. Under nitrogen protection, the mixture was warmed to 105°C and reacted for 2 h. The reaction solution was cooled to room temperature, quenched with saturated aqueous sodium bicarbonate solution (50 mL), and extracted with dichloromethane (50 mL × 3). The organic phases were combined, washed with saturated brine (50 mL), dried over anhydrous sodium sulfate, and filtered. The filtrate was concentrated under reduced pressure. The residue was purified by silica gel column chromatography (eluent: ethyl acetate/petroleum ether = 4/1) to obtain (*R*)-1-chloro-6-cyclobutyl-4-((1-(3-(difluoromethyl)-2-fluorophenyl)ethyl)amino)pyrido[3,4-*d*]pyridazin-7(6*H*)-one, yield 43.4%; ESI-MS (m/z): 423.0 $[M+H]^+$.

step g): Preparation of (*R*)-6-cyclobutyl-4-((1-(3-(difluoromethyl)-2-fluorophenyl)ethyl)amino)-1- (4-methylpiperazin-1-yl)pyrido[3,4-*d*]pyridazin-7(6*H*)-one

(*R*)-1-Chloro-6-cyclobutyl-4-((1-(3-(difluoromethyl)-2-fluorophenyl)ethyl)amino)pyrido[3,4-*d*]pyridazin-7(6*H*)-one (50 mg, 0.118 mmol), N-methylpiperazine (24 mg, 0.237 mmol) and 1,4-dioxane (2 mL) were added to a reaction flask. The mixture was warmed to 120°C and reacted for 1h in a microwave reactor. After the reaction was completed, the mixture was concentrated under reduced pressure. The residue was purified by Prep-HPLC (separation method 3) to obtain (*R*)-6-cyclobutyl-4-((1-(3-(difluoromethyl)-2-fluorophenyl)ethyl)amino)-1-(4-methylpiperazin-1-yl) pyrido[3,4-*d*]pyridazin-7(6*H*)-one, yield 18.0%; $^1H$ NMR (400 MHz, DMSO-$d_6$) $\delta$ 9.07 (s, 1H), 7.63 (t, *J* = 7.2 Hz, 2H), 7.49 (t, *J* = 7.2 Hz, 1H), 7.28 (t, *J* = 7.6 Hz, 1H), 7.24 (t, *J* = 52.0 Hz, 1H), 6.41 (s, 1H), 5.65-5.56 (m, 1H), 5.22-5.11 (m, 1H), 3.07-2.89 (m, 4H), 2.49-2.41 (m, 8H), 2.22 (s, 3H), 1.93-1.83 (m, 2H), 1.56 (d, *J* = 7.2 Hz, 3H); ESI-MS (m/z): 487.0 $[M+H]^+$.

According to the preparation method of **Example 88,** with the corresponding raw materials, the compounds in the following Examples were prepared.

| Number | Chemical Name | Structure | $^1$H NMR and MS |
|---|---|---|---|
| **Example 89** | (*R*)-6-cyclopropyl-1-(4-methylpiperazin-1-yl)-4-((1-(3-(pentafluoro-$\lambda^6$-sulfaneyl)phenyl)et hyl)amino)pyrido[3, 4-*d*]pyridazin-7(6*H*)-one | 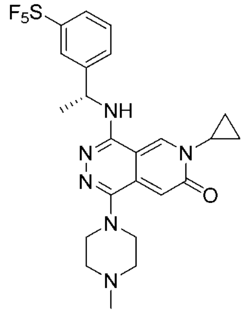 | $^1$H NMR (400 MHz, DMSO-$d_6$) δ 8.94 (s, 1H), 7.88 (s, 1H), 7.76-7.71 (m, 1H), 7.69 (d, *J* = 7.6 Hz, 1H), 7.59-7.53 (m, 2H), 6.42 (s, 1H), 5.45-5.36 (m, 1H), 3.66-3.58 (m, 1H), 3.00 (s, 4H), 2.47 (s, 4H), 2.22 (s, 3H), 1.54 (d, *J* = 7.2 Hz, 3H), 1.18-1.13 (m, 2H), 1.11-1.03 (m, 2H); ESI-MS (m/z): 531.0 $[M+H]^+$. |
| **Example 90** | (*R*)-6-(3,3-difluorocyclobutyl)-4-((1-(3-(difluoromethyl)-2-fluorophenyl)ethyl) amino)-1-(4-methylpiperazin-1-yl)pyrido [3,4-*d*]pyridazin-7(6*H*)-one | 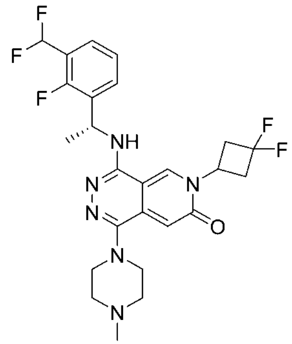 | $^1$H NMR (400 MHz, DMSO-$d_6$) δ 8.97 (s, 1H), 7.65-7.58 (m, 2H), 7.49 (t, *J* = 7.2 Hz, 1H), 7.28 (t, *J* = 7.6 Hz, 1H), 7.25 (t, *J* = 54.4 Hz, 1H), 6.45 (s, 1H), 5.64-5.57 (m, 1H), 4.93-4.85 (m, 1H), 3.29-3.15 (m, 4H), 3.05-2.96 (m, 4H), 2.49-2.44 (m, 4H), 2.23 (s, 3H), 1.56 (d, *J* = 6.8 Hz, 3H); ESI-MS(m/z): 523.0 $[M+H]^+$. |
| **Example 91** | (*R*)-1-(4-isopropylpiperazin-1-yl)-4-((1-(2-methyl-3-(trifluoromethyl)phe nyl)ethyl) amino)-6-(1-methylcyclopropyl) pyrido [3,4-*d*]pyridazin-7(6*H*)-one | 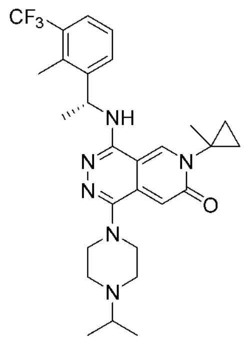 | $^1$H NMR (400MHz,DMSO-$d_6$) δ 9.22 (s, 1H), 7.74 (d, *J* = 7.6 Hz, 1H), 7.57 (d, *J* = 6.8 Hz, 1H), 7.54 (d, *J* = 8.0 Hz, 1H), 7.35 (t, *J* = 7.6 Hz, 1H), 6.39 (s, 1H), 5.60-5.49 (m, 1H), 3.04-2.90 (m, 4H), 2.71-2.64 (m, 1H), 2.63-2.54 (m, 4H), 2.52 (s, 3H), 1.54 (s, 3H), 1.48 (d, *J* = 6.8 Hz, 3H), 1.20-1.04 (m, 4H), 1.00 (d, *J* = 6.4 Hz, 6H); ESI-MS(m/z): 529.0 $[M+H]^+$. |

(continued)

| Number | Chemical Name | Structure | $^1$H NMR and MS |
|---|---|---|---|
| **Example 92** | (*R*)-1-(4-cyclopropylpiperazi n-1-yl)-4-((1-(2-methyl-3-(trifluoromethyl)phenyl)ethyl)amino)-6-(1-methylcyclopropyl) pyrido[3,4-*d*]pyridazin-7(6*H*)-one | 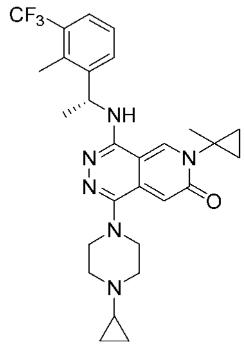 | $^1$H NMR (400MHz,DMSO-$d_6$) δ 9.22 (s, 1H), 7.74 (d, *J* = 7.6 Hz, 1H), 7.58 (d, *J* = 6.8 Hz, 1H), 7.54 (d, *J* = 8.0 Hz, 1H), 7.35 (t, *J* = 7.8 Hz, 1H), 6.40 (s, 1H), 5.60-5.49 (m, 1H), 3.05-2.80 (m, 4H), 2.73-2.64 (m, 4H), 2.53 (s, 3H), 1.73-1.64 (m, 1H), 1.54 (s, 3H), 1.48 (d, *J* = 6.8 Hz, 3H), 1.20- 1.04 (m, 4H), 0.46-0.27 (m, 4H); ESI-MS(m/z): 527.0 $[M+H]^+$. |
| **Example 93** | (*R*)-1-(4-(cyclopropylmethyl) piperazin-1-yl)-4-((1-(2-methyl-3-(trifluoromethyl)phenyl)ethyl)amino)-6-(1-methylcyclopropyl) pyrido[3,4-*d*]pyridazin-7(6*H*)-one | 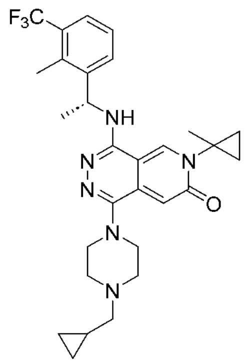 | $^1$H NMR (400MHz,DMSO-$d_6$) δ 9.22 (s, 1H), 7.74 (d, *J* = 7.6 Hz, 1H), 7.58 (d, *J* = 6.4 Hz, 1H), 7.54 (d, *J* = 8.0 Hz, 1H), 7.35 (t, *J* = 7.8 Hz, 1H), 6.38 (s, 1H), 5.60-5.49 (m, 1H), 3.11-2.75 (m, 4H), 2.65-2.53 (m, 7H), 2.23 (d, *J* = 6.4 Hz, 2H), 1.54 (s, 3H), 1.49 (d, *J* = 6.8 Hz, 3H), 1.19- 1.03 (m, 4H), 0.89-0.78 (m, 1H), 0.50-0.43 (m, 2H), 0.12-0.05 (m, 2H); ESI-MS(m/z): 541.0 $[M+H]^+$. |
| **Example 94** | (*R*)-1-(4-cyclobutylpiperazin -1-yl)-4-((1-(2-methyl-3-(trifluoromethyl)phenyl)ethyl)amino)-6-(1-methylcyclopropyl) pyrido[3,4-*d*]pyridazin-7(6*H*)-one | 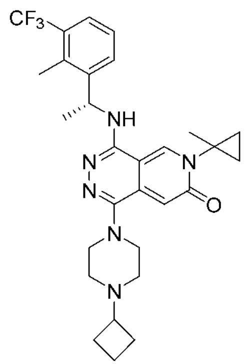 | $^1$H NMR (400MHz,DMSO-$d_6$) δ 9.22 (s, 1H), 7.74 (d, *J* = 8.0 Hz, 1H), 7.58 (d, *J* = 6.4 Hz, 1H), 7.54 (d, *J* = 8.0 Hz, 1H), 7.35 (t, *J* = 7.8 Hz, 1H), 6.37 (s, 1H), 5.60-5.49 (m, 1H), 3.06-2.85 (m, 4H), 2.82-2.72 (m, 1H), 2.52 (s, 3H), 2.46-2.34 (m, 4H), 2.02-1.92 (m, 2H), 1.86-1.75 (m, 2H), 1.68-1.58 (m, 2H), 1.54 (s, 3H), 1.48 (d, *J* = 6.8 Hz, 3H), 1.19-1.04 (m, 4H); ESI-MS(m/z): 541.0 $[M+H]^+$. |

(continued)

| Number | Chemical Name | Structure | $^1$H NMR and MS |
|---|---|---|---|
| **Example 95** | (*R*)-1-(4-(tert-butyl)piperazin-1-yl)-4-((1-(2-methyl-3-(trifluoromethyl)phe nyl)ethyl)amino)-6-(1-methylcyclopropyl) pyrido[3,4-*d*]pyridazin-7(6*H*)-one | 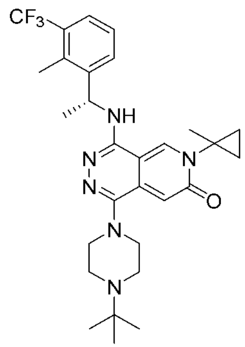 | $^1$H NMR (400MHz,DMSO-$d_6$) $\delta$ 9.22 (s, 1H), 7.74 (d, *J* = 8.0 Hz, 1H), 7.60-7.48 (m, 2H), 7.35 (t, *J* = 8.0 Hz, 1H), 6.40 (s, 1H), 5.61-5.48 (m, 1H), 3.05-2.88 (m, 4H), 2.71-2.58(m, 4H), 2.52 (s, 3H), 1.54 (s, 3H), 1.48 (d, *J* = 6.8 Hz, 3H), 1.18-1.01 (m, 13H); ESI-MS(m/z): 543.0 $[M+H]^+$. |
| **Example 96** | (*R*)-1-(4-cyclopentylpiperazi n-1-yl)-4-((1-(2-methyl-3-(trifluoromethyl)phe nyl)ethyl)amino)-6-(1-methylcyclopropyl) pyrido[3,4-*d*]pyridazin-7(6*H*)-one | 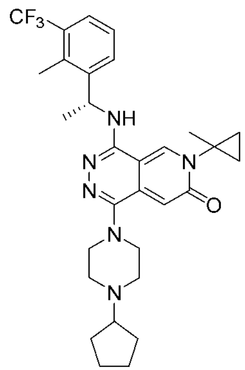 | $^1$H NMR (400MHz,DMSO-$d_6$) $\delta$ 9.22 (s, 1H), 7.74 (d, *J* = 7.6 Hz, 1H), 7.58 (d, *J* = 6.8 Hz, 1H),7.54 (d, *J* = 8.0 Hz, 1H), 7.35 (t, *J* = 8.0 Hz, 1H), 6.38 (s, 1H), 5.60-5.49 (m, 1H), 3.05-2.89 (m, 4H), 2.60-2.52 (m, 7H), 2.35-2.30 (m, 1H), 1.83-1.75 (m, 2H), 1.64-1.57 (m, 2H), 1.54 (s, 3H), 1.52-1.45 (m, 5H), 1.36-1.29 (m, 2H), 1.18-1.05 (m, 4H); ESI-MS(m/z): 555.0 $[M+H]^+$. |
| **Example 97** | (*R*)-1-(ethylamino)-4-((1-(2-methyl-3-(trifluoromethyl)phe nyl)ethyl)amino)-6-(1-methylcyclopropyl) pyrido[3,4-*d*]pyridazin-7(6*H*)-one | 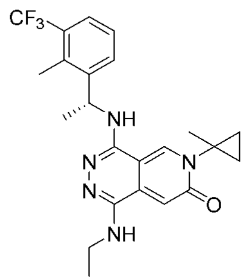 | $^1$H NMR (400MHz,DMSO-$d_6$) $\delta$ 9.02 (s, 1H), 7.73 (d, *J* = 8.0 Hz, 1H), 7.52 (d, *J* = 8.0 Hz, 1H), 7.33 (t, *J* = 8.0 Hz, 1H), 6.97 (d, *J* = 6.8 Hz, 1H), 6.85 (s, 1H), 6.30-6.21 (m, 1H), 5.49-5.38 (m, 1H), 3.29-3.18 (m, 2H), 2.52 (s, 3H), 1.53 (s, 3H), 1.45 (d, *J* = 7.2 Hz, 3H), 1.18-1.03 (m, 7H); ESI-MS(m/z): 446.0 $[M+H]^+$. |

(continued)

| Number | Chemical Name | Structure | $^1$H NMR and MS |
|---|---|---|---|
| **Example 98** | (*R*)-6-(3,3-difluorocyclobutyl)-4-((1-(3-(difluoromethyl)-2-fluorophenyl)ethyl) amino)-1-(4-ethylpiperazin-1-yl)pyrido[3,4-*d*] pyridazin-7(6*H*)-one | 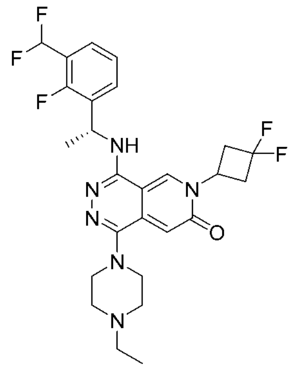 | $^1$H NMR (400 MHz, DMSO-$d_6$) δ 8.97 (s, 1H), 7.65-7.58 (m, 2H), 7.49 (t, *J* = 7.2 Hz, 1H), 7.28 (t, *J* = 7.6 Hz, 1H), 7.25 (t, *J* = 54.4 Hz, 1H), 6.45 (s, 1H), 5.64-5.57 (m, 1H), 4.94-4.85 (m, 1H), 3.32-3.27 (m, 4H), 3.07-2.96 (m, 4H), 2.57-2.52 (m, 4H), 2.41-2.35 (m, 2H), 1.56 (d, *J* = 6.8 Hz, 3H), 1.01 (t, *J* = 7.2 Hz, 3H); ESI-MS(m/z): 537.0 $[M+H]^+$. |
| **Example 99** | (*R*)-3-(1-((6-cyclopropyl-1-(4-cyclopropylpiperazi n-1-yl)-7-oxo-6,7-dihydropyrido [3,4-*d*]pyridazin-4-yl)amino)ethyl)-2-methylbenzonitrile | 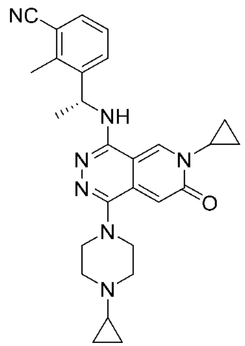 | $^1$H NMR (400 MHz, DMSO-$d_6$) δ 8.97 (s, 1H), 7.73 (d, *J* = 7.6 Hz, 1H), 7.63 (d, *J* = 7.6 Hz, 1H), 7.59 (d, *J* = 6.4 Hz, 1H), 7.35 (t, *J* = 8.0 Hz, 1H), 6.43 (s, 1H), 5.50-5.43 (m, 1H), 3.64-3.59 (m, 1H), 2.95 (s, 4H), 2.69 (s, 4H), 2.61 (s, 3H), 1.72-1.67 (m, 1H), 1.48 (d, *J* = 6.8 Hz, 3H), 1.19-1.09 (m, 4H), 0.45-0.40 (m, 2H), 0.34-0.31 (m, 2H); ESI-MS (m/z): 470.3 $[M+H]^+$. |
| **Example 100** | (*R*)-3-(1-((1-(4-cyclobutylpiperazin -1-yl)-6-cyclopropyl-7-oxo-6,7-dihydropyrido [3,4-*d*]pyridazin-4-yl)amino)ethyl)-2-methylbenzonitrile | 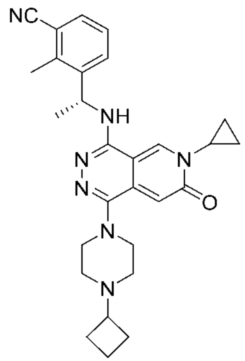 | $^1$H NMR (400 MHz, DMSO-$d_6$) δ 8.97 (s, 1H), 7.73 (d, *J* = 8.0 Hz, 1H), 7.64 (d, *J* = 7.6 Hz, 1H), 7.59 (d, *J* = 6.4 Hz, 1H), 7.35 (t, *J* = 7.6 Hz, 1H), 6.40 (s, 1H), 5.50-5.43 (m, 1H), 3.64-3.58 (m, 1H), 2.98 (s, 4H), 2.81-2.73 (m, 1H), 2.62 (s, 3H), 2.40 (s, 4H), 2.00-1.94 (m, 2H), 1.85-1.75 (m, 2H), 1.67-1.59 (m, 2H), 1.48 (d, *J* = 6.8 Hz, 3H), 1.18-1.08 (m, 4H); ESI-MS (m/z): 484.3 $[M+H]^+$. |

(continued)

| Number | Chemical Name | Structure | $^1$H NMR and MS |
|---|---|---|---|
| **Example 101** | (*R*)-3-(1-((1-(4-cyclopentylpiperazi n-1-yl)-6-cyclopropyl-7-oxo-6,7-dihydropyrido [3,4-*d*]pyridazin-4-yl)amino)ethyl)-2-methylbenzonitrile | 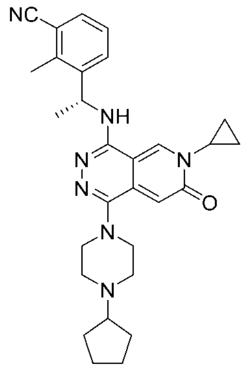 | $^1$H NMR (400 MHz, DMSO-$d_6$) δ 8.96 (s, 1H), 7.73 (d, *J* = 7.6 Hz, 1H), 7.64 (d, *J* = 7.6 Hz, 1H), 7.58 (d, *J* = 6.4 Hz, 1H), 7.35 (t, *J* = 7.6 Hz, 1H), 6.41 (s, 1H), 5.50-5.43 (m, 1H), 3.64-3.58 (m, 1H), 2.99 (s, 4H), 2.62 (s, 3H), 2.56-2.53 (m, 5H), 1.83-1.76 (m, 2H), 1.66-1.58 (m, 2H), 1.55-1.51 (m, 2H), 1.48 (d, *J* = 7.2 Hz, 3H), 1.39-1.32 (m, 2H), 1.19-1.09 (m, 4H); ESI-MS (m/z): 498.3 [M+H]$^+$. |
| **Example 102** | (*R*)-3-(1-((6-cyclopropyl-1-(4-(cyclopropylmethyl) piperazin-1-yl)-7-oxo-6,7-dihydropyrido[3,4-*d*]pyridazin-4-yl)amino)ethyl)-2-methylbenzonitrile | 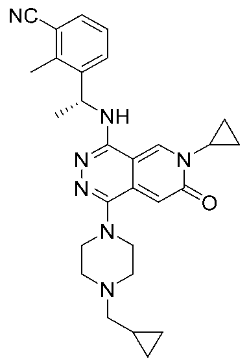 | $^1$H NMR (400 MHz, DMSO-$d_6$) δ 8.97 (s, 1H), 7.73 (d, *J* = 7.6 Hz, 1H), 7.64 (d, *J* = 7.6 Hz, 1H), 7.59 (d, *J* = 6.8 Hz, 1H), 7.35 (t, *J* = 7.6 Hz, 1H), 6.41 (s, 1H), 5.50-5.43 (m, 1H), 3.64-3.59 (m, 1H), 3.01 (s, 4H), 2.62 (s, 3H), 2.60 (s, 4H), 2.24 (d, *J* = 6.4 Hz, 2H), 1.48 (d, *J* = 7.2 Hz, 3H), 1.19-1.09 (m, 4H), 0.90-0.80 (m, 1H), 0.49-0.45 (m, 2H), 0.11-0.08 (m, 2H); ESI-MS (m/z): 484.3 [M+H]$^+$. |
| **Example 103** | (*R*)-6-(3,3-difluorocyclobutyl)-4-((1-(3-(di-fluoromethyl)-2-fluorophenyl)ethyl) ami-no)-1-(4-cyclopropylpiperazi n-1-yl)pyrido [3,4-*d*]pyridazin-7(6*H*)-one | 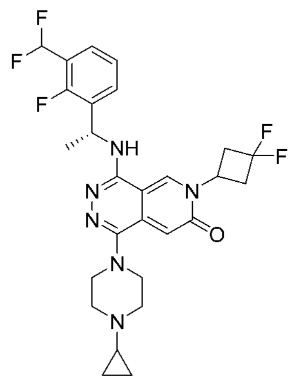 | $^1$H NMR (400 MHz, DMSO-$d_6$) δ 8.97 (s, 1H), 7.64-7.57 (m, 2H), 7.49 (t, *J* = 7.2 Hz, 1H), 7.28 (t, *J* = 7.6 Hz, 1H), 7.24 (t, *J* = 54.4 Hz, 1H), 6.47 (s, 1H), 5.64-5.57 (m, 1H), 4.92-4.87 (m, 1H), 3.29-3.15 (m, 4H), 3.01-2.92 (m, 4H), 2.71-2.66 (m, 4H), 1.72-1.67 (m, 1H), 1.56 (d, *J* = 6.8 Hz, 3H), 0.45-0.40 (m, 2H), 0.34-0.31 (m, 2H); ESI-MS(m/z): 549.0 [M+H]$^+$. |

(continued)

| Number | Chemical Name | Structure | $^1$H NMR and MS |
|---|---|---|---|
| **Example 104** | (*R*)-1-(4-ethylpiperazin-1-yl)-4-((1-(2-methyl-3-(trifluoromethyl)phe nyl)ethyl) amino)-6-(1-methylcyclopropyl) pyrido [3,4-*d*]pyridazin-7(6*H*)-one | 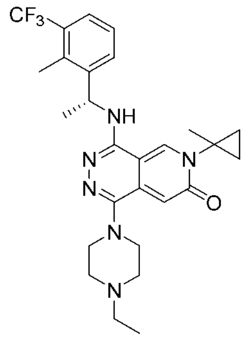 | $^1$H NMR (400MHz,DMSO-$d_6$) δ 9.22 (s, 1H), 7.74 (d, *J* = 7.6 Hz, 1H), 7.58 (d, *J* = 6.8 Hz, 1H), 7.54 (d, *J* = 8.0 Hz, 1H), 7.35 (t, *J* = 8.0 Hz, 1H), 6.38 (s, 1H), 5.61-5.49 (m, 1H), 3.05-2.91 (m, 4H), 2.54-2.52 (m, 7H),2.40-2.35 (m, 2H), 1.54 (s, 3H), 1.48 (d, *J* = 7.2 Hz, 3H), 1.18-1.05 (m, 4H), 1.01 (t, *J* = 7.2 Hz, 3H); ESI-MS(m/z): 515.0 $[M+H]^+$. |
| **Example 105** | (*R*)-4-((1-(3-(difluoromethyl)-2-fluorophenyl)ethyl) amino)-1-(ethylamino)-6-(1-methylcyclopropyl) pyrido[3,4-*d*]pyridazin-7(6*H*)-one | 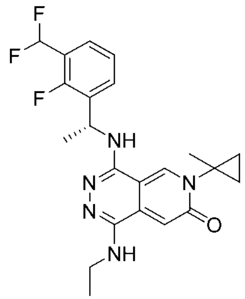 | $^1$H NMR (400 MHz, DMSO-$d_6$) δ 9.03 (s, 1H), 7.60 (t, J = 7.6 Hz, 1H), 7.46 (t, J = 7.2 Hz, 1H), 7.25 (t, J = 7.6 Hz, 1H), 7.24 (t, J = 52.0 Hz, 1H), 6.95 (d, J = 6.8 Hz, 1H), 6.86 (s, 1H), 6.28 (t, J = 5.2 Hz, 1H), 5.50-5.40 (m, 1H), 3.28-3.17 (m, 2H), 1.56-1.49 (m, 6H), 1.20-1.05 (m, 7H); ESI-MS (m/z): 432.0 $[M+H]^+$. |
| **Example 106** | (*R*)-6-(3,3-difluorocyclobutyl)-4-((1-(3-(difluoromethyl)-2-fluorophenyl)ethyl) amino)-1-(4-cyclopentylpiperazi n-1-yl)pyrido [3,4-*d*]pyridazin-7(6*H*)-one | 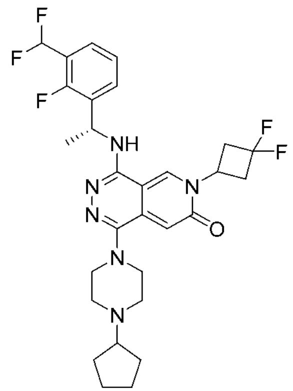 | $^1$H NMR (400 MHz, DMSO-$d_6$) δ 8.96 (s, 1H), 7.63 (t, *J* = 7.6 Hz, 1H), 7.56 (d, *J* = 7.6 Hz, 1H), 7.49 (t, *J* = 7.2 Hz, 1H), 7.28 (t, *J* = 7.6 Hz, 1H), 7.24 (t, *J* = 54.4 Hz, 1H), 6.46 (s, 1H), 5.64-5.58 (m, 1H), 4.92-4.87 (m, 1H), 3.36-3.32 (m, 1H), 3.29-3.15 (m, 4H), 3.04-2.95 (m, 4H), 2.60-2.53 (m, 4H), 1.83-1.75 (m, 2H), 1.63-1.47 (m, 7H), 1.39-1.30 (m, 2H); ESI-MS(m/z): 577.0 $[M+H]^+$. |

(continued)

| Number | Chemical Name | Structure | $^1$H NMR and MS |
|---|---|---|---|
| **Example 107** | (*R*)-6-(3,3-difluorocyclobutyl)-4-((1-(3-(difluoromethyl)-2-fluorophenyl)ethyl) amino)-1-(4-(cyclopropylmethyl) piperazin-1-yl)pyrido[3,4-*d*]pyridazin-7(6*H*)-one | 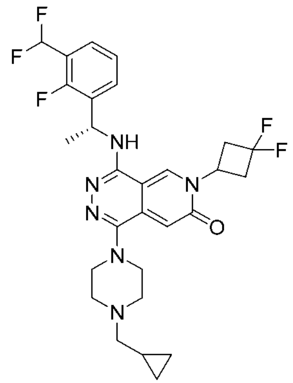 | $^1$H NMR (400 MHz, DMSO-$d_6$) δ 8.96 (s, 1H), 7.63 (t, *J* = 7.6 Hz, 1H), 7.56 (d, *J* = 7.6 Hz, 1H), 7.49 (t, *J* = 7.2 Hz, 1H), 7.28 (t, *J* = 7.6 Hz, 1H), 7.24 (t, *J* = 54.4 Hz, 1H), 6.46 (s, 1H), 5.65-5.58 (m, 1H), 4.94-4.87 (m, 1H), 3.29-3.15 (m, 4H), 3.06-2.99 (m, 4H), 2.67-2.57 (m, 4H), 2.24 (d, *J* = 6.8 Hz, 2H), 1.56 (d, *J* = 7.2 Hz, 3H), 0.88-0.82 (m, 1H), 0.49-0.44 (m, 2H), 0.11-0.07 (m, 2H); ESI-MS(m/z): 563.0 $[M+H]^+$. |
| **Example 108** | (*R*)-6-(3,3-difluorocyclobutyl)-4-((1-(3-(difluoromethyl)-2-fluorophenyl)ethyl) amino)-1-(4-(tert-butyl)piperazin-1-yl)pyrido [3,4-*d*]pyridazin-7(6*H*)-one | 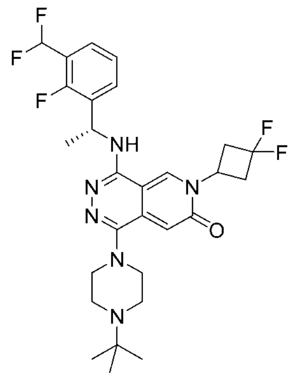 | $^1$H NMR (400 MHz, DMSO-$d_6$) δ 8.96 (s, 1H), 7.63 (t, *J* = 7.6 Hz, 1H), 7.56 *(d, J= 7.6 Hz,* 1H), 7.49 (t, *J* = 7.2 Hz, 1H), 7.29 (t, *J* = 7.6 Hz, 1H), 7.25 (t, *J* = 54.4 Hz, 1H), 6.47 (s, 1H), 5.64-5.57 (m, 1H), 4.94-4.88 (m, 1H), 3.30-3.14 (m, 4H), 3.04-2.95 (m, 4H), 2.69-2.63 (m, 4H), 1.56 (d, *J* = 7.2 Hz, 3H), 1.04 (s, 9H); ESI-MS(m/z): 565.0 $[M+H]^+$. |

(continued)

| Number | Chemical Name | Structure | $^1$H NMR and MS |
|---|---|---|---|
| **Example 109** | (*R*)-4-((1-(3-(difluoromethyl)-2-fluorophenyl)ethyl) amino)-1-(4-ethylpiperazin-1-yl)-6-morpholinopyrido[3 ,4-*d*]pyridazin-7(6*H*)-one | 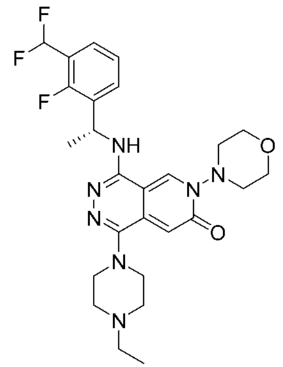 | $^1$H NMR (400 MHz, DMSO-$d_6$) δ 9.26 (s, 1H), 7.65-7.60 (m, 2H), 7.48 (t, *J* = 7.2 Hz, 1H), 7.27 (t, *J* = 7.6 Hz, 1H), 7.24 (t, *J* = 54.4 Hz, 1H), 6.48 (s, 1H), 5.60-5.53 (m, 1H), 3.81-3.75 (m, 4H), 3.67-3.45 (m, 4H), 3.04-2.95 (m, 4H), 2.58-2.51 (m, 4H), 2.41-2.35 (m, 2H), 1.52 (d, *J* = 6.8 Hz, 3H), 1.01 (t, *J* = 7.2 Hz, 3H); ESI-MS(m/z): 532.0 $[M+H]^+$. |
| **Example 110** | (*R*)-4-((1-(3-(difluoromethyl)-2-fluorophenyl)ethyl) amino)-1-(4-methylpiperazin-1-yl)-6-morpholinopyrido[3 ,4-*d*]pyridazin-7(6*H*)-one | 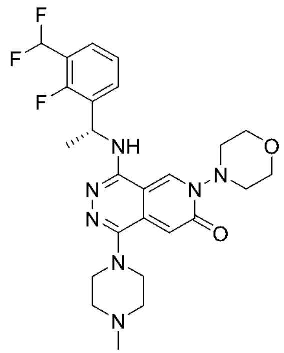 | $^1$H NMR (400 MHz, DMSO-$d_6$) δ 9.30 (s, 1H), 7.75 (s, 1H), 7.64 (t, *J* = 7.2 Hz, 1H), 7.49 (t, *J* = 7.2 Hz, 1H), 7.27 (t, *J* = 7.6 Hz, 1H), 7.24 (t, *J* = 54.4 Hz, 1H), 6.52 (s, 1H), 5.61-5.54 (m, 1H), 3.82-3.76 (m, 4H), 3.62-3.50 (m, 3H), 3.18-3.08 (m, 4H), 2.99-2.74 (m, 4H), 2.58-2.51 (m, 4H), 1.56 (d, *J* = 6.8 Hz, 3H); ESI-MS(m/z): 518.0 $[M+H]^+$. |
| **Example 111** | (*R*)-4-((1-(3-(difluoromethyl)-2-fluorophenyl)ethyl) amino)-1-(4-isopropylpiperazin-1-yl)-6-morpholinopyrido[3 ,4-*d*]pyridazin-7(6*H*)-one | 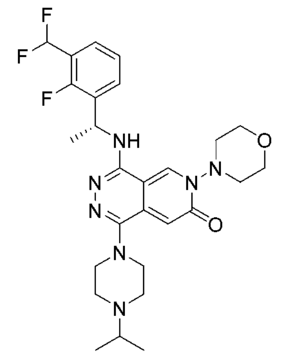 | $^1$H NMR (400 MHz, DMSO-$d_6$) δ 9.33 (s, 1H), 7.76 (s, 1H), 7.65 (t, *J* = 7.6 Hz, 1H), 7.48 (t, *J* = 7.2 Hz, 1H), 7.27 (t, *J* = 7.6 Hz, 1H), 7.24 (t, *J* = 54.4 Hz, 1H), 6.57 (s, 1H), 5.62-5.55 (m, 1H), 3.82-3.75 (m, 4H), 3.65-3.45 (m, 4H), 3.26-2.85 (m, 9H), 1.53 (d, *J* = 6.8 Hz, 3H), 1.24-1.05 (m, 6H); ESI-MS(m/z): 546.0 $[M+H]^+$. |

(continued)

| Number | Chemical Name | Structure | $^1$H NMR and MS |
|---|---|---|---|
| **Example 112** | (*R*)-6-cyclobutyl-4-((1-(3-(difluoromethyl)-2-fluorophenyl)ethyl) amino)-1-(4-ethylpiperazin-1-yl)pyrido[3,4-*d*]pyridazin-7(6*H*)-one | 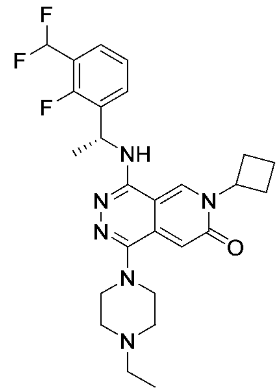 | $^1$H NMR (400 MHz, DMSO-$d_6$) δ 9.07 (s, 1H), 7.63 (t, *J* = 6.4 Hz, 2H), 7.49 (t, *J* = 7.2 Hz, 1H), 7.28 (t, *J* = 7.6 Hz, 1H), 7.25 (t, *J* = 56.0 Hz, 1H), 6.41 (s, 1H), 5.65-5.56 (m, 1H), 5.23-5.11 (m, 1H), 3.09-2.91 (m, 4H), 2.49-2.42 (m, 8H), 2.41-2.35 (m, 2H), 1.92-1.84 (m, 2H), 1.56 (d, *J* = 7.2 Hz, 3H), 1.01 (t, *J* = 7.2 Hz, 3H); ESI-MS (m/z): 501.0 $[M+H]^+$. |
| **Example 113** | (*R*)-6-cyclobutyl-4-((1-(3-(difluoromethyl)-2-fluorophenyl)ethyl) amino)-1-(4-isopropylpiperazin-1-yl)pyrido[3,4-*d*]pyridazin-7(6*H*)-one | 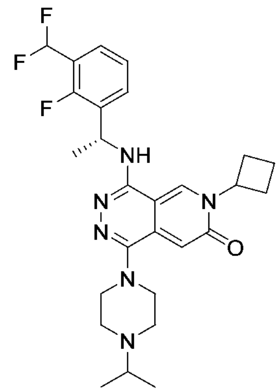 | $^1$H NMR (400 MHz, DMSO-$d_6$) δ 9.07 (s, 1H), 7.62 (d, *J* = 6.8 Hz, 2H), 7.49 (t, *J* = 7.2 Hz, 1H), 7.28 (t, *J* = 7.6 Hz, 1H), 7.25 (t, *J* = 56.0 Hz, 1H), 6.42 (s, 1H), 5.65-5.56 (m, 1H), 5.23-5.12 (m, 1H), 3.05-2.90 (m, 4H), 2.70-2.64 (m, 1H), 2.62-2.54 (m, 4H), 2.48-2.41 (m, 4H), 1.92-1.83 (m, 2H), 1.56 (d, *J* = 7.2 Hz, 3H), 1.00 (d, *J* = 6.4 Hz, 6H); ESI-MS (m/z): 515.0 $[M+H]^+$. |

(continued)

| Number | Chemical Name | Structure | $^1H$ NMR and MS |
|---|---|---|---|
| **Example 114** | (*R*)-4-((1-(3-(difluoromethyl)-2-fluorophenyl)ethyl) amino)-6-isopropyl-1-(4-methylpiperazin-1-yl)pyrido[3,4-*d*]pyridazin-7(6*H*)-one formate | 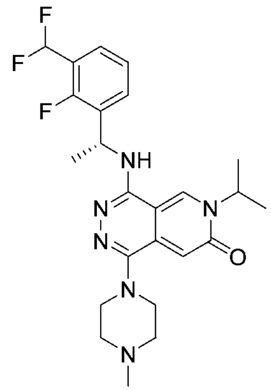 | The crude product was purified by Prep-HPLC (separation method 2) to obtain (R)-4-((1-(3-(difluoromethyl)-2-fluorophenyl)ethyl)amino)-6-isopropyl-1-(4-methylpiperazin-1-yl)pyrido[3,4-*d*]pyridazin-7(6*H*)-one formate; $^1H$ NMR (400 MHz, DMSO-$d_6$) δ 12.74 (s, 1H), 9.14 (s, 1H), 8.14 (s, 1H), 7.75-7.58 (m, 2H), 7.48 (t, *J* = 7.2 Hz, 1H), 7.28 (t, *J* = 7.6 Hz, 1H), 7.24 (t, *J* = 54.4 Hz, 1H), 6.47 (s, 1H), 5.59 (s, 1H), 5.27 (p, *J* = 6.8 Hz, 1H), 3.10-3.00 (m, 4H), 2.79-2.61 (m, 4H), 2.36 (s, 3H), 1.56 (d, *J* = 6.4 Hz, 3H), 1.48 (d, *J* = 6.4 Hz, 6H); ESI-MS (m/z): 475.2 $[M+H]^+$. |
| **Example 115** | (*R*)-4-((1-(3-(difluoromethyl)-2-fluorophenyl)ethyl) amino)-6-(1-methylcyclopropyl)-1-(oxetan-3-ylamino)pyrido[3,4-*d*]pyridazin-7(6*H*)-one | 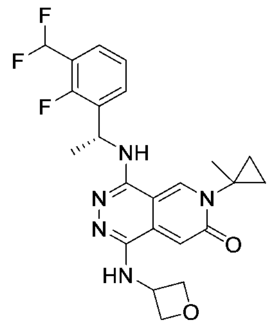 | $^1H$ NMR (400MHz,DMSO-$d_6$) δ 9.02 (s, 1H), 7.68 (t, *J* = 7.2 Hz, 1H), 7.53-7.45 (m, 1H), 7.41-7.07 (m, 3H), 6.92 (s, 1H), 5.28-5.11 (m, 1H), 5.00-4.84 (m, 1H), 4.19-4.06 (m, 1H), 3.96- 3.77 (m, 1H), 3.67-3.32 (m, 3H), 1.57-1.49 (m, 6H), 1.19-1.03 (m, 4H); ESI-MS(m/z): 460.0 $[M+H]^+$. |
| **Example 116** | (*R*)-4-((1-(3-(difluoromethyl)-2-fluorophenyl)ethyl) amino)-6-ethyl-1-(4-methylpiperazin-1-yl)pyrido[3,4-*d*]pyridazin-7(6*H*)-one | 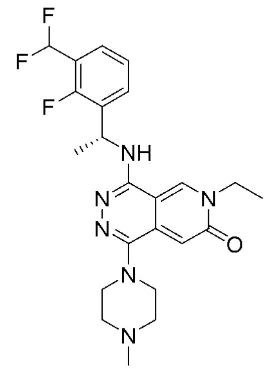 | $^1H$ NMR (400 MHz, DMSO-$d_6$) δ 9.30 (s, 1H), 7.64 (t, *J* = 7.6 Hz, 1H), 7.52-7.41 (m, 2H), 7.27 (t, *J* = 7.6 Hz, 1H), 7.24 (t, *J* = 56.0 Hz, 1H), 6.46 (s, 1H), 5.62-5.52 (m, 1H), 4.22-4.09 (m, 2H), 3.16-2.97 (m, 4H), 2.93-2.53 (m, 4H), 2.39 (s, 3H), 1.54 (d, *J* = 7.2 Hz, 3H), 1.38 (t, *J* = 7.2 Hz, 3H); ESI-MS (m/z): 461.0 $[M+H]^+$. |

(continued)

| Number | Chemical Name | Structure | $^1$H NMR and MS |
|---|---|---|---|
| **Example 117** | (*R*)-4-((1-(3-(difluoromethyl)-2-fluorophenyl)ethyl) amino)-6-ethyl-1-(4-ethylpiperazin-1-yl)pyrido[3,4-*d*]pyridazin-7(6*H*)-one | 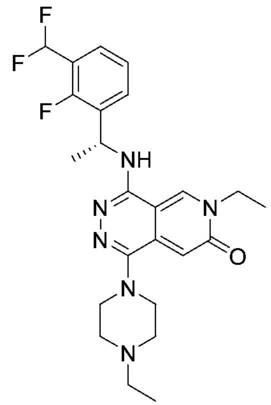 | $^1$H NMR (400 MHz, DMSO-$d_6$) δ 9.29 (s, 1H), 7.64 (t, *J* = 7.6 Hz, 1H), 7.52-7.39 (m, 2H), 7.27 (t, *J* = 7.6 Hz, 1H), 7.24 (t, *J* = 56.0 Hz, 1H), 6.46 (s, 1H), 5.62-5.52 (m, 1H), 4.20-4.12 (m, 2H), 3.16-2.92 (m, 4H), 2.53-2.50 (m, 6H), 1.54 (d, *J* = 7.2 Hz, 3H), 1.38 (t, *J* = 7.2 Hz, 3H), 1.08 (s, 3H); ESI-MS (m/z): 475.0 $[M+H]^+$. |
| **Example 118** | (*R*)-4-((1-(3-(difluoromethyl)-2-fluorophenyl)ethyl) amino)-6-ethyl-1-(4-isopropylpiperazin-1-yl)pyrido[3,4-*d*]pyridazin-7(6*H*)-one | 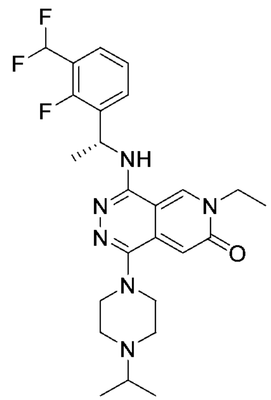 | $^1$H NMR (400 MHz, DMSO-$d_6$) δ 9.33 (s, 1H), 7.65 (t, *J* = 7.6 Hz, 1H), 7.57-7.41 (m, 2H), 7.28 (t, *J* = 7.6 Hz, 1H), 7.24 (t, *J* = 56.0 Hz, 1H), 6.49 (s, 1H), 5.64-5.52 (m, 1H), 4.22-4.09 (m, 2H), 3.20-2.85 (m, 4H), 2.76-2.50 (m, 5H), 1.54 (d, *J* = 7.2 Hz, 3H), 1.38 (t, *J* = 7.2 Hz, 3H), 1.31-0.92 (m, 6H); ESI-MS (m/z): 489.0 $[M+H]^+$. |

(continued)

| Number | Chemical Name | Structure | $^1$H NMR and MS |
|---|---|---|---|
| **Example 119** | 4-(((*R*)-1-(3-(difluoromethyl)-2-fluorophenyl)ethyl) amino)-6-(1-methylcyclopropyl)-1-((tetrahydrofuran-3-yl)amino)pyrido [3,4 -*d*]pyridazin-7(6*H*)-one formate | 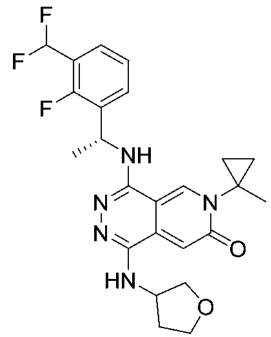 | The crude product was purified by Prep-HPLC (separation method 2), to obtain 4-(((*R*)-1-(3-(difluoromethyl)-2-fluorophenyl)ethyl)amino)-6-(1-methylcyclopropyl)-1-((tetrahydrofuran-3-yl)amino)pyrido[3,4-*d*]pyridazin-7(6*H*)-one formate; $^1$H NMR (400MHz,DMSO-d6) δ 12.74 (s, 1H), 10.08 (s, 1H), 9.37-9.26 (m, 1H), 8.11-7.92 (m, 1H), 7.74-7.66 (m, 1H), 7.56-7.47 (m, 1H), 7.45-7.08 (m, 4H), 5.37-5.23 (m, 1H), 4.50-3.91 (m, 2H), 3.85-3.51 (m, 3H), 2.08-1.95 (m, 1H), 1.87-1.78 (m, 1H), 1.61-1.51 (m, 6H), 1.21-1.07 (m, 4H); ESI-MS(m/z): 474.0 $[M+H]^+$. |
| **Example 120** | (*R*)-4-((1-(3-(difluoromethyl)-2-fluorophenyl)ethyl) amino)-1-(4-cyclopentylpiperazin-1-yl)-6-morpholinopyrido[3 ,4-*d*]pyridazin-7(6*H*)-one | 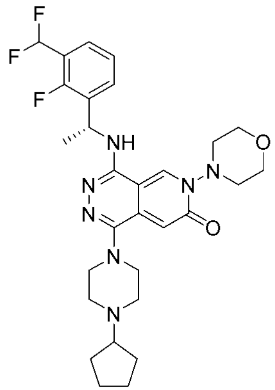 | $^1$H NMR (400 MHz, DMSO-$d_6$) δ 9.29 (s, 1H), 7.69 (s, 1H), 7.64 (t, *J* = 7.6 Hz, 1H), 7.48 (t, *J* = 7.2 Hz, 1H), 7.27 (t, *J* = 7.6 Hz, 1H), 7.24 (t, *J* = 54.4 Hz, 1H), 6.52 (s, 1H), 5.61-5.54 (m, 1H), 3.84-3.72 (m, 4H), 3.65-3.43 (m, 4H), 3.15-2.93 (m, 4H), 2.70-2.53 (m, 3H), 2.48-2.32 (m, 2H), 1.93-1.78 (m, 2H), 1.70-1.60 (m, 2H), 1.58-1.46 (m, 6H), 1.44-1.32 (m, 1H); ESI-MS(m/z): 572.0 $[M+H]^+$. |
| **Example 121** | (*R*)-4-((1-(3-(difluoromethyl)-2-fluorophenyl)ethyl) amino)-1-(4-isopropylpiperidin-1-yl)-6-(1-methylcyclopropyl) pyrido [3,4-*d*]pyridazin-7(6*H*)-one | 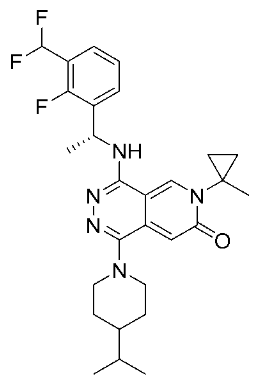 | $^1$H NMR (400 MHz, DMSO-$d_6$) δ 9.26 (s, 1H), 7.66-7.61 (m, 2H), 7.49 (t, *J* = 7.2 Hz, 1H), 7.28 (t, *J* = 7.6 Hz, 1H), 7.28 (t, *J* = 54.4 Hz, 1H), 6.40 (s, 1H), 5.60-5.54 (m, 1H), 3.44-3.38 (m, 3H), 2.69-2.56 (m, 3H), 2.43 (s, 6H), 1.94-1.91 (m, 2H), 1.71-1.62 (m, 2H), 1.56-1.53 (m, 6H), 1.22-1.08 (m, 4H); ESI-MS (m/z): 514.3 $[M+H]^+$. |

(continued)

| Number | Chemical Name | Structure | $^1$H NMR and MS |
|---|---|---|---|
| **Example 122** | (*R*)-4-((1-(3-(difluoromethyl)-2-fluorophenyl)ethyl) amino)-1-(ethyl(methyl)amino)-6-(1-methylcyclopropyl) pyrido[3,4-*d*] pyridazin-7(6*H*)-one | 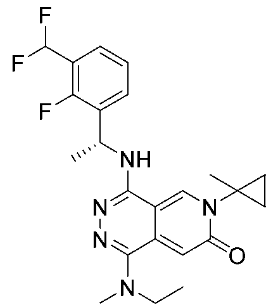 | $^1$H NMR (400MHz,DMSO-$d_6$) δ 9.20 (s, 1H), 7.63 (t, J = 7.2 Hz, 1H), 7.52-7.45 (m, 2H), 7.39-7.09 (m, 2H), 6.40 (s, 1H), 5.63-5.50 (m, 1H), 3.00 (dd, J = 7.2 Hz, 2H), 2.66 (s, 3H), 1.58-1.51 (m, 6H), 1.21-1.04 (m, 7H); ESI-MS(m/z): 446.0 [M+H]+. |
| **Example 123** | (*R*)-4-((1-(3-(difluoromethyl)-2-fluorophenyl)ethyl) amino)-6-(1-methylcyclopropyl)-1-((1-methylpiperidin-4-yl)amino)pyrido[3,4 -*d*]pyridazin-7(6*H*)-one formate | 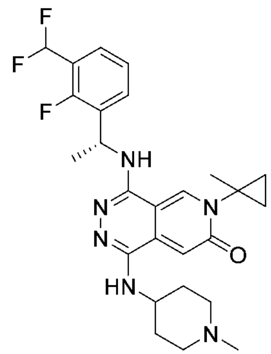 | The crude product was purified by Prep-HPLC (separation method 2), to obtain (*R*)-4-((1-(3-(difluoromethyl)-2-fluorophenyl)ethyl)amino)-6-(1-methylcyclopropyl)-1-((1-methylpiperidin-4-yl)amino)pyrido[3,4-d]pyridazin-7(6*H*)-one formate; $^1$H NMR (400 MHz, DMSO-$d_6$) δ 12.73 (s, 1H), 10.40 (s, 1H), 9.27 (s, 1H), 8.14 (s, 1H), 7.66 (t, *J* = 7.6 Hz, 1H), 7.51 (s, 1H), 7.40-7.04 (m, 3H), 5.34-5.48 (m, 1H), 3.89 (s, 1H), 3.45-3.34 (m, 2H), 3.15-2.78 (m, 2H), 2.69 (s, 3H), 2.14-1.72 (m, 4H), 1.67-1.48 (m, 6H), 1.24-1.04 (m, 4H); ESI-MS(m/z): 501.2 $[M+H]^+$ |
| **Example 124** | (*R*)-4-((1-(3-(difluoromethyl)-2-fluorophenyl)ethyl) amino)-1-((2-(dimethylamino)ethyl)(methyl)amino)-6-(1-methylcyclopropyl) pyrido[3,4-*d*]pyridazin-7(6*H*)-one formate | 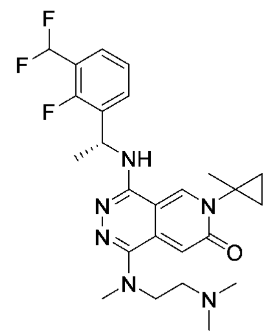 | The crude product was purified by Prep-HPLC (separation method 2), to obtain (R)-4-((1-(3-(difluoromethyl)-2-fluorophenyl)ethyl)amino)-1-((2-(dimethylamino) ethyl)(methyl)amino) -6-(1-methylcyclopropyl)pyrido[3,4-*d*]pyridazin-7(6*H*)-one formate; $^1$H NMR (400 MHz, DMSO-$d_6$) δ 13.20 (s, 1H), 10.62-9.85 (m, 2H), 8.14 (s, 1H), 7.78 (t, *J* = 7.6 Hz, 1H), 7.58 (d, *J* = 8.4 Hz, 1H), 7.34 (t, *J* = 7.6 Hz, 1H), 7.25 (t, *J* = 54.4 Hz, 1H), 6.89 (s, 1H), 5.55 (p, *J* = 7.2 Hz, 1H), 3.55-3.42 (m, 2H), 3.30 (s, 3H), 2.83 (s, 2H), 2.74 (s, 6H), 1.70 (s, 3H), 1.55 (s, 3H), 1.31-1.15 (m, 2H), 1.14-1.04 (m, 2H); ESI-MS(m/z): 489.2 $[M+H]^{+.}$ |

(continued)

| Number | Chemical Name | Structure | $^1$H NMR and MS |
|---|---|---|---|
| **Example 125** | (*R*)-1-(bicyclo[1.1.1]penta n-1-ylamino)-4-((1-(3-(difluoromethyl)-2-fluorophenyl)ethyl) amino)-6-(1-methylcyclopropyl) pyrido[3,4-*d*]pyridazin-7(6*H*)-one | 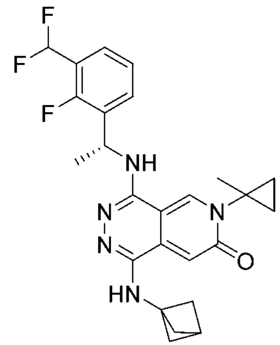 | $^1$H NMR (400 MHz, DMSO-$d_6$) δ 9.05 (s, 1H), 7.62 (t, *J* = 7.2 Hz, 1H), 7.48 (t, *J* = 7.2 Hz, 1H), 7.27 (t, *J* = 7.6 Hz, 1H), 7.24 (t, *J* = 54.0 Hz, 1H), 7.02-6.80 (m, 3H), 5.52 (p, *J* = 7.2 Hz, 1H), 2.40 (s, 1H), 2.05-1.98 (m, 6H), 1.56-1.48 (m, 6H), 1.19-1.03 (m, 4H); ESI-MS(m/z): 470.2 $[M+H]^+$. |
| **Example 126** | 4-(((*R*)-1-(3-(difluoromethyl)-2-fluorophenyl)ethyl) amino)-6-(1-methylcyclopropyl)-1-((1-methylpyrrolidin-3-yl)amino)pyrido[3,4 *d*]pyridazin-7(6*H*)-one | 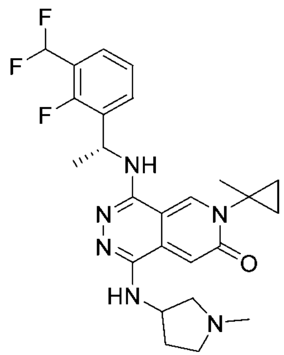 | $^1$H NMR (400MHz,DMSO-$d_6$) δ 9.06 (s, 1H), 7.61 (t, *J* = 7.2 Hz, 1H), 7.47 (t, *J* = 6.8 Hz, 1H), 7.38-7.09 (m, 2H), 7.03 (d, *J* = 6.0 Hz, 1H), 6.97 (s, 1H), 6.41-6.24 (m, 1H), 5.53-5.42 (m, 1H), 4.38-4.25 (m, 1H), 2.94-2.52 (m, 4H), 2.35 (s, 3H), 2.21-2.13 (m, 1H), 1.82-1.70 (m, 1H), 1.55-1.50 (m, 6H), 1.20-1.04 (m, 4H); ESI-MS(m/z): 487.0 $[M+H]^+$. |
| **Example 127** | 4-(((*R*)-1-(3-(difluoromethyl)-2-fluorophenyl)ethyl) amino)-1-(4-methylpiperazin-1-yl)-6-(tetrahydrofuran-3-yl)pyrido[3,4-*d*] pyridazin-7(6*H*)-one | 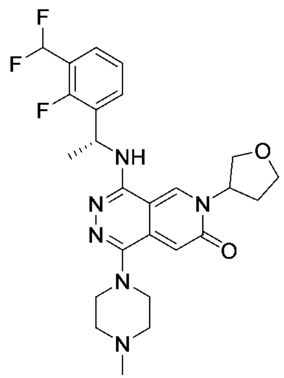 | $^1$H NMR (400 MHz, DMSO-$d_6$) δ 8.91 (d, *J* = 6.8 Hz, 1H), 7.65-7.60 (m, 2H), 7.48 (t, *J* = 7.2 Hz, 1H), 7.27 (t, *J* = 7.6 Hz, 1H), 7.24 (t, *J* = 54.4 Hz, 1H), 6.47 (s, 1H), 5.60-5.47 (m, 2H), 4.21-4.13 (m, 1H), 4.06-3.94 (m, 2H), 3.87-3.82 (m, 1H), 3.20-3.13 (m, 1H), 3.02-2.97 (m, 4H), 2.48-2.44 (m, 3H), 2.35-2.23 (m, 5H), 1.55 (d, *J* = 7.2 Hz, 3H); ESI-MS(m/z): 503.0 $[M+H]^+$. |

(continued)

| Number | Chemical Name | Structure | $^1$H NMR and MS |
|---|---|---|---|
| **Example 128** | 4-(((*R*)-1-(3-(difluoromethyl)-2-fluorophenyl)ethyl) amino)-1-(3-(hydroxymethyl)pyrrolidin-1-yl)-6-(1-methylcyclopropyl) pyrido [3,4-*d*]pyridazin-7(6*H*)-one | 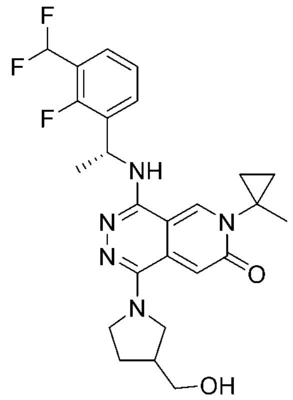 | $^1$H NMR (400 MHz, DMSO-$d_6$) δ 9.11 (s, 1H), 7.62 (t, *J* = 7.2 Hz, 1H), 7.48 (t, *J* = 6.8 Hz, 1H), 7.38-7.10 (m, 3H), 6.63 (s, 1H), 5.55-5.48 (m, 1H), 4.64-4.60 (m, 1H), 3.46-3.34 (m, 5H), 3.30-3.24 (m, 1H), 2.33-2.25 (m, 1H), 1.94-1.86 (m, 1H), 1.62-1.52 (m, 7H), 1.22-1.07 (m, 4H); ESI-MS (m/z): 488.2 $[M+H]^+$. |
| **Example 129** | (*R*)-1-((dicyclopropylmeth yl)amino)-4-((1-(3-(difluoromethyl)-2-fluorophenyl)ethyl) amino)-6-(1-methylcyclopropyl) pyrido[3,4-*d*]pyridazin-7(6*H*)-one | 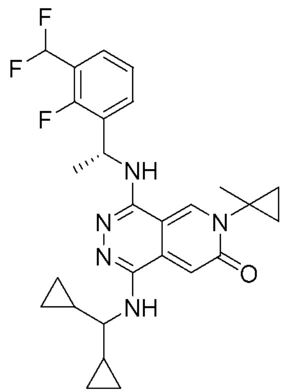 | $^1$H NMR (400 MHz, DMSO-$d_6$) δ 9.01 (s, 1H), 7.60 (t, *J* = 7.6 Hz, 1H), 7.46 (t, *J* = 7.2 Hz, 1H), 7.26 (d, *J* = 7.6 Hz, 1H), 7.23 (t, *J* =54.4 Hz, 1H), 7.03 (s, 1H), 6.92 (d, *J* = 6.8 Hz, 1H), 6.11 (d, *J* = 8.4 Hz, 1H), 5.43 (p, *J* = 6.8 Hz, 1H), 3.36 (q, *J* = 7.6 Hz, 1H), 1.54 (s, 3H), 1.50 (d, *J* = 7.2 Hz, 3H), 1.20-1.12 (m, 2H), 1.08-0.98 (m, 4H), 0.40-0.34 (m, 2H), 0.30-0.15 (m, 6H); ESI-MS(m/z): 498.2 $[M+H]^+$. |

(continued)

| Number | Chemical Name | Structure | $^1$H NMR and MS |
|---|---|---|---|
| **Example 130** | (*R*)-4-((1-(3-(difluoromethyl)-2-fluorophenyl)ethyl) amino)-1-(3-methylazetidin-1-yl)-6-(1-methylcyclopropyl) pyrido[3,4-*d*] pyridazin-7(6*H*)-one | 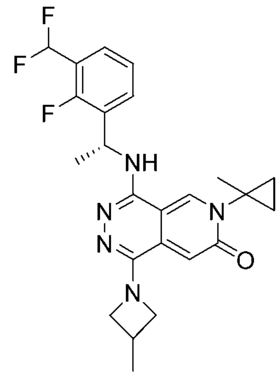 | $^1$H NMR (400MHz,DMSO-$d_6$) δ 8.64 (s, 1H), 7.67-7.56 (m, 1H), 7.46 (t, *J* = 6.8 Hz, 1H), 7.35-7.08 (m, 2H), 6.87 (s, 1H), 6.79-6.66 (m, 1H), 5.17-5.06 (m, 1H), 3.64-3.36 (m, 2H), 3.27-2.67 (m, 2H), 1.97-1.70 (m, 1H), 1.56-1.47 (m, 6H),1.17-0.83 (m, 7H); ESI-MS(m/z): 458.0 [M+H]+. |
| **Example 131** | (*R*)-4-((1-(3-(difluoromethyl)-2-fluorophenyl)ethyl) amino)-1-(3-(hydroxymethyl)azetidin-1-yl)-6-(1-methylcyclopropyl) pyrido [3,4-*d*]pyridazin-7(6*H*)-one | 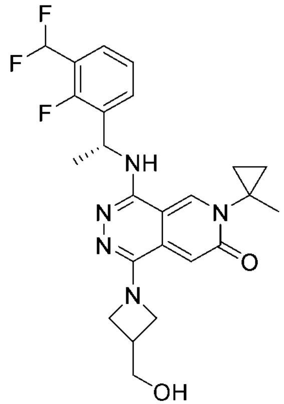 | $^1$H NMR (400 MHz, DMSO-$d_6$) δ 8.62 (s, 1H), 7.62 (t, *J* = 7.6 Hz, 1H), 7.47 (t, *J* = 6.8 Hz, 1H), 7.29 (t, *J* = 7.6 Hz, 1H), 7.22 (t, *J* = 54.4 Hz, 1H), 6.85 (s, 1H), 6.66-6.64 (m, 1H), 5.16-5.10 (m, 1H), 4.62-4.57 (m, 1H), 3.44-3.33 (m, 3H), 3.29-3.00 (m, 3H), 1.91-1.82 (m, 1H), 1.51-1.49 (m, 6H), 1.14-1.00 (m, 4H); ESI-MS (m/z): 474.2 $[M+H]^+$. |
| **Example 132** | (*R*)-3-(1-((1-(4-isopropylpiperazin-1-yl)-6-(1-methylpiperidin-4-yl)-7-oxo-6,7-dihydropyrido[3,4-*d*]pyridazin-4-yl)amino) ethyl)-2-methylbenzonitrile | 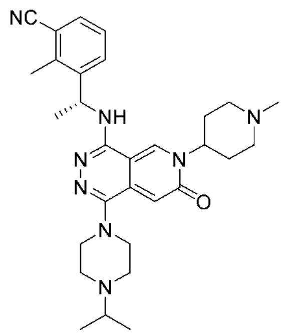 | $^1$H NMR (400 MHz, DMSO-$d_6$) δ 9.11 (s, 1H), 7.72 (d, *J* = 8.0 Hz, 1H), 7.66-7.63 (m, 2H), 7.37 (t, *J* = 8.0 Hz, 1H), 6.46 (s, 1H), 5.51-5.44 (m, 1H), 4.95-4.89 (m, 1H), 2.99 (s, 6H), 2.69-2.67 (m, 1H), 2.63-2.58 (m, 7H), 2.26 (s, 3H), 2.16-2.07 (m, 4H), 1.82-1.80 (m, 2H), 1.49 (d, *J* = 6.8 Hz, 3H), 1.01 (d, *J* = 6.4 Hz, 6H); ESI-MS (m/z): 529.3 $[M+H]^+$. |

(continued)

| Number | Chemical Name | Structure | $^1$H NMR and MS |
|---|---|---|---|
| **Example 133** | 4-(((*R*)-1-(3-(difluoromethyl)-2-fluorophenyl)ethyl) amino)-6-(1-methylcyclopropyl)-1-(quinuclidin-3-ylamino)pyrido[3,4-*d*] pyridazin-7(6*H*)-one | 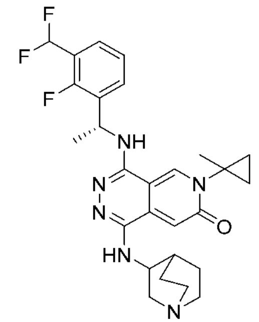 | $^1$H NMR (400MHz,DMSO-$d_6$) δ 9.03 (s, 1H), 7.58 (t, *J* = 7.2 Hz, 1H), 7.46 (t, *J* = 6.8 Hz, 1H), 7.38-7.08 (m, 3H), 6.98 (d, *J* = 6.8 Hz, 1H), 6.10-6.01 (m, 1H), 5.51-5.40 (m, 1H), 3.97-3.81 (m, 1H), 3.12-3.02 (m, 1H), 2.96-2.82 (m, 1H), 2.77-2.59 (m, 4H), 2.03-1.95 (m, 1H), 1.85-1.67 (m, 1H), 1.58-1.49 (m, 8H), 1.25-1.05 (m, 5H); ESI-MS(m/z): 513.0 [M+H]$^+$. |
| **Example 134** | (*R*)-1-((2,2-difluoroethyl)amino )-4-((1-(3-(difluoromethyl)-2-fluorophenyl)ethyl) amino)-6-(1-methylcyclopropyl) pyrido[3,4-*d*]pyridazin-7(6*H*)-one | 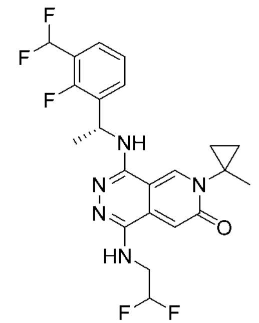 | $^1$H NMR (400 MHz, DMSO-$d_6$) δ 9.11 (s, 1H), 7.61 (t, *J* = 7.6 Hz, 1H), 7.48 (t, *J* = 7.2 Hz, 1H), 7.27 (t, *J* = 7.6 Hz, 1H), 7.24 (t, *J* = 54.4 Hz, 1H), 7.13 (s, 1H), 6.87 (s, 2H), 6.24-5.94 (m, 1H), 5.52-5.45 (m, 1H), 3.73-2.55 (m, 2H), 1.58-1.52 (m, 6H), 1.19-1.06 (m, 4H); ESI-MS (m/z): 468.2 [M+H]$^+$. |
| **Example 135** | (*R*)-2-methyl-3-(1-((1-(4-methylpiperazin-1-yl)-6-(1-methylpiperidin-4-yl)-7-oxo-6,7-dihydropyrido[3,4-*d*]pyridazin-4-yl)amino)ethyl)benz onitrile formate | 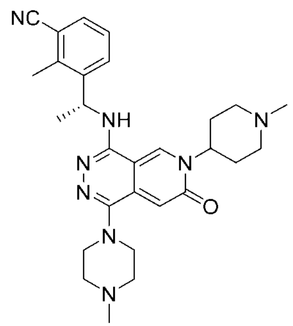 | The crude product was purified by Prep-HPLC (separation method 2), to obtain (*R*)-2-methyl-3-(1-((1-(4-methylpiperazin-1-yl)-6-(1-methylpiperidin-4-yl)-7-oxo-6,7-dihydropyrido[3,4-*d*]pyridazin-4-yl)amino)ethyl)benzonitrile formate; $^1$H NMR (400 MHz, DMSO-$d_6$) δ 9.12 (s, 1H), 8.20 (s, 1H), 7.73-7.63 (m, 3H), 7.37 (t, *J* = 8.0 Hz, 1H), 6.45 (s, 1H), 5.50-5.44 (m, 1H), 4.95-4.89 (m, 1H), 3.01-2.98 (m, 6H), 2.63 (s, 3H), 2.48 (s, 4H), 2.26 (s, 3H), 2.23 (m, 3H), 2.17-2.08 (m, 4H), 1.82-1.80 (m, 2H), 1.49 (d, *J* = 6.8 Hz, 3H); ESI-MS (m/z): 501.3 [M+H]$^+$. |

(continued)

| Number | Chemical Name | Structure | $^1$H NMR and MS |
|---|---|---|---|
| **Example 136** | 4-(((*R*)-1-(3-(difluoromethyl)-2-fluorophenyl)ethyl) amino)-6-(hexahydrocyclopen ta [c]pyrrol-2(1*H*)-yl)-1-(4-methylpiperazin-1-yl)pyrido[3,4-*d*]pyridazin-7(6*H*)-one formate | 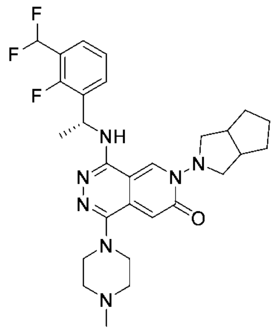 | The crude product was purified by Prep-HPLC (separation method 2), to obtain 4-(((*R*)-1-(3-(difluoromethyl)-2-fluorophenyl)ethyl)amino)-6-(hexahydrocyclopenta[c]pyrrol-2(1*H*)-yl)-1-(4-methylpiperazin-1-yl)pyrido[3,4-*d*]pyridazin-7(6*H*)-one formate; $^1$H NMR (400 MHz, DMSO-$d_6$) δ 12.52 (s, 1H), 9.39 (s, 1H), 8.14 (s, 1H), 7.68 (t, *J* = 7.2 Hz, 1H), 7.53 (t, *J* = 7.2 Hz, 1H), 7.30 (t, *J* = 7.6 Hz, 1H), 7.24 (t, *J* = 54.4 Hz, 1H), 6.93 (s, 1H), 5.42-5.35 (m, 1H), 5.10-5.03 (m, 1H), 4.37-4.33 (m, 1H), 3.82-3.74 (m, 2H), 3.60-3.51 (m, 1H), 3.40-3.35 (m, 1H), 3.31 (s, 6H), 2.10-1.80 (m, 3H), 1.61-1.53 (m, 7H), 1.27-1.06 (m, 5H); ESI-MS(m/z): 542.0 [M+H]$^+$. |
| **Example 137** | 4-(((*R*)-1-(3-(difluoromethyl)-2-fluorophenyl)ethyl) amino)-1-((R)-3-hydroxypyrrolidin-1-yl)-6-(1-methylcyclopropyl) pyrido [3,4-*d*]pyridazin-7(6*H*)-one formate | 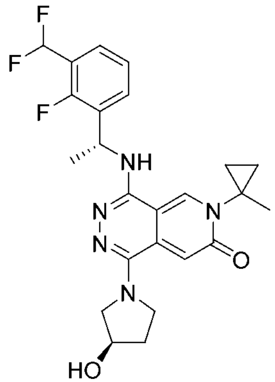 | The crude product was purified by Prep-HPLC (separation method 2), to obtain 4-(((*R*)-1-(3-(difluoromethyl)-2-fluorophenyl)ethyl)amino)-1-((R)-3-hydroxypyrrolidin-1-yl)-6-(1-methylcyclopropyl)pyrido[3,4-*d*]pyridazin-7(6*H*)-one formate; $^1$H NMR (400 MHz, DMSO-$d_6$) δ 10.27 (s, 1H), 9.36 (s, 1H), 7.81 (s, 1H), 7.65 (t, *J* = 7.2 Hz, 1H), 7.50 (d, *J* = 7.2 Hz, 1H), 7.29 (t, *J* = 7.6 Hz, 1H), 7.25 (t, *J* = 54.4 Hz, 1H), 6.55 (s, 1H), 5.62-5.55 (m, 1H), 3.71-3.66 (m, 2H), 3.29-3.26 (m, 3H), 2.78-2.71 (m, 5H), 1.84-1.70 (m, 3H), 1.58-1.53 (m, 5H); ESI-MS (m/z): 474.2 [M+H]$^+$. |

(continued)

| Number | Chemical Name | Structure | $^1$H NMR and MS |
|---|---|---|---|
| **Example 138** | (*R*)-4-((1-(3-(difluoromethyl)-2-fluorophenyl)ethyl) amino)-6-methyl-1-(4-methylpiperazin-1-yl)pyrido[3,4-*d*]pyridazin-7(6*H*)-one formate | 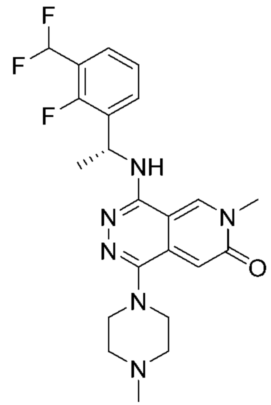 | The crude product was purified by Prep-HPLC (separation method 2) to obtain (*R*)-4-((1-(3-(difluoromethyl)-2-fluorophenyl)ethyl)amino)-6-methyl-1-(4-methylpiperazin-1-yl)pyrido[3,4-*d*]pyridazin-7(6*H*)-one formate; $^1$H NMR (400 MHz, DMSO-$d_6$) δ 12.71 (s, 1H), 9.37 (s, 1H), 8.14 (s, 1H), 7.69-7.60 (m, 1H), 7.48 (t, *J* = 7.2 Hz, 2H), 7.30-7.24 (m, 1H), 7.24 (t, *J* = 54.4 Hz, 1H), 6.46 (s, 1H), 5.63-5.52 (m, 1H), 3.69 (s, 3H), 3.16-3.05 (m, 4H), 2.89-2.71 (m, 4H), 2.45 (s, 3H), 1.53 (d, *J* = 9.2 Hz, 3H); ESI-MS (m/z): 447.2 $[M+H]^+$. |
| **Example 139** | 4-(((*R*)-1-(3-(difluoromethyl)-2-fluorophenyl)ethyl) amino)-1-((R)-2-(hydroxymethyl) pyr rolidin-1-yl)-6-(1-methylcyclopropyl) pyrido[3,4-*d*]pyridazin-7(6*H*)-one | 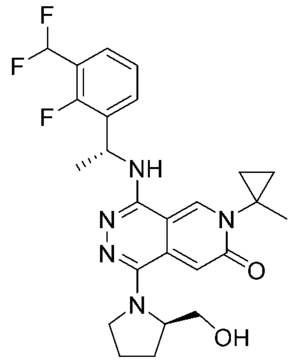 | $^1$H NMR (400 MHz, DMSO-$d_6$) δ 9.12 (s, 1H), 7.62 (t, *J* = 7.6 Hz, 1H), 7.48 (t, *J* = 7.2 Hz, 1H), 7.34 (d, *J* = 6.8 Hz, 1H), 7.28 (t, *J* = 7.6 Hz, 1H), 7.24 (t, *J* = 54.4 Hz, 1H), 6.61 (s, 1H), 5.58-5.52 (m, 1H), 4.52 (s, 1H), 4.27-4.21 (m, 1H), 3.63-3.57 (m, 1H), 3.44-3.40 (m, 1H), 3.19-3.14 (m, 2H), 2.03-1.97 (m, 1H), 1.89-1.80 (m, 1H), 1.76-1.62 (m, 2H), 1.55-1.53 (m, 6H), 1.21-1.07 (m, 4H); ESI-MS (m/z): 488.2 $[M+H]^+$. |

(continued)

| Number | Chemical Name | Structure | $^1$H NMR and MS |
|---|---|---|---|
| **Example 140** | 4-(((*R*)-1-(3-(difluoromethyl)-2-fluorophenyl)ethyl) amino)-6-(hexahydrocyclopen ta [c]pyrrol-2(1*H*)-yl)-1-(4-cyclopentylpiperazin-1-yl)-pyrido[3,4-*d*]pyridazin-7(6*H*)-one | 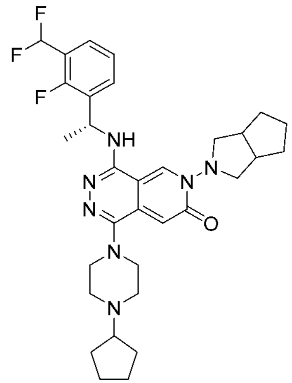 | $^1$H NMR (400 MHz, DMSO-$d_6$) δ 9.27 (s, 1H), 7.63-7.59 (m, 2H), 7.48 (t, *J* = 7.2 Hz, 1H), 7.27 (t, *J* = 7.2 Hz, 1H), 7.24 (t, *J* = 54.4 Hz, 1H), 6.47 (s, 1H), 5.60-5.53 (m, 1H), 3.70-3.64 (m, 2H), 3.30-3.25 (m, 3H), 3.06-2.95 (m, 4H), 2.72-2.57 (m, 6H), 1.84-1.70 (m, 5H), 1.64-1.48 (m, 10H), 1.40-1.32 (m, 2H); ESI-MS(m/z): 596.0 $[M+H]^+$. |
| **Example 141** | (*R*)-4-((1-(5-amino-2-fluoro-3-methylphenyl)ethyl) amino)-6-cyclopropyl-1-(4-methylpiperazin-1-yl)pyrido[3,4-*d*]pyridazin-7(6*H*)-one | 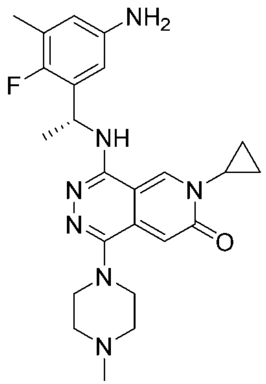 | $^1$H NMR (400 MHz, DMSO-$d_6$) δ 9.00 (s, 1H), 7.44 (d, *J* = 6.8 Hz, 1H), 6.43 (s, 1H), 6.39-6.36 (m, 1H), 6.26-6.24 (m, 1H), 5.48-5.41 (m, 1H), 4.74 (s, 2H), 3.66-3.60 (m, 1H), 3.24 (s, 4H), 3.02-3.00 (m, 4H), 2.24 (s, 3H), 2.11 (s, 3H), 1.46 (d, *J* = 7.2 Hz, 3H), 1.20-1.08 (m, 4H); ESI-MS (m/z): 452.2 $[M+H]^+$. |

(continued)

| Number | Chemical Name | Structure | $^1$H NMR and MS |
|---|---|---|---|
| **Example 142** | 4-(((*R*)-1-(3-(difluoromethyl)-2-fluorophenyl)ethyl) amino)-6-(hexahydrocyclopen ta [c]pyrrol-2(1*H*)-yl)-1-(4-cyclopropylpiperazi n-1-yl)-pyrido[3,4-*d*]pyridazin-7(6*H*)-one | 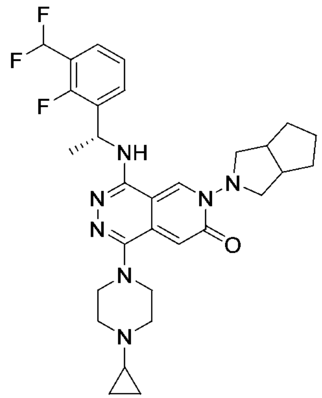 | $^1$H NMR (400 MHz, DMSO-$d_6$) δ 9.28 (s, 1H), 7.66-7.58 (m, 2H), 7.48 (t, *J* = 7.2 Hz, 1H), 7.27 (t, *J* = 7.2 Hz, 1H), 7.23 (t, *J* = 54.4 Hz, 1H), 6.48 (s, 1H), 5.59-5.52 (m, 1H), 3.70-3.65 (m, 2H), 3.28-3.25 (m, 1H), 3.01-2.92 (m, 4H), 2.74-2.65 (m, 6H), 1.80-1.70 (m, 4H), 1.60-1.49 (m, 7H), 0.47-0.40 (m, 2H), 0.38-0.31 (m, 2H); ESI-MS(m/z): 568.0 $[M+H]^+$. |
| **Example 143** | (*R*)-4-((1-(5-amino-2-fluoro-3-methylphenyl)ethyl) amino)-6-cyclopropyl-1-(4-cyclopropylpiperazi n-1-yl)pyrido[3,4-*d*]pyridazin-7(6*H*)-one | 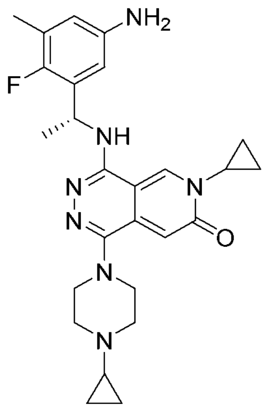 | $^1$H NMR (400 MHz, DMSO-$d_6$) δ 9.06 (s, 1H), 7.49 (d, *J* = 7.2 Hz, 1H), 6.50 (s, 1H), 6.44-6.42 (m, 1H), 6.32-6.29 (m, 1H), 5.52-5.46 (m, 1H), 4.79 (s, 2H), 3.72-3.66 (m, 1H), 3.02 (s, 4H), 2.77-2.74 (m, 4H), 2.16 (s, 3H), 1.78-1.73 (m, 1H), 1.52 (d, *J* = 6.8 Hz, 3H), 1.26-1.14 (m, 4H), 0.51-0.37 (m, 4H); ESI-MS (m/z): 478.3 $[M+H]^+$. |

(continued)

| Number | Chemical Name | Structure | $^1$H NMR and MS |
|---|---|---|---|
| **Example 144** | (*R*)-4-((1-(5-amino-2-fluoro-3-methylphenyl)ethyl) amino)-6-cyclopropyl-1-(4-ethylpiperazin-1-yl)pyrido[3,4-*d*]pyridazin-7(6*H*)-one formate | 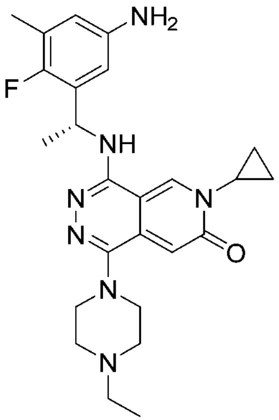 | The crude product was purified by Prep-HPLC (separation method 2) to obtain (*R*)-4-((1-(5-amino-2-fluoro-3-methylphenyl)ethyl)amino)-6-cyclopropyl-1-(4-ethylpiperazin-1-yl)pyrido[3,4-*d*]pyridazin-7(6*H*)-one formate; $^1$H NMR (400 MHz, DMSO-$d_6$) δ 10.18 (s, 1H), 9.11 (s, 1H), 7.66 (s, 1H), 6.54 (s, 1H), 6.41-6.39 (m, 1H), 6.29-6.27 (m, 1H), 5.49-5.42 (m, 1H), 4.81 (s, 2H), 3.67-3.61 (m, 1H), 3.48 (s, 3H), 3.15 (s, 6H), 2.11 (s, 3H), 1.49 (d, *J* = 6.8 Hz, 3H), 1.27-1.09 (m, 8H); ESI-MS (m/z): 466.3 $[M+H]^+$. |
| **Example 145** | (*R*)-3-(1-((1-(4-ethylpiperazin-1-yl)-6-methyl-7-oxo-6,7-dihydropyrido[3,4-*d*]pyridazin-4-yl)amino)ethyl)-2-methylbenzonitrile formate | 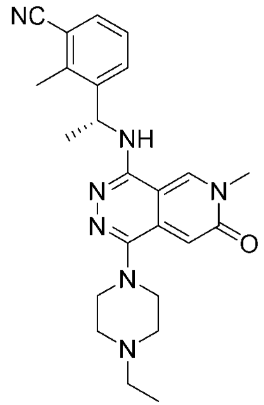 | The crude product was purified by Prep-HPLC (separation method 2) to obtain (*R*)-3-(1-((1-(4-ethylpiperazin-1-yl)-6-methyl-7-oxo-6,7-dihydropyrido[3,4-*d*]pyridazin-4-yl)amino)ethyl)-2-methylbenzonitrile formate; $^1$H NMR (400 MHz, DMSO-$d_6$) δ 10.32 (s, 1H), 9.42 (s, 1H), 8.14 (s, 1H), 7.78 (d, *J* = 8.0 Hz, 1H), 7.63 (d, *J* = 7.6 Hz, 2H), 7.35 (t, *J* = 7.6 Hz, 1H), 6.49 (s, 1H), 5.52-5.42 (m, 1H), 3.68 (s, 3H), 3.26-3.03 (m, 8H), 3.00-2.88 (m, 2H), 2.62 (s, 3H), 1.48 (d, *J* = 6.8 Hz, 3H), 1.20 (t, *J* = 7.2 Hz, 3H); ESI-MS(m/z): 432.2 $[M+H]^+$. |

(continued)

| Number | Chemical Name | Structure | $^1H$ NMR and MS |
|---|---|---|---|
| **Example 146** | (*R*)-1-(4-cyclopentylpiperazi n-1-yl)-4-((1-(3-(difluoromethyl)-2-fluorophenyl)ethyl) amino)-6-methylpyrido[3,4-*d*] pyridazin-7(6*H*)-one | 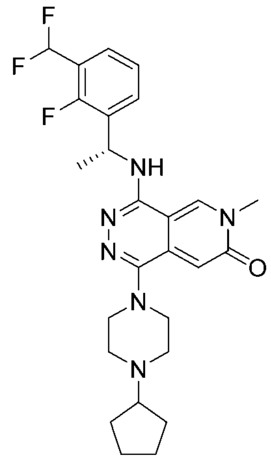 | $^1H$ NMR (400 MHz, DMSO-$d_6$) δ 9.26 (s, 1H), 7.62 (t, *J* = 7.2 Hz, 1H), 7.49 (t, *J* = 7.2 Hz, 1H), 7.35 (d, *J* = 6.8 Hz, 1H), 7.27 (t, *J* = 7.6 Hz, 1H), 7.24 (t, *J* = 54.4 Hz, 1H), 6.43 (s, 1H), 5.57 (p, *J* = 6.8 Hz, 1H), 3.68 (s, 3H), 3.07-2.94 (m, 4H), 2.63-2.53 (m, 4H), 2.53-2.51 (m, 1H), 1.83-1.75 (m, 2H), 1.64-1.57 (m, 2H), 1.55-1.47 (m, 5H), 1.39-1.31 (m, 2H); ESI-MS(m/z): 501.2 $[M+H]^+$. |
| **Example 147** | (*R*)-4-((1-(5-amino-2-fluoro-3-methylphenyl)ethyl) amino)-6-cyclopropyl-1-(4-isopropylpiperazin-1-yl)pyrido[3,4-*d*]pyridazin-7(6*H*)-one formate | 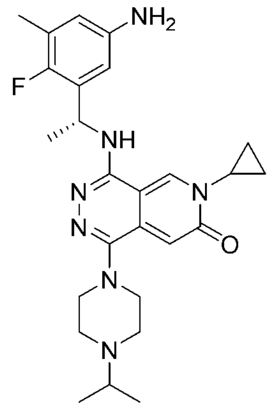 | The crude product was purified by Prep-HPLC (separation method 2) to obtain (*R*)-4-((1-(5-amino-2-fluoro-3-methylphenyl)ethyl)amino)-6-cyclopropyl-1-(4-isopropylpiperazin-1-yl)pyrido[3,4-*d*]pyridazin-7(6*H*)-one formate; $^1H$ NMR (400 MHz, DMSO-$d_6$) δ 9.03 (s, 1H), 8.14 (s, 1H), 7.52 (s, 1H), 6.53 (s, 1H), 6.40-6.38 (m, 1H), 6.27-6.25 (m, 1H), 5.48-5.41 (m, 1H), 3.67-3.61 (m, 1H), 3.17-3.01 (m, 11H), 2.11 (s, 3H), 1.47 (d, *J* = 7.2 Hz, 3H), 1.24-1.08 (m, 10H); ESI-MS (m/z): 480.3 $[M+H]^+$. |

(continued)

| Number | Chemical Name | Structure | $^1$H NMR and MS |
|---|---|---|---|
| **Example 148** | (*R*)-4-((1-(5-amino-2-fluoro-3-methylphenyl)ethyl) amino)-1-(4-cyclopentylpiperazin-1-yl)-6-cyclopropylpyrido[3 ,4-*d*]pyridazin-7(6*H*)-one | 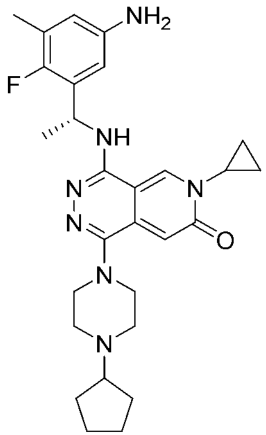 | $^1$H NMR (400 MHz, DMSO-$d_6$) δ 8.99 (s, 1H), 7.41 (d, *J* = 7.2 Hz, 1H), 6.43 (s, 1H), 6.39-6.37 (m, 1H), 6.26-6.24 (m, 1H), 5.48-5.41 (m, 1H), 4.73 (s, 2H), 3.66-3.61 (m, 1H), 3.01 (s, 4H), 2.59-2.54 (m, 5H), 2.11 (s, 3H), 1.82-1.77 (m, 2H), 1.66-1.58 (m, 2H), 1.56-1.49 (m, 2H), 1.46 (d, *J* = 7.2 Hz, 3H), 1.41-1.32 (m, 2H), 1.20-1.08 (m, 4H); ESI-MS (m/z): 506.3 $[M+H]^+$. |
| **Example 149** | 4-(((*R*)-1-(3-(difluoromethyl)-2-fluorophenyl)ethyl) amino)-6-(hexahydrocyclopen ta [c]pyrrol-2(1*H*)-yl)-1-(4-isopropylpiperazin-1-yl)pyrido[3,4-*d*]pyridazin-7(6*H*)-one | 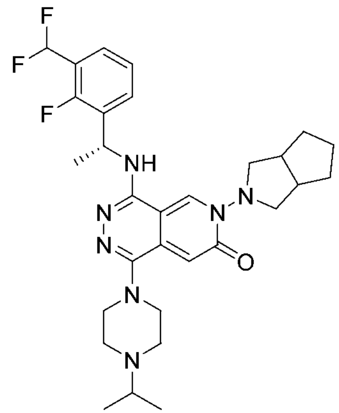 | $^1$H NMR (400 MHz, DMSO-$d_6$) δ 9.3 (s, 1H), 7.72-7.62 (m, 2H), 7.49 (t, *J* = 7.2 Hz, 1H), 7.28 (t, *J* = 7.2 Hz, 1H), 7.24 (t, *J* = 54.4 Hz, 1H), 6.59 (s, 1H), 5.62-5.55 (m, 1H), 3.71-3.66 (m, 2H), 3.55-3.35 (m, 4H), 3.27-3.05 (m, 8H), 2.73-2.67 (m, 2H), 1.82-1.71 (m, 3H), 1.61-1.49 (m, 6H), 1.32-1.16 (m, 5H); ESI-MS(m/z): 570.0 [M+H]+. |

(continued)

| Number | Chemical Name | Structure | $^1$H NMR and MS |
|---|---|---|---|
| **Example 150** | 3-((1*R*)-1-((6-(hexahydrocyclopen ta[c] pyrrol-2(1*H*)-yl)-1-(4-methylpiperazin-1-yl)-7-oxo-6,7-dihydropyrido[3,4-*d*]pyridazin-4-yl)amino)ethyl)-2-methylbenzonitrile | 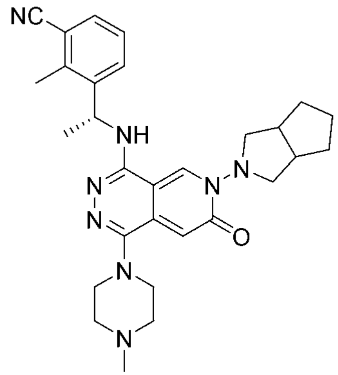 | $^1$H NMR (400 MHz, DMSO-$d_6$) δ 9.31 (s, 1H), 7.83-7.73 (m, 2H), 7.63 (d, *J* = 7.2 Hz, 1H), 7.35 (t, *J* = 7.2 Hz, 1H), 6.51 (s, 1H), 5.49-5.42 (m, 1H), 3.69-3.64 (m, 2H), 3.25-3.07 (m, 8H), 2.75-2.59 (m, 9H), 1.80-1.71 (m, 3H), 1.62-1.44 (m, 7H); ESI-MS(m/z): 513.0 [M+H]+. |
| **Example 151** | (*R*)-4-((1-(3-(difluoromethyl)-2-fluorophenyl)ethyl) amino)-1,6-bis(4-methylpiperazin-1-yl)pyrido[3,4-*d*]pyridazin-7(6*H*)-one | 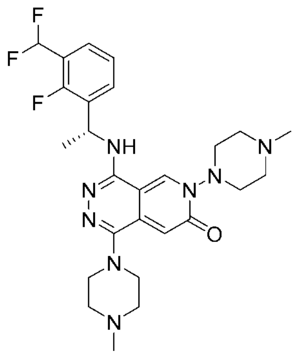 | $^1$H NMR (400MHz,DMSO-$d_6$) □δ 9.23 (s, 1H), 7.66-7.59 (m, 2H), 7.48 (t, *J* = 7.1 Hz, 1H), 7.38-7.09 (m, 2H), 6.46 (s, 1H), 5.62-5.50 (m, 1H), 3.07-2.93 (m, 4H), 2.49-2.38 (m, 12H), 2.23 (d, *J* = 8.8 Hz, 6H), 1.52 (d, *J* = 7.2 Hz, 3H); ESI-MS(m/z): 531.0 $[M+H]^+$. |
| **Example 152** | (*R*)-4-((1-(5-amino-2-fluoro-3-methylphenyl)ethyl) amino)-6-(1-methylcyclopropyl)-1-(4-methylpiperazin-1-yl)pyrido [3,4-*d*]pyridazin-7(6*H*)-one | 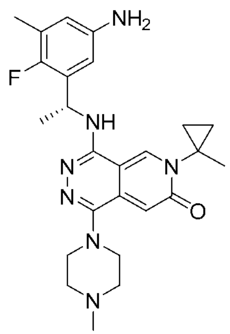 | $^1$H NMR (400 MHz, DMSO-$d_6$) δ 9.25 (s, 1H), 7.42 (d, *J* = 6.8 Hz, 1H), 6.40-6.37 (m, 2H), 6.27-6.24 (m, 1H), 5.46-5.39 (m, 1H), 4.72 (s, 2H), 3.00 (s, 4H), 2.47 (s, 4H), 2.23 (s, 3H), 2.11 (s, 3H), 1.55 (s, 3H), 1.47 (d, *J* = 6.8 Hz, 3H), 1.19-1.08 (m, 4H); ESI-MS (m/z): 466.3 $[M+H]^+$. |

(continued)

| Number | Chemical Name | Structure | $^1$H NMR and MS |
|---|---|---|---|
| **Example 153** | 3-((1*R*)-1-((6-(hexahydrocyclopen ta[c] pyrrol-2(1*H*)-yl)-1-(4-ethylpiperazin-1-yl)-7-oxo-6,7-dihydropyrido[3,4-d]pyridazin-4-yl)amino)ethyl)-2-methylbenzonitrile | 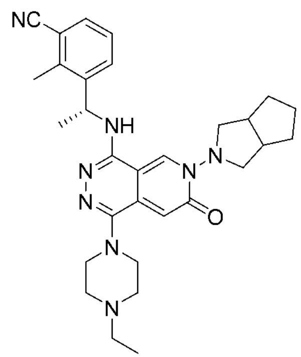 | $^1$H NMR (400 MHz, DMSO-$d_6$) δ 9.31 (s, 1H), 7.82-7.70 (m, 2H), 7.63 (d, *J* = 7.2 Hz, 1H), 7.35 (t, *J* = 7.2 Hz, 1H), 6.51 (s, 1H), 5.49-5.42 (m, 1H), 3.69-3.64 (m, 2H), 3.27-2.83 (m, 11H), 2.75-2.59 (m, 6H), 1.80-1.70 (m, 3H), 1.79-1.70 (m, 3H), 1.59-1.50 (m, 3H), 1.47 (d, *J* = 6.8 Hz, 3H); ESI-MS(m/z): 527.0 $[M+H]^+$. |
| **Example 154** | (*R*)-1-(4-(cyclopropylmethyl) piperazin-1-yl)-4-((1-(3-(difluoromethyl)-2-fluorophenyl)ethyl) amino)-6-(4-methylpiperazin-1-yl)pyrido[3,4-*d*]pyridazin-7(6*H*)-one | 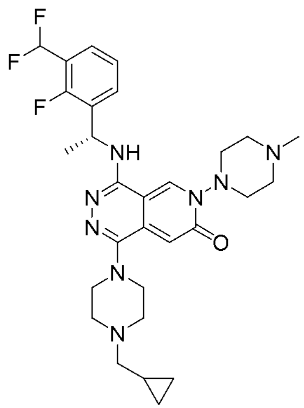 | $^1$H NMR (400MHz,DMSO-$d_6$) δ 9.23 (s, 1H), 7.66-7.59 (m, 2H), 7.48 (t, *J* = 6.8 Hz, 1H), 7.37-7.10 (m, 2H), 6.46 (s, 1H), 5.62-5.49 (m, 1H), 3.94-3.31 (m, 4H), 3.11-2.94 (m, 4H), 2.68-2.51 (m, 8H), 2.27-2.19 (m, 5H), 1.52 (d, *J* = 7.2 Hz, 3H), 0.91-0.78 (m, 1H), 0.54-0.37 (m, 2H), 0.19-0.05 (m, 2H); ESI-MS(m/z): 571.0 $[M+H]^+$. |

(continued)

| Number | Chemical Name | Structure | $^1$H NMR and MS |
|---|---|---|---|
| **Example 155** | (*R*)-1-(4-isopropylpiperazin-1-yl)-6-(1-methylcyclopropyl)-4-((1-(3-(trifluoromethyl)phe nyl)ethyl)amino)pyr ido[3,4-*d*]pyridazin-7(6*H*)-one formate | 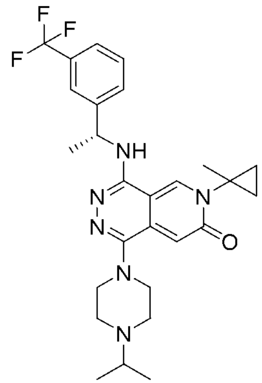 | The crude product was purified by Prep-HPLC (separation method 2) to obtain (*R*)-1-(4-isopropylpiperazin-1-yl)-6-(1-methylcyclopropyl)-4-((1-(3-(trifluoromethyl)phenyl)ethyl)amino) pyrido[3,4-*d*]pyridazin-7(6*H*)-one formate; $^1$H NMR (400 MHz, DMSO-$d_6$) δ 9.18 (s, 1H), 8.15 (s, 1H), 7.75-7.71 (m, 2H), 7.59-7.54 (m, 3H), 6.42 (s, 1H), 5.44- 5.38 (m, 1H), 3.00 (s, 4H), 2.74-2.68 (m, 1H), 2.62 (s, 4H), 1.55 (s, 6H), 1.16-1.03 (m, 4H), 1.00 (d, *J* = 8.8 Hz, 6H);ESI-MS(m/z): 515.2 $[M+H]^+$. |
| **Example 156** | (*R*)-1-(4-ethylpiperazin-1-yl)-6-(1-methylcyclopropyl)-4-((1-(3-(trifluoromethyl)phe nyl)ethyl)amino)pyr ido[3,4-*d*]pyridazin-7(6*H*)-one formate | 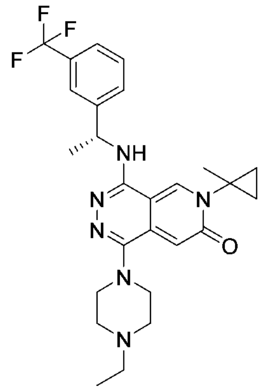 | The crude product was purified by Prep-HPLC (separation method 2) to obtain (*R*)-1-(4-ethylpiperazin-1-yl)-6-(1-methylcyclopropyl)-4-((1-(3-(trifluoromethyl) phenyl)ethyl)amino) pyrido[3,4-*d*]pyridazin-7(6*H*)-one formate; $^1$H NMR (400 MHz, DMSO-$d_6$) δ 9.19 (s, 1H), 8.19 (s, 1H), 7.75-7.71 (m, 2H), 7.58-7.54 (m, 3H), 6.40 (s, 1H), 5.44- 5.39 (m, 1H), 3.01 (s, 4H), 2.54-2.50 (m, 4H), 2.44-2.40 (m, 2H), 1.55 (s, 6H), 1.15-1.01 (m, 7H); ESI-MS (m/z): 501.0 [M+H]+. |

(continued)

| Number | Chemical Name | Structure | $^1$H NMR and MS |
|---|---|---|---|
| **Example 157** | (*R*)-1-(4-methylpiperazin-1-yl)-6-(1-methylcyclopropyl)-4-((1-(3-(trifluoromethyl)phe nyl)ethyl)amino)pyr ido[3,4-*d*]pyridazin-7(6*H*)-one formate | 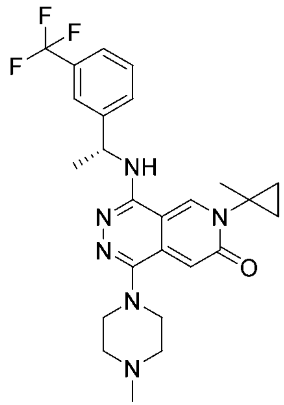 | The crude product was purified by Prep-HPLC (separation method 2) to obtain (*R*)-1-(4-methylpiperazin-1-yl)-6-(1-methylcyclopropyl)-4-((1-(3-(trifluoromethyl) phenyl)ethyl)amino) pyrido[3,4-*d*]pyridazin-7(6*H*)-one formate; $^1$H NMR (400 MHz, DMSO-$d_6$) δ 9.24 (s, 1H), 8.14 (s, 1H), 7.75-7.56 (m, 5H), 6.45 (s, 1H), 5.45-5.41 (m, 1H), 3.32- 2.96 (m, 8H), 2.56 (s, 3H), 1.58-1.54 (m, 6H), 1.16-1.08 (m, 4H); ESI-MS(m/z): 487.0 [M+H]$^+$. |
| **Example 158** | (*R*)-6-(1-methylcyclopropyl)-1-(morpholinoamino)-4-((1-(3-(trifluoromethyl)phe nyl) ethyl)amino)pyr ido[3,4-*d*]pyridazin-7(6*H*)-one formate | 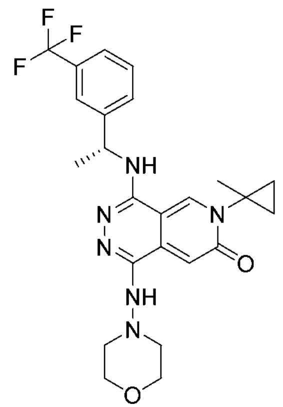 | The crude product was purified by Prep-HPLC (separation method 2) to obtain (*R*)-6-(1-methylcyclopropyl)-1-(morpholinoamino)-4-((1-(3-(trifluoromethyl)phenyl)ethyl)amino) pyrido[3,4-*d*]pyridazin-7(6*H*)-one formate; $^1$H NMR (400 MHz, DMSO-$d_6$) δ 8.68 (s, 1H), 8.16 (s, 1H), 7.69-7.55(m,4H), 6.83 (s, 1H), 6.64 (s, 1H), 5.02-4.97 (m, 1H), 2.56-2.47 (m, 6H), 2.19 (s, 3H), 1.50 (s, 6H), 1.13-1.03 (m, 4H); ESI-MS (m/z): 489.0 [M+H]$^+$. |

(continued)

| Number | Chemical Name | Structure | $^1$H NMR and MS |
|---|---|---|---|
| **Example 159** | (*R*)-6-(1-methylcyclopropyl)-1-((4-methylpiperazin-1-yl)amino)-4-((1-(3-(trifluoromethyl)phe nyl)ethyl)amino)pyr ido[3,4-*d*] pyridazin-7(6*H*)-one formate | 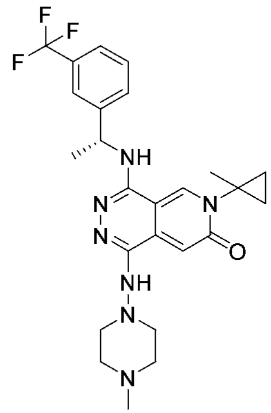 | The crude product was purified by Prep-HPLC (separation method 2) to obtain (*R*)-6-(1-methylcyclopropyl)-1-((4-methylpiperazin-1-yl)amino)-4-((1-(3-(trifluoromethyl)phenyl)ethyl)amino) pyrido[3,4-*d*]pyridazin-7(6*H*)-one formate; $^1$H NMR (400 MHz, DMSO-$d_6$) δ 8.73 (s, 1H), 8.24 (s, 1H), 8.16 (s, 1H), 7.74- 7.60 (m, 4H), 6.88 (s, 1H), 6.69 (s, 1H), 5.08-5.00 (m, 1H), 2.62-2.53 (m, 8H), 2.24 (s, 3H), 1.55 (s, 6H), 1.19-1.08 (m, 4H);ESI-MS(m/z): 502.0 $[M+H]^+$. |
| **Example 160** | (*R*)-4-((1-(5-amino-2-fluoro-3-methylphenyl)ethyl) amino)-1-(4-ethylpiperazin-1-yl)-6-(1-methylcyclopropyl) pyrido[3,4-*d*] pyridazin-7(6*H*)-one formate | 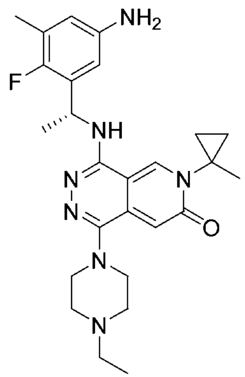 | The crude product was purified by Prep-HPLC (separation method 2) to obtain (*R*)-4-((1-(5-amino-2-fluoro-3-methylphenyl)ethyl)amino)-1-(4-ethylpiperazin-1-yl)-6-(1-methylcyclopropyl)pyrido[3,4-*d*]pyridazin-7(6*H*)-one formate; $^1$H NMR (400 MHz, DMSO-$d_6$) δ 9.39 (s, 1H), 8.14 (s, 1H), 7.76 (s, 1H), 6.52 (s, 1H), 6.44-6.41 (m, 1H), 6.30-6.28 (m, 1H), 5.48-5.41 (m, 1H), 3.59-3.30 (m, 8H), 3.12-3.08 (m, 4H), 2.11 (s, 3H), 1.55 (s, 3H), 1.50 (d, $J$ = 7.2 Hz, 3H), 1.26 (t, $J$ = 7.2 Hz, 3H), 1.20-1.07 (m, 4H); ESI-MS (m/z): 480.3 $[M+H]^+$. |

(continued)

| Number | Chemical Name | Structure | $^1$H NMR and MS |
|---|---|---|---|
| **Example 161** | (*R*)-4-((1-(5-amino-2-fluoro-3-methylphenyl)ethyl) amino)-1-(4-isopropylpiperazin-1-yl)-6-(1-methylcyclopropyl) pyrido [3,4-*d*]pyridazin-7(6*H*)-one | 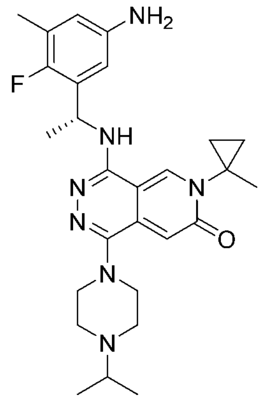 | $^1$H NMR (400 MHz, DMSO-$d_6$) δ 9.24 (s, 1H), 7.40 (d, *J* = 7.2 Hz, 1H), 6.41 (s, 1H), 6.40-6.37 (m, 1H), 6.27-6.24 (m, 1H), 5.46-5.39 (m, 1H), 4.72 (s, 2H), 3.00-2.98 (m, 4H), 2.72-2.65 (m, 1H), 2.61-2.58 (m, 4H), 2.11 (s, 3H), 1.55 (s, 3H), 1.47 (d, *J* = 7.2 Hz, 3H), 1.19-1.08 (m, 4H), 1.01 (d, *J* = 6.4 Hz, 6H); ESI-MS (m/z): 494.3 $[M+H]^+$. |
| **Example 162** | (*R*)-4-((1-(5-amino-2-fluoro-3-methylphenyl)ethyl) amino)-1-(4-cyclopropylpiperazin-1-yl)-6-(1-methylcyclopropyl) pyrido [3,4-*d*]pyridazin-7(6*H*)-one | 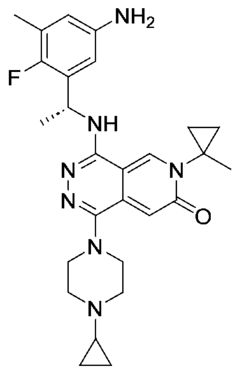 | $^1$H NMR (400 MHz, DMSO-$d_6$) δ 9.25 (s, 1H), 7.41 (d, *J* = 7.2 Hz, 1H), 6.42 (s, 1H), 6.39-6.37 (m, 1H), 6.26-6.24 (m, 1H), 5.46-5.39 (m, 1H), 4.72 (s, 2H), 2.96 (s, 4H), 2.70-2.68 (m, 4H), 2.11 (s, 3H), 1.72-1.67 (m, 1H), 1.55 (s, 3H), 1.47 (d, *J* = 7.2 Hz, 3H), 1.20-1.08 (m, 4H), 0.45-0.32 (m, 4H); ESI-MS (m/z): 492.3 $[M+H]^+$. |

(continued)

| Number | Chemical Name | Structure | $^1$H NMR and MS |
|---|---|---|---|
| **Example 163** | 3-((1*R*)-1-((1-(4-(tert-butyl)piperazin-1-yl)-6-(hexahydrocyclopen ta[c]pyrrol-2(1*H*)-yl)-7-oxo-6,7-dihydropyrido [3,4-*d*]pyridazin-4-yl)amino)ethyl)-2-methylbenzonitrile formate | 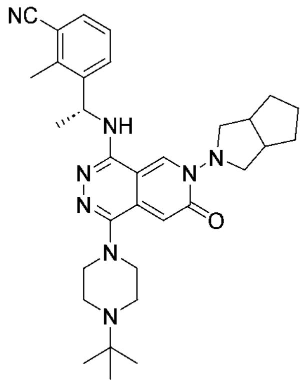 | The crude product was purified by Prep-HPLC (separation method 2) to obtain 3-((1*R*)-1-((1-(4-(tert-butyl)piperazin-1-yl)-6-(hexahydrocyclopenta[c]pyrrol-2(1*H*)-yl)-7-oxo-6,7-dihydropyrido[3,4-*d*]pyridazin-4-yl)amino)ethyl)-2-methylbenzonitrile formate; $^1$H NMR (400 MHz, DMSO-$d_6$) δ 10.18 (s, 1H), 9.32 (s, 1H), 8.14 (s, 1H), 7.84-7.72 (m, 2H), 7.64 (d, *J* = 8.0 Hz, 1H), 7.36 (t, *J* = 8.0 Hz, 1H), 6.64 (s, 1H), 5.50-5.43 (m, 1H), 3.71-3.65 (m, 2H), 3.55-2.41 (m, 4H), 3.28-3.18 (m, 6H), 2.72-2.67 (m, 2H), 2.61 (s, 3H), 1.80-1.71 (m, 3H), 1.57-1.47 (m, 6H), 1.38 (s, 9H); ESI-MS(m/z): 555.0 $[M+H]^+$. |
| **Example 164** | (*R*)-4-((1-(2-fluoro-3-(trifluoromethyl)phe nyl)ethyl)amino)-6-(1-methylcyclopropyl)-1-(4-methylpiperazin-1-yl)pyrido [3,4-*d*]pyridazin-7(6*H*)-one | 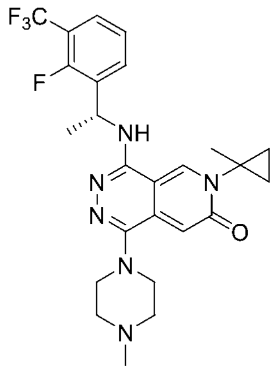 | $^1$H NMR (400 MHz, DMSO-$d_6$) δ 9.24 (s, 1H), 7.77 (d, *J* = 7.2 Hz, 1H), 7.68-7.56 (m, 2H), 7.34 (t, *J* = 7.6 Hz, 1H), 6.41 (s, 1H), 5.59-5.54 (m, 1H), 3.06-2.97 (m, 4H), 3.59-2.52 (m, 4H), 2.30-2.25 (m, 3H), 1.56 (s, 3H), 1.55 (s, 3H), 1.21-1.07 (m, 4H); ESI-MS(m/z): 505.0 $[M+H]^+$. |

(continued)

| Number | Chemical Name | Structure | $^1$H NMR and MS |
|---|---|---|---|
| **Example 165** | 3-((1*R*)-1-((1-(4-cyclopentylpiperazi n-1-yl)-6-(hexahydrocyclopen ta[c]pyrrol-2(1*H*)-yl)-7-oxo-6,7-dihydropyrido [3,4-*d*]pyridazin-4-yl)amino)ethyl)-2-methylbenzonitrile | 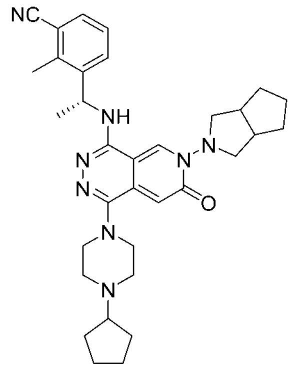 | $^1$H NMR (400 MHz, DMSO-$d_6$) δ 9.27 (s, 1H), 7.75-7.62 (m, 3H), 7.35 (t, *J* = 7.6 Hz, 1H), 6.49 (s, 1H), 5.48-5.41 (m, 1H), 3.69-3.64 (m, 2H), 3.29-3.24 (m, 3H), 3.16-3.04 (m, 4H), 2.96-2.78 (m, 4H), 2.71-2.67 (m, 2H), 2.61 (s, 3H), 1.91-1.83 (m, 2H), 1.77-1.71 (m, 3H), 1.67-1.62 (m, 2H), 1.56-1.45 (m, 10H); ESI-MS(m/z): 567.0 $[M+H]^+$. |
| **Example 166** | 3-((1*R*)-1-((6-(hexahydrocyclopen ta[c] pyrrol-2(1*H*)-yl)-1-((S)-3-(hydroxymethyl) pyr rolidin-1-yl)-7-oxo-6,7-dihydropyrido [3,4-*d*]pyridazin-4-yl)amino)ethyl)-2-methylbenzonitrile | 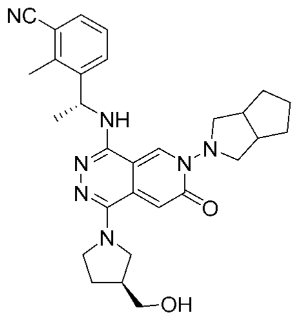 | $^1$H NMR (400 MHz, DMSO-$d_6$) δ 12.34 (s, 1H), 9.30 (s, 1H), 7.75 (d, *J* = 7.6 Hz, 1H), 7.66 (d, *J* = 7.6 Hz, 1H), 7.37 (t, *J* = 7.2 Hz, 1H), 6.99 (s, 1H), 5.29-5.22 (m, 1H), 4.74-4.69 (m, 1H), 3.68-3.59 (m, 4H), 3.47-3.35 (m, 3H), 3.29-3.25 (m, 3H), 2. 74-2.66 (m, 2H), 2.60 (s, 3H), 2.39-2.33 (m, 1H), 2.01-1.93 (m, 1H), 1.81-1.65 (m, 4H), 1.61-1.49 (m, 6H); ESI-MS(m/z): 514.0 $[M+H]^+$. |
| **Example 167** | 3-((1*R*)-1-((1-((*R*)-3,4-dimethylpiperazin-1-yl)-6-(hexahydrocyclopen ta[c]pyrrol-2(1*H*)-yl)-7-oxo-6,7-dihydropyrido [3,4-*d*]pyridazin-4-yl)amino)ethyl)-2-methylbenzonitrile | 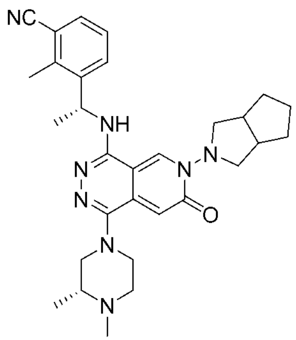 | $^1$H NMR (400 MHz, DMSO-$d_6$) δ 9.27 (s, 1H), 7.74 (d, *J* = 7.6 Hz, 1H), 6.69 (s, 1H), 7.63 (d, *J* = 7.6 Hz, 1H), 7.35 (t, *J* = 7.6 Hz, 1H), 6.49 (s, 1H), 5.48-5.41 (m, 1H), 3.69-3.64 (m, 2H), 3.26-3.19 (m, 3H), 3.10-2.88 (m, 3H), 2.75-2.61 (m, 8H), 2.47-2.35 (m, 3H), 1.79-1.69 (m, 3H), 1.57-1.45 (m, 6H), 1.01 (s, 3H); ESI-MS(m/z): 527.0 $[M+H]^+$. |

(continued)

| Number | Chemical Name | Structure | $^1$H NMR and MS |
|---|---|---|---|
| **Example 168** | (*R*)-1-(4-ethylpiperazin-1-yl)-4-((1-(2-fluoro-3-(trifluoromethyl)phe nyl)ethyl)amino)-6-(1-methylcyclopropyl) pyrido[3,4-*d*] pyridazin-7(6*H*)-one formate | 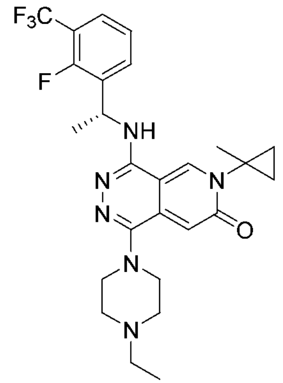 | The crude product was purified by Prep-HPLC (separation method 2) to obtain (*R*)-1-(4-ethylpiperazin-1-yl)-4-((1-(2-fluoro-3-(trifluoromethyl)phenyl)ethyl)amino)-6-(1-methylcyclopropyl)pyrido[3,4-*d*]pyridazin-7(6*H*)-one formate; $^1$H NMR (400 MHz, DMSO-$d_6$) δ 9.23 (s, 1H), 8.15 (s, 1H), 7.77 (t, *J* = 7.2 Hz, 1H), 7.65-7.55 (m, 2H), 7.35 (t, *J* = 8.0 Hz, 1H), 6.41 (s, 1H), 5.60-5.53 (m, 1H), 3.32-3.15 (m, 1H), 3.01 (s, 4H), 2.57 (s, 3H), 2.46-2.41 (m, 2H), 1.57-1.54 (m, 6H), 1.21-1.13 (m, 2H), 1.10-1.01 (m, 5H); ESI-MS(m/z): 519.0 $[M+H]^+$. |
| **Example 169** | (*R*)-4-((1-(2-fluoro-3-(trifluoromethyl)phenyl)ethyl)amino)-1-(4-isopropylpiperazin-1-yl)-6-(1-methylcyclopropyl) pyrido [3,4-*d*]pyridazin-7(6*H*)-one | 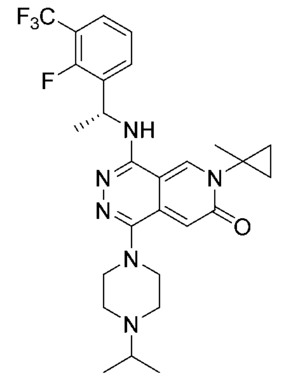 | $^1$H NMR (400 MHz, DMSO-$d_6$) δ 9.24 (s, 1H), 7.77 (t, *J* = 7.6 Hz, 1H), 7.67-7.58 (m, 2H), 7.35 (t, *J* = 7.6 Hz, 1H), 6.43 (s, 1H), 5.60-5.54 (m, 1H), 3.01 (s, 4H), 2.80-2.63 (m, 5H), 1.56 (s, 2H), 1.55 (s, 4H), 1.21-1.13 (m, 2H), 1.10-1.12 (m, 8H); ESI-MS(m/z): 533.0 $[M+H]^+$. |

(continued)

| Number | Chemical Name | Structure | $^1$H NMR and MS |
|---|---|---|---|
| **Example 170** | (*R*)-4-((1-(2-fluoro-3-(trifluoromethyl)phenyl)ethyl)amino)-1-(4-cyclopropylpiperazin-1-yl)-6-(1-methylcyclopropyl) pyrido [3,4-*d*]pyridazin-7(6*H*)-one | 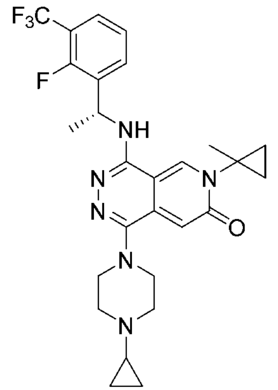 | $^1$H NMR (400 MHz, DMSO-$d_6$) δ 9.23 (s, 1H), 7.76 (t, *J* = 7.2 Hz, 1H), 7.65-7.58 (m, 2H), 7.34 (t, *J* = 8.0 Hz, 1H), 6.42 (s, 1H), 5.60-5.53 (m, 1H), 2.94 (s, 4H), 2.67 (s, 4H), 1.71-1.66 (m, 1H), 1.56 (s, 2H), 1.55 (s, 4H), 1.21-1.08 (m, 4H), 0.44-0.30 (m, 4H); ESI-MS(m/z): 531.0 $[M+H]^+$. |
| **Example 171** | (*R*)-4-((1-(2-fluoro-3-(trifluoromethyl)phenyl)ethyl)amino)-1-(4-cyclopentylpiperazin-1-yl)-6-(1-methylcyclopropyl) pyrido [3,4-*d*]pyridazin-7(6*H*)-one | 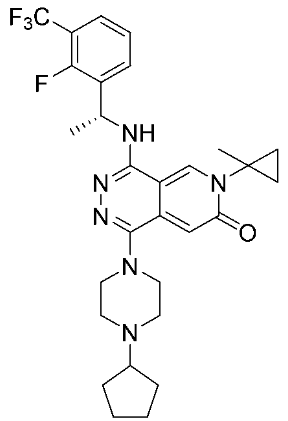 | $^1$H NMR (400 MHz, DMSO-$d_6$) δ 9.22 (s, 1H), 7.76 (t, *J* = 7.6 Hz, 1H), 7.63 (t, *J* = 7.6 Hz, 1H), 7.58 (d, *J* = 6.4 Hz, 1H), 7.34 (t, *J* = 8.0 Hz, 1H), 6.40 (s, 1H), 5.60-5.53 (m, 1H), 2.98 (s, 4H), 2.60-2.51 (m, 5H), 1.83-1.75 (m, 2H), 1.65-1.45 (m, 10H), 1.38-1.29 (m, 2H), 1.20-1.07 (m, 4H); ESI-MS(m/z): 559.0 $[M+H]^+$. |

(continued)

| Number | Chemical Name | Structure | $^1$H NMR and MS |
|---|---|---|---|
| **Example 172** | (*R*)-4-((1-(2-fluoro-3-(trifluoromethyl)phenyl)ethyl)amino)-1-(4-tert-butylpiperazin-1-yl)-6-(1-methylcyclopropyl) pyrido [3,4-*d*]pyridazin-7(6*H*)-one | 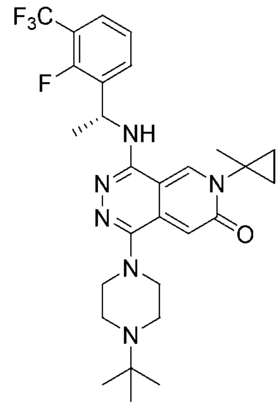 | $^1$H NMR (400 MHz, DMSO-$d_6$) δ 9.22 (s, 1H), 7.76 (t, *J* = 7.2 Hz, 1H), 7.63 (t, *J* = 7.2 Hz, 1H), 7.57 (d, *J* = 6.4 Hz, 1H), 7.34 (t, *J* = 8.0 Hz, 1H), 6.41 (s, 1H), 5.60-5.53 (m, 1H), 2.97 (s, 4H), 2.67-2.61 (m, 4H), 1.56 (s, 2H), 1.55 (s, 4H), 1.21-1.13 (m, 2H), 1.10-1.03 (m, 11H); ESI-MS(m/z): 547.0 $[M+H]^+$. |
| **Example 173** | (*R*)-4-((1-(2-fluoro-3-(trifluoromethyl)phenyl)ethyl)amino)-1-(4-cyclobutylpiperazin-1-yl)-6-(1-methylcyclopropyl) pyrido [3,4-*d*]pyridazin-7(6*H*)-one | 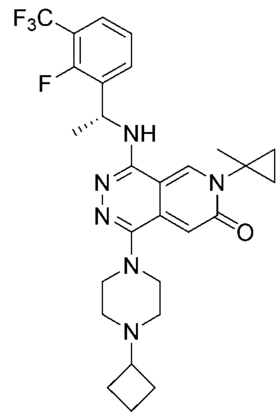 | $^1$H NMR (400 MHz, DMSO-$d_6$) δ 9.23 (s, 1H), 7.77 (t, *J* = 7.6 Hz, 1H), 7.64 (t, *J* = 7.2 Hz, 1H), 7.59 (d, *J* = 6.4 Hz, 1H), 7.35 (t, *J* = 8.0 Hz, 1H), 6.39 (s, 1H), 5.61-5.54 (m, 1H), 2.98 (s, 4H), 2.81-2.73 (m, 1H), 2.40 (s, 4H), 2.01-1.93 (m, 2H), 1.85-1.76 (m, 2H), 1.67-1.61 (m, 2H), 1.57 (s, 2H), 1.55 (s, 4H), 1.21-1.14 (m, 2H), 1.11-1.07 (m, 2H); ESI-MS (m/z): 545.0 $[M+H]^+$. |

(continued)

| Number | Chemical Name | Structure | $^1$H NMR and MS |
|---|---|---|---|
| **Example 174** | (*R*)-4-((1-(2-fluoro-3-(trifluoromethyl)phe nyl)ethyl)amino)-1-(4-methyl-3-oxopiperazin-1-yl)-6-(1-methylcyclopropyl) pyrido [3,4-*d*]pyridazin-7(6*H*)-one | 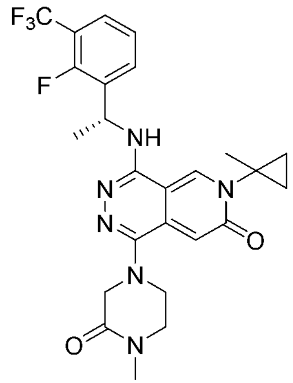 | $^1$H NMR (400 MHz, DMSO-$d_6$) δ 9.26 (s, 1H), 7.77 (t, *J* = 7.6 Hz, 1H), 7.67 (d, *J* = 6.4 Hz, 1H), 7.63 (t, *J* = 7.2 Hz, 1H), 7.35 (t, *J* = 7.6 Hz, 1H), 6.43 (s, 1H), 5.62-5.55 (m, 1H), 3.64 (s, 2H), 3.39-3.35 (m, 2H), 3.30-3.28 (m, 2H), 2.86 (s, 3H), 1.57 (s, 2H), 1.55 (s, 4H), 1.21-1.08 (m, 4H); ESI-MS(m/z): 519.0 $[M+H]^+$. |
| **Example 175** | 1-(2-oxa-5-azabicyclo[2.2.1]he ptan-5-yl)-4-(((*R*)-1-(2-fluoro-3-(trifluoromethyl) phe nyl)ethyl)amino)-6-(1-methylcyclopropyl) pyrido[3,4-*d*]pyridazin-7(6*H*)-one | 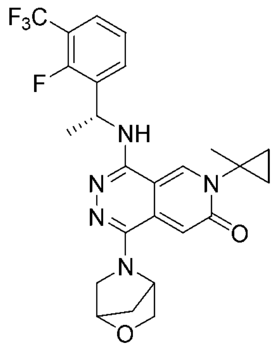 | $^1$H NMR (400 MHz, DMSO-$d_6$) δ 9.15 (s, 1H), 7.78-7.73 (m, 1H), 7.65-7.60 (m, 1H), 7.37-7.71 (m, 2H), 6.50 (d, *J* = 8.8 Hz, 1H), 5.57-5.48 (m, 1H), 4.50-4.45 (m, 2H), 3.99-3.85 (m, 1H), 3.77-3.63 (m, 2H), 3.28-3.17 (m, 1H), 1.79 (d, *J* = 10.0 Hz, 1H), 1.70 (d, *J* = 9.6 Hz, 1H), 1.55 (s, 4H), 1.54 (s, 2H), 1.21-1.33 (m, 2H), 1.01-1.05 (m, 2H); ESI-MS(m/z): 504.0 $[M+H]^+$. |
| **Example 176** | *1-((R)-3,4*-dimethylpiperazin-1-yl)-6-(1-methylcyclopropyl)-4-(((R)-1-(3-(trifluoromethyl)phe nyl)ethyl)amino)pyr ido[3,4-*d*] pyridazin-7(6*H*)-one formate | 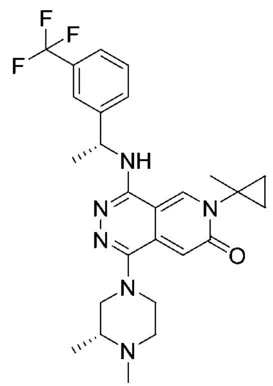 | The crude product was purified by Prep-HPLC (separation method 2), to obtain 1-((*R*)-3,4-dimethylpiperazin-1-yl)-6-(1-methylcyclopropyl)-4-(((*R*)-1-(3-(trifluoromethyl)phenyl)ethyl)amino) pyrido[3,4-*d*]pyridazin-7(6*H*)-one formate; $^1$H NMR (400 MHz, DMSO-$d_6$) δ 13.03 (s, 1H), 10.75 (s, 1H), 9.51 (s, 1H), 8.14 (s, 1H), 7.82-7.56 (m, 4H), 6.55 (s, 1H), 5.42 (s, 1H), 3.49-3.42 (m, 4H), 3.31- 2.50 (m, 9H), 1.62-1.54 (m, 6H), 1.30-1.08 (m, 4H); ESI-MS(m/z): 501.0 $[M+H]^+$. |

(continued)

| Number | Chemical Name | Structure | $^1H$ NMR and MS |
|---|---|---|---|
| **Example 177** | (*R*)-1-(3-(hydroxymethyl)aze tidin-1-yl)-6-(1-methylcyclopropyl)-4-((1-(3-(trifluoromethyl)phe nyl)ethyl)amino)pyr ido [3,4-*d*]pyridazin-7(6*H*)-one formate | 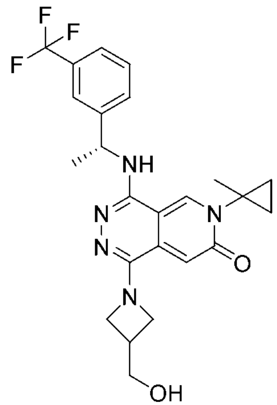 | The crude product was purified by Prep-HPLC (separation method 2), to obtain (*R*)-1-(3-(hydroxymethyl)azetidin-1-yl)-6-(1-methylcyclopropyl)-4-((1-(3-(trifluoromethyl)phenyl)ethyl)amino) pyrido[3,4-*d*]pyridazin-7(6*H*)-one formate; $^1H$ NMR (400 MHz, DMSO-$d_6$) δ 9.01 (s, 1H), 8.20 (s, 1H), 7.78-7.73 (m, 2H), 7.60-7.48 (m, 3H), 7.14 (s, 1H), 5.05- 5.00 (m, 1H), 3.69-3.64 (m, 1H), 3.50- 3.38 (m, 5H), 3.19-3.15 (m, 1H), 2.12- 2.08 (m, 1H), 1.56-1.49 (m, 6H); 1.16- 1.06 (m, 4H); ESI-MS(m/z): 474.0 $[M+H]^+$. |
| **Example 178** | 1-((*R*)-3-(hydroxymethyl)pyr rolidin-1-yl)-6-(1-methylcyclopropyl)-4-(((R)-1-(3-(trifluoromethyl)phe nyl) ethyl)amino)pyr ido[3,4-*d*]pyridazin-7(6*H*)-one formate | 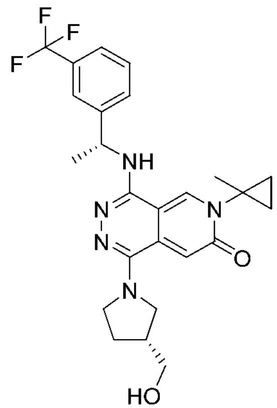 | The crude product was purified by Prep-HPLC (separation method 2), to obtain 1-((*R*)-3-(hydroxymethyl)pyrrolidin-1-yl)-6-(1-methylcyclopropyl)-4-(((*R*)-1-(3-(trifluoromethyl)phenyl)ethyl)amino) pyrido[3,4-*d*]pyridazin-7(6*H*)-one formate; $^1H$ NMR (400 MHz, DMSO-$d_6$) δ 9.03(s, 1H), 8.16 (s, 1H), 7.74-7.53 (m, 4H), 7.32-7.27 (m, 1H), 6.65 (s, 1H), 5.36- 5.31 (m, 1H), 4.63 (s, 1H), 3.46-3.28 (m, 6H), 2.32-2.27 (m, 1H), 1.94-1.90 (m, 1H), 1.59-1.53 (m, 7H), 1.17-1.07 (m, 4H); ESI-MS(m/z): 488.0 $[M+H]^+$. |

(continued)

| Number | Chemical Name | Structure | $^1$H NMR and MS |
|---|---|---|---|
| **Example 179** | 1-((S)-3-(hydroxymethyl)pyr rolidin-1-yl)-6-(1-methylcyclopropyl)-4-(((R)-1-(3-(trifluoromethyl)phe nyl) ethyl)amino)pyr ido[3,4-*d*]pyridazin-7(6*H*)-one formate | 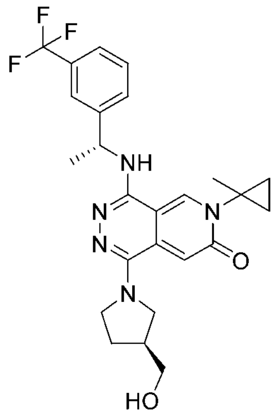 | The crude product was purified by Prep-HPLC (separation method 2) to obtain 1-((S)-3-(hydroxymethyl)pyrrolidin-1-yl)-6-(1-methylcyclopropyl)-4-(((*R*)-1-(3-(trifluoromethyl)phenyl)ethyl)amino) pyrido[3,4-*d*]pyridazin-7(6*H*)-one formate; $^1$H NMR (400 MHz, DMSO-$d_6$) δ 9.12 (s, 1H), 8.14 (s, 1H), 7.75-7.71 (m, 2H), 7.59-7.53 (m, 3H), 6.69 (s, 1H), 5.35- 5.29 (m, 1H), 4.64 (s, 1H), 3.47-3.29 (m, 6H), 2.34-2.30 (m, 1H), 1.93-1.91 (m, 1H), 1.62-1.55 (s, 7H), 1.17-1.08 (m, 4H); ESI-MS(m/z): 488.0 [M+H]$^+$. |
| **Example 180** | 1-((S)-3,4-dimethylpiperazin-1-yl)-6-(1-methylcyclopropyl)-4-(((*R*)-1-(3-(trifluoromethyl)phe nyl)ethyl)amino)pyr ido[3,4-*d*] pyridazin-7(6*H*)-one formate | 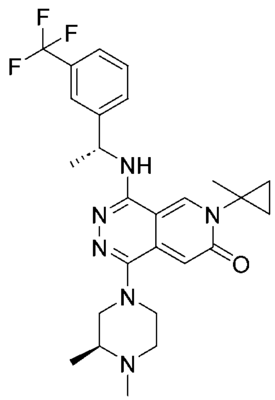 | The crude product was purified by Prep-HPLC (separation method 2) to obtain 1-((S)-3,4-dimethylpiperazin-1-yl)-6-(1-methylcyclopropyl)-4-(((*R*)-1-(3-(trifluoromethyl)phenyl)ethyl)amino) pyrido[3,4-*d*]pyridazin-7(6*H*)-one formate; $^1$H NMR (400 MHz, DMSO-$d_6$) δ 10.85-9.38 (m, 2H), 8.14 (s, 1H), 7.86- 7.61 (m, 4H), 6.61 (s, 1H), 5.45-5.38 (m, 1H), 3.56-3.20 (m, 5H), 2.82-2.74 (m, 4H), 1.66-1.31 (m, 6H), 1.19-1.09 (m, 8H); ESI-MS(m/z): 501.0 [M+H]$^+$. |

(continued)

| Number | Chemical Name | Structure | $^1$H NMR and MS |
|---|---|---|---|
| **Example 181** | (*R*)-1-(4-cyclopropylpiperazi n-1-yl)-6-(1-methylcyclopropyl)-4-((1-(3-(trifluoromethyl)phe nyl)ethyl)amino)pyr ido[3,4-*d*]pyridazin-7(6*H*)-one formate | 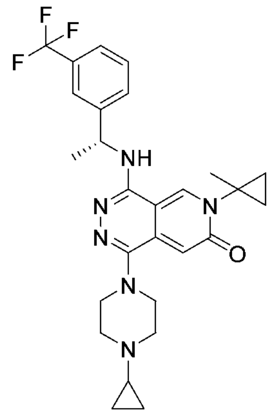 | The crude product was purified by Prep-HPLC (separation method 2) to obtain (*R*)-1-(4-cyclopropylpiperazin-1-yl)-6-(1-methylcyclopropyl)-4-((1-(3-(trifluoromethyl)phenyl)ethyl)amino) pyrido[3,4-*d*]pyridazin-7(6*H*)-one formate; $^1$H NMR (400 MHz, DMSO-$d_6$) δ 9.24 (s, 1H), 8.15 (s, 1H), 7.74-7.53 (m, 5H), 6.42 (s, 1H), 5.42-8.37 (m, 1H), 2.96 (s, 4H), 2.69 (s, 4H), 1.71 (s, 1H), 1.56-1.52 (m, 6H), 1.15-1.07 (m, 4H), 0.44-0.33 (m, 4H); ESI-MS(m/z): 513.0 [M+H]$^+$. |
| **Example 182** | (*R*)-4-((1-(3-(difluoromethyl)-2-fluorophenyl)ethyl) amino)-6-(1-methylcyclopropyl)-1-(7-oxa-2-azaspiro[3.5]nonan-2-yl) pyrido[3,4-*d*]pyridazin-7(6*H*)-one | 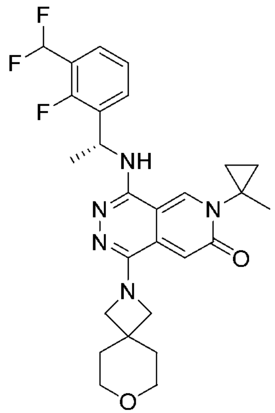 | $^1$H NMR (400 MHz, DMSO-$d_6$) δ 8.60 (s, 1H), 7.60 (t, *J* = 7.6 Hz, 1H), 7.46 (t, *J* = 6.8 Hz, 1H), 7.26 (t, *J* = 7.6 Hz, 1H), 7.22 (t, *J* = 54.4 Hz, 1H), 6.84 (s, 1H), 6.67 (d, *J* = 6.4 Hz, 1H), 5.12-5.05 (m, 1H), 3.59-3.54 (m, 1H), 3.50-3.43 (m, 2H), 3.40-3.37 (m, 1H), 3.23-3.19 (m, 3H), 1.54-1.49 (m, 6H), 1.33-0.99 (m, 9H); ESI-MS (m/z): 514.2 [M+H]$^+$. |

(continued)

| Number | Chemical Name | Structure | $^1$H NMR and MS |
|---|---|---|---|
| **Example 183** | 4-(((*R*)-1-(3-(difluoromethyl)-2-fluorophenyl)ethyl) amino)-1-(7-methyl-2,7-diazaspiro[4.4]nona n-2-yl)-6-(1-methylcyclopropyl) pyrido[3,4-*d*]pyridazin-7(6*H*)-one | 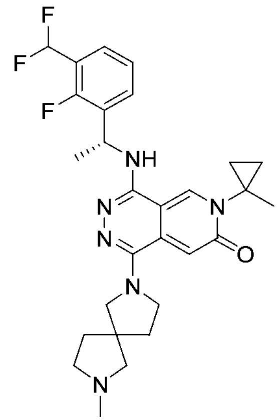 | $^1$H NMR (400 MHz, DMSO-$d_6$) δ 9.09 (s, 1H), 7.61 (t, *J* = 7.2 Hz, 1H), 7.47 (t, *J* = 7.2 Hz, 1H), 7.37-7.10 (m, 3H), 6.60 (s, 1H), 5.53-5.46 (m, 1H), 3.51-3.38 (m, 4H), 2.48-2.30 (m, 4H), 2.19 (s, 3H), 1.88-1.69 (m, 4H), 1.55-1.52 (m, 6H), 1.23-1.05 (m, 4H); ESI-MS (m/z): 527.3 $[M+H]^+$. |
| **Example 184** | (*R*)-4-((1-(3-(difluoromethyl)-2-fluorophenyl)ethyl) amino)-6-(1-methylcyclopropyl)-1-(7-azaspiro[3.5]nonan-7-yl)pyrido [3,4-*d*]pyridazin-7(6*H*)-one | 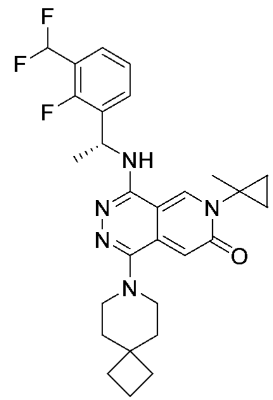 | $^1$H NMR (400 MHz, DMSO-$d_6$) δ 13.09 (s, 1H), 9.72 (s, 1H), 7.70 (t, *J* = 7.2 Hz, 1H), 7.60 (s, 1H), 7.40-7.10(m, 2H), 6.54 (s, 1H), 5.54-5.38(m, 1H), 3.10-2.94 (m, 4H), 1.90-1.65 (m, 13H), 1.55 (s, 3H), 1.25-1.09 (m, 4H); ESI-MS(m/z): 512.0 $[M+H]^+$. |

(continued)

| Number | Chemical Name | Structure | $^1$H NMR and MS |
|---|---|---|---|
| **Example 185** | (*R*)-4-((1-(3-(difluoromethyl)-2-fluorophenyl)ethyl) amino)-6-(1-methylcyclopropyl)-1-(8-azaspiro[4.5]decan-8-yl)pyrido [3,4-*d*]pyridazin-7(6*H*)-one | 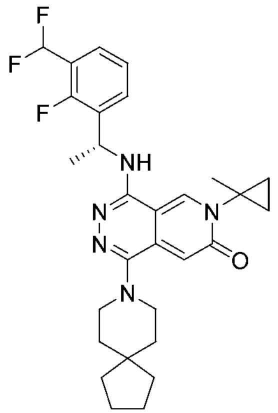 | $^1$H NMR (400 MHz, DMSO-$d_6$) δ 13.10 (s, 1H), 9.78 (s, 1H), 7.72 (t, *J* = 7.2 Hz, 1H), 7.62 (t, *J* = 6.4 Hz, 1H), 7.41-7.11 (m, 2H), 6.57 (s, 1H), 5.50-5.39(m, 1H), 3.16-3.03 (m, 4H), 1.70 (d, *J* = 6.4 Hz, 3H), 1.62-1.54 (m, 11H), 1.48-1.41 (m, 4H), 1.24-1.08 (m, 4H); ESI-MS(m/z): 526.0 $[M+H]^+$. |
| **Example 186** | (*R*)-1-(1,1-difluoro-6-azaspiro[2.5]octan-6-yl)-4-((1-(3-(difluoromethyl)-2-fluorophenyl)ethyl) amino)-6-(1-methylcyclopropyl) pyrido[3,4-*d*]pyridazin-7(6*H*)-one | 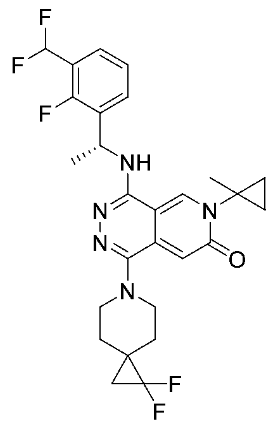 | $^1$H NMR (400 MHz, DMSO-$d_6$) δ 13.15 (s, 1H), 9.68 (s, 1H), 7.79-7.69 (m, 1H), 7.64-7.55 (m, 1H), 7.42-7.11 (m, 2H), 6.57 (s, 1H), 5.60-5.43 (m, 1H), 3.17-3.05 (m, 4H), 1.86-1.64 (m, 7H), 1.56 (s, 3H), 1.34 (t, *J* = 8.4 Hz, 2H), 1.26-1.09 (m, 4H); ESI-MS(m/z): 534.0 $[M+H]^+$. |

(continued)

| Number | Chemical Name | Structure | $^1$H NMR and MS |
|---|---|---|---|
| **Example 187** | (*R*)-4-((1-(3-(difluoromethyl)-2-fluorophenyl)ethyl) amino)-6-(1-methylcyclopropyl)-1-(5-azaspiro[2.4]heptan -5-yl)pyrido [3,4-*d*]pyridazin-7(6*H*)-one | 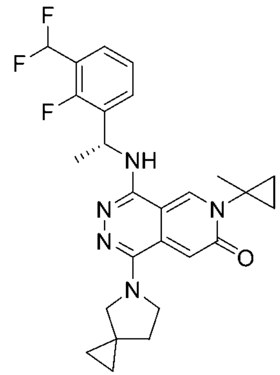 | $^1$H NMR (400 MHz, DMSO-$d_6$) δ 12.52 (s, 1H), 9.32 (s, 1H), 7.66 (t, $J$ = 7.2 Hz, 1H), 7.52 (t, $J$ = 6.8 Hz, 1H), 7.38-7.09 (m, 2H), 6.88 (s, 1H), 5.50-5.35 (m, 1H), 3.80-3.39 (m, 4H), 1.84 (t, $J$ = 6.8 Hz, 2H), 1.65-1.51 (m, 6H), 1.24-1.07 (m, 4H), 0.65-0.54 (m, 4H); ESI-MS(m/z): 484.0 [M+H]$^+$. |
| **Example 188** | 4-(((*R*)-1-(3-(difluoromethyl)-2-fluorophenyl)ethyl) amino)-1-(hexahydrocyclopen ta [c]pyrrol-2(1*H*)-yl)-6-(1-methylcyclopropyl) pyrido[3,4-*d*]pyridazin-7(6*H*)-one | 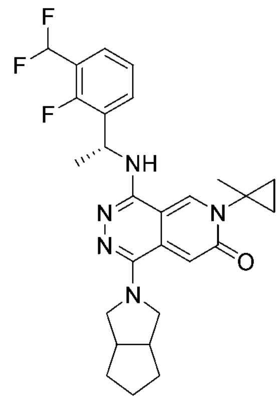 | $^1$H NMR (400 MHz, DMSO-$d_6$) δ 12.52 (s, 1H), 9.48 (s, 1H), 7.69 (t, $J$ = 7.2 Hz, 1H), 7.60-7.51 (m, 1H), 7.38-7.09 (m, 2H), 6.94 (s, 1H), 5.50-5.30 (m, 1H), 3.90-3.46(m, 2H), 2.78-2.64 (m, 2H), 1.83-1.05 (m, 18H); ESI-MS(m/z): 498.0 [M+H]$^+$. |

(continued)

| Number | Chemical Name | Structure | $^1H$ NMR and MS |
|---|---|---|---|
| **Example 189** | 4-(((*R*)-1-(3-(difluoromethyl)-2-fluorophenyl)ethyl) amino)-1-(6,6-dimethyl-3-azabicyclo[3.1.0]he xan-3-yl)-6-(1-methylcyclopropyl) pyrido[3,4-*d*]pyridazin-7(6*H*)-one | 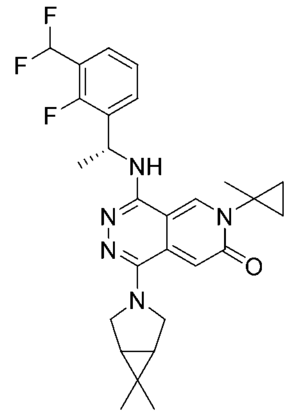 | $^1H$ NMR (400 MHz, DMSO-$d_6$) δ 9.07 (s, 1H), 7.60 (t, *J* = 7.2 Hz, 1H), 7.46 (t, *J* = 6.8 Hz, 1H), 7.38-7.09 (m, 3H), 6.70 (s, 1H), 5.55-5.43 (m, 1H), 3.78-3.43 (m, 4H), 1.57-1.48 (m, 6H), 1.45-1.40 (m, 2H), 1.23-1.06 (m, 4H), 1.01 (s, 3H), 0.91 (s, 3H); ESI-MS(m/z): 498.0 $[M+H]^+$. |
| **Example 190** | (*R*)-4-((1-(3-(difluoromethyl)-2-fluorophenyl)ethyl) amino)-6-(1-methylcyclopropyl)-1-(2-oxa-6-azaspiro[3.3]heptan -6-yl) pyrido[3,4-*d*]pyridazin-7(6*H*)-one | 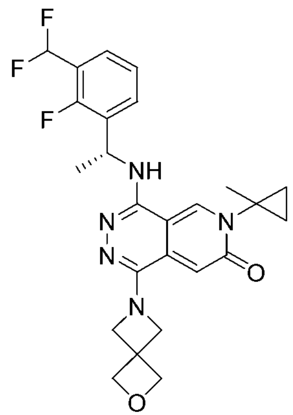 | $^1H$ NMR (400 MHz, DMSO-$d_6$) δ 9.14 (s, 1H), 7.60 (t, *J* = 7.2 Hz, 1H), 7.47 (t, *J* = 6.8 Hz, 1H), 7.38-7.09 (m, 3H), 6.36 (s, 1H), 5.54-5.42 (m, 1H), 4.67 (s, 4H), 4.23-4.14 (m, 4H), 1.55-1.51 (m, 6H), 1.22-1.06 (m, 4H); ESI-MS(m/z): 486.0 $[M+H]^+$. |

(continued)

| Number | Chemical Name | Structure | $^1$H NMR and MS |
|---|---|---|---|
| **Example 191** | (*R*)-4-((1-(3-(difluoromethyl)-2-fluorophenyl)ethyl) amino)-6-(1-methylcyclopropyl)-1-(2-azaspiro[3.3]heptan -2-yl)pyrido [3,4-*d*]pyridazin-7(6*H*)-one | 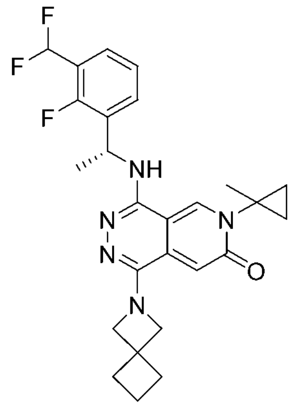 | $^1$H NMR (400 MHz, DMSO-$d_6$) δ 8.59 (s, 1H), 7.62 (t, *J* = 7.2 Hz, 1H), 7.47 (t, *J* = 6.8 Hz, 1H), 7.38-7.09 (m, 2H), 6.83 (s, 1H), 6.65 (d, *J* = 6.8 Hz, 1H), 5.16-5.04 (m, 1H), 3.54-3.31 (m, 4H), 1.94-1.60 (m, 6H), 1.54-1.48 (m, 6H), 1.23-1.02 (m, 4H); ESI-MS(m/z): 484.0 $[M+H]^+$. |
| **Example 192** | 4-(((*R*)-1-(3-(difluoromethyl)-2-fluorophenyl)ethyl) amino)-1-(hexahydropyrrolo [ 1,2-a]pyrazin-2(1*H*)-yl)-6-(1-methylcyclopropyl) pyrido[3,4-*d*]pyridazin-7(6*H*)-one | 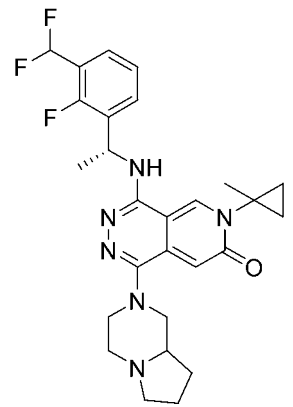 | $^1$H NMR (400 MHz, DMSO-$d_6$) δ 9.23 (s, 1H), 7.63 (t, *J* = 7.2 Hz, 1H), 7.56 (d, *J* = 6.8 Hz, 1H), 7.48 (t, *J* = 6.8 Hz, 1H), 7.38-7.09 (m, 2 H), 6.40 (s, 1H), 5.62-5.50 (m, 1H), 3.42-3.21 (m, 2H), 3.01-2.93 (m, 2H), 2.85-2.76 (m, 1H), 2.49-2.43 (m, 2H), 2.33-1.64 (m, 6H), 1.57-1.50 (m, 6H), 1.30-1.08 (m, 4H); ESI-MS(m/z): 513.0 $[M+H]^+$. |

(continued)

| Number | Chemical Name | Structure | $^1$H NMR and MS |
|---|---|---|---|
| **Example 193** | 4-(((*R*)-1-(3-(difluoromethyl)-2-fluorophenyl)ethyl) amino)-1-8-methyl-3,8-diazabicyclo[3.2.1]o ctan-3-yl)-6-(1-methylcyclopropyl) pyrido[3,4-*d*]pyridazin-7(6*H*)-one | 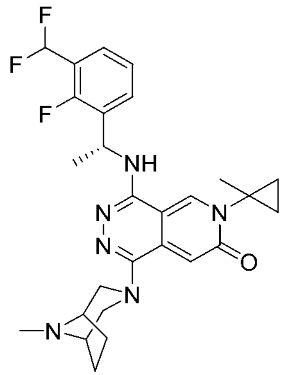 | $^1$H NMR (400 MHz, DMSO-$d_6$) δ 9.22 (s, 1H), 7.61 (t, *J* = 7.2 Hz, 1H), 7.54 (d, *J* = 6.8 Hz, 1H), 7.48 (t, *J* = 6.8 Hz, 1H), 7.38-7.09 (m, 2H), 6.46 (s, 1H), 5.60-5.50 (m, 1H), 3.15-3.06 (m, 2H), 3.01-2.87 (m, 4H), 2.18 (s, 3H), 2.01-1.93 (m, 2H), 1.85-1.76 (m, 2H), 1.56-1.51 (m, 6H), 1.24-1.07 (m, 4H); ESI-MS(m/z): 513.0 $[M+H]^+$. |
| **Example 194** | (*R*)-4-((1-(3-(difluoromethyl)-2-fluorophenyl)ethyl) amino)-1-(4-(2-hydroxyethyl)pipera zin-1-yl)-6-(1-methylcyclopropyl) pyrido[3,4-*d*]pyridazin-7(6*H*)-one | 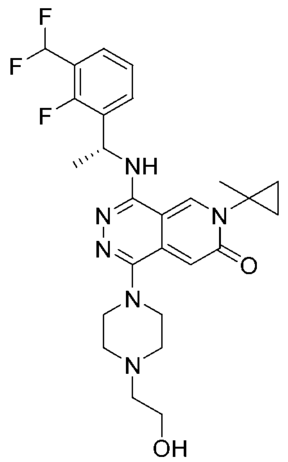 | $^1$H NMR (400 MHz, DMSO-$d_6$) δ 9.22 (s, 1H), 7.62 (t, *J* = 7.2 Hz, 1H), 7.56 (d, *J* = 6.8 Hz, 1H), 7.48 (t, *J* = 7.2 Hz, 1H), 7.39-7.09 (m, 2H), 6.39 (s, 1H), 5.62-5.52 (m, 1H), 4.38 (t, *J* = 5.2 Hz, 1H), 3.51 (q, *J* = 6.0 Hz, 2H), 3.05-2.93 (m, 4H), 2.63-2.53 (m, 4H), 2.44 (t, *J* = 6.2 Hz, 2H), 1.56-1.52 (m, 6H), 1.21-1.06 (m, 4H); ESI-MS(m/z): 517.0 $[M+H]^+$. |

(continued)

| Number | Chemical Name | Structure | $^1$H NMR and MS |
|---|---|---|---|
| **Example 195** | 4-(((*R*)-1-(3-(difluoromethyl)-2-fluorophenyl)ethyl) amino)-1-((S)-2,4-dimethylpiperazin-1-yl)-6-(1-methylcyclopropyl) pyrido [3,4-*d*]pyridazin-7(6*H*)-one | 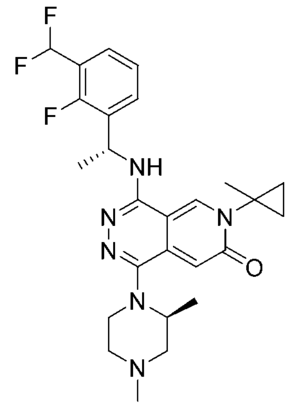 | $^1$H NMR (400 MHz, DMSO-$d_6$) δ 9.24 (s, 1H), 7.70-7.60 (m, 2H), 7.49 (t, $J$ = 6.8 Hz, 1H), 7.39-7.10 (m, 2H), 6.48 (s, 1H), 5.70-5.55 (m, 1H), 3.50-3.39 (m, 1H), 3.11-3.02 (m, 1H), 2.71-2.60 (m, 2H), 2.56-2.50 (m, 1H), 2.36-2.26 (m, 1H), 2.18 (s, 3H), 2.08-1.98 (m, 1H), 1.58-1.52 (m, 6H), 1.24-1.06 (m, 4H), 0.86 (d, $J$ = 6.0 Hz, 3H); ESI-MS(m/z): 501.0 $[M+H]^+$. |
| **Example 196** | (*R*)-4-((1-(3-(difluoromethyl)-2-fluorophenyl)ethyl) amino)-1-(6-hydroxy-2-azaspiro [3.3]heptan -2-yl)-6-(1-methylcyclopropyl) pyrido[3,4-*d*]pyridazin-7(6*H*)-one | 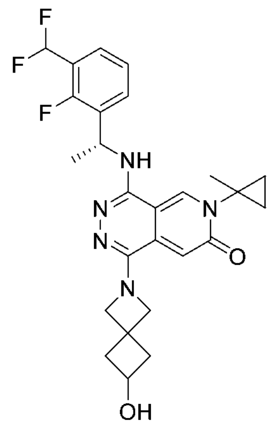 | $^1$H NMR (400 MHz, DMSO-$d_6$) δ 9.03 (s, 1H), 8.25 (s, 1H),7.68 (t, $J$ = 7.2 Hz, 1H), 7.58-7.45 (m, 2H), 7.38-7.09 (m, 3H), 5.24-5.16(m, 1H), 4.24-4.13 (m, 1H), 3.76-3.71 (m, 1H), 3.55-3.48 (m, 1H), 3.35-3.29 (m, 2H), 2.15-1.60 (m, 4H), 1.58-1.50 (m, 6H), 1.23-1.03 (m, 4H); ESI-MS(m/z): 500.0 $[M+H]^+$. |

(continued)

| Number | Chemical Name | Structure | $^1$H NMR and MS |
|---|---|---|---|
| **Example 197** | (*R*)-4-((1-(3-(difluoromethyl)-2-fluorophenyl)ethyl) amino)-1-(2,2-dioxido-2-thia-6-azaspiro[3.4]octan-6-yl)-6-(1-methylcyclopropyl) pyrido[3,4-*d*]pyridazin-7(6*H*)-one | 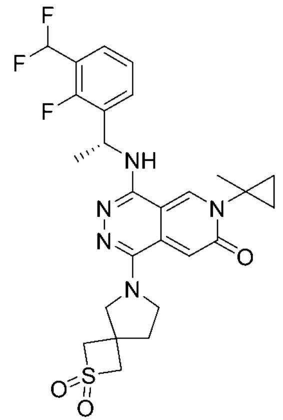 | $^1$H NMR (400 MHz, DMSO-$d_6$) δ 9.12 (s, 1H), 7.61 (t, *J* = 7.2 Hz, 1H), 7.47 (t, *J* = 6.8 Hz, 1H), 7.38-7.09 (m, 3H), 6.58 (s, 1H), 5.54-5.46 (m, 1H), 4.25-4.06 (m, 4H), 3.66 (s, 2H), 3.54-3.44 (m, 2H), 2.20-2.12(m, 2H), 1.59-1.48 (m, 6H), 1.24-1.07 (m, 4H); ESI-MS(m/z): 548.0 $[M+H]^+$. |
| **Example 198** | 1-(8-oxa-3-azabicyclo[3.2.1]oct an-3-yl)-4-(((*R*)-1-(3-(difluoromethyl)-2-fluorophenyl)ethyl) amino)-6-(1-methylcyclopropyl) pyrido[3,4-*d*]pyridazin-7(6*H*)-one | 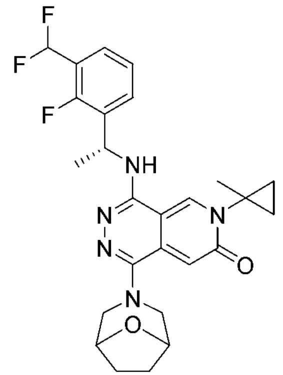 | $^1$H NMR (400 MHz, DMSO-$d_6$) δ 9.23 (s, 1H), 7.65-7.54 (m, 2H), 7.48 (t, *J* = 6.8 Hz, 1H), 7.38-7.09 (m, 2H), 6.44 (s, 1H), 5.62-5.50 (m, 1H), 4.36-4.26 (m, 2H), 3.06-2.89 (m, 4H), 2.04-1.83 (m, 4H), 1.58-1.50 (m, 6H), 1.25-1.07 (m, 4H); ESI-MS(m/z): 500.0 $[M+H]^+$. |

(continued)

| Number | Chemical Name | Structure | $^1$H NMR and MS |
|---|---|---|---|
| **Example 199** | (*R*)-4-((1-(3-(difluoromethyl)-2-fluorophenyl)ethyl) amino)-6-(1-methylcyclopropyl)-1-(1-oxa-8-azaspiro[4.5]decan-8-yl) pyrido[3,4-*d*]pyridazin-7(6*H*)-one | 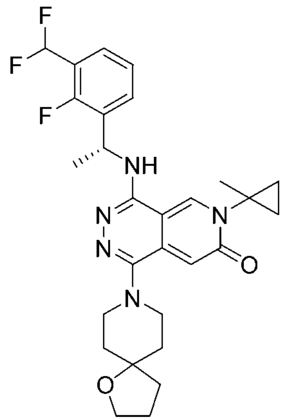 | $^1$H NMR (400 MHz, DMSO-$d_6$) δ 9.21 (s, 1H), 7.62 (t, $J$ = 7.2 Hz, 1H), 7.56-7.45 (m, 2H), 7.38-7.09 (m, 2H), 6.39 (s, 1H), 5.63-5.50 (m, 1H), 3.72 (t, $J$ = 6.8 Hz, 2H), 3.10-2.90 (m, 4H), 1.92-1.81 (m, 2H), 1.75-1.60 (m, 6H), 1.58-1.50 (m, 6H), 1.25-1.05 (m, 4H); ESI-MS(m/z): 528.0 [M+H]$^+$. |
| **Example 200** | (*R*)-4-((1-(3-(difluoromethyl)-2-fluorophenyl)ethyl) amino)-6-(1-methylcyclopropyl)-1-(8-oxa-2-azaspiro[4.5]decan-2-yl) pyrido[3,4-*d*]pyridazin-7(6*H*)-one | 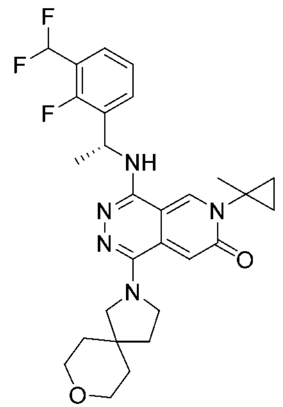 | $^1$H NMR (400 MHz, DMSO-$d_6$) δ 9.10 (s, 1H), 7.61 (t, $J$ = 7.2 Hz, 1H), 7.47 (t, $J$ = 6.8 Hz, 1H), 7.38-7.09 (m, 3H), 6.62 (s, 1H), 5.50-5.45 (m, 1H), 3.63-3.42 (m, 6H), 3.29 (s, 2H), 1.76 (t, $J$ = 7.2 Hz, 2H), 1.57-1.47 (m, 10H), 1.24-1.06 (m, 4H); ESI-MS(m/z): 528.0 [M+H]$^+$. |

(continued)

| Number | Chemical Name | Structure | $^1H$ NMR and MS |
|---|---|---|---|
| **Example 201** | 1-(2-oxa-5-azabicyclo[2.2.1]he ptan-5-yl)-4-(((*R*)-1-(3-(difluoromethyl)-2-fluorophenyl)ethyl) amino)-6-(1-methylcyclopropyl) pyrido[3,4-*d*]pyridazin-7(6*H*)-one | 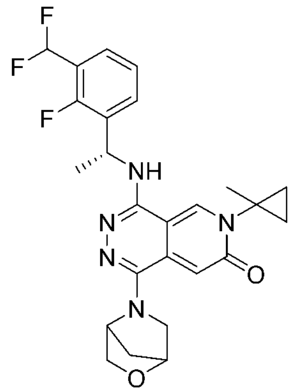 | $^1H$ NMR (400 MHz, DMSO-$d_6$) δ 9.15 (s, 1H), 7.67-7.58 (m, 1H), 7.52-7.44(m, 1H), 7.38-7.09 (m, 3H), 6.50 (d, *J* = 4.4 Hz, 1H), 5.60-5.45 (m, 1H), 4.55-4.43 (m, 2H), 4.02-3.84 (m, 1H) 3.78-3.61 (m, 2H), 3.29-3.16 (m, 1H), 1.84-1.66 (m, 2H), 1.58-1.48 (m, 6H), 1.23-1.05 (m, 4H); ESI-MS(m/z): 486.0 $[M+H]^+$. |
| **Example 202** | 1-(3-oxa-8-azabicyclo[3.2.1]oct an-8-yl)-4-(((*R*)-1-(3-(difluoromethyl)-2-fluorophenyl)ethyl) amino)-6-(1-methylcyclopropyl) pyrido[3,4-*d*]pyridazin-7(6*H*)-one | 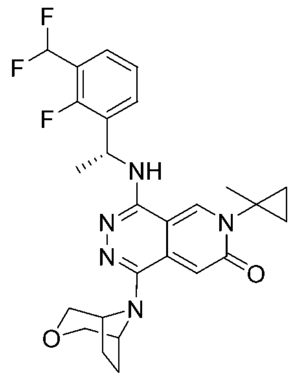 | $^1H$ NMR (400 MHz, DMSO-$d_6$) δ 9.27 (s, 1H), 7.56-7.09 (m, 5H), 6.55 (s, 1H), 5.63-5.48 (m, 1H), 3.98-3.70 (m, 4H), 3.62-3.51 (m, 2H), 1.91-1.73 (m, 4H), 1.61-1.52 (m, 6H), 1.24-1.08 (m, 4H); ESI-MS(m/z): 500.0 $[M+H]^+$. |

(continued)

| Number | Chemical Name | Structure | $^1$H NMR and MS |
|---|---|---|---|
| **Example 203** | 4-(((*R*)-1-(3-(difluoromethyl)-2-fluorophenyl)ethyl) amino)-6-(1-methylcyclopropyl)-1-(octahydro-2H-pyrido[1,2-a]pyrazin-2-yl)pyrido[3,4-*d*]pyridazin-7(6*H*)-one | 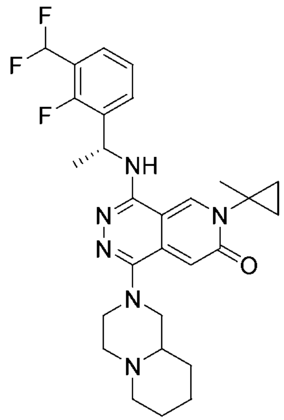 | $^1$H NMR (400 MHz, DMSO-$d_6$) δ 9.22 (s, 1H), 7.62 (t, *J* = 7.2 Hz, 1H), 7.55 (d, *J* = 6.8 Hz, 1H), 7.48 (t, *J* = 6.8 Hz, 1H), 7.38-7.09 (m, 2H), 6.40 (s, 1H), 5.62-5.50 (m, 1H), 3.27-3.06 (m, 2H), 2.87-2.63 (m, 3H), 2.48-2.25 (m, 2H), 2.11-1.94 (m, 2H), 1.71-1.43 (m, 10H), 1.27-1.04 (m, 6H); ESI-MS(m/z): 527.0 [M+H]$^+$. |
| **Example 204** | (*R*)-4-((1-(3-(difluoromethyl)-2-fluorophenyl)ethyl) amino)-6-(1-methylcyclopropyl)-1-(3-oxo-2,8-diazaspiro[4.5]deca n-8-yl)pyrido[3,4-*d*]pyridazin-7(6*H*)-one | 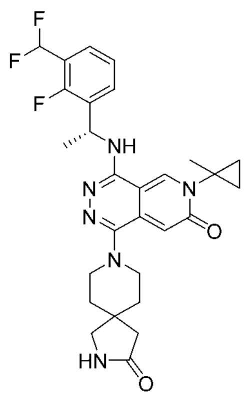 | $^1$H NMR (400 MHz, DMSO-$d_6$) δ 13.13 (s, 1H), 9.34 (s, 1H), 7.72-7.61 (m, 1H), 7.60-7.48 (m, 2H), 7.39-7.10 (m, 2H), 6.46 (s, 1H), 5.62-5.43 (m, 1H), 3.14-2.92 (m, 6H), 2.09 (s, 2H), 1.74-1.52 (m, 10H), 1.24-1.07 (m, 4H); ESI-MS(m/z): 541.0 [M+H]$^+$. |

(continued)

| Number | Chemical Name | Structure | $^1$H NMR and MS |
|---|---|---|---|
| **Example 205** | 4-(((*R*)-1-(3-(difluoromethyl)-2-fluorophenyl)ethyl) amino)-6-(1-methylcyclopropyl)-1-(tetrahydro-1*H*-furo[3,4-c]pyrrol-5(3*H*)-yl)pyrido[3,4-*d*]pyridazin-7(6*H*)-one | 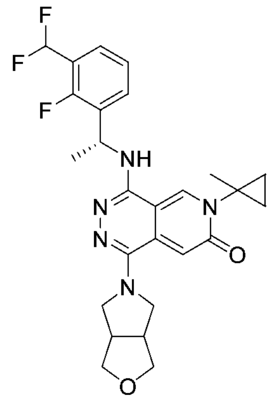 | $^1$H NMR (400 MHz, DMSO-$d_6$) δ 9.19 (s, 1H), 7.61 (t, *J* = 7.2 Hz, 1H), 7.53-7.43 (m, 2H), 7.37-7.09 (m, 2H), 6.50 (s, 1H), 5.60-5.48 (m, 1H), 3.87-3.75 (m, 2H), 3.54-3.42 (m, 2H), 3.30-3.22 (m, 2H), 3.09-2.99 (m, 2H), 2.90-2.80 (m, 2H), 1.59-1.49 (m, 6H), 1.23-1.06 (m, 4H); ESI-MS(m/z): 500.0 $[M+H]^+$. |
| **Example 206** | 4-(((*R*)-1-(3-(difluoromethyl)-2-fluorophenyl)ethyl) amino)-6-(1-methylcyclopropyl)-1-(5-methylhexahydropy rrolo[3,4-c] pyrrol-2(1*H*)-yl)pyrido[3,4-*d*]pyridazin-7(6*H*)-one | 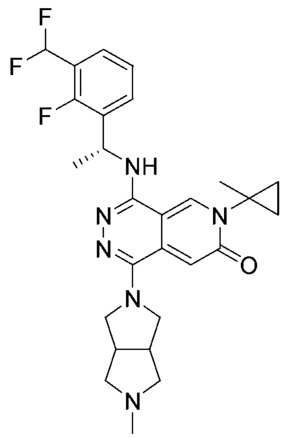 | $^1$H NMR (400 MHz, DMSO-$d_6$) δ 9.19 (s, 1H), 7.61 (t, *J* = 7.3 Hz, 1H), 7.53-7.43 (m, 2H), 7.38-7.09 (m, 2H), 6.49 (s, 1H), 5.60-5.50 (m, 1H), 3.27-3.18 (m, 2H), 3.02-2.92 (m, 2H), 2.79-2.69 (m, 2H), 2.65-2.54 (m, 2H), 2.28-2.18 (m, 5H), 1.59-1.49 (m, 6H), 1.25-1.05 (m, 4H); ESI-MS(m/z): 513.0 $[M+H]^+$. |

(continued)

| Number | Chemical Name | Structure | $^1$H NMR and MS |
|---|---|---|---|
| **Example 207** | 4-(((*R*)-1-(1,1-difluoro-2,3-dihydro-1H-inden-4-yl)ethyl)amino)-1-((S)-3-(hydroxymethyl)pip eridin-1-yl)-6-(1-methylcyclopropyl) pyrido[3,4-*d*]pyridazin-7(6*H*)-one | 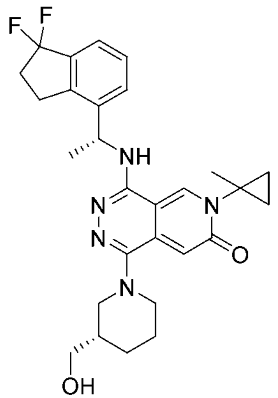 | $^1$H NMR (400MHz,DMSO-$d_6$) δ 9.18 (s, 1H), 7.57 (d, *J* = 7.2 Hz, 1H), 7.47 (d, *J* = 6.8 Hz, 1H), 7.43-7.34 (m, 2H), 6.40 (s, 1H), 5.46-5.31 (m, 1H), 4.50-4.46 (m, 1H), 3.40-3.31 (m, 2H), 3.28-3.05 (m, 4H), 2.71-2.56 (m, 3H), 2.42-2.32 (m, 1H), 1.83-1.57(m, 4H), 1.56-1.49 (m, 6H), 1.20-1.00 (m, 5H); ESI-MS(m/z): 510.0 $[M+H]^+$. |
| **Example 208** | (*R*)-4-((1-(1,1-difluoro-2,3-dihydro-1*H*-inden-4-yl)ethyl)amino)-6-(1-methylcyclopropyl)-1-(4-methylpiperazin-1-yl)pyrido [3,4-*d*]pyridazin-7(6*H*)-one | 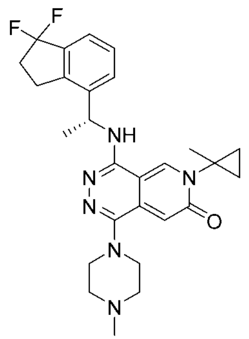 | $^1$H NMR (400MHz,DMSO-$d_6$) δ 9.20 (s, 1H), 7.57 (d, *J* = 6.8 Hz, 1H), 7.50 (d, *J* = 6.0 Hz, 1H), 7.44-7.32 (m, 2H), 6.38 (s, 1H), 5.45-5.33 (m, 1H), 3.25-3.08 (m, 2H), 3.05-2.90 (m, 4H), 2.68-2.58 (m, 2H), 2.48-2.42 (m, 4H), 2.22 (s, 3H), 1.59-1.45 (m, 6H), 1.20-0.97 (m, 4H); ESI-MS(m/z): 495.0 $[M+H]^+$. |

**Example 209**

**Preparation of (*R*)-4-((1-(3-((difluoromethyl)sulfonyl)-2-methylphenyl)ethyl)amino)-6-(1-methylcyclopropyl)-1-(4-methylpiperazin-1-yl)pyrido[3,4-*d*]pyridazin-7(6*H*)-one**

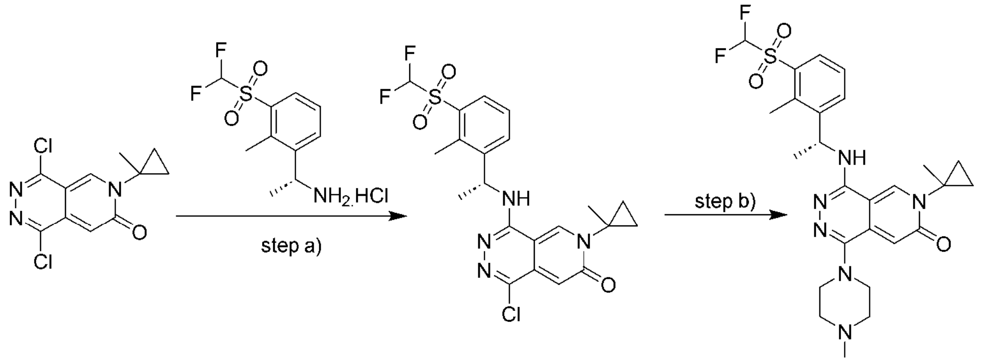


step a): Preparation of (*R*)-1-chloro-4-((1-(3-((difluoromethyl)sulfonyl)-2-methylphenyl)ethyl)amino)-6- (1-methylcyclopropyl)pyrido[3,4-*d*]pyridazin-7(6*H*)-one

1,4-Dichloro-6-(1-methylcyclopropyl)pyrido[3,4-*d*]pyridazin-7(6*H*)-one (50 mg, 0.185 mmol), (*R*)-1-(3-((difluoromethyl)sulfonyl)-2-methylphenyl)ethan-1-amine hydrochloride (58 mg, 0.204 mmol), *N,N*-diisopropylethylamine (72 mg, 0.555 mmol), and dimethyl sulfoxide (5 mL) were added to a reaction flask. The mixture was warmed to 120°C and reacted for 1.5 h. After the reaction was completed, the mixture was cooled to room temperature, quenched with saturated aqueous ammonium chloride solution (50 mL), extracted with ethyl acetate (30 mL × 3). The organic phases were combined, washed with saturated brine (60 mL), dried over anhydrous sodium sulfate, filtered, and the filtrate was concentrated under reduced pressure. The residue was purified by silica gel column chromatography (eluent: petroleum ether/ethyl acetate=1:3) to obtain (*R*)-1-chloro-4-((1-(3-((difluoromethyl)sulfonyl)-2-methylphenyl) ethyl)amino)-6-(1-methylcyclopropyl)pyrido[3,4-*d*]pyridazin-7(6*H*)-one; yield: 67.2%; ESI-MS (m/z): 483.1 $[M+H]^+$

step b): Preparation of (*R*)-4-((1-(3-((difluoromethyl)sulfonyl)-2-methylphenyl)ethyl)amino)-6-(1-methylcyclopropyl) -1-(4-methylpiperazin-1-yl)pyrido[3,4-*d*]pyridazin-7(6*H*)-one

(*R*)-1-Chloro-4-((1-(3-((difluoromethyl)sulfonyl)-2-methylphenyl)ethyl)amino)-6-(1-methylcyclopropyl)pyrido [3,4-*d*]pyridazin-7(6*H*)-one (60 mg, 0.124 mmol), *N*- methylpiperazine (62 mg, 0.620 mmol) and 1,4-dioxane (2 mL) were added to a microwave tube. The mixture was warmed to 110 °C in a microwave reactor and reacted for 1 h. After the reaction was completed,the mixture was cooled to room temperature and concentrated under reduced pressure. The residue was purified by Prep-HPLC (separation method 3) to obtain (R)-4-((1-(3-((difluoromethyl)sulfonyl)-2-methylphenyl)ethyl)amino) -6-(1-methylcyclopropyl)-1-(4-methylpiperazin-1-yl)pyrido[3,4-*d*]pyridazin-7(6*H*)-one; yield: 30.9%; $^1H$ NMR (400 MHz, DMSO-$d_6$) δ 9.21 (s, 1H), 7.96 (d, *J* = 7.6 Hz, 1H), 7.89 (d, *J* = 8.0 Hz, 1H), 7.62 (d, *J* = 6.8 Hz, 1H), 7.54 (t, *J* = 8.0 Hz, 1H), 7.34 (t, *J* = 52.0 Hz, 1H), 6.38 (s, 1H), 5.57 (p, *J* = 6.8 Hz, 1H), 3.08-2.88 (m, 4H), 2.76 (s, 3H), 2.49-2.37 (m, 4H), 2.22 (s, 3H), 1.54 (s, 3H), 1.50 (d, *J* = 6.9 Hz, 3H), 1.20-1.03 (m, 4H); ESI-MS (m/z): 547.2 $[M+H]^+$.

According to the preparation method of **Example 209,** with the corresponding raw materials, the compounds in the following Examples were prepared.

| Number | Chemical Name | Structure | $^1$H NMR and MS |
|---|---|---|---|
| **Example 210** | (*R*)-6-cyclopropyl-4-((1-(3-(difluoromethyl)-2-methylphenyl)ethyl) amino)-1-(4-methylpiperazin-1-yl)pyrido [3,4-*d*]pyridazin-7(6*H*)-one | 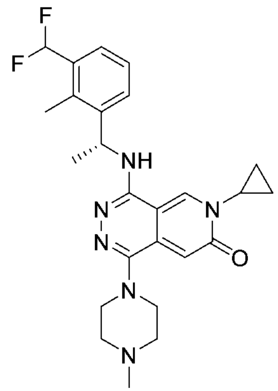 | $^1$H NMR (400 MHz, DMSO-$d_6$) δ 8.98 (s, 1H), 7.61 (d, *J* = 7.6 Hz, 1H), 7.56 (d, *J* = 6.8 Hz, 1H), 7.39 (d, *J* = 7.6 Hz, 1H), 7.28 (t, *J* = 7.6 Hz, 1H), 7.22 (t, *J* = 55.2 Hz, 1H), 6.41 (s, 1H), 5.60-5.54 (m, 1H), 3.64-3.58 (m, 1H), 2.99 (s, 4H), 2.46 (s, 7H), 2.23 (s, 3H), 1.47 (d, *J* = 6.8 Hz, 3H), 1.18-1.08 (m, 4H); ESI-MS (m/z): 469.2 $[M+H]^+$. |
| **Example 211** | (*R*)-6-cyclopropyl-4-((1-(3-(difluoromethyl)-2-methylphenyl)ethyl) amino)-1-(4-isopropylpiperazin-1-yl)pyrido [3,4-*d*]pyridazin-7(6*H*)-one | 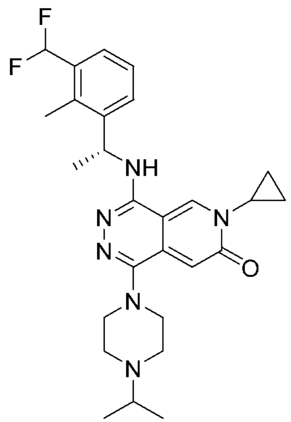 | $^1$H NMR (400 MHz, DMSO-$d_6$) δ 8.98 (s, 1H), 7.61 (d, *J* = 7.6 Hz, 1H), 7.54 (d, *J* = 6.8 Hz, 1H), 7.39 (d, *J* = 7.6 Hz, 1H), 7.28 (t, *J* = 8.0 Hz, 1H), 7.22 (t, *J* = 55.2 Hz, 1H), 6.42 (s, 1H), 5.60-5.54 (m, 1H), 3.64-3.59 (m, 1H), 2.99 (s, 4H), 2.70-2.58 (m, 5H), 2.46 (s, 3H), 1.47 (d, *J* = 6.8 Hz, 3H), 1.17-1.09 (m, 4H), 1.01 (d, *J* = 6.8 Hz, 6H); ESI-MS (m/z): 497.3 $[M+H]^+$. |

(continued)

| Number | Chemical Name | Structure | $^1$H NMR and MS |
|---|---|---|---|
| **Example 212** | (*R*)-6-cyclopropyl-1-(4-cyclopropylpiperazin-1-yl)-4-((1-(3-(difluoromethyl)-2-methylphenyl)ethyl) amino)pyrido[3,4-*d*]pyridazin-7(6*H*)-one | 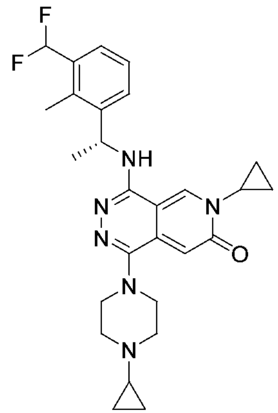 | $^1$H NMR (400 MHz, DMSO-$d_6$) δ 9.04 (s, 1H), 7.66 (d, *J* = 7.6 Hz, 1H), 7.60 (d, *J* = 6.8 Hz, 1H), 7.45 (d, *J* = 7.6 Hz, 1H), 7.34 (t, *J* = 7.6 Hz, 1H), 7.27 (t, *J* = 55.2 Hz, 1H), 6.49 (s, 1H), 5.66-5.59 (m, 1H), 3.70-3.64 (m, 1H), 3.01 (s, 4H), 2.76-2.73 (m, 4H), 2.51 (s, 3H), 1.78-1.72 (m, 1H), 1.53 (d, *J* = 6.8 Hz, 3H), 1.24-1.14 (m, 4H), 0.51-0.37 (m, 4H); ESI-MS (m/z): 495.3 $[M+H]^+$. |
| **Example 213** | (*R*)-4-((1-(3-(difluoromethyl)-2-methylphenyl)ethyl) amino)-6-(1-methylcyclopropyl)-1-(4-methylpiperazin-1-yl)pyrido [3,4-*d*]pyridazin-7(6*H*)-one formate | 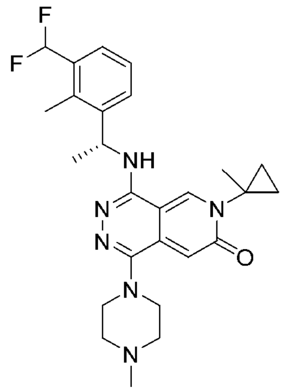 | The crude product was purified by Prep-HPLC (separation method 2) to obtain (*R*)-4-((1-(3-(difluoromethyl)-2-methylphenyl)ethyl)amino)-6-(1-methylcyclopropyl)-1-(4-methylpiperazin-1-yl)pyrido[3,4-*d*]pyridazin-7(6*H*)-one formate; $^1$H NMR (400MHz,DMSO-*d6*) δ 9.22 (s, 1H), 8.16 (s, 1H), 7.62 (d, *J* = 7.6 Hz, 1H), 7.55 (d, *J* = 6.8 Hz, 1H), 7.39 (d, *J* = 7.6 Hz, 1H), 7.36-7.08 (m, 2H), 6.39 (s, 1H), 5.58-5.51 (m, 1H), 3.08-2.94 (m, 4H), 2.49-2.42 (m, 7H), 2.24 (s, 3H), 1.54 (s, 3H), 1.48 (d, *J* = 6.8 Hz, 3H), 1.19-1.04 (m, 4H); ESI-MS(m/z): 483.0 $[M+H]^+$. |

(continued)

| Number | Chemical Name | Structure | $^1$H NMR and MS |
|---|---|---|---|
| **Example 214** | (*R*)-6-(1-methylcyclopropyl)-1-(4-methylpiperazin-1-yl)-4-((1-(3-(pentafluoro-$\lambda^6$-sul-faneyl)phenyl)et hyl)amino)pyrido[3, 4-*d*] pyridazin-7(6*H*)-one formate | 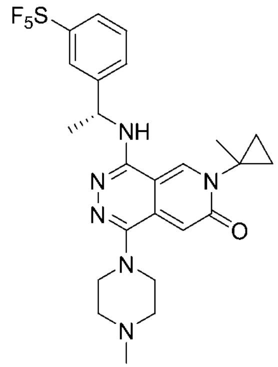 | The crude product was purified by Prep-HPLC (separation method 2) to obtain (*R*)-6-(1-methylcyclopropyl)-1-(4-methylpiperazin-1-yl)-4-((1-(3-(pentafluoro-$\lambda^6$-sulfaneyl)phenyl)ethyl)amino)pyrido[ 3,4-*d*]pyridazin-7(6*H*)-one formate; $^1$H NMR (400MHz,DMSO-*d*6) $\delta$ 9.18 (s, 1H), 8.18 (s, 1H), 7.89 (s, 1H), 7.76-7.67 (m, 2H), 7.59-7.52 (m, 2H), 6.39 (s, 1H), 5.44-5.36 (m, 1H), 3.06-2.94 (m, 4H), 2.50-2.43 (m, 4H), 2.22 (s, 3H), 1.58-1.52 (m, 6H), 1.17-1.11 (m, 2H), 1.10-1.05 (m, 2H); ESI-MS(m/z): 545.0 [M+H]$^+$. |
| **Example 215** | (*R*)-3-(1-((6-cyclopropyl-1-(4-isopropylpiperazin-1-yl)-7-oxo-6,7-dihydropyrido [3,4-*d*]pyridazin-4-yl)amino)ethyl)-2-methylbenzonitrile | 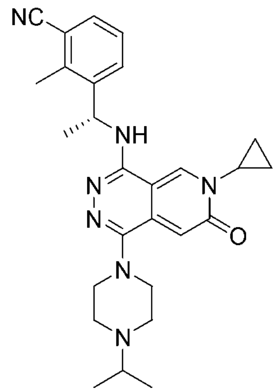 | $^1$H NMR (400 MHz, DMSO-$d_6$) $\delta$ 8.96 (s, 1H), 7.73 (d, *J* = 7.6 Hz, 1H), 7.64 (d, *J* = 7.2 Hz, 1H), 7.57 (d, *J* = 6.8 Hz, 1H), 7.35 (t, *J* = 7.6 Hz, 1H), 6.42 (s, 1H), 5.50-5.43 (m, 1H), 3.64-3.59 (m, 1H), 2.99 (s, 4H), 2.71-2.67 (m, 1H), 2.62-2.58 (m, 7H), 1.48 (d, *J* = 6.8 Hz, 3H), 1.19-1.08 (m, 4H), 1.01 (d, *J* = 6.4 Hz, 6H); ESI-MS (m/z): 472.3 [M+H]$^+$. |

(continued)

| Number | Chemical Name | Structure | $^1$H NMR and MS |
|---|---|---|---|
| **Example 216** | (*R*)-4-((1-(3-(difluoromethyl)-2-fluorophenyl)ethyl) amino)-1-(4-ethylpiperazin-1-yl)-6-(1-methylcyclopropyl) pyrido[3,4-*d*] pyridazin-7(6*H*)-one | 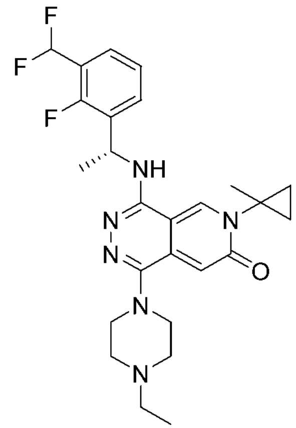 | $^1$H NMR (400 MHz, DMSO-$d_6$) δ 9.22 (s, 1H), 7.62 (t, *J* = 7.2 Hz, 1H), 7.56 (d, *J* = 6.8 Hz, 1H), 7.48 (t, *J* = 7.2 Hz, 1H), 7.27 (t, *J* = 7.6 Hz, 1H), 7.24 (t, *J* = 52.0 Hz, 1H), 6.39 (s, 1H), 5.61-5.52 (m, 1H), 3.09-2.87 (m, 4H), 2.52-2.51 (m, 4H), 2.41-2.35 (m, 2H), 1.58-1.50 (m, 6H), 1.22-1.06 (m, 4H), 1.01 (t, *J* = 7.2 Hz, 3H); ESI-MS(m/z): 501.0 [M+H]$^+$. |
| **Example 217** | (*R*)-4-((1-(3-(difluoromethyl)-2-fluorophenyl)ethyl) amino)-1-(4-isopropylpiperazin-1-yl)-6-(1-methylcyclopropyl) pyrido [3,4-*d*]pyridazin-7(6*H*)-one | 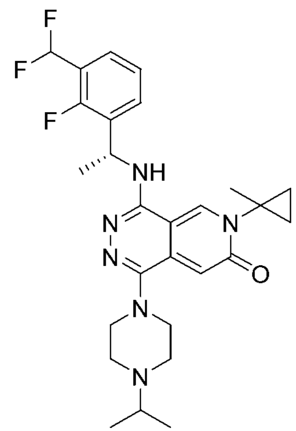 | $^1$H NMR (400 MHz, DMSO-$d_6$) δ 9.22 (s, 1H), 7.62 (t, *J* = 7.6 Hz, 1H), 7.55 (d, *J* = 6.8 Hz, 1H), 7.48 (t, *J* = 7.2 Hz, 1H), 7.27 (t, *J* = 7.6 Hz, 1H), 7.27 (t, *J* = 52.0 Hz, 1H), 6.40 (s, 1H), 5.62-5.51 (m, 1H), 3.05-2.91 (m, 4H), 2.70-2.63 (m, 1H), 2.61-2.55 (m, 4H), 1.58-1.51 (m, 6H), 1.21-1.06 (m, 4H), 1.00 (d, *J* = 6.4 Hz, 6H); ESI-MS (m/z): 515.0 [M+H]$^+$. |

(continued)

| Number | Chemical Name | Structure | $^1$H NMR and MS |
|---|---|---|---|
| **Example 218** | (*R*)-1-(4-cyclopropylpiperazi n-1-yl)-4-((1-(3-(difluoromethyl)-2-fluorophenyl)ethyl) amino)-6-(1-methylcyclopropyl) pyrido[3,4-*d*]pyridazin-7(6*H*)-one | 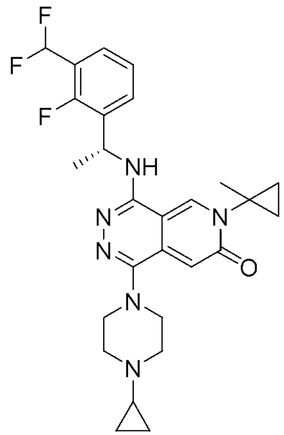 | $^1$H NMR (400 MHz, DMSO-$d_6$) δ 9.22 (s, 1H), 7.62 (t, *J* = 7.6 Hz, 1H), 7.55 (d, *J* = 6.8 Hz, 1H), 7.48 (t, *J* = 7.2 Hz, 1H), 7.27 (t, *J* = 7.6 Hz, 1H), 7.24 (t, *J* = 52.0 Hz, 1H), 6.41 (s, 1H), 5.60-5.51 (m, 1H), 3.05-2.84 (m, 4H), 2.72-2.62 (m, 4H), 1.72-1.65 (m, 1H), 1.54 (d, *J* = 6.8 Hz, 6H), 1.21-1.05 (m, 4H), 0.46-0.38 (m, 2H), 0.35-0.26 (m, 2H); ESI-MS (m/z): 513.0 $[M+H]^+$. |
| **Example 219** | (*R*)-1-(4-(cyclopropylmethyl) piperazin-1-yl)-4-((1-(3-(difluoromethyl)-2-fluorophenyl)ethyl) amino)-6-(1-methylcyclopropyl) pyrido[3,4-*d*]pyridazin-7(6*H*)-one | 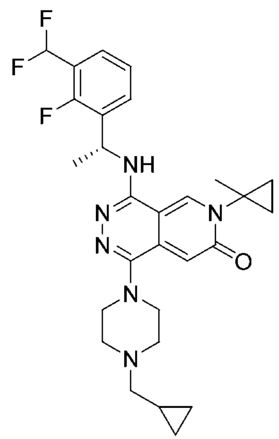 | $^{11}$H NMR (400 MHz, DMSO-$d_6$) δ 9.22 (s, 1H), 7.62 (t, *J* = 7.6 Hz, 1H), 7.56 (d, *J* = 6.8 Hz, 1H), 7.48 (t, *J* = 7.2 Hz, 1H), 7.27 (t, *J* = 7.6 Hz, 1H), 7.24 (t, *J* = 52.0 Hz, 1H), 6.39 (s, 1H), 5.61-5.51 (m, 1H), 3.15-2.90 (m, 4H), 2.65-2.52 (m, 4H), 2.23 (d, *J* = 6.4 Hz, 2H), 1.59-1.49 (m, 6H), 1.22-1.04 (m, 4H), 0.90-0.78 (m, 1H), 0.50-0.42 (m, 2H), 0.12-0.05 (m, 2H); ESI-MS(m/z): 527.0 $[M+H]^+$. |

(continued)

| Number | Chemical Name | Structure | $^1$H NMR and MS |
|---|---|---|---|
| **Example 220** | (*R*)-1-(4-cyclobutylpiperazin -1-yl)-4-((1-(3-(difluoromethyl)-2-fluorophenyl)ethyl) amino)-6-(1-methylcyclopropyl) pyrido[3,4-*d*]pyridazin-7(6*H*)-one | 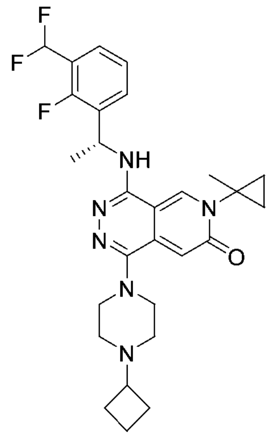 | $^1$H NMR (400 MHz, DMSO-$d_6$) δ 9.22 (s, 1H), 7.62 (t, *J* = 7.2 Hz, 1H), 7.56 *(d, J= 6.8 Hz,* 1H), 7.48 (t, *J* = 7.2 Hz, 1H), 7.27 (t, *J* = 7.6 Hz, 1H), 7.24 (t, *J* = 52.0 Hz, 1H), 6.38 (s, 1H), 5.60-5.52 (m, 1H), 3.08-2.88 (m, 4H), 2.81-2.71 (m, 1H), 2.45-2.32 (m, 4H), 2.01-1.91 (m, 2H), 1.85-1.75 (m, 2H), 1.67-1.59 (m, 2H), 1.56-1.51 (m, 6H), 1.20-1.06 (m, 4H); ESI-MS(m/z): 527.0 $[M+H]^+$. |
| **Example 221** | (*R*)-1-(4-cyclopentylpiperazi n-1-yl)-4-((1-(3-(difluoromethyl)-2-fluorophenyl)ethyl) amino)-6-(1-methylcyclopropyl) pyrido[3,4-*d*]pyridazin-7(6*H*)-one | 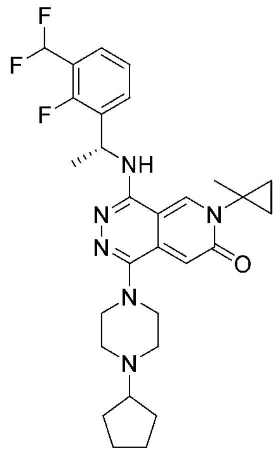 | $^1$H NMR (400 MHz, DMSO-$d_6$) δ 9.22 (s, 1H), 7.62 (t, *J* = 7.6 Hz, 1H), 7.56 (d, *J* = 6.8 Hz, 1H), 7.48 (t, *J* = 7.2 Hz, 1H), 7.27 (t, *J* = 7.6 Hz, 1H), 7.24 (t, *J* = 52.0 Hz, 1H), 6.39 (s, 1H), 5.60-5.51 (m, 1H), 3.06-2.88 (m, 4H), 2.61-2.51 (m, 5H), 1.84-1.75 (m, 2H), 1.64-1.57 (m, 2H), 1.56-1.52 (m, 6H), 1.52-1.44 (m, 2H), 1.38-1.27 (m, 2H), 1.22-1.03 (m, 4H); ESI-MS(m/z): 541.0 $[M+H]^+$. |

(continued)

| Number | Chemical Name | Structure | $^1$H NMR and MS |
|---|---|---|---|
| **Example 222** | (*R*)-1-(4-(tert-butyl)piperazin-1-yl)-4-((1-(3-(difluoromethyl)-2-fluorophenyl)ethyl) amino)-6-(1-methylcyclopropyl) pyrido[3,4-*d*]pyridazin-7(6*H*)-one formate | 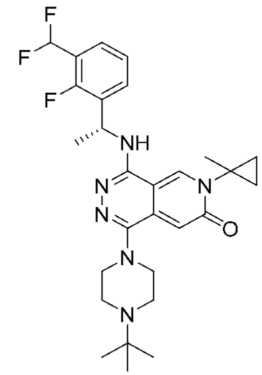 | The crude product was purified by Prep-HPLC (separation method 2) to obtain (*R*)-1-(4-(tert-butyl)piperazin-1-yl)-4-((1-(3-(difluoromethyl)-2-fluorophenyl)ethyl) amino)-6-(1-methylcyclopropyl)pyrido[3,4-*d*]pyridazin-7(6*H*)-one formate; $^1$H NMR (400 MHz, DMSO-$d_6$) δ 13.21 (s, 1H), 10.83 (s, 1H), 10.22 (s, 1H), 9.93 (s, 1H), 7.74 (s, 1H), 7.59 (s, 1H), 7.34 (s, 1H), 7.26 (t, *J* = 52.0 Hz, 1H), 6.80 (s, 1H), 5.60-5.42 (m, 1H), 3.61-3.48 (m, 4H), 2.52-2.51 (m, 4H), 1.78-1.58 (m, 3H), 1.55 (s, 3H), 1.39 (s, 9H), 1.24-1.07 (m, 4H); ESI-MS(m/z): 529.0 $[M+H]^+$. |
| **Example 223** | (*R*)-4-((1-(3-(difluoromethyl)-2-fluorophenyl)ethyl) amino)-1-(4-methylpiperazin-1-yl)-6-(1-methylpiperidin-4-yl)pyrido[3,4-*d*] pyridazin-7(6*H*)-one | 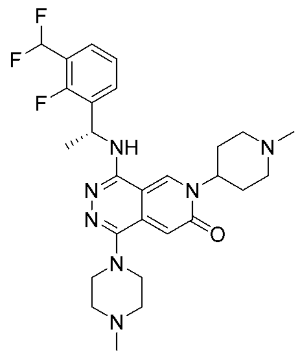 | $^1$H NMR (400 MHz, DMSO-$d_6$) δ 9.15 (s, 1H), 7.66 (d, *J* = 6.8 Hz, 1H), 7.60 (*t, J* = 7.6 Hz, 1H), 7.49 (t, *J* = 7.2 Hz, 1H), 7.29 (t, *J* = 7.6 Hz, 1H), 7.25 (t, *J* = 54.0 Hz, 1H), 6.46 (s, 1H), 5.61-5.54 (m, 1H), 4.96-4.90 (m, 1H), 3.00-2.97 (m, 6H), 2.47 (s, 4H), 2.24 (d, *J* = 14.8 Hz, 6H), 2.17-2.07 (m, 4H), 1.83-1.80 (m, 2H), 1.55 (d, *J* = 6.8 Hz, 3H); ESI-MS (m/z): 530.3 $[M+H]^+$. |

(continued)

| Number | Chemical Name | Structure | $^1$H NMR and MS |
|---|---|---|---|
| **Example 224** | (*R*)-3-(1-((6-cyclopropyl-1-(4-ethylpiperazin-1-yl)-7-oxo-6,7-dihydropyrido[3,4-*d*]pyridazin-4-yl)amino)ethyl)-2-methylbenzonitrile | 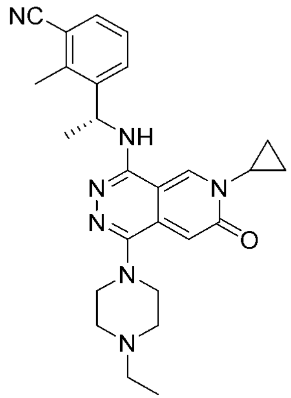 | $^1$H NMR (400 MHz, DMSO-$d_6$) δ 8.97 (s, 1H), 7.73 (d, $J$ = 7.6 Hz, 1H), 7.64 (d, $J$ = 7.6 Hz, 1H), 7.59 (d, $J$ = 6.8 Hz, 1H), 7.35 (t, $J$ = 8.0 Hz, 1H), 6.41 (s, 1H), 5.50-5.43 (m, 1H), 3.64-3.59 (m, 1H), 3.00 (s, 4H), 2.62 (s, 3H), 2.52 (s, 4H), 2.41-2.36 (m, 2H), 1.48 (d, $J$ = 7.2 Hz, 3H), 1.17-1.09 (m, 4H), 1.02 (t, $J$ = 7.2 Hz, 3H); ESI-MS (m/z): 458.3 $[M+H]^+$. |
| **Example 225** | (*R*)-1-(cyclopropyl(methyl )amino)-4-((1-(3-(difluoromethyl)-2-fluorophenyl)ethyl) amino)-6-(1-methylcyclopropyl) pyrido[3,4-*d*]pyridazin-7(6*H*)-one | 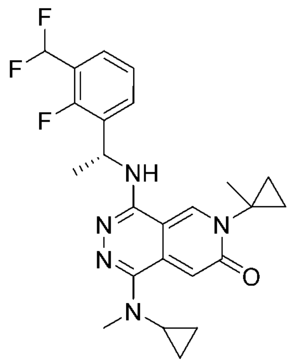 | $^1$H NMR (400 MHz, DMSO-$d_6$) δ 9.24 (s, 1H), 7.70-7.55 (m, 2H), 7.49 (t, $J$ = 7.2 Hz, 1H), 7.28 (t, $J$ = 7.6 Hz, 1H), 7.24 (t, $J$ = 54.4 Hz, 1H), 6.47 (s, 1H), 5.55 (p, $J$ = 7.2 Hz, 1H), 2.80-2.68 (m, 4H), 1.59-1.51 (m, 6H), 1.24-1.04 (m, 4H), 0.62-0.49 (m, 2H), 0.39-0.21 (m, 2H); ESI-MS (m/z): 458.2 $[M+H]^+$. |

(continued)

| Number | Chemical Name | Structure | $^1$H NMR and MS |
|---|---|---|---|
| **Example 226** | (*R*)-4-((1-(3-(1,1-difluoro-2-hydroxyethyl) pheny l)ethyl)amino)-6-(1-methylcyclopropyl)-1-(4-methylpiperazin-1-yl)pyrido [3,4-*d*]pyridazin-7(6H)-one | 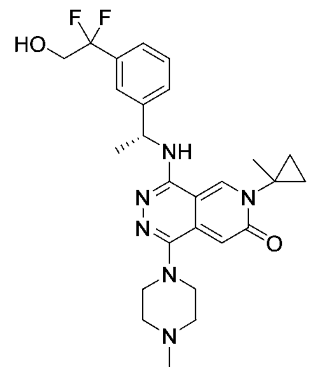 | $^1$H NMR (400 MHz, DMSO-$d_6$) δ 9.18 (s, 1H), 7.55 (s, 1H), 7.52 (t, *J* = 7.6 Hz, 2H), 7.42 (t, *J* = 7.6 Hz, 1H), 7.36 (d, *J* = 7.6 Hz, 1H), 6.39 (s, 1H), 5.60 (t, *J* = 6.4 Hz, 1H), 5.41 (p, *J* = 7.2 Hz, 1H), 3.90-3.79 (m, 2H), 3.08-2.91 (m, 4H), 2.49-2.42 (m, 4H), 2.22 (s, 3H), 1.57-1.50 (m, 6H), 1.17-1.02 (m, 4H); ESI-MS(m/z): 499.2 $[M+H]^+$. |
| **Example 227** | (*R*)-4-((1-(3-(1,1-difluoro-2-hydroxyethyl) pheny l)ethyl)amino)-1-(4-ethylpiperazin-1-yl)-6-(1-methylcyclopropyl) pyrido [3,4-*d*]pyridazin-7(6*H*)-one | 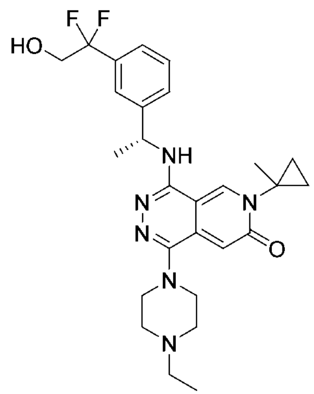 | $^1$H NMR (400 MHz, DMSO-$d_6$) δ 9.18 (s, 1H), 7.55 (s, 1H), 7.52 (t, *J* = 8.0 Hz, 2H), 7.42 (t, *J* = 7.6 Hz, 1H), 7.36 (d, *J* = 7.6 Hz, 1H), 6.39 (s, 1H), 5.60 (t, *J* = 6.4 Hz, 1H), 5.41 (p, *J* = 7.2 Hz, 1H), 3.89-3.78 (m, 2H), 3.12-2.88 (m, 4H), 2.52 (s, 4H), 2.38 (q, *J* = 7.2 Hz, 2H), 1.57-1.48 (m, 6H), 1.18-1.04 (m, 4H), 1.02 (t, *J* = 7.2 Hz, 3H); ESI-MS(m/z): 513.3 $[M+H]^+$. |

(continued)

| Number | Chemical Name | Structure | $^1$H NMR and MS |
|---|---|---|---|
| **Example 228** | 4-(((*R*)-1-(3-(1,1-difluoro-2-hydroxyethyl) pheny l)ethyl)amino)-1-(3-(hydroxymethyl) pyr rolidin-1-yl)-6-(1-methylcyclopropyl) pyrido[3,4-*d*]pyridazin-7(6*H*)-one | 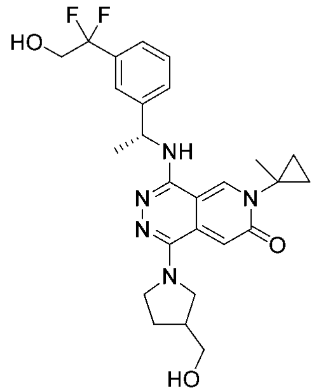 | $^1$H NMR (400 MHz, DMSO-$d_6$) δ 9.07 (s, 1H), 7.57-7.49 (m, 2H), 7.41 (t, *J* = 7.6 Hz, 1H), 7.35 (d, *J* = 7.6 Hz, 1H), 7.20 (d, *J* = 7.2 Hz, 1H), 6.61 (s, 1H), 5.60 (t, *J* = 6.4 Hz, 1H), 5.35 (p, *J* = 7.2 Hz, 1H), 4.62 (t, *J* = 5.2 Hz, 1H), 3.89-3.78 (m, 2H), 3.45-3.32 (m, 5H), 3.29-3.23 (m, 1H), 2.29 (p, *J* = 6.8 Hz, 1H), 1.94-1.85 (m, 1H), 1.63-1.47 (m, 7H), 1.19-1.03 (m, 4H); ESI-MS(m/z): 500.2 $[M+H]^+$. |
| **Example 229** | (*R*)-4-((1-(3-(1,1-difluoro-2-hydroxyethyl) pheny l)ethyl)amino)-1-(3-(hydroxymethyl) aze tidin-1-yl)-6-(1-methylcyclopropyl) pyrido[3,4-*d*]pyridazin-7(6*H*)-one | | $^1$H NMR (400 MHz, DMSO-$d_6$) δ 8.59 (s, 1H), 7.60-7.47 (m, 2H), 7.42 (t, *J* = 7.6 Hz, 1H), 7.35 (d, *J* = 7.6 Hz, 1H), 6.86 (s, 1H), 6.56 (s, 1H), 5.55 (s, 1H), 4.89 (p, *J* = 7.2 Hz, 1H), 4.67-4.53 (m, 1H), 3.84 (t, *J* = 14.0 Hz, 2H), 3.50-3.29 (m, 5H), 3.12-3.02 (m, 1H), 1.96-1.82 (m, 1H), 1.55-1.39 (m, 6H), 1.14-0.96 (m, 4H); ESI-MS(m/z): 486.2 $[M+H]^+$. |

**Biological Activity Test**

**Test Example 1: Assay for SOS1 Inhibitory Activity**

The effect of SOS1 inhibitors on the interaction between SOS1 and KRAS proteins was detected using homogeneous time-resolved fluorescence (HTRF) technology, thereby evaluating their inhibitory level on SOS1 proteins. The KRAS-G12D/SOS1 BINDING ASSAY KIT (Cisbio) was used for the proteins and detection reagents. First, the 2 mM test compound stock solution was diluted 20-fold (100 μM) with the Diluent reagent, followed by serial 5-fold dilutions using Diluent reagent (5% DMSO) to obtain total 8 concentrations of working solutions of the test compounds. In a 384-well plate, 4 μL of tag2-$KRAS^{G12D}$ protein (containing 50 μM GTP), 2 μL of the test compounds, and 4 μL of tag1-SOS1 protein were sequentially added to each well. The assay was performed in duplicate wells, incubated at room temperature for 15 min. 5 μL of Anti-tag1-$Tb^{3+}$ working solution and 5 μL of Anti-tag2-XL665 working solution were added sequentially to each well, and incubated at 4°C for 3 h. The 384-well plate was placed on a multifunctional microplate reader for reading, with the excitation wavelength set at 337 nm, and the readings at 620 nm and 665 nm were recorded. The data results are presented as the ratio of the 665 nm signal value to the 620 nm signal value for each well, i.e.: Ratio = $10^4 \times$ 665 nm signal value / 620 nm signal value. The inhibition rate was calculated using the Ratio value:

$$\%\text{Inhibition Rate} = \left[\frac{\overline{\text{Ratio}}_{\text{negative control}} - \overline{\text{Ratio}}_{\text{test compound}}}{\overline{\text{Ratio}}_{\text{negative control}}}\right] * 100\%$$

$IC_{50}$ was calculated by the inhibition rates using GraphPad Prism software. Each compound was assayed in 2 replicate wells per measurement. The $IC_{50}$ values of the compounds of the present disclosure for inhibiting SOS1 activity is 1.4~100.0 nM, and the test data of some Example compounds are shown in Table 1:

Table 1. Inhibitory activity of some Example compounds against SOS1

| Example Number | SOS1 inhibitory activity ($IC_{50}$, nM) |
|---|---|
| Example 1 | 13.5 |
| Example 4 | 4.9 |
| Example 5 | 4.7 |
| Example 6 | 14.8 |
| Example 7 | 17.7 |
| Example 8 | 7.7 |
| Example 9 | 4.5 |
| Example 10 | 5.8 |
| Example 11 | 1.4 |
| Example 12 | 4.9 |
| Example 13 | 4.6 |
| Example 14 | 17.6 |
| Example 15 | 5.5 |
| Example 16 | 4.1 |
| Example 17 | 11.2 |
| Example 18 | 5.7 |
| Example 19 | 27.2 |
| Example 20 | 23.8 |
| Example 21 | 6.9 |
| Example 22 | 16.4 |
| Example 23 | 22.4 |
| Example 24 | 12.7 |

(continued)

| Example Number | SOS1 inhibitory activity ($IC_{50}$, nM) |
|---|---|
| Example 25 | 12.0 |
| Example 26 | 18.8 |
| Example 27 | 16.5 |
| Example 28 | 5.7 |
| Example 29 | 10.6 |
| Example 30 | 33.5 |
| Example 31 | 19.5 |
| Example 32 | 25.5 |
| Example 33 | 22.5 |
| Example 34 | 14.4 |
| Example 35 | 14.8 |
| Example 36 | 10.7 |
| Example 37 | 9.2 |
| Example 38 | 5.00 |
| Example 39 | 23.8 |
| Example 40 | 32.5 |
| Example 41 | 57.9 |
| Example 42 | 51.3 |
| Example 43 | 84.2 |
| Example 44 | 44.2 |
| Example 45 | 6.2 |
| Example 46 | 9.3 |
| Example 47 | 66.2 |
| Example 48 | 46.5 |
| Example 49 | 6.0 |
| Example 50 | 5.8 |
| Example 51 | 11.8 |
| Example 52 | 5.7 |
| Example 53 | 24.7 |
| Example 54 | 50.3 |
| Example 55 | 49.2 |
| Example 56 | 10.6 |
| Example 57 | 25.6 |
| Example 58 | 100.0 |
| Example 59 | 27.4 |
| Example 60 | 20.9 |
| Example 61 | 6.1 |
| Example 62 | 9.4 |
| Example 63 | 7.8 |

(continued)

| Example Number | SOS1 inhibitory activity ($IC_{50}$, nM) |
|---|---|
| Example 64 | 10.7 |
| Example 65 | 61.0 |
| Example 66 | 30.9 |
| Example 67 | 29.0 |
| Example 68 | 18.9 |
| Example 69 | 34.2 |
| Example 70 | 6.7 |
| Example 71 | 30.8 |
| Example 72 | 50.4 |
| Example 73 | 4.4 |
| Example 74 | 4.6 |
| Example 75 | 4.8 |
| Example 76 | 9.2 |
| Example 77 | 5.9 |
| Example 78 | 4.6 |
| Example 79 | 12.7 |
| Example 80 | 4.5 |
| Example 81 | 6.3 |
| Example 82 | 9.0 |
| Example 83 | 5.6 |
| Example 84 | 18.7 |
| Example 85 | 11.7 |
| Example 86 | 3.6 |
| Example 87 | 5.7 |
| Example 88 | 4.5 |
| Example 89 | 4.1 |
| Example 90 | 4.2 |
| Example 91 | 5.7 |
| Example 92 | 10.2 |
| Example 93 | 5.4 |
| Example 94 | 7.3 |
| Example 95 | 6.8 |
| Example 96 | 7.4 |
| Example 97 | 5.1 |
| Example 98 | 4.3 |
| Example 99 | 11.3 |
| Example 100 | 10.9 |
| Example 101 | 8.9 |
| Example 102 | 12.5 |

(continued)

| Example Number | SOS1 inhibitory activity ($IC_{50}$, nM) |
|---|---|
| Example 103 | 5.9 |
| Example 104 | 7.5 |
| Example 105 | 4.7 |
| Example 106 | 5.2 |
| Example 107 | 4.2 |
| Example 108 | 5.5 |
| Example 109 | 4.2 |
| Example 110 | 3.3 |
| Example 111 | 4.4 |
| Example 112 | 5.0 |
| Example 113 | 4.5 |
| Example 114 | 6.0 |
| Example 115 | 4.1 |
| Example 116 | 6.6 |
| Example 117 | 8.2 |
| Example 118 | 8.4 |
| Example 119 | 4.3 |
| Example 120 | 4.1 |
| Example 121 | 5.5 |
| Example 122 | 10.6 |
| Example 123 | 3.8 |
| Example 124 | 5.8 |
| Example 125 | 14.1 |
| Example 126 | 9.4 |
| Example 127 | 6.7 |
| Example 128 | 4.2 |
| Example 129 | 6.1 |
| Example 130 | 3.4 |
| Example 131 | 4.6 |
| Example 132 | 16.6 |
| Example 133 | 6.5 |
| Example 134 | 9.5 |
| Example 135 | 14.1 |
| Example 136 | 6.2 |
| Example 137 | 4.8 |
| Example 138 | 10.7 |
| Example 139 | 6.2 |
| Example 140 | 8.5 |
| Example 141 | 7.8 |

(continued)

| Example Number | SOS1 inhibitory activity ($IC_{50}$, nM) |
|---|---|
| Example 142 | 10.6 |
| Example 143 | 11.5 |
| Example 144 | 6.7 |
| Example 145 | 7.8 |
| Example 146 | 12.5 |
| Example 147 | 6.3 |
| Example 148 | 6.5 |
| Example 149 | 8.6 |
| Example 150 | 5.5 |
| Example 151 | 3.8 |
| Example 152 | 4.2 |
| Example 153 | 4.6 |
| Example 154 | 5.4 |
| Example 155 | 5.6 |
| Example 156 | 5.4 |
| Example 157 | 5.1 |
| Example 158 | 6.9 |
| Example 159 | 6.4 |
| Example 160 | 5.7 |
| Example 161 | 5.2 |
| Example 162 | 6.7 |
| Example 163 | 8.4 |
| Example 164 | 4.2 |
| Example 165 | 9.6 |
| Example 166 | 9.7 |
| Example 167 | 6.3 |
| Example 168 | 4.1 |
| Example 169 | 4.7 |
| Example 170 | 6.7 |
| Example 171 | 5.0 |
| Example 172 | 4.7 |
| Example 173 | 5.3 |
| Example 174 | 20.2 |
| Example 175 | 13.0 |
| Example 176 | 6.0 |
| Example 177 | 6.2 |
| Example 178 | 6.3 |
| Example 179 | 6.4 |
| Example 180 | 7.2 |

(continued)

| Example Number | SOS1 inhibitory activity ($IC_{50}$, nM) |
|---|---|
| Example 181 | 7.8 |
| Example 182 | 5.0 |
| Example 183 | 4.9 |
| Example 184 | 40.2 |
| Example 185 | 84.3 |
| Example 186 | 31.1 |
| Example 187 | 4.8 |
| Example 188 | 3.1 |
| Example 189 | 4.3 |
| Example 190 | 4.2 |
| Example 191 | 4.5 |
| Example 192 | 3.8 |
| Example 193 | 4.3 |
| Example 194 | 3.9 |
| Example 195 | 3.7 |
| Example 196 | 3.7 |
| Example 197 | 5.0 |
| Example 198 | 13.6 |
| Example 199 | 12.5 |
| Example 200 | 4.2 |
| Example 201 | 5.3 |
| Example 202 | 12.4 |
| Example 203 | 3.8 |
| Example 204 | 8.0 |
| Example 205 | 4.1 |
| Example 206 | 4.9 |
| Example 207 | 7.3 |
| Example 208 | 4.5 |
| Example 209 | 4.4 |
| Example 210 | 2.7 |
| Example 211 | 5.2 |
| Example 212 | 11.9 |
| Example 213 | 6.2 |
| Example 214 | 5.0 |
| Example 215 | 8.6 |
| Example 216 | 4.6 |
| Example 217 | 4.6 |
| Example 218 | 6.0 |
| Example 219 | 4.7 |

(continued)

| Example Number | SOS1 inhibitory activity ($IC_{50}$, nM) |
|---|---|
| Example 220 | 4.0 |
| Example 221 | 5.0 |
| Example 222 | 5.0 |
| Example 223 | 5.4 |
| Example 224 | 8.4 |
| Example 225 | 7.6 |
| Example 226 | 4.3 |
| Example 227 | 3.8 |
| Example 228 | 4.4 |
| Example 229 | 4.5 |

It can be seen that the compounds of the present disclosure have strong SOS1 inhibitory activity, and by comparing with the corresponding SOS1 inhibitory activity data in Table 1 of PCT/CN2023/104412, it can be known that the compounds of the present disclosure, with the $-NR_{11}R_{12}$ functional group introduced at the $R_4$ position, show significantly enhanced inhibitory activity against SOS1 compared with those where $R_4$ is other types of substituents such as cycloalkyl, cycloalkenyl, etc.

**Test Example 2: Assay of Inhibitory Activity on NCI-H358 Cell Proliferation**

**Comparative compound 1**

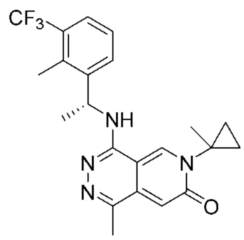

The synthesis method is as follows:

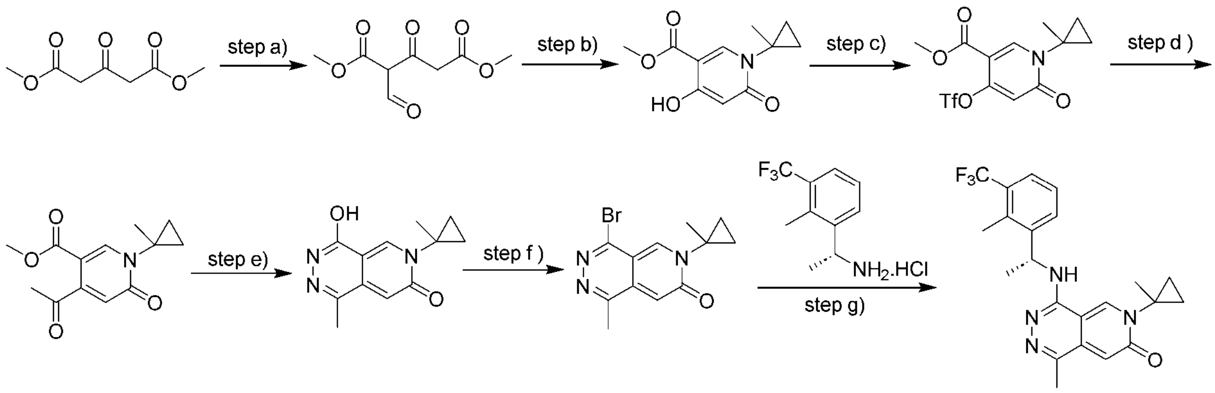


step a): Preparation of dimethyl 2-formyl-3-oxopentanedioate

Dimethyl 3-oxopentanedioate (10.0 g, 57.421 mmol) and 1-methyl tetrahydrofuran (100 mL) were added to a reaction flask. Under an ice-water bath, DMF-DMA (6.8 g, 57.42 mmol) was added dropwise. The mixture was stirred to

react at room temperature for 2 h. To the reaction solution was added 4N dilute hydrochloric acid (30 mL), and the mixture was continued to react at room temperature for 1 h. After the reaction was completed, the reaction solution was extracted with methyl tert-butyl ether (100 mL). The organic phases were combined and washed with saturated brine (100 mL), dried over anhydrous sodium sulfate, and filtered. The filtrate was concentrated under reduced pressure to obtain dimethyl 2-formyl-3-oxopentanedioate, which was used directly in the next step without further purification, yield 98.6%; ESI-MS (m/z): 203.2 $[M+H]^+$.

step b): Preparation of methyl 4-hydroxy-1-(1-methylcyclopropyl)-6-oxo-1,6-dihydropyridine-3-carboxylate

1-Methylcyclopropan-1-amine hydrochloride (4.7 g, 43.925 mmol) and dimethyl 2-formyl-3-oxopentanedioate (8.9 g, 43.925 mmol) were dissolved with methanol (120 mL). Under an ice-water bath, sodium methoxide (5.2 g, 96.635 mmol) was added. The mixture was warmed to 80 °C and reacted for 6 h. After the reaction was completed, the reaction solution was cooled to room temperature and concentrated under reduced pressure to remove most of the solvent. The reaction was quenched by adding saturated aqueous ammonium chloride (100 mL). The reaction solution was adjusted to pH 2-3 with 4N hydrochloric acid, and a large amount of solid precipitated. The precipitate was filtered, and the filter cake was washed with 1N hydrochloric acid, collected, and dried under reduced pressure. The filter cake was triturated with a mixed solvent of methyl tert-butyl ether and petroleum ether (80 mL, v/v = 4/1), filtered, and the filter cake was dried under reduced pressure to obtain methyl 4-hydroxy-1-(1-methylcyclopropyl)-6-oxo-1,6-dihydropyridine-3-carboxylate (yield 83.8%); ESI-MS (m/z): 224.2 $[M+H]^+$.

step c): Preparation of methyl 1-(1-methylcyclopropyl)-6-oxo-4-(((trifluoromethyl)sulfonyl)oxy)-1,6-dihydropyridine -3-carboxylate

Methyl 4-hydroxy-1-(1-methylcyclopropyl)-6-oxo-1,6-dihydropyridine-3-carboxylate (6.4 g, 28.662 mmol), pyridine (4.5 g, 57.324 mmol), and 1,2-dichloroethane (60 mL) were added to a reaction flask. Under an ice-water bath, trifluoromethanesulfonic anhydride (12.1 g, 42.993 mmol) was added. Under an ice-water bath, the mixture was stirred to react for 2 min. The reaction was quenched by adding saturated aqueous ammonium chloride (50 mL), allowed to stand for liquid separation, and the aqueous phase was extracted with dichloromethane (100 mL) The organic phases were combined, washed with saturated brine (100 mL), dried over anhydrous sodium sulfate, filtered, and the filtrate was concentrated under reduced pressure. The residue was purified by silica gel column chromatography (eluent: petroleum ether/ethyl acetate = 10/1 to 3/1) to obtain methyl 1-(1-methylcyclopropyl)-6-oxo-4-(((trifluoromethyl)sulfonyl)oxy)-1,6-dihydropyridine-3- carboxylate, yield 91.2%; ESI-MS (m/z): 356.2 $[M+H]^+$.

step d): Preparation of methyl 4-acetyl-1-(1-methylcyclopropyl)-6-oxo-1,6-dihydropyridine-3-carboxylate

Methyl 1-(1-methylcyclopropyl)-6-oxo-4-(((trifluoromethyl)sulfonyl)oxy)-1,6-dihydropyridine-3-carboxylate (9.0 g, 25.352 mmol), 1,4-dioxane (60 mL), tributyl(1-ethoxyethenyl)tin (11.9 g, 32.958 mmol), triethylamine (3.3 g, 32.958 mmol), and bis(triphenylphosphine)palladium(II) chloride (356 mg, 0.507 mmol) were sequentially added to a reaction flask. The mixture was purged with nitrogen three times, warmed to 100°C, and stirred to react overnight. After the reaction was completed, the reaction solution was cooled to room temperature, and the solvent was removed under reduced pressure. The residue was adjusted to pH 2-3 with 4N dilute hydrochloric acid, and continued to stir at room temperature for 1 h. The reaction was quenched by adding a saturated potassium fluoride aqueous solution (80 mL), then ethyl acetate (100 mL) was added. The mixture was continued to stir for 30 min, filtered, and the filter cake was washed with ethyl acetate (50 mL). The filtrate was separated, and the organic phase was washed with saturated brine (50 mL), dried over anhydrous sodium sulfate, filtered, and concentrated under reduced pressure. The residue was purified by silica gel column chromatography (eluent: petroleum ether/ethyl acetate = 1/1) to obtain methyl 4-acetyl-1-(1-methylcyclopropyl)-6-oxo-1,6-dihydropyridine-3- carboxylate, yield 83.3%; ESI-MS (m/z): 250.2 $[M+H]^+$.

step e): Preparation of 4-hydroxy-1-methyl-6-(1-methylcyclopropyl)pyrido[3,4-*d*]pyridazin-7(6*H*)-one

Methyl 4-acetyl-1-(1-methylcyclopropyl)-6-oxo-1,6-dihydropyridine-3-carboxylate (5.0 g, 20.059 mmol) and absolute ethanol (50 mL) were added to a reaction flask. Hydrazine hydrate (5 mL, 80%) was added at room temperature, and the mixture was stirred to react at 80°C for 1 h. The reaction solution was cooled to room temperature and concentrated under reduced pressure. Ethyl acetate (30 mL) was added to the residue, and the mixture was triturated at room temperature for 1 h, filtered. The filter cake was washed with ethyl acetate (30 mL), collected, and dried under reduced pressure to obtain 4-hydroxy-1-methyl-6-(1-methylcyclopropyl)pyrido[3,4-*d*]pyridazin-7(6*H*)-one, yield 95.6%; ESI-MS(m/z): 232.1 $[M+H]^+$.

step f): Preparation of 4-bromo-1-methyl-6-(1-methylcyclopropyl)pyrido[3,4-d]pyridazin-7(6H)-one

4-Hydroxy-1-methyl-6-(1-methylcyclopropyl)pyrido[3,4-*d*]pyridazin-7(6*H*)-one (950 mg, 4.108 mmol), phosphorus oxybromide (2.4 g, 8.720 mmol), and acetonitrile (100 mL) were added to a reaction flask. The mixture was warmed to reflux and reacted for 2 h. The reaction solution was cooled to room temperature, poured into a saturated sodium bicarbonate aqueous solution (200 mL) for quenching, and extracted with dichloromethane (200 mL × 2). The organic phases were combined, washed with saturated brine (100 mL), dried over anhydrous sodium sulfate, and filtered. The filtrate was concentrated under reduced pressure to obtain 4-bromo-1-methyl-6-(1-methylcyclopropyl)pyrido[3,4-*d*]pyridazin-7(6*H*)-one, yield 82.7%; ESI-MS (m/z): 294.0 $[M+H]^+$.

step g): Preparation of (*R*)-1-methyl-4-((1-(2-methyl-3-(trifluoromethyl)phenyl)ethyl)amino)-6-(1-methylcyclopropyl) pyrido[3,4-*d*]pyridazin-7(6*H*)-one

4-Bromo-1-methyl-6-(1-methylcyclopropyl)pyrido[3,4-*d*]pyridazin-7(6*H*)-one (100 mg, 0.340 mmol), (*R*)-1-(2-methyl -3-(trifluoromethyl)phenyl)ethan-1-amine hydrochloride (160 mg, 0.668 mmol), DIPEA (260 mg, 2.012 mmol), cesium fluoride (310 mg, 2.041 mmol), and DMSO (3 mL) were added to a reaction flask. The mixture was warmed to 80°C and reacted for 3 h. After the reaction was completed, the mixture was cooled to room temperature, quenched by adding saturated aqueous ammonium chloride solution (40 mL), and extracted with dichloromethane (40 mL × 2). The organic phases were combined, washed with saturated brine (30 mL × 3), dried over anhydrous sodium sulfate, filtered, and the filtrate was concentrated under reduced pressure. The resulting crude product was purified by silica gel column chromatography (eluent: methanol/dichloromethane = 5/95) and further purified by Prep-HPLC (separation method 3) to obtain (*R*)-1-methyl-4-((1-(2-methyl-3-(trifluoromethyl)phenyl)ethyl)amino)-6-(1-methylcyclopropyl)pyrido[3,4-*d*] pyridazin-7(6*H*)-one, yield 14.3%; $^1$H NMR (400 MHz, DMSO-$d_6$) δ 9.24 (s, 1H), 7.76 (d, *J* = 6.8 Hz, 1H),7.73 (d, *J* = 7.6 Hz, 1H), 7.54 (d, *J* = 7.6 Hz, 1H), 7.35 (t, *J* = 7.6 Hz, 1H), 6.46 (s, 1H), 5.64-5.54 (m, 1H), 2.53 (s, 3H), 2.32 (s, 3H), 1.54 (s, 3H), 1.51 (d, *J* = 7.2 Hz, 3H), 1.20-1.04 (m, 4H); ESI-MS(m/z): 417.0 $[M+H]^+$.

## Comparative compound 2

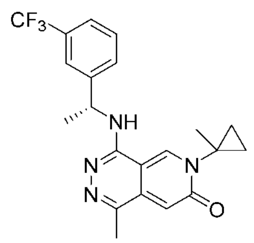

The synthesis method is as follows:

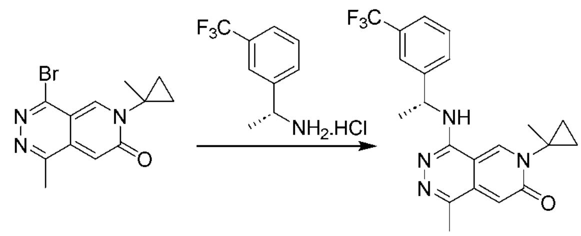

4-Bromo-1-methyl-6-(1-methylcyclopropyl)pyrido[3,4-*d*]pyridazin-7(6*H*)-one (450 mg, 1.530 mmol), (*R*)-1-(3-(trifluoromethyl)phenyl)ethan-1-amine hydrochloride (690 mg, 3.058 mmol), DIPEA (1.2 g, 9.285 mmol), cesium fluoride (0.9 g, 5.925 mmol), and DMSO (10 mL) were added to a reaction flask. The mixture was warmed to 80°C and reacted for 3 h. After the reaction was completed, the mixture was cooled to room temperature, quenched by adding saturated aqueous ammonium chloride solution (40 mL), and extracted with dichloromethane (50 mL × 2). The organic phases were combined, washed with saturated brine (50 mL × 3), dried over anhydrous sodium sulfate, filtered, and concentrated under reduced pressure. The resulting crude product was purified by Prep-HPLC (separation method 1) to obtain (*R*)-1-methyl-6-(1-methylcyclopropyl)-4-((1-(3-(trifluoromethyl)phenyl)ethyl)amino)pyrido[3,4-*d*]pyridazin-7(6*H*)-one hydrochloride, yield 12.8%; $^1$H NMR (400 MHz, DMSO-$d_6$) δ 13.33 (s, 1H), 10.63 (s, 1H), 10.16 (s, 1H), 8.00-7.97 (m, 2H), 7.69-7.62 (m, 2H), 6.81 (s, 1H), 5.53-5.46 (m, 1H), 2.42 (s, 3H), 1.77 (d, *J* = 6.4 Hz, 3H), 1.55 (s, 3H), 1.28-1.18 (m, 2H), 1.12-1.06 (m, 2H); ESI-MS (m/z): 403.2 $[M+H]^+$.

The number of viable cells were detected by CellTiter-Glo 3D® reagent, thereby evaluating the inhibitory effect of compounds on cell proliferation. NCI-H358 non-small cell lung cancer cells (Nanjing cobioer) in the exponential growth phase were digested with trypsin-EDTA, and seeded into 96-well ultra-low attachment microplates at 100 μL per well with a density of 2000 cells/well. The cells were cultured overnight at 37°C with 5% $CO_2$ to form microspheres. The test compounds were serially diluted 5-fold with DMSO to obtain 8 concentration gradient dilutions, then diluted with RPMI-1640 (10% FBS) cell culture medium respectively to obtain compound working solutions (2×). 100 μL per well was added to the cell supernatant, and the culture was continued for 7 days under the conditions of 37°C and 5% $CO_2$. The well plate was removed, and 100 μL of CellTiter-Glo 3D® reagent was added. After lysis at room temperature for 10-30 minutes, 70 μL of the lysate was aspirated and transferred to a white opaque 384-well plate. The 384-well plate was placed on a multifunctional microplate reader to record the luminescence value (RLU).

The inhibition rate was calculated by the following formula:

Inhibition rate (%)={ $[(RLU_{negative\ control} - RLU_{blank}) - (RLU_{test\ compound} - RLU_{blank})] / (RLU_{negative\ control} - RLU_{blank})\} \times 100\%$

Note: The negative control refers to the group without inhibitor, and the blank refers to the group without cells.

$IC_{50}$ was calculated by the inhibition rates using GraphPad Prism software. The $IC_{50}$ values of the compounds of the present disclosure for inhibiting NCI-H358 cell proliferation is 9.6 ~ 65.3 nM. The inhibitory activities of some Example compounds and comparative compounds against NCI-H358 cell proliferation are shown in Table 2.

Table 2. Inhibitory activity of some Example compounds against NCI-H358 cell proliferation

| Example Number | Inhibitory activity against NCI-H358 cell proliferation ($IC_{50}$, nM) |
|---|---|
| Example 5 | 50.1 |
| Example 9 | 42.6 |
| Example 12 | 36.3 |
| Example 16 | 57.8 |
| Example 49 | 55.1 |
| Example 52 | 61.5 |
| Example 61 | 31.8 |
| Example 73 | 14.7 |
| Example 77 | 39.6 |
| Example 80 | 20.8 |
| Example 81 | 65.3 |
| Example 88 | 23.1 |
| Example 89 | 58.6 |
| Example 90 | 49.8 |
| Example 91 | 38.5 |
| Example 97 | 36.8 |
| Example 98 | 37.6 |
| Example 104 | 29.7 |
| Example 105 | 58.1 |
| Example 108 | 27.0 |
| Example 109 | 16.6 |
| Example 110 | 9.6 |
| Example 111 | 14.4 |
| Example 112 | 20.2 |
| Example 113 | 24.2 |

(continued)

| Example Number | Inhibitory activity against NCI-H358 cell proliferation ($IC_{50}$, nM) |
|---|---|
| Example 119 | 59.1 |
| Example 120 | 24.9 |
| Example 130 | 47.8 |
| Example 151 | 39.4 |
| Example 156 | 47.4 |
| Example 157 | 52.2 |
| Example 164 | 32.8 |
| Example 168 | 35.5 |
| Example 176 | 32.3 |
| Example 191 | 39.3 |
| Example 192 | 28.8 |
| Example 194 | 44.9 |
| Example 195 | 25.6 |
| Example 216 | 36.4 |
| Example 217 | 47.1 |
| Example 219 | 25.0 |
| Example 220 | 56.7 |
| Example 221 | 53.6 |
| Example 222 | 33.4 |
| Example 227 | 48.2 |
| comparative compound 1 | 282 |
| comparative compound2 | 377 |

As can be seen from the data in Table 2, the compounds of the present disclosure exhibit significantly improved anti-tumor activity compared with comparative compound 1 and comparative compound 2. It can be seen that the compounds of the present disclosure, with the $-NR_{11}R_{12}$ functional group introduced at the $R_4$ position, show significantly enhanced anti-tumor activity compared with those where $R_4$ is other types of substituents such as methyl.

**Test Example 3: Assay of Hepatocyte Microsomal Metabolic Stability**

Metabolic stability affects the clearance rate, half-life, and oral bioavailability of compounds in the body, and is one of the most important ADME drug-like properties of compounds. Hepatocyte microsomal stability assay is a commonly used method for studying metabolic stability. In the present disclosure, rat liver microsomes are used to determine and investigate the metabolic stability of compounds.

Test Method

a. Solution Preparation:

Preparation of compound stock solution: an appropriate amount of the test compound and testosterone was taken, and a 1 mM stock solution was prepared with DMSO, which was stored in a refrigerator at 4°C.

Preparation of compound working solution: The 1 mM compound stock solution was taken respectively, and then diluted to 100 μM with acetonitrile-water (v:v = 1:1) to obtain the working solution.

Preparation of NADPH working solution: an appropriate amount of NADPH was weighed and dissolved in 16 mM $MgCl_2$ to prepare a NADPH working solution with a concentration of 4.0 mM.

Preparation of rat liver microsome working solution: an appropriate amount of rat liver microsomes was taken and

diluted with PBS to obtain a liver microsome working solution with a concentration of 1 mg/mL.

b. Sample Incubation:

In the incubation system, 48 μL of PBS, 100 μL of rat liver microsome working solution, and 2 μL of the test compound working solution were sequentially added and mixed well. After pre-incubation at 37°C for 5 min, 50 μL of NADPH was added to initiate the reaction. After incubation for the corresponding time points, an appropriate amount of ice-cold acetonitrile containing an internal standard was added to terminate the reaction. The mixture was vortexed to mix well, centrifuged, and the supernatant was collected. The supernatant was injected into LC-MS/MS for detection of the residual metabolic amount of the compound.

c. Data Processing:

Softwares of Excel, etc. were used for data processing according to the following formula.

$$C_t = C_0 \bullet e^{-k_e \bullet t}$$

when $C_t = \frac{1}{2} C_0$ ,

$$T_{1/2} = \frac{Ln2}{k_e} = \frac{0.693}{k_e}$$

$$CL_{int(mic)} = \frac{0.693}{\text{In vitro } T_{1/2}} \bullet \frac{1}{\text{mg / mL microsomal protein in reaction system}}$$

$$CL_{int(liver)} = CL_{int(mic)} \bullet \frac{\text{mg microsomes}}{\text{g liver}} \bullet \frac{\text{g liver}}{\text{kg body weight}}$$

$$CL_{(liver)} = (CL_{int(liver)} \times f_u \times Q_h) / (CL_{int(liver)} \times f_u + Q_h)$$

fu (unbound fraction in blood) was defaulted to be 1.

The results of rat liver microsomal stability test for some compounds in the Examples and the reference compound MRTX0902 are shown in Table 3:

Table 3. Results of rat liver microsomal stability test

| Example Number | $T_{1/2}$ (min) | $CL_{(liver)}$ (ml/min/kg) |
|---|---|---|
| Example 9 | 260 | 8.2 |
| Example 10 | 29.0 | 33.6 |
| Example 11 | 51.3 | 25.8 |
| Example 12 | 150 | 12.8 |
| Example 13 | 238 | 8.8 |
| Example 15 | 47.2 | 27.0 |
| Example 38 | 213 | 9.7 |
| Example 50 | 131 | 14.2 |
| Example 52 | 84.6 | 19.2 |
| Example 73 | 95.4 | 17.7 |
| Example 74 | >300 | <7.23 |
| Example 75 | >300 | <7.23 |
| Example 77 | >300 | <7.23 |

(continued)

| Example Number | $T_{1/2}$ (min) | $CL_{(liver)}$ (ml/min/kg) |
|---|---|---|
| Example 78 | 253 | 8.4 |
| Example 80 | 161 | 12.1 |
| Example 88 | 128 | 14.4 |
| Example 90 | 139 | 13.5 |
| Example 91 | 151 | 12.68 |
| Example 97 | 105 | 16.66 |
| Example 98 | 187 | 10.75 |
| Example 104 | 156 | 12.4 |
| Example 108 | 134 | 13.91 |
| Example 109 | >300 | <7.23 |
| Example 110 | >300 | <7.23 |
| Example 111 | >300 | <7.23 |
| Example 112 | 199 | 10.22 |
| Example 113 | 135 | 13.88 |
| Example 120 | >300 | <7.23 |
| Example 121 | >300 | <7.23 |
| Example 216 | 291 | 7.43 |
| Example 217 | 260 | 8.18 |
| Example 219 | 168 | 11.7 |
| Example 220 | 80.4 | 19.9 |
| Example 221 | 67.3 | 22.2 |
| Example 222 | 218 | 9.47 |
| Compound MRTX0902 | 2.9 | 51.9 |

MRTX0902 is a compound disclosed in WO2021127429 (Example 12-10), which has been reported to have SOS1 inhibitory activity and that compound has entered Phase I clinical trials. As can be seen from the data in Table 3, by comparing with compound MRTX0902, the compounds of the present disclosure exhibit better metabolic stability and superior druggable properties.

In addition, by comparing with the corresponding rat liver microsomal stability test data in Table 2 of PCT/CN2023/104412, it can be known that the compounds of the present disclosure, with the $-NR_{11}R_{12}$ functional group introduced at the $R_4$ position, show significantly improved metabolic stability compared with those where $R_4$ is other types of substituents such as methyl, hydroxyl.

**Test Example 4: Assay of Inhibitory Activity on Hepatic Drug-Metabolizing Enzymes**

The main site of drug metabolism is the liver, and the main component of the mixed-function oxidase system present in the liver is the CYP450 enzyme. This enzyme system causes most clinically observed drug-drug interactions, which in turn leads to an increased incidence of adverse drug reactions. CYP450 is a group of supergene family composed of many isozymes, wherein CYP3A4 acts as a major metabolizing enzyme and is involved in the metabolism of nearly half of clinically used drugs. Similarly, CYP2C9 and CYP2C19 also act as important metabolizing enzymes and are involved in the metabolism of various drugs clinically. Therefore, evaluating the risk of drug-drug interactions by testing the inhibitory activity of compounds on CYP450 enzymes at an early stage can improve the safety of medication use.

In the present disclosure, human liver microsomes were used as the CYP450 enzyme source. The specific probe substrates for each CYP isoenzyme (wherein CYP3A4 used two substrates, midazolam and testosterone, denoted as CYP3A4-M and CYP3A4-T respectively) were incubated with a series of concentrations of the test compound in the presence of the cofactor NADPH. The production amount of metabolites of probe substrates in the incubation system was

determined by LC-MS/MS, and the $IC_{50}$ values of the test compound against each CYP450 enzyme subtype were calculated to evaluate its inhibitory effect on each CYP450 enzyme subtype. During the experiment, 49 μL of PBS, 50 μL of probe substrate, and 50 μL of human liver microsome working solution were sequentially added to the incubation system, followed by the addition of 1 μL of the test compound working solution at each concentration, and the mixture was mixed well. After pre-incubation at 37°C for 5 min, 50 μL of NADPH was added to initiate the reaction. After incubation for the corresponding time, an appropriate amount of ice-cold acetonitrile containing an internal standard was added to terminate the reaction. The mixture was vortexed to mix well, centrifuged, and the supernatant was taken. The supernatant was injected into LC-MS/MS to determine the production amount of metabolites of the probe substrate. Taking the enzyme activity at the 0-concentration point (characterized by the production amount of metabolites) as 100%, the percentage of residual enzyme activity of metabolites at different concentrations of the test compound was calculated. $IC_{50}$ was calculated by the residual enzyme activity using Graphpad Prism software.

The experimental results showed that the inhibitory activity of the compounds of the present disclosure against CYP2C9, CYP2C19, CYP3A4-M, and CYP3A4-T was significantly weaker than that of the reference compound MRTX0902, which is beneficial for reducing the risk of drug-drug interactions. For example, Table 4 below lists the test data of some Example compounds of the present disclosure and the reference compound MRTX0902:

Table 4. Test data of inhibitory activity on drug-metabolizing enzymes

| | Inhibitory activity on drug-metabolizing enzymes ($IC_{50}$, μM) | | | |
|---|---|---|---|---|
| | CYP2C9 | CYP2C19 | CYP3A4-M | CYP3A4-T |
| Example 9 | >10 | >10 | >10 | >10 |
| Example 12 | >10 | >10 | >10 | >10 |
| Example 13 | >10 | >10 | >10 | >10 |
| Example 52 | >10 | >10 | >10 | >10 |
| Example 73 | >10 | >10 | >10 | >10 |
| Example 74 | >10 | >10 | >10 | >10 |
| Example 75 | >10 | >10 | >10 | >10 |
| Example 77 | >10 | >10 | 7.81 | >10 |
| Example 80 | >10 | >10 | >10 | >10 |
| Example 88 | >10 | >10 | >10 | >10 |
| Example 98 | >10 | >10 | >10 | >10 |
| Example 109 | >10 | >10 | >10 | >10 |
| Example 110 | >10 | >10 | >10 | >10 |
| Example 111 | >10 | >10 | >10 | >10 |
| Example 112 | >10 | >10 | >10 | >10 |
| Example 120 | >10 | >10 | >10 | >10 |
| Example 216 | >10 | >10 | >10 | >10 |
| Example 217 | >10 | >10 | >10 | >10 |
| Example 222 | >10 | >10 | >10 | >10 |
| MRTX0902 | 4.00 | 8.62 | 6.90 | 8.75 |

As can be seen from the data in Table 4, compared with compound MRTX0902, the compounds of the present disclosure show weaker inhibitory effects on liver drug-metabolizing enzymes and have better druggability.

In addition, by comparing with the corresponding liver drug-metabolizing enzyme inhibitory activity test data in Table 4 of PCT/CN2023/104412, it can be known that the compounds of the present disclosure, with the $-NR_{11}R_{12}$ functional group introduced at the $R_4$ position, show significantly weaker inhibitory effects on CYP2C9 compared with those where $R_4$ is other types of substituents such as methyl.

**Test Example 5: Rat Pharmacokinetic Test**

Male SD rats, weighing 200-250 g, were used and divided into two groups based on the principle of similar body weight, with 3 rats in each of the IV group and the PO group. The IV group was administered the test compound at a dose of 1 mg/kg via intravenous injection, and the PO group was administered the test compound at a dose of 5 mg/kg via gavage. Whole blood was collected from the IV group respectively before administration and at 0.083, 0.25, 0.5, 1, 2, 4, 6, 8, and 24 h after administration; for the PO group, whole blood was collected before administration and at 0.25, 0.5, 1, 2, 4, 6, 8, and 24 h after administration. The blood samples were placed in $K_2$-EDTA anticoagulant tubes, centrifuged (4°C) within 30 min to separate plasma, and stored at -80°C for subsequent testing. During sample processing, an appropriate amount of acetonitrile containing an internal standard was used for protein precipitation, vortexed, and after centrifugation, the supernatant was taken for injection into LC-MS/MS for detection. Based on the measured plasma drug concentrations, the main pharmacokinetic parameters were calculated to evaluate its pharmacokinetic behavior. The main pharmacokinetic parameters were analyzed by the non-compartmental model using WinNonLin 8.3 software.

The results of the rat pharmacokinetic test showed that when rats were administered the compounds of the present disclosure via oral gavage, the oral bioavailability could reach 100%-110%. However, the oral bioavailability of the reference compound MRTX0902 was 22.6%. Compared with MRTX0902, the oral bioavailability of the compounds of the present disclosure was significantly improved.

**Test Example 6: Mouse Pharmacokinetic Test**

Female Balb/c mice, weighing 19-21 g, were divided into two groups based on the principle of similar body weight, with 9 mice in each of the IV group and the PO group (for cross blood collection). The IV group was administered the test compound at a dose of 1 mg/kg via intravenous injection, and the PO group was administered the test compound at a dose of 10 mg/kg via gavage. Whole blood was collected from the IV group before administration and at 0.083, 0.25, 0.5, 1, 2, 4, 6, 8, and 24 h after administration; for the PO group, whole blood was collected before administration and at 0.25, 0.5, 1, 2, 4, 6, 8, and 24 h after administration. The blood samples were placed in $K_2$-EDTA anticoagulant tubes, centrifuged (4°C) within 30 minutes to separate plasma, and stored at -80°C for subsequent testing. During sample processing, an appropriate amount of acetonitrile containing an internal standard was used for protein precipitation, vortexed, and after centrifugation, the supernatant was taken for injection into LC-MS/MS for detection. Based on the measured plasma drug concentrations, the main pharmacokinetic parameters were calculated to evaluate its pharmacokinetic behavior. The main pharmacokinetic parameters were analyzed by the non-compartmental model using WinNonLin 8.3 software.

The results of the mouse pharmacokinetic test showed that when mice were administered the compounds of the present disclosure via oral gavage, the oral bioavailability could reach 100%-110%. However, the oral bioavailability of the reference compound MRTX0902 was 40.4%. Compared with MRTX0902, the oral bioavailability of the compounds of the present disclosure was significantly improved.

**Test Example 7: Beagle Dog Pharmacokinetic Test**

Male Beagle dogs, weighing 9-12 kg, were divided into two groups based on the principle of similar body weight, with 3 dogs in each of the IV group and the PO group. The IV group was administered the test compound at a dose of 1 mg/kg via intravenous injection, and the PO group was administered the test compound at a dose of 5 mg/kg via gavage. Whole blood was collected from the IV group before administration and at 0.083, 0.25, 0.5, 1, 2, 4, 6, 8, and 24 h after administration; for the PO group, whole blood was collected before administration and at 0.083, 0.25, 0.5, 1, 2, 4, 6, 8, and 24 h after administration. The blood samples were placed in $K_2$-EDTA anticoagulant tubes, centrifuged (4°C) within 30 minutes to separate plasma, and stored at -80°C for subsequent analysis. During sample processing, an appropriate amount of acetonitrile containing an internal standard was used for protein precipitation, vortexed, and after centrifugation, the supernatant was taken for injection into LC-MS/MS for detection. Based on the measured plasma drug concentrations, the main pharmacokinetic parameters were calculated to evaluate its pharmacokinetic behavior. The main pharmacokinetic parameters were analyzed by the non-compartmental model using WinNonLin 8.3 software.

The results of the Beagle Dog Pharmacokinetic test showed that when Beagle dogs were administered the compounds of the present disclosure via oral gavage, the oral bioavailability could reach 100%-110%, exhibiting high oral bioavailability.

**Test Example 8: In Vivo Pharmacodynamic Test**

In this experiment, a subcutaneous xenograft tumor model of human pancreatic cancer MIA PaCa-2 cells was used to evaluate the anti-tumor activity of the compounds of the present disclosure. Balb/c-nu nude mice were selected for the experiment, which were female, 6-8 weeks old, and weighed approximately 18-22 grams. $5\times10^6$ MIA PaCa-2 cells

(suspended in 0.10 mL of 50% Matrigel suspension) were inoculated subcutaneously into the right scapula of the mice. When the average tumor volume grew to approximately 100-200 $mm^3$, grouping and administration were initiated. The test compounds of the present disclosure were administered by oral gavage (P.O), twice a day (BID). Tumor volume and body weight were measured twice a week, wherein the tumor volume was measured in cubic millimeters and calculated by the following formula: $V = 0.5a \times b^2$, wherein a and b are the major diameter and minor diameter of the tumor, respectively. The anti-tumor efficacy of the test compounds was evaluated using the tumor growth inhibition rate TGI (%). The calculation of TGI (%): TGI (%) = [1 - (Average tumor volume at the end of administration in a treatment group - Average tumor volume at the start of administration in the same treatment group) / (Average tumor volume at the end of administration in the solvent control group - Average tumor volume at the start of administration in the solvent control group)] × 100%. The calculation of body weight change (%): Body weight change (%) = (Average body weight of animals at the end of administration in a treatment group - Average body weight of animals at the start of administration in the same treatment group) / Average body weight of animals at the start of administration in the same treatment group × 100%.

The experimental results showed that the TGI (%) of the compounds of the present disclosure ranged from 45% ~ 90%, exhibiting good anti-tumor activity. No significant decrease in animal body weight was observed, and the tolerance was good.

Based on the comprehensive analysis of the above experimental results, it can be seen that the compounds of the present disclosure exhibit significant inhibitory effects on SOS1 and can serve as SOS1 inhibitors. They have broad application prospects in the fields of diseases mediated by an SOS1 protein, such as cancer and pathogenic skin rash diseases. Furthermore, compared with MRTX0902, the compounds of the present disclosure exhibit better metabolic stability in liver microsomes, weaker inhibitory effects on liver drug-metabolizing enzymes, and higher oral bioavailability. Thus, they possess excellent druggability and promising clinical application prospects.

Although examples of the present disclosure have been shown and described, it will be understood by those skilled in the art that various changes, modifications, substitutions, and variations may be made to these examples without departing from the principles and spirit of the present disclosure. The scope of the present disclosure is defined by the appended claims and their equivalents.

## Claims

1. A compound represented by Formula I, or a tautomer, stereoisomer, solvate, metabolite, isotopically labeled compound, pharmaceutically acceptable salt or co-crystal thereof:

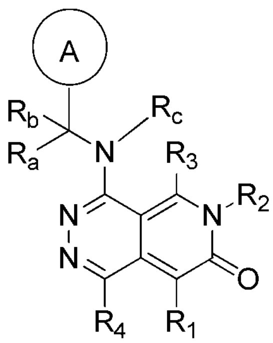

Formula I

wherein:

ring A is selected from aryl, heteroaryl, the aryl, heteroaryl are optionally substituted with a substituent selected from halogen, alkyl, alkoxy, -OH, -CN, $-NR_5R_6$, $-SF_5$, $-SO_2R_{10}$, the alkyl, alkoxy are further optionally substituted with a substituent selected from halogen, $-NR_5R_6$, -OH, wherein, $R_5$, $R_6$ are independently selected from H, alkyl, $R_{10}$ is alkyl and optionally substituted with halogen; or, two substituents on the aryl or heteroaryl are connected to form an aliphatic carbocyclic ring, the aliphatic carbocyclic ring is optionally substituted with halogen;
$R_a$, $R_b$, $R_c$ are independently selected from H, alkyl;
$R_1$, $R_3$ are independently selected from H, halogen, alkyl, alkoxy, -OH, -CN, $-NR_7R_8$, the alkyl, alkoxy are optionally substituted with a substituent selected from halogen, $-NR_7R_8$, wherein $R_7$, $R_8$ are independently selected from H, alkyl;
$R_2$ is selected from H, alkyl, cycloalkyl, heterocycloalkyl, cycloalkenyl, heterocycloalkenyl, the cycloalkyl,

heterocycloalkyl, cycloalkenyl, heterocycloalkenyl are optionally substituted with a substituent selected from halogen, alkyl, alkoxy,

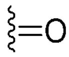
,

$-C(=O)R_9$, $R_9$ is selected from alkyl, cycloalkyl, heterocycloalkyl;
$R_4$ is $-NR_{11}R_{12}$, $R_{11}$, $R_{12}$ are independently selected from H, alkyl, cycloalkyl, heterocycloalkyl, wherein, when $R_{11}$ and/or $R_{12}$ are alkyl, the alkyl is optionally substituted with a substituent selected from -OH, alkenyl, halogen, $-NR_{14}R_{15}$, cycloalkyl, $R_{14}$, $R_{15}$ are independently selected from H, alkyl; when $R_{11}$ and/or $R_{12}$ are selected from cycloalkyl, heterocycloalkyl, the cycloalkyl, heterocycloalkyl are optionally substituted with alkyl; or,
$R_{11}$, $R_{12}$ and the nitrogen atom to which they are attached collectively form a heterocycloalkyl, the heterocycloalkyl is optionally substituted with a substituent selected from halogen, alkyl, cycloalkyl, - $C(=O)R_{13}$,

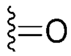
,

-OH, the alkyl is further optionally substituted with a substituent selected from -OH, cycloalkyl, wherein, $R_{13}$ is alkyl; or,
$R_{11}$, $R_{12}$ and the nitrogen atom to which they are attached collectively form a heteroaryl.

**2.** The compound according to claim 1, wherein, ring A is selected from aryl, heteroaryl, the aryl, heteroaryl are optionally substituted with a substituent selected from halogen, alkyl, alkoxy, -OH, -CN, - $NR_5R_6$, $-SF_5$, $-SO_2R_{10}$, the alkyl, alkoxy are further optionally substituted with a substituent selected from halogen, $-NR_5R_6$, wherein, $R_5$, $R_6$ are independently selected from H, alkyl, $R_{10}$ is alkyl and optionally substituted with halogen.

**3.** The compound according to claim 1 or 2, wherein, ring A is selected from 6~12 membered aryl, 5-12 membered heteroaryl, the heteroaryl contains heteroatoms selected from N, O, S, the substituents of the above groups are as defined in claim 1 or 2;
preferably, ring A is phenyl, the substituents thereof are as defined in claim 1 or 2.

**4.** The compound according to any one of claims 1-3, wherein, the substituent contained in ring A is selected from halogen, C1~C6 alkyl, C1-C6 alkoxy, -OH, -CN, $-NR_5R_6$, $-SF_5$, $-SO_2R_{10}$, the alkyl, alkoxy are further optionally substituted with a substituent selected from halogen, $-NR_5R_6$, -OH, wherein, $R_5$, $R_6$ are independently selected from H, C1-C6 alkyl, $R_{10}$ is C1~C6 alkyl and optionally substituted with halogen; or, two substituents on ring A are connected to form a 5-6 membered aliphatic carbocyclic ring, the aliphatic carbocyclic ring is optionally substituted with halogen;

preferably, the substituent contained in ring A is selected from halogen, C1-C6 alkyl, -CN, $-NR_5R_6$, - $SF_5$, $-SO_2R_{10}$, the alkyl is further optionally substituted with a substituent selected from halogen, -OH, wherein, $R_5$, $R_6$ are independently selected from H, C1-C6 alkyl, $R_{10}$ is C1~C6 alkyl and optionally substituted with halogen; or, two substituents on ring A are connected to form a 5~6 membered aliphatic carbocyclic ring, the aliphatic carbocyclic ring is optionally substituted with halogen;
preferably, the substituent contained in ring A is selected from halogen, C1-C6 alkyl, C1-C6 alkoxy, - OH, -CN, $-NR_5R_6$, $-SF_5$, $-SO_2R_{10}$, the alkyl, alkoxy are further optionally substituted with a substituent selected from halogen, $-NR_5R_6$, wherein, $R_5$, $R_6$ are independently selected from H, C1-C6 alkyl, $R_{10}$ is C1~C6 alkyl and optionally substituted with halogen;
preferably, the substituent contained in ring A is selected from halogen, C1~C6 alkyl, -CN, $-NR_5R_6$, the alkyl is further optionally substituted with halogen, $R_5$, $R_6$ are independently selected from H, C1~C6 alkyl;
preferably, the substituent contained in ring A is selected from -F, $-CH_3$, $-CHF_2$, $-CF_3$, -CN, $-NH_2$, $-SF_5$, $-SO_2CHF_2$, $-CF_2CH_2OH$; or, two substituents on ring A are connected to form

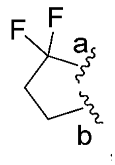

wherein the two ends of a and b are respectively connected to two adjacent ring carbon atoms on ring A; preferably, the substituent contained in ring A is selected from -F, $-CH_3$, $-CHF_2$, $-CF_3$, -CN, $-NH_2$.

**5.** The compound according to any one of claims 1-4, wherein, ring A is selected from:

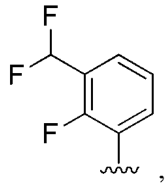 ,

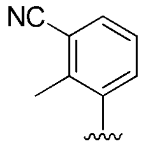 , 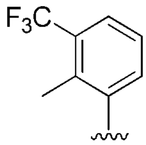 , 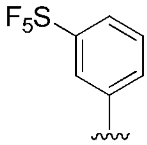 , 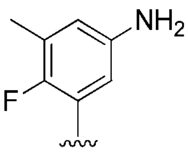 , 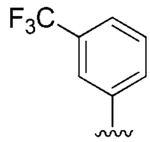 , 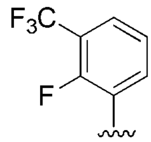 ,

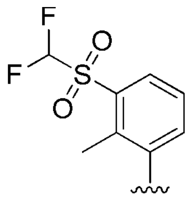 , 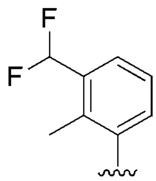 , 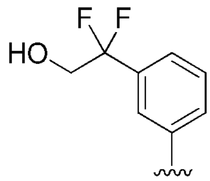 , 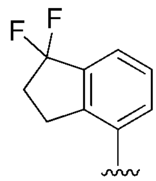 ;

preferably, ring A is selected from:

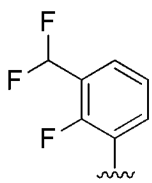 , 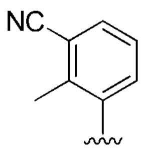 , 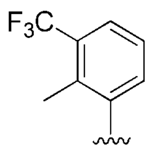 .

**6.** The compound according to any one of claims 1~5, wherein, $R_a$, $R_b$, $R_c$ are independently selected from H, C1~C6 alkyl;

preferably, $R_a$, $R_b$, $R_c$ are independently selected from H, methyl;
preferably, $R_a$ is methyl, $R_b$ is H, $R_c$ is H.

**7.** The compound according to any one of claims 1~6, wherein, the structure is represented by Formula II:

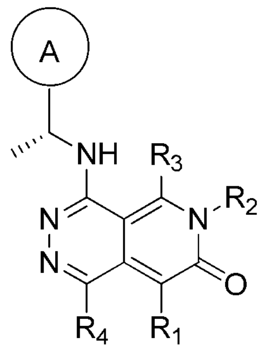

Formula II

wherein each group is as defined in any one of claims 1~5.

**8.** The compound according to any one of claims 1~7, wherein, $R_1$, $R_3$ are independently selected from H, halogen, C1~C6 alkyl, C1-C6 alkoxy, -OH, -CN, $-NR_7R_8$, the alkyl, alkoxy are optionally substituted with a substituent selected from halogen, $-NR_7R_8$, wherein $R_7$, $R_8$ are independently selected from H, C1~C6 alkyl.

**9.** The compound according to any one of claims 1~7, wherein, $R_1$, $R_3$ are independently selected from H, C1-C6 alkyl, C1-C6 alkoxy, -OH, -CN, $-NR_7R_8$, the alkyl, alkoxy are optionally substituted with a substituent selected from halogen, $-NR_7R_8$, wherein $R_7$, $R_8$ are independently selected from H, alkyl;

preferably, $R_1$, $R_3$ are independently selected from H, C1-C6 alkyl, C1-C6 alkoxy, -OH, -CN, $-NR_7R_8$, the alkyl, alkoxy are optionally substituted with a substituent selected from halogen, $-NR_7R_8$, wherein $R_7$, $R_8$ are independently selected from H, C1~C6 alkyl;
preferably, both $R_1$, $R_3$ are H.

**10.** The compound according to any one of claims 1~9, wherein, $R_2$ is selected from H, C1-C6 alkyl, 3~10 membered cycloalkyl, 3~10 membered heterocycloalkyl, 3~10 membered cycloalkenyl, 3~10 membered heterocycloalkenyl, the cycloalkyl, heterocycloalkyl, cycloalkenyl, heterocycloalkenyl are optionally substituted with a substituent selected from halogen, C1~C6 alkyl, C1~C6 alkoxy,

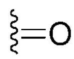
,

- $C(=O)R_9$, $R_9$ is selected from C1~C6 alkyl, 3-10 membered cycloalkyl, 3-10 membered heterocycloalkyl, the heterocycloalkyl, heterocycloalkenyl contain heteroatoms selected from N, O, S;

preferably, $R_2$ is selected from H, C1~C6 alkyl, 3~10 membered cycloalkyl, 3~10 membered heterocycloalkyl, 3~10 membered heterocycloalkenyl, the cycloalkyl, heterocycloalkyl, heterocycloalkenyl are optionally substituted with a substituent selected from C1-C6 alkyl, C1-C6 alkoxy, $-C(=O)R_9$, $R_9$ is selected from C1~C6 alkyl, 3-10 membered heterocycloalkyl, the heterocycloalkyl, heterocycloalkenyl contain 1~2 heteroatoms selected from N, O, S;
preferably, $R_2$ is selected from H, C1-C6 alkyl, 3 membered cycloalkyl, 5-6 membered heterocycloalkyl, 6 membered heterocycloalkenyl, the cycloalkyl, heterocycloalkyl, heterocycloalkenyl are optionally substituted with a substituent selected from C1-C6 alkyl, C1-C6 alkoxy, $-C(=O)R_9$, $R_9$ is selected from C1~C6 alkyl, 6 membered heterocycloalkyl, the heterocycloalkyl, heterocycloalkenyl contain 1~2 heteroatoms selected from N, O;
preferably, $R_2$ is selected from C1-C6 alkyl, 3-8 membered cycloalkyl, 5-8 membered heterocycloalkyl, the cycloalkyl, heterocycloalkyl are optionally substituted with a substituent selected from halogen, C1-C6 alkyl, the heterocycloalkyl contains 1-2 heteroatoms selected from N, O;
preferably, $R_2$ is C1~C6 alkyl; or,
$R_2$ is selected from cyclopropyl, cyclobutyl, tetrahydrofuranyl, piperidinyl, piperazinyl, morpholinyl, 3-azabicyclo [3.3.0]octy, the above groups are optionally substituted with a substituent selected from halogen, C1~C6 alkyl;
preferably, $R_2$ is cyclopropyl, the cyclopropyl is optionally substituted with C1~C6 alkyl;

preferably, $R_2$ is selected from: methyl, ethyl,

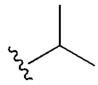, 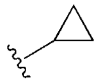, 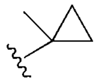, 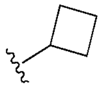, 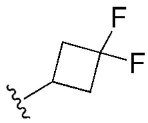,

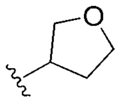, 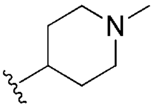, 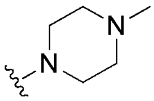, 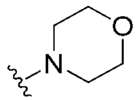, 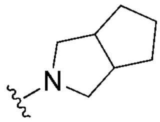;

preferably, $R_2$ is selected from:

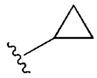, 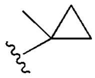.

**11.** The compound according to any one of claims 1-10, wherein, $R_4$ is $-NR_{11}R_{12}$, wherein $R_{11}$, $R_{12}$ are independently selected from H, alkyl, cycloalkyl, the alkyl is optionally substituted with a substituent selected from -OH, alkenyl, the cycloalkyl is optionally substituted with alkyl; or, $R_{11}$, $R_{12}$ and the nitrogen atom to which they are attached collectively form a heterocycloalkyl, the heterocycloalkyl is optionally substituted with a substituent selected from halogen, alkyl, cycloalkyl, $-C(=O)R_{13}$,

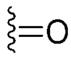,

$R_{13}$ is alkyl; or, $R_{11}$, $R_{12}$ and the nitrogen atom to which they are attached collectively form a heteroaryl.

**12.** The compound according to any one of claims 1-11, wherein, $R_4$ is $-NR_{11}R_{12}$, $R_{11}$, $R_{12}$ are independently selected from H, C1-C6 alkyl, 3-10 membered cycloalkyl, 3~10 membered heterocycloalkyl, the heterocycloalkyl contains heteroatoms selected from N, O, S; wherein, when $R_{11}$ and/or $R_{12}$ are C1~C6 alkyl, the alkyl is optionally substituted with a substituent selected from halogen, $-NR_{14}R_{15}$, 3-10 membered cycloalkyl, $R_{14}$, $R_{15}$ are independently selected from H, C1~C6 alkyl; when $R_{11}$ and/or $R_{12}$ are selected from 3~10 membered cycloalkyl, 3~10 membered heterocycloalkyl, the cycloalkyl, heterocycloalkyl are optionally substituted with C1~C6 alkyl;

preferably, $R_4$ is $-NR_{11}R_{12}$, $R_{11}$, $R_{12}$ are independently selected from H, C1~C6 alkyl, 3~8 membered cycloalkyl, 4-8 membered heterocycloalkyl, the heterocycloalkyl contains 1-2 heteroatoms selected from N, O; wherein, when $R_{11}$ and/or $R_{12}$ are C1~C6 alkyl, the alkyl is optionally substituted with a substituent selected from halogen, $-NR_{14}R_{15}$, 3-8 membered cycloalkyl, $R_{14}$, $R_{15}$ are independently selected from H, C1~C6 alkyl; when $R_{11}$ and/or $R_{12}$ are selected from 3-8 membered cycloalkyl, 4-8 membered heterocycloalkyl, the cycloalkyl, heterocycloalkyl are optionally substituted with C1~C6 alkyl;
preferably, $R_4$ is $-NR_{11}R_{12}$, wherein $R_{11}$, $R_{12}$ are independently selected from H, C1-C6 alkyl, 3-10 membered cycloalkyl, the C1-C6 alkyl is optionally substituted with a substituent selected from -OH, C2~C6 alkenyl, the 3~10 membered cycloalkyl is optionally substituted with C1~C6 alkyl;
preferably, $R_4$ is $-NR_{11}R_{12}$, wherein $R_{11}$, $R_{12}$ are independently selected from H, C1-C6 alkyl, 3~5 membered cycloalkyl, the C1-C6 alkyl is optionally substituted with a substituent selected from -OH, C2~C6 alkenyl, the 3~5 membered cycloalkyl is optionally substituted with C1~C6 alkyl;
preferably, $R_4$ is $-NR_{11}R_{12}$, $R_{11}$, $R_{12}$ are independently selected from H, C1-C6 alkyl, cyclopropyl, bicyclo[1.1.1]pentyl, oxacyclobutyl, tetrahydropyrrolyl, tetrahydrofuranyl, piperidinyl, piperazinyl, morpholinyl, 1-azabicyclo[2.2.2]octyl; wherein, when $R_{11}$ and/or $R_{12}$ are C1-C6 alkyl, the alkyl is optionally substituted with a substituent selected from halogen, $-NR_{14}R_{15}$, 3 membered cycloalkyl, $R_{14}$, $R_{15}$ are independently selected from H, C1~C6 alkyl; when $R_{11}$ and/or $R_{12}$ are selected from cyclopropyl, bicyclo[1.1.1]pentyl, oxacyclobutyl, tetrahydropyrrolyl, tetrahydrofuranyl, piperidinyl, piperazinyl, morpholinyl, 1-azabicyclo[2.2.2]octyl, the above groups are optionally substituted with C1~C6 alkyl;
preferably, $R_4$ is $-NR_{11}R_{12}$, wherein $R_{11}$, $R_{12}$ are independently selected from H, methyl, ethyl, propyl, cyclopropyl, the cyclopropyl is optionally substituted with methyl;
preferably, $R_4$ is selected from:

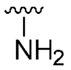, 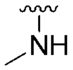, 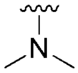, 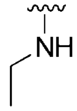, 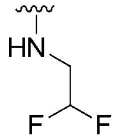, 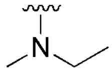,

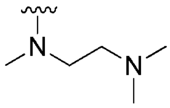, 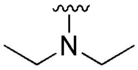, 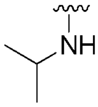, 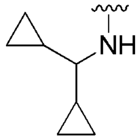, 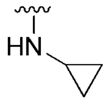, 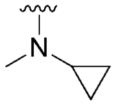, 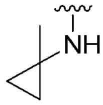, 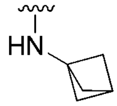,

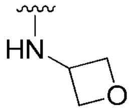, 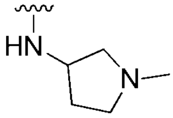, 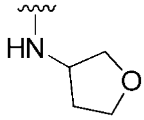, 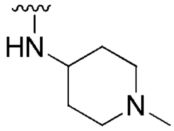, 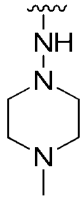, 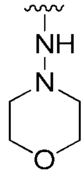, 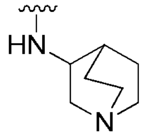;

preferably, $R_4$ is selected from:

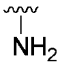 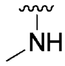 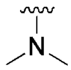 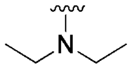 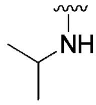 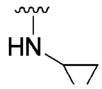 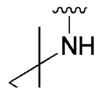

preferably, $R_4$ is

;

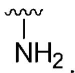.

**13.** The compound according to any one of claims 1-11, wherein, $R_4$ is $-NR_{11}R_{12}$, $R_{11}$, $R_{12}$ and the nitrogen atom to which they are attached collectively form a 3-10 membered heterocycloalkyl, the heterocycloalkyl contains heteroatoms selected from N, O, S, the heterocycloalkyl is optionally substituted with a substituent selected from halogen, C1-C6 alkyl, 3-10 membered cycloalkyl, $-C(=O)R_{13}$,

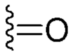, -

OH, the alkyl is further optionally substituted with a substituent selected from -OH, 3-10 membered cycloalkyl, wherein, $R_{13}$ is C1~C6 alkyl;

preferably, $R_4$ is $-NR_{11}R_{12}$, wherein $R_{11}$, $R_{12}$ and the nitrogen atom to which they are attached collectively form a 3-10 membered heterocycloalkyl, the heterocycloalkyl contains heteroatoms selected from N, O, S, the heterocycloalkyl is optionally substituted with a substituent selected from halogen, C1~C6 alkyl, 3~10 membered cycloalkyl, $-C(=O)R_{13}$,

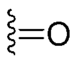,

$R_{13}$ is C1~C6 alkyl;

preferably, $R_4$ is $-NR_{11}R_{12}$, $R_{11}$, $R_{12}$ and the nitrogen atom to which they are attached collectively form a 4-10 membered heterocycloalkyl, the heterocycloalkyl contains 1-2 heteroatoms selected from N, O, S, the heterocycloalkyl is optionally substituted with a substituent selected from halogen, C1-C6 alkyl, 3~5 membered cycloalkyl, $-C(=O)R_{13}$,

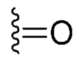,

-OH, the alkyl is further optionally substituted with a substituent selected from -OH, 3~5 membered cycloalkyl, wherein, $R_{13}$ is C1~C6 alkyl;

preferably, $R_4$ is $-NR_{11}R_{12}$, wherein $R_{11}$, $R_{12}$ and the nitrogen atom to which they are attached collectively form a 4~6 membered heterocycloalkyl, the heterocycloalkyl contains 1~2 heteroatoms selected from N, O, S, the heterocycloalkyl is optionally substituted with a substituent selected from halogen, C1~C6 alkyl, $-C(=O)R_{13}$,

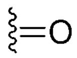,

$R_{13}$ is C1~C6 alkyl;

preferably, $R_4$ is $-NR_{11}R_{12}$, $R_{11}$, $R_{12}$ and the nitrogen atom to which they are attached collectively form

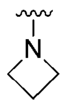, 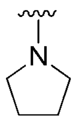, 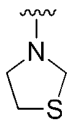, 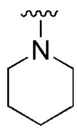, 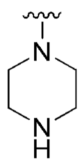, 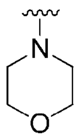, 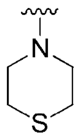, 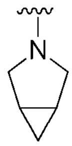, 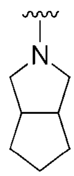, 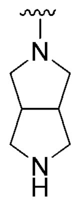, 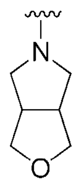, 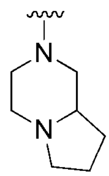,

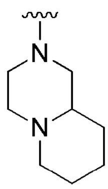, 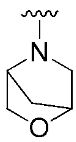, 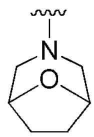, 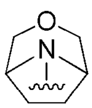, 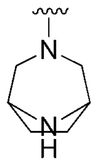, 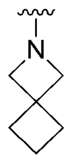, 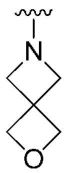, 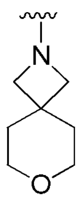, 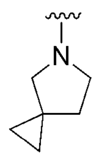, 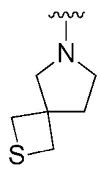,

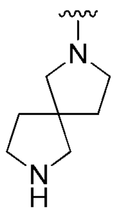, 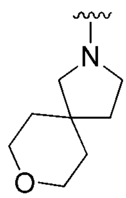, 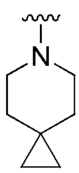, 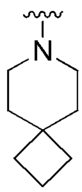, 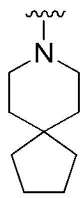, 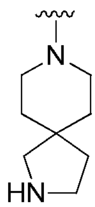, 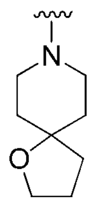,

the above groups are optionally substituted with a substituent selected from halogen, C1-C6 alkyl, 3~5 membered cycloalkyl, $-C(=O)R_{13}$,

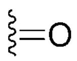,

-OH, the alkyl is further optionally substituted with a substituent selected from -OH, 3~5 membered cycloalkyl, wherein, $R_{13}$ is C1~C6 alkyl;

preferably, $R_4$ is $-NR_{11}R_{12}$, wherein $R_{11}$, $R_{12}$ and the nitrogen atom to which they are attached collectively form azetidinyl, tetrahydropyrrolyl, tetrahydrothiazolyl, piperidinyl, piperazinyl, morpholinyl, thiomorpholinyl, 3-azabicyclo [3.1.0] hexyl, the above groups are optionally substituted with a substituent selected from fluorine, methyl, $-C(=O)R_{13}$,

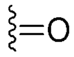,

$R_{13}$ is methyl;

preferably, $R_4$ is $-NR_{11}R_{12}$, wherein $R_{11}$, $R_{12}$ and the nitrogen atom to which they are attached collectively form azetidinyl, piperazinyl, the above groups are optionally substituted with a substituent selected from fluorine, methyl, $-C(=O)R_{13}$,

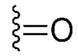

$R_{13}$ is methyl;

preferably, $R_4$ is selected from:

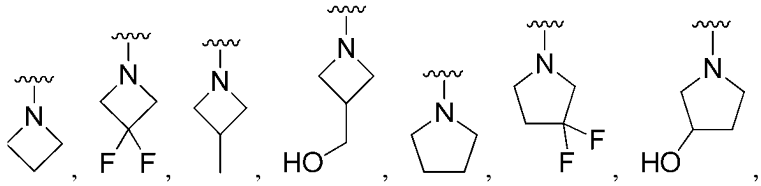,

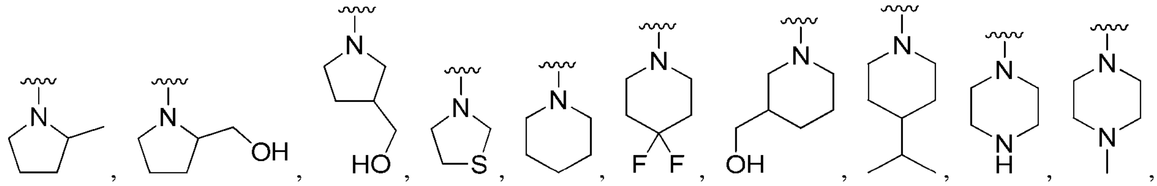,

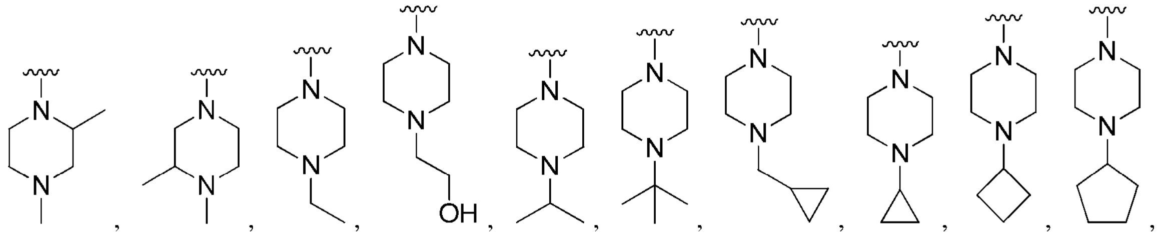,

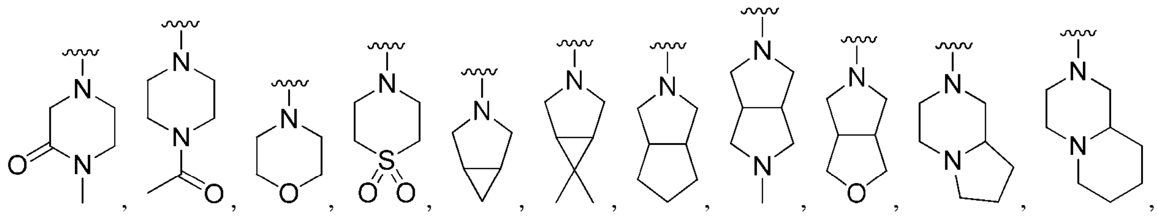,

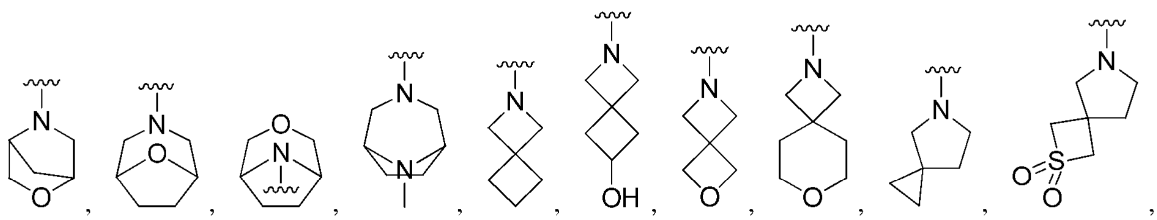,

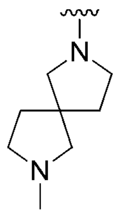, 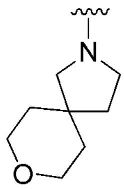, 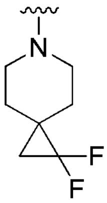, 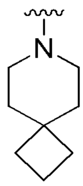, 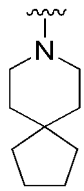, 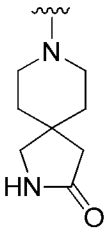, 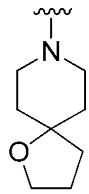;

preferably, the configuration of $R_4$ is selected from at least one of the following:

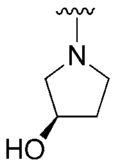, 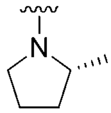,

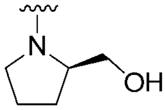, 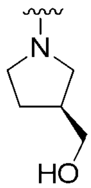, 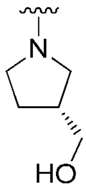, 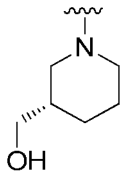, 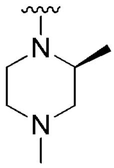, 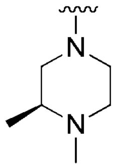, 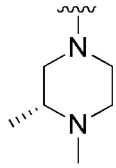, 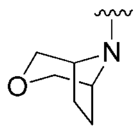;

preferably, $R_4$ is selected from:

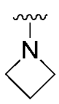 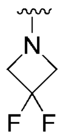 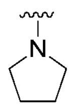 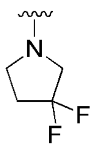 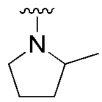 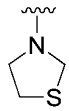 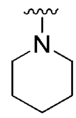

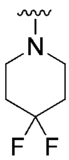 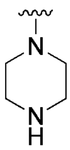 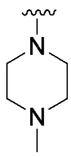 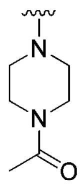 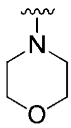 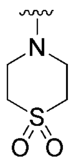 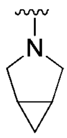;

preferably,

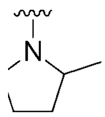 is 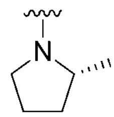.

**14.** The compound according to any one of claims 1-11, wherein, $R_4$ is $-NR_{11}R_{12}$, wherein $R_{11}$, $R_{12}$ and the nitrogen atom to which they are attached collectively form a 5-10 membered heteroaryl, the heteroaryl contains heteroatoms selected from N, O, S;

preferably, $R_4$ is $-NR_{11}R_{12}$, wherein $R_{11}$, $R_{12}$ and the nitrogen atom to which they are attached collectively form a 5 membered heteroaryl, the heteroaryl contains 1~3 nitrogen heteroatoms;
preferably, $R_4$ is $-NR_{11}R_{12}$, wherein $R_{11}$, $R_{12}$ and the nitrogen atom to which they are attached collectively form pyrazolyl, imidazolyl, 1,2,3-triazolyl, 1,2,4-triazolyl;
preferably, $R_4$ is selected from:

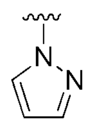 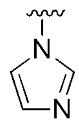 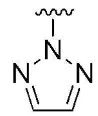 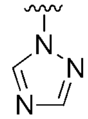 .

**15.** The compound according to any one of claims 1-14, wherein, the compound is selected from one of the following:

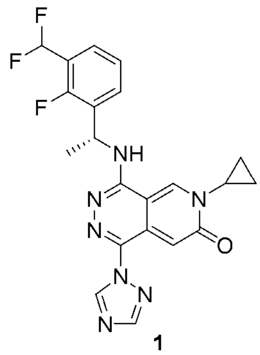
1

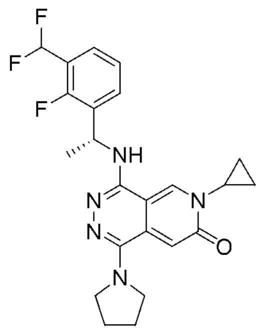
4

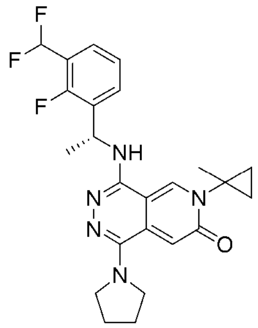
5

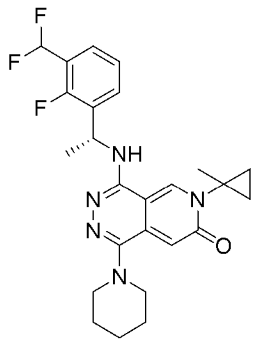
6

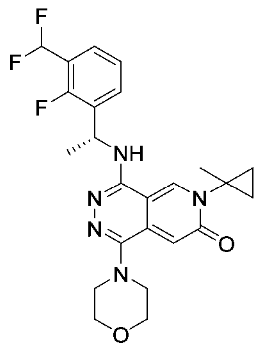
7

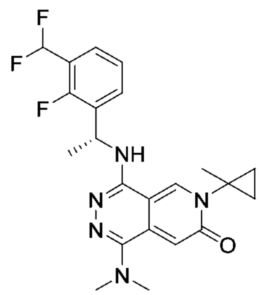
8

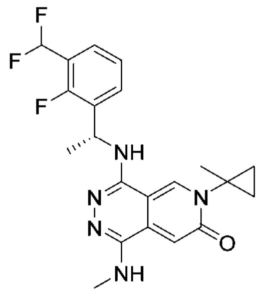
9

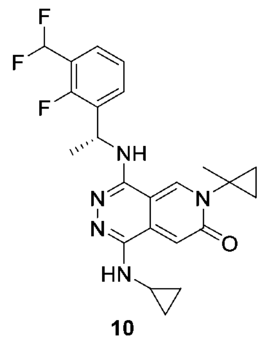
10

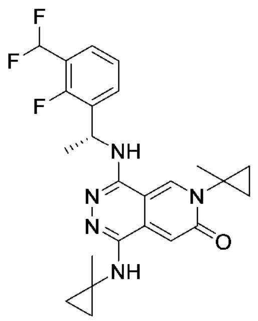
11

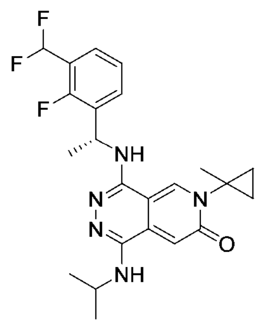
12

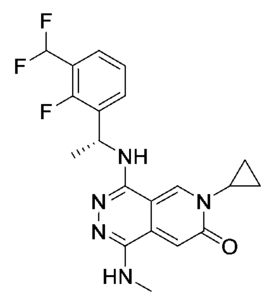
13

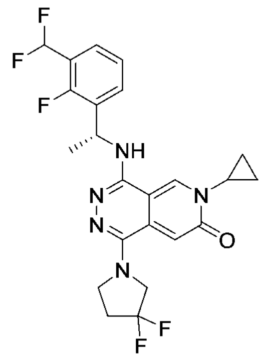
14

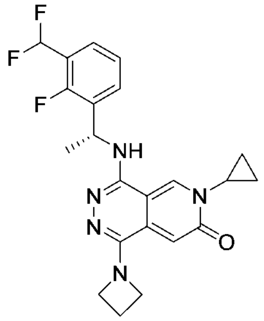
**15**

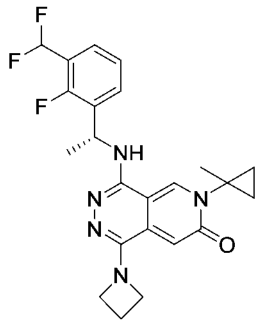
**16**

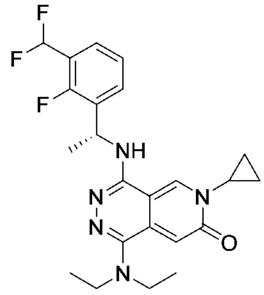
**17**

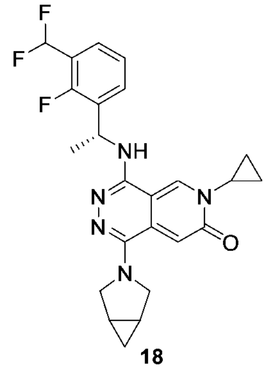
**18**

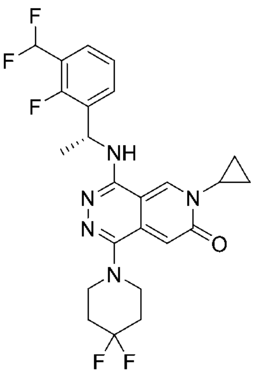
**19**

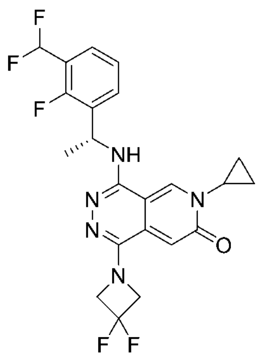
**20**

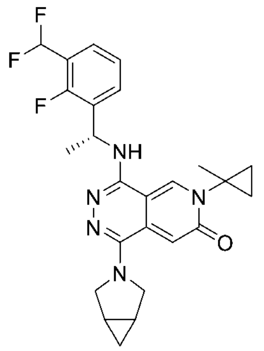
**21**

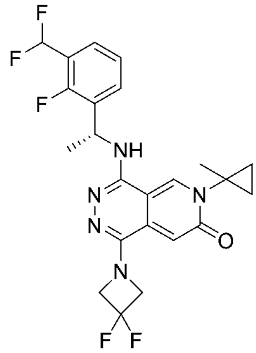
**22**

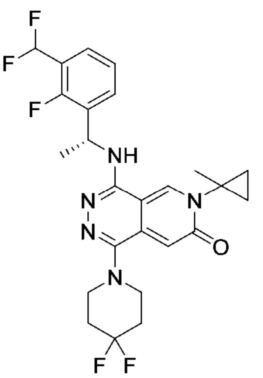
**23**

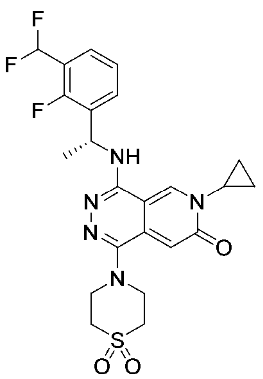
**24**

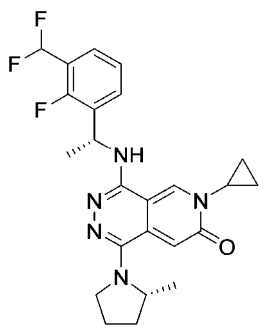
**25**

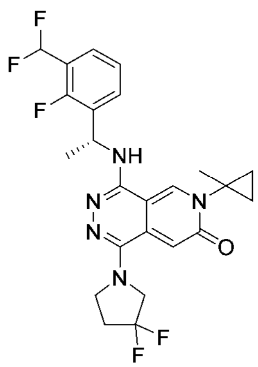
**26**

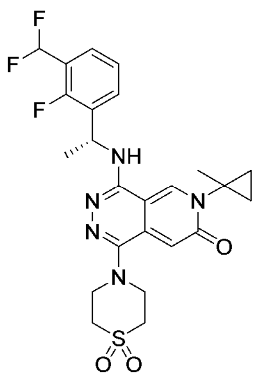
**27**

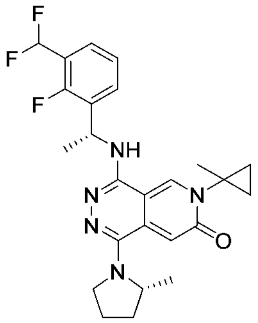
**28**

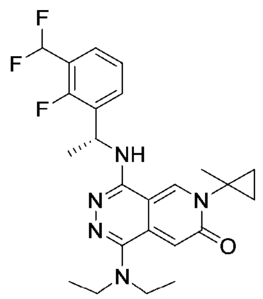
**29**

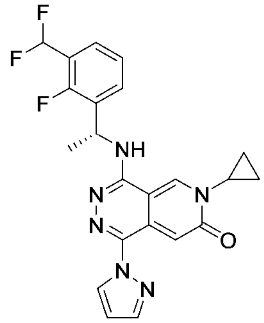
**30**

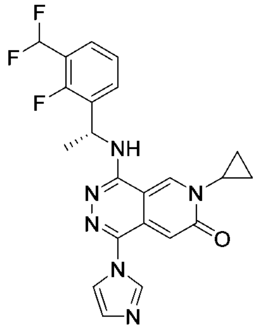
31

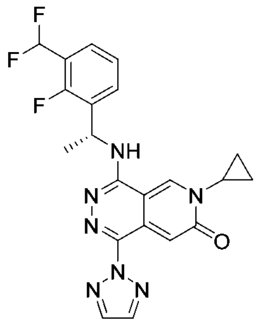
32

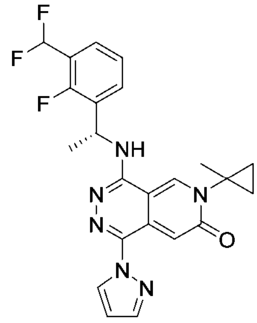
33

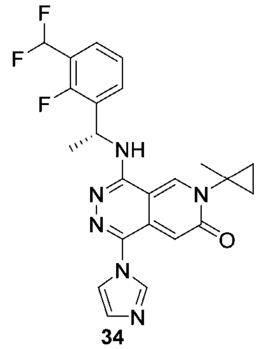
34

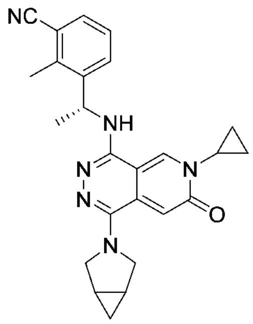
35

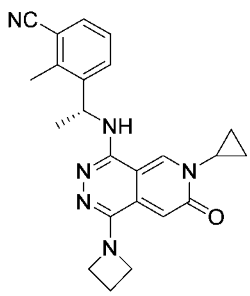
36

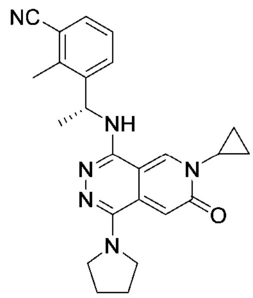
37

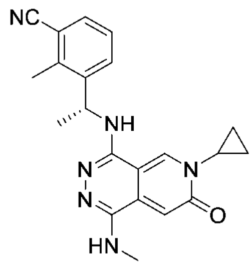
38

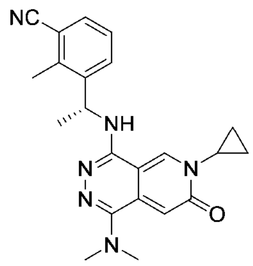
39

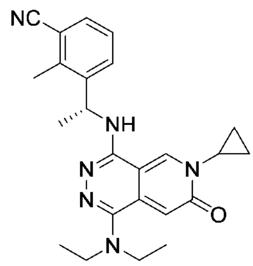
40

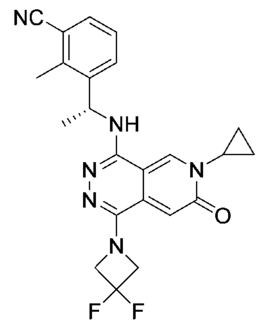
41

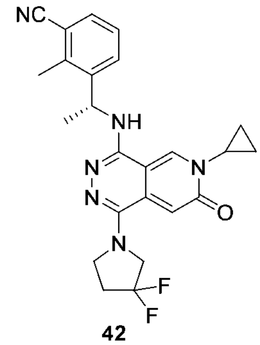
42

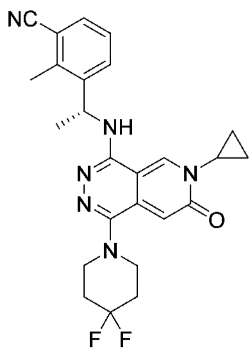
43

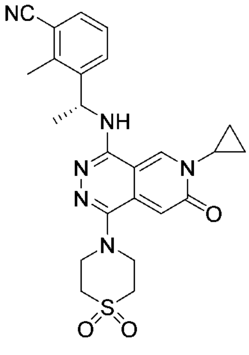
44

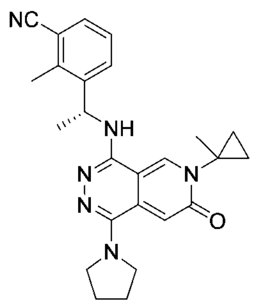
45

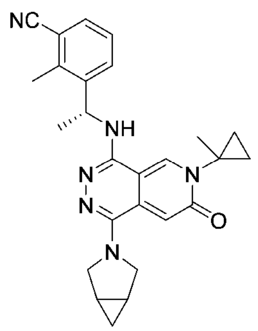
46

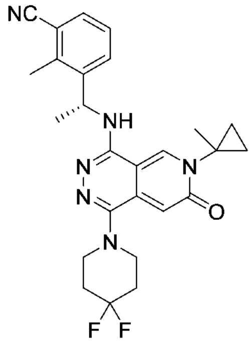
47

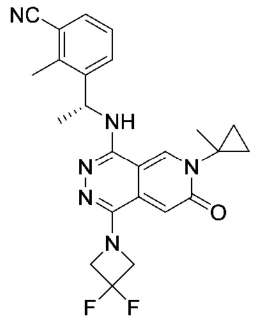
48

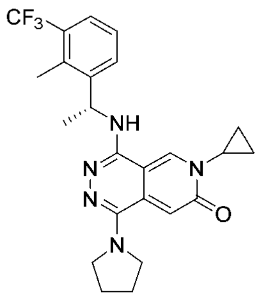
49

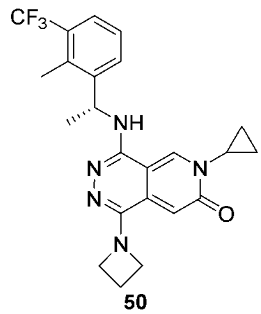
50

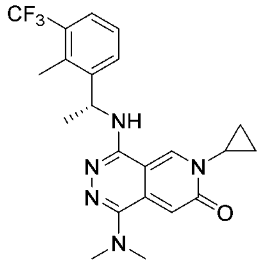
51

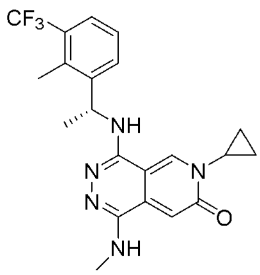
52

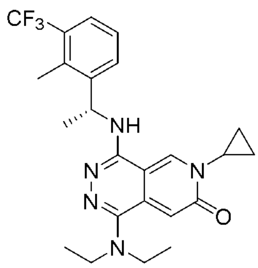
53

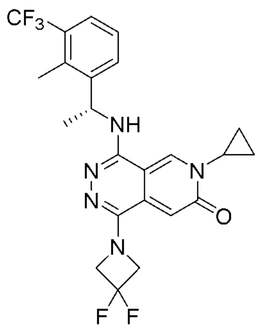
54

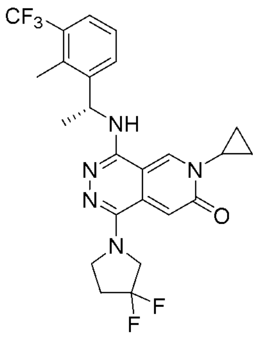
55

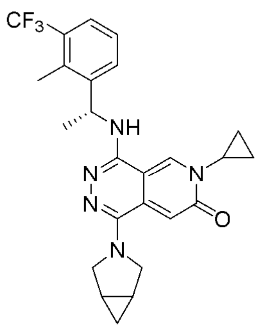
56

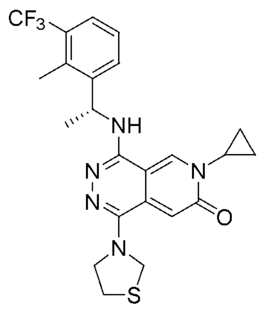
57

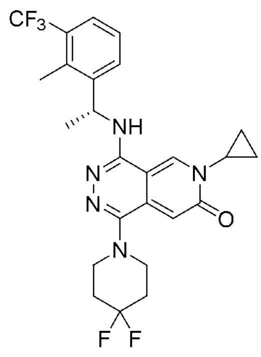
58

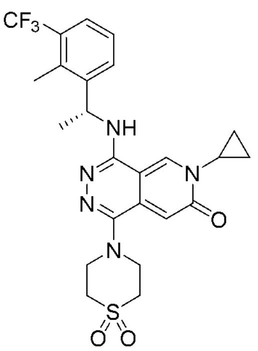
59

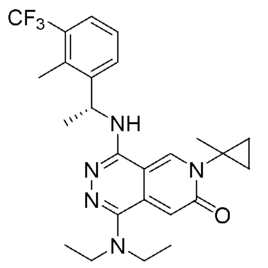
60

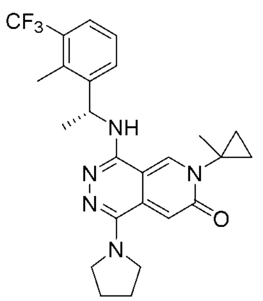
61

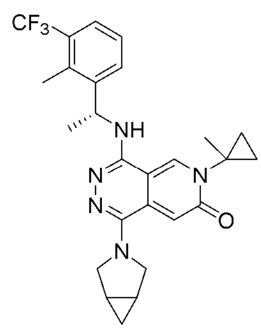
62

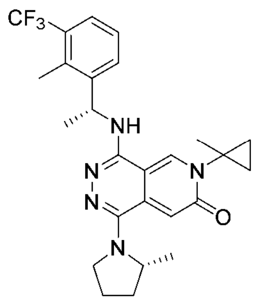
63

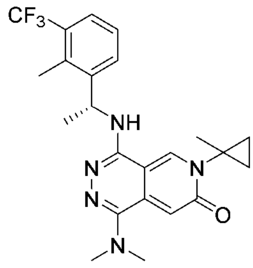
64

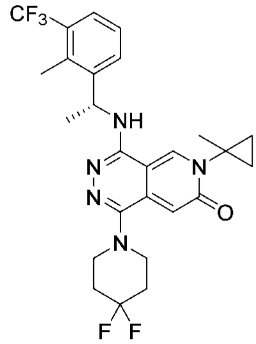
65

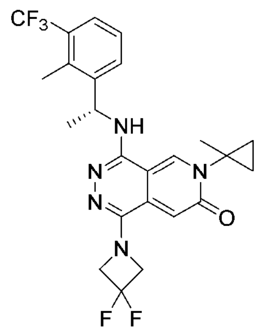
66

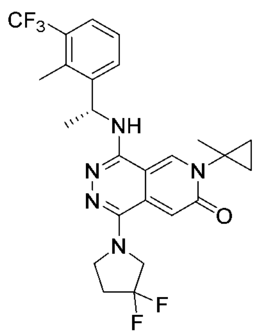
67

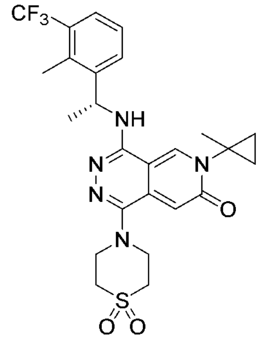
68

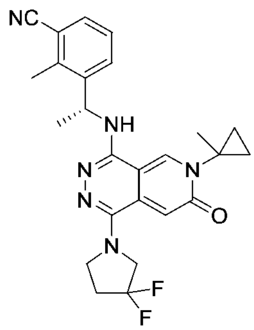
69

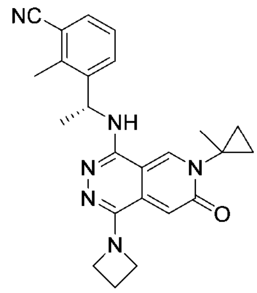
70

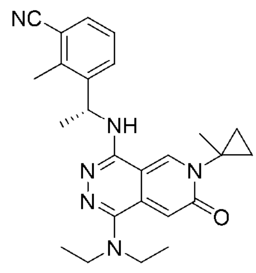
71

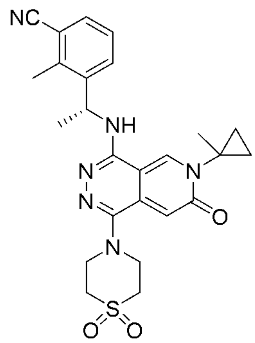
72

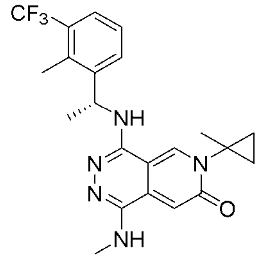
73

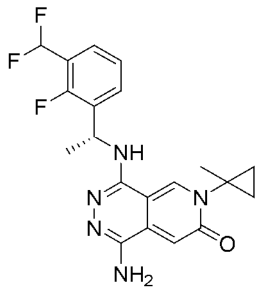
74

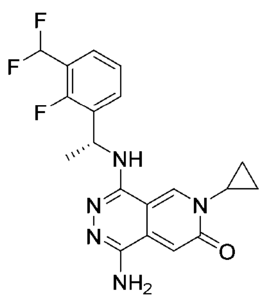
75

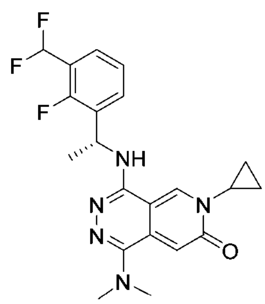
76

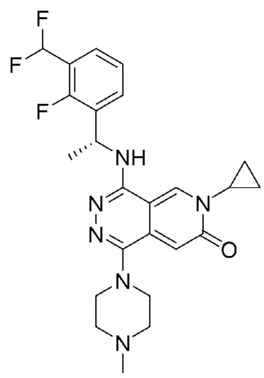
77

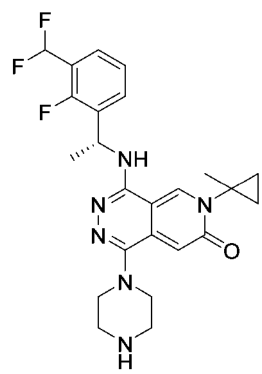
78

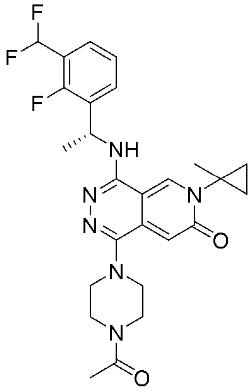

**79**

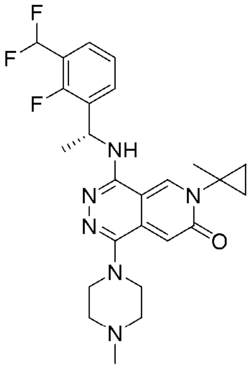

**80**

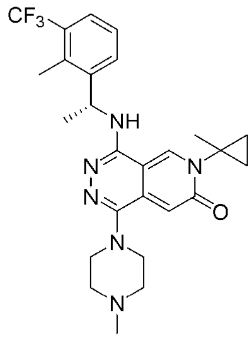

**81**

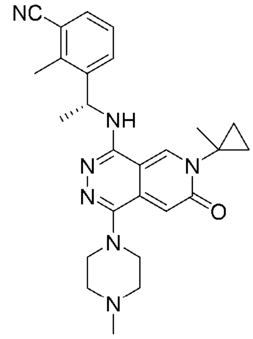

**82**

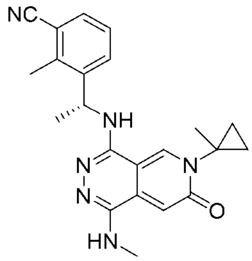

**83**

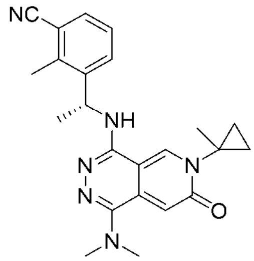

**84**

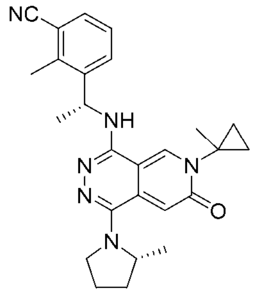

**85**

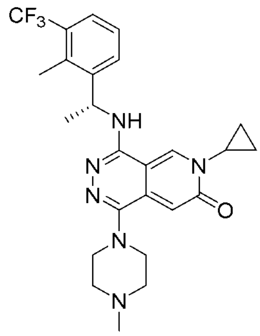

**86**

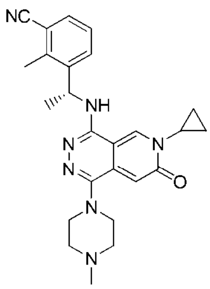

**87**

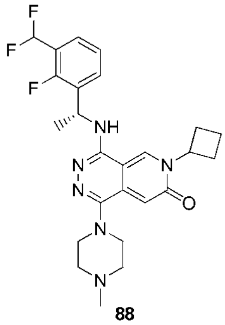

**88**

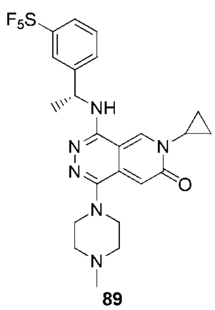

**89**

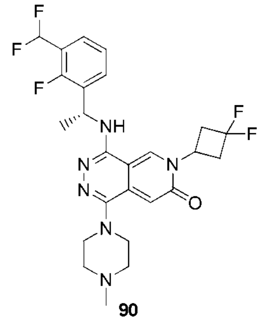

**90**

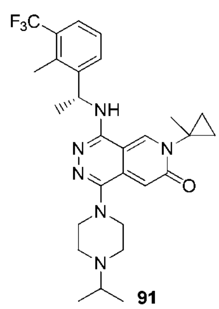

**91**

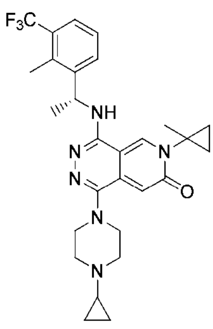

**92**

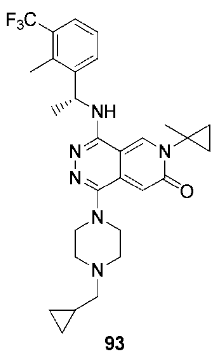

**93**

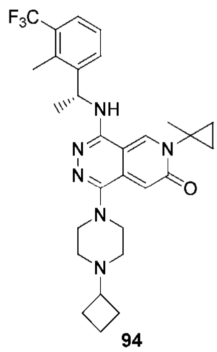

**94**

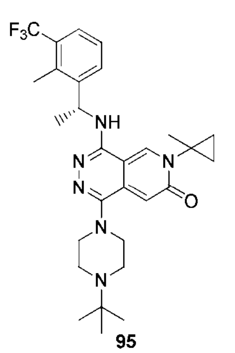

**95**

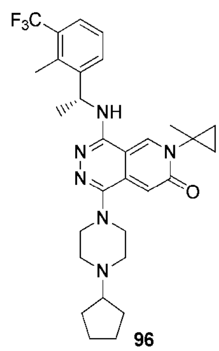

**96**

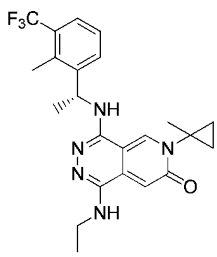
97

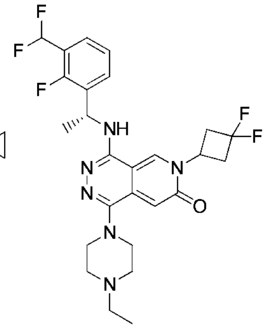
98

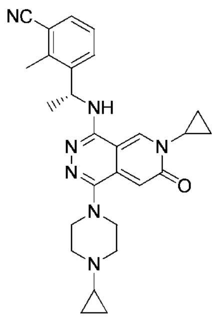
99

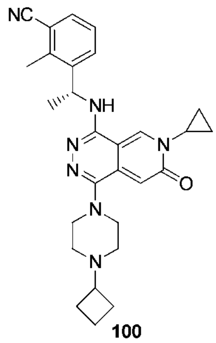
100

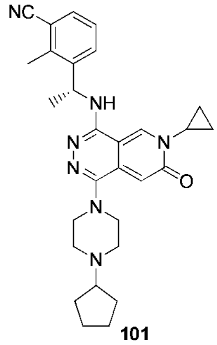
101

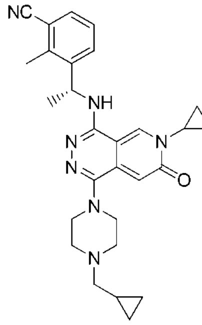
102

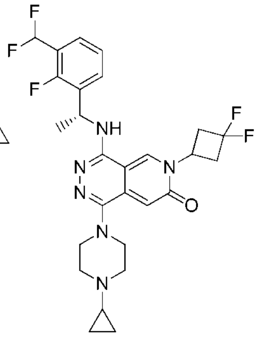
103

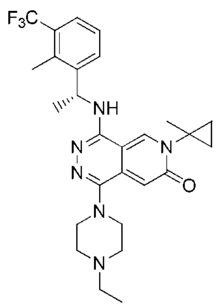
104

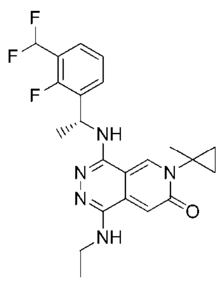
105

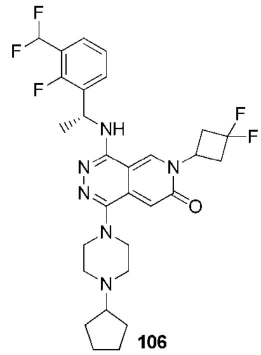
106

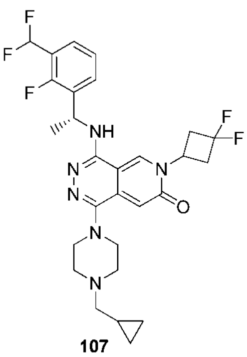
107

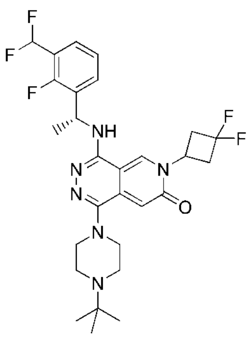
108

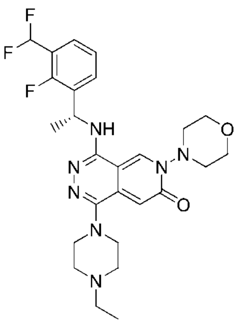
109

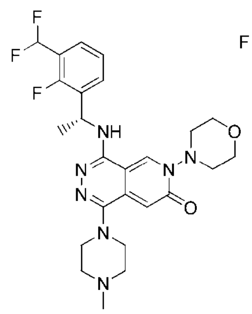
110

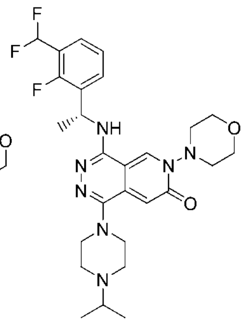
111

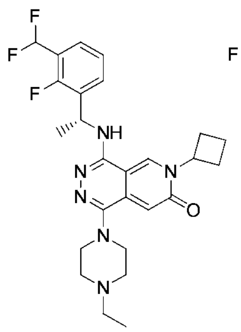
112

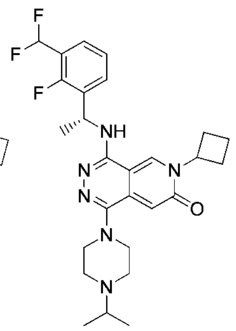
113

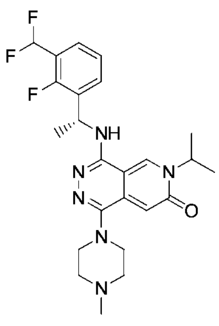
114

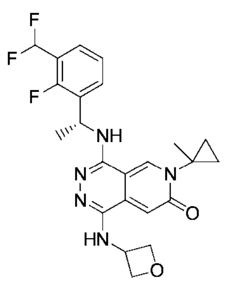
115

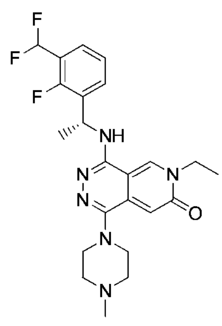
116

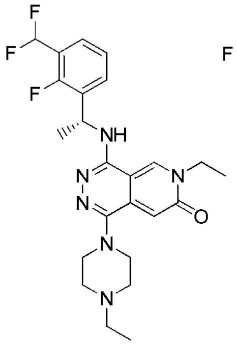
117
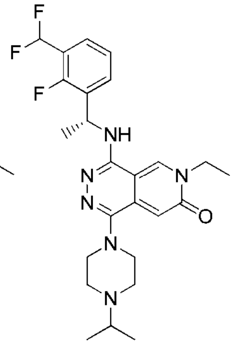
118
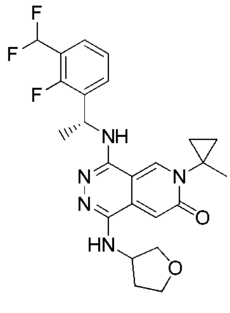
119
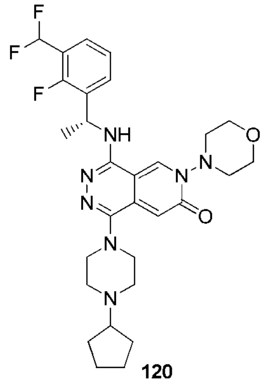
120
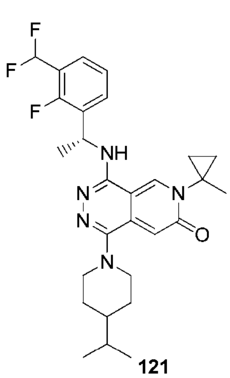
121

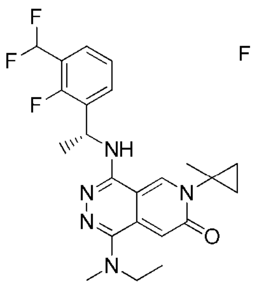
122
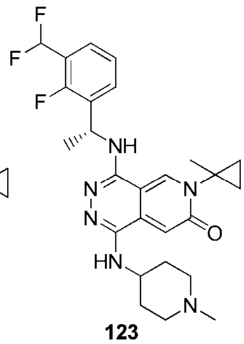
123
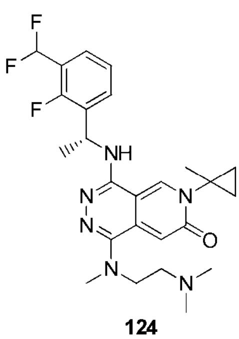
124
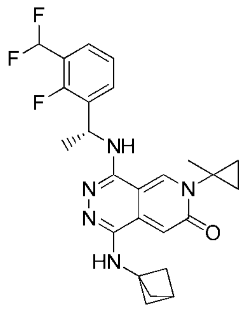
125
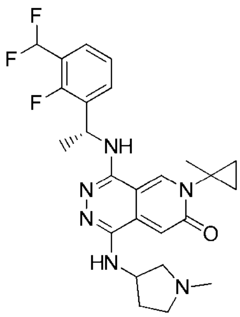
126

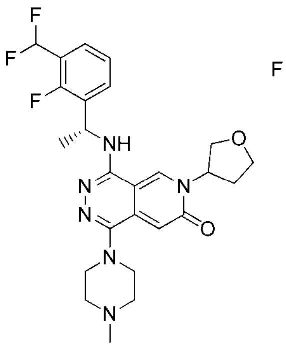
127
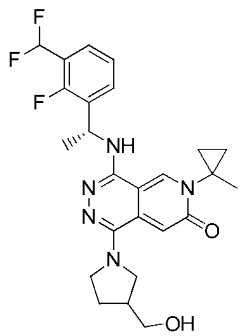
128
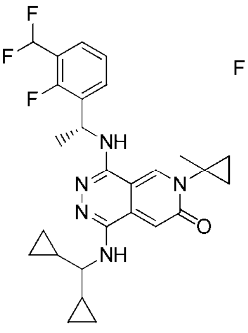
129
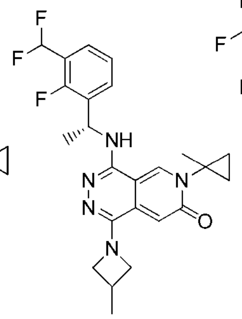
130
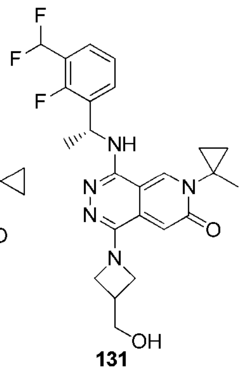
131

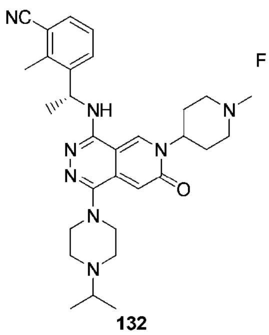
132
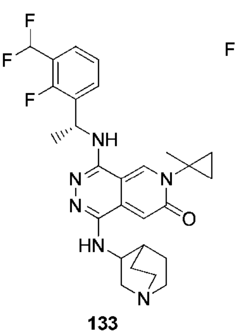
133
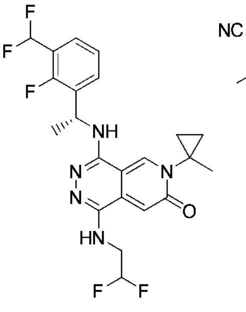
134
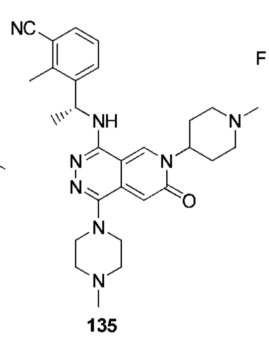
135
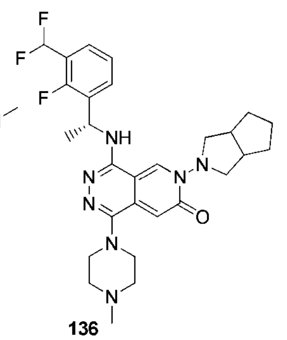
136

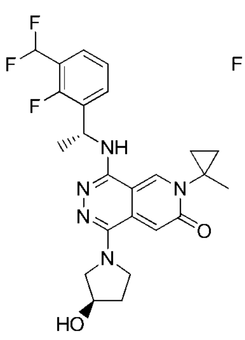

137

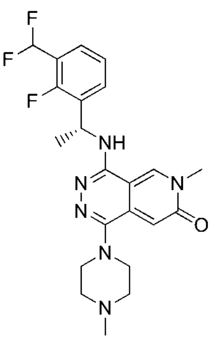

138

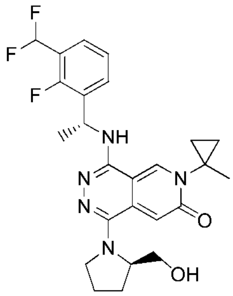

139

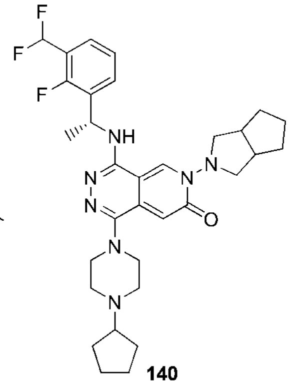

140

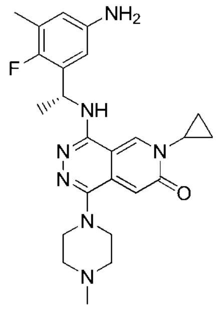

141

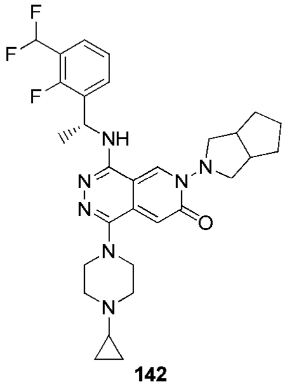

142

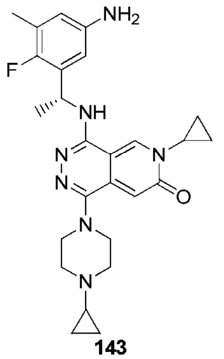

143

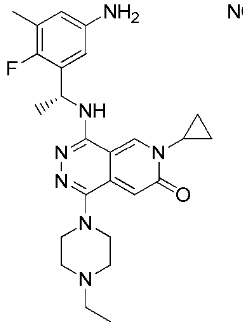

144

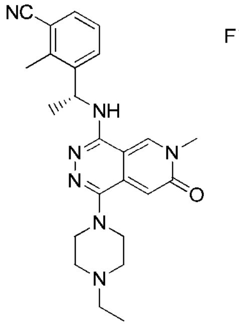

145

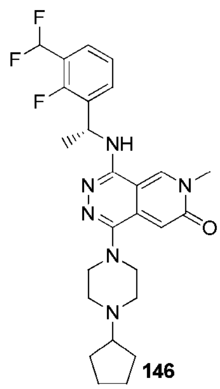

146

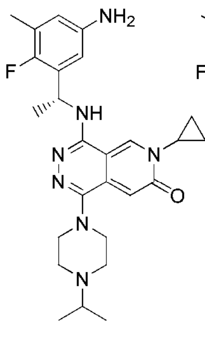

147

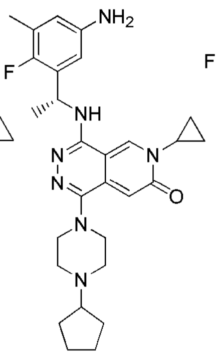

148

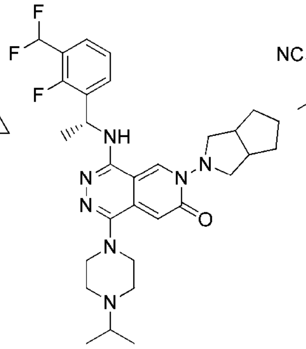

149

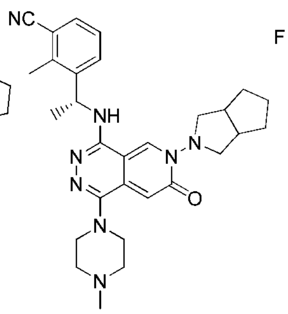

150

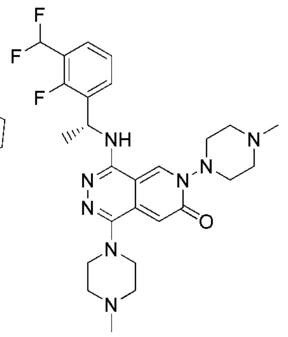

151

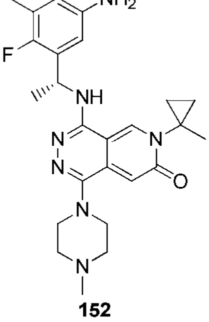

152

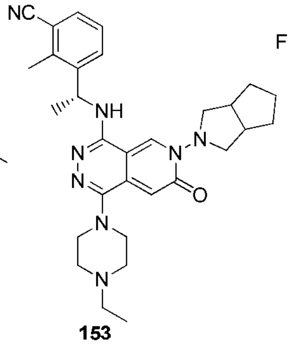

153

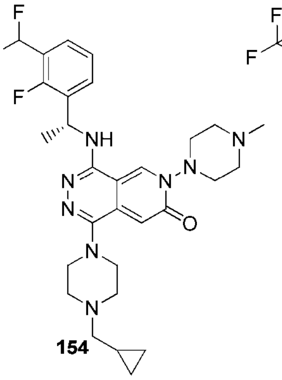

154

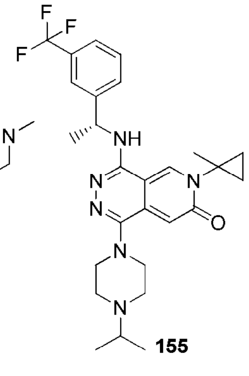

155

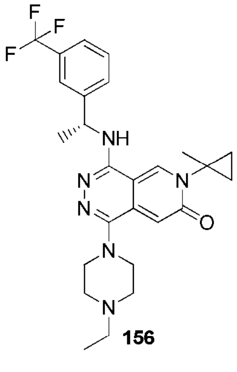

156

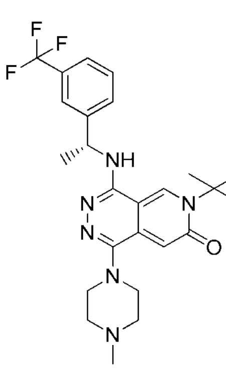
157

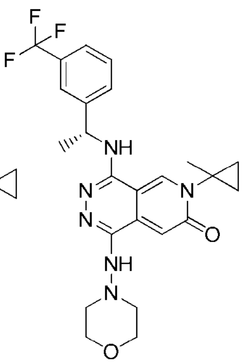
158

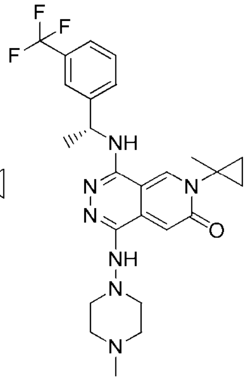
159

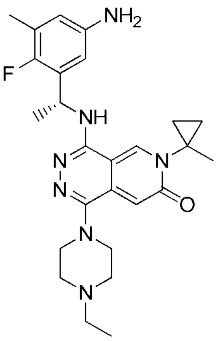
160

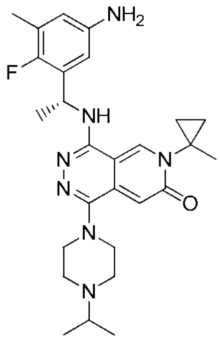
161

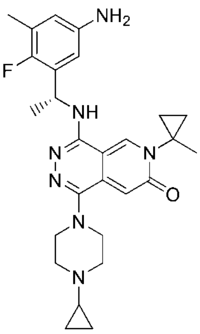
162

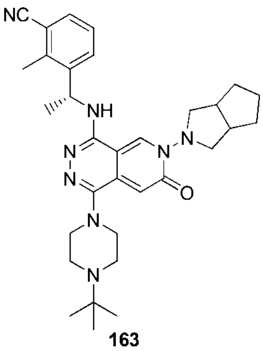
163

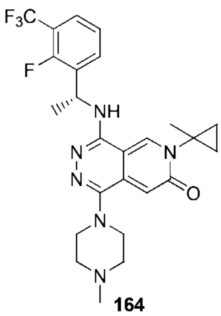
164

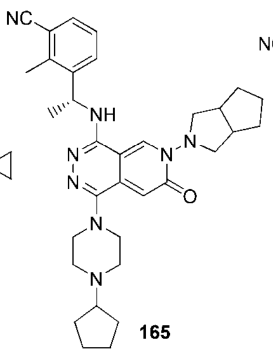
165

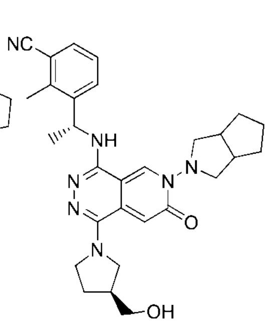
166

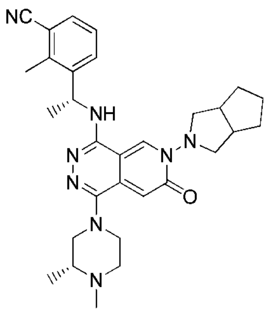
167

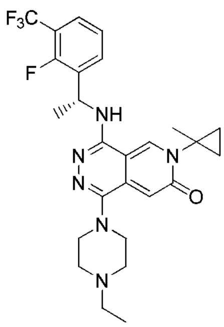
168

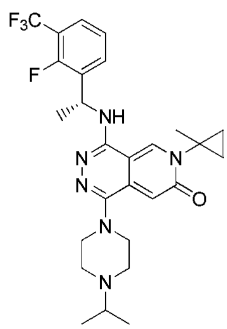
169

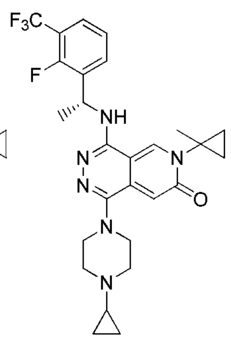
170

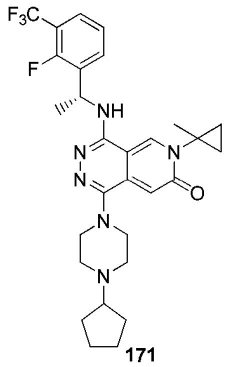
171

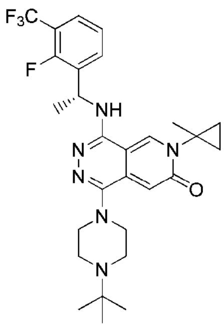
172

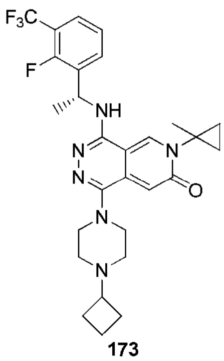
173

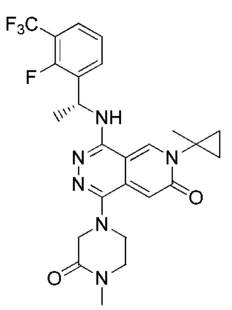
174

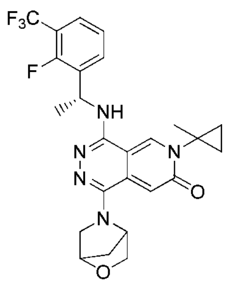
175

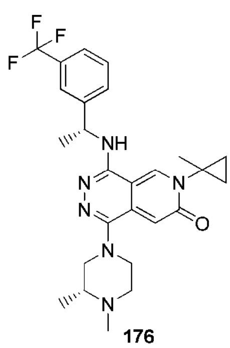
176

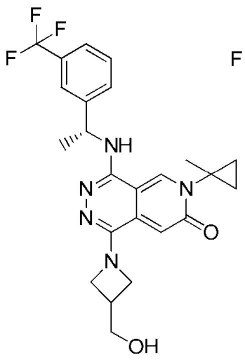

177

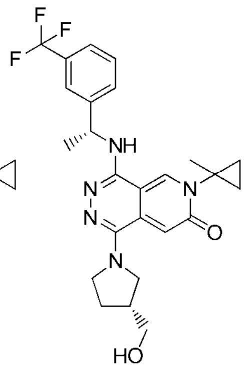

178

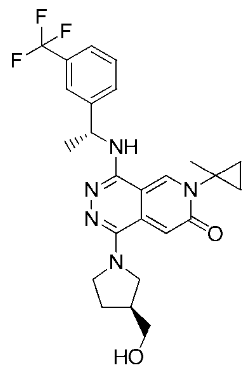

179

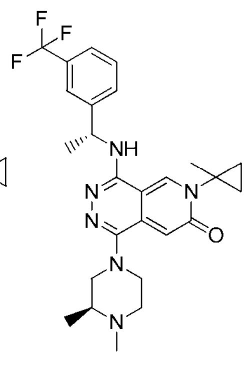

180

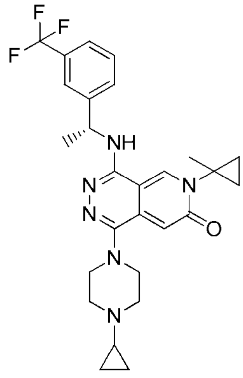

181

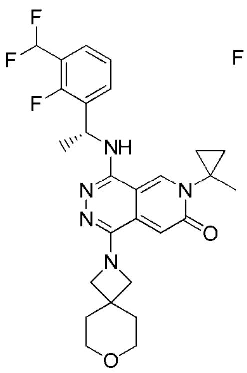

182

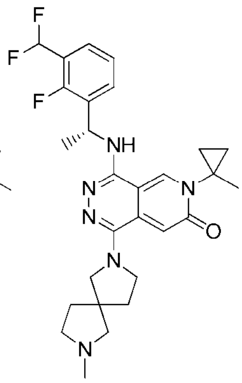

183

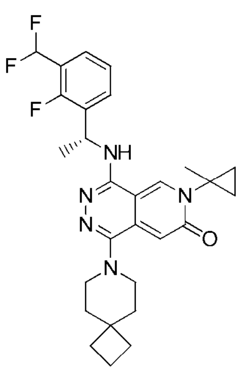

184

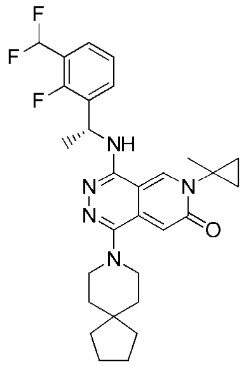

185

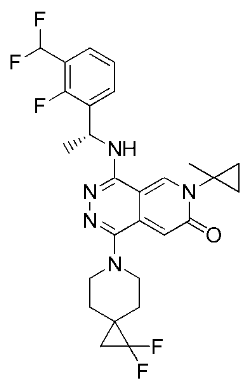

186

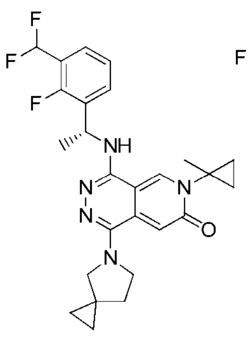

187

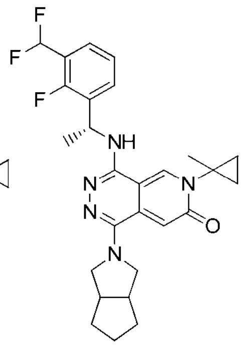

188

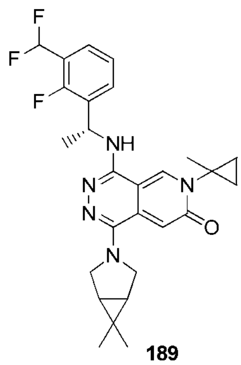

189

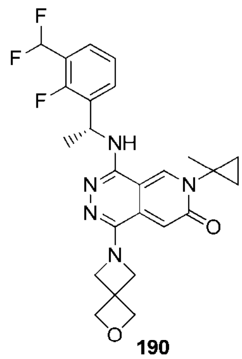

190

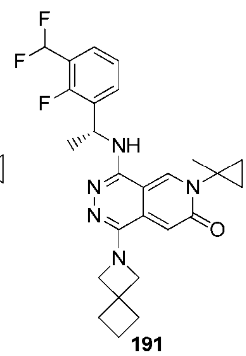

191

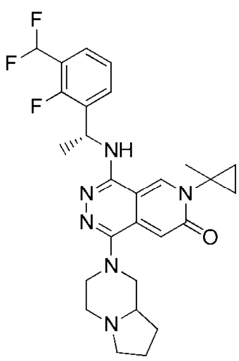

192

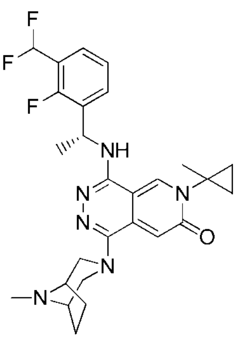

193

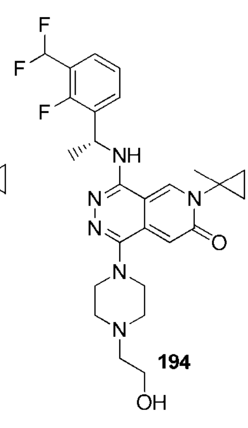

194

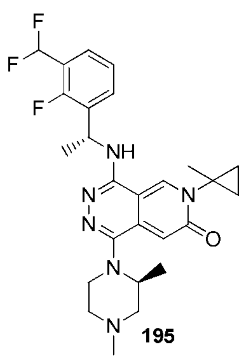

195

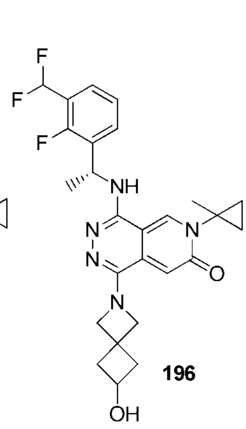

196

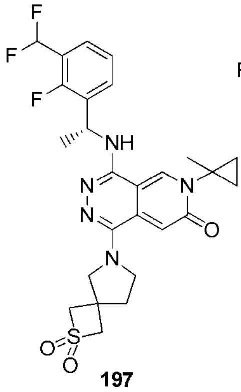
197

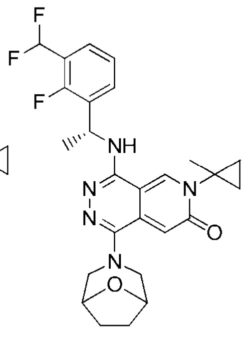
198

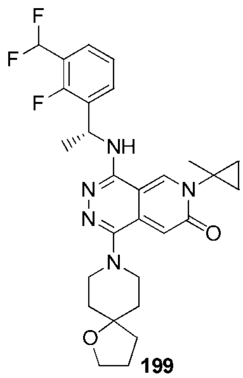
199

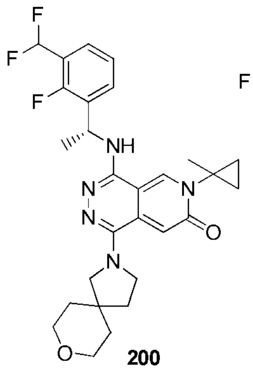
200

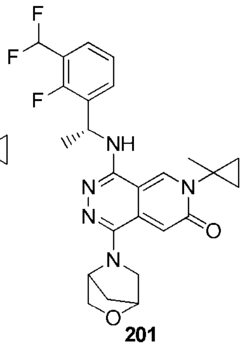
201

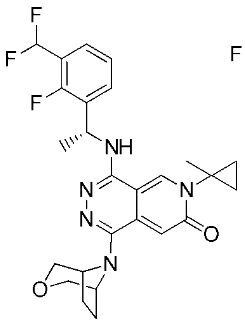
202

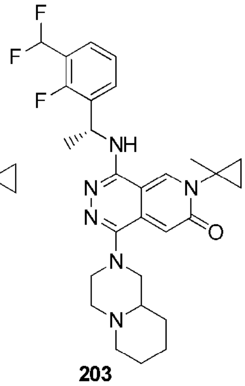
203

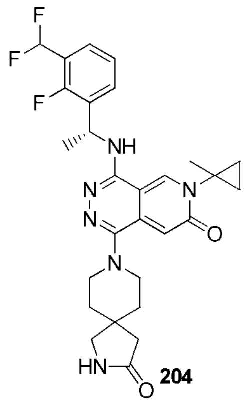
204

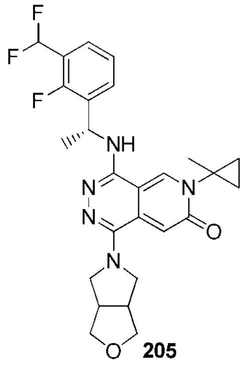
205

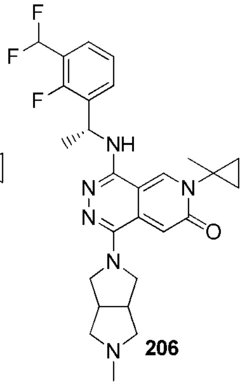
206

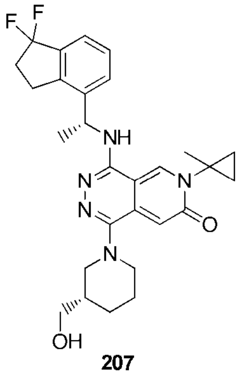
207

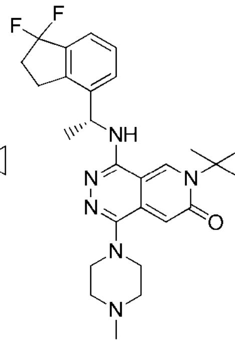
208

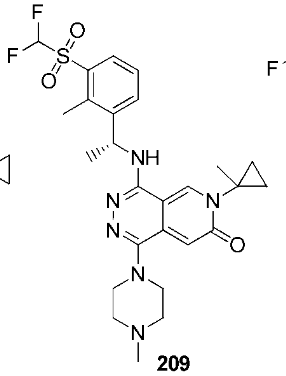
209

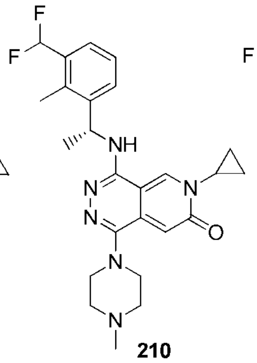
210

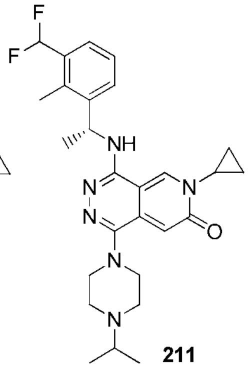
211

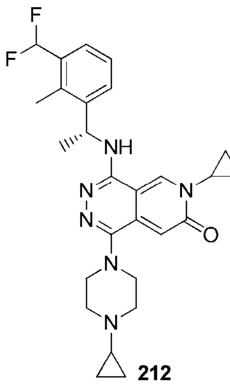
212

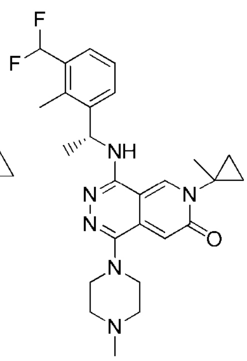
213

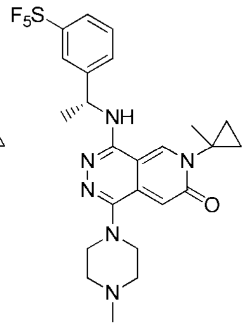
214

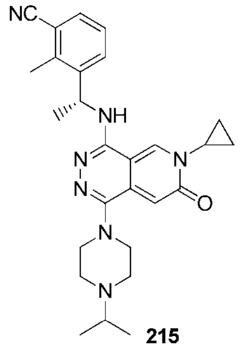
215

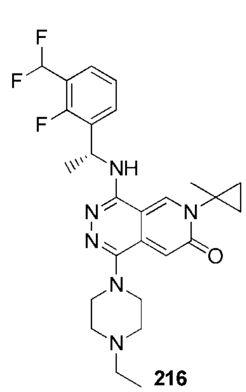
216

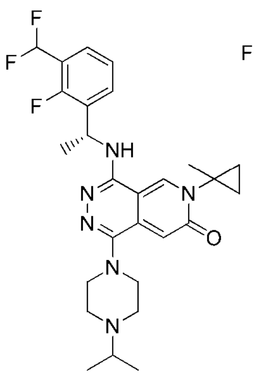
217

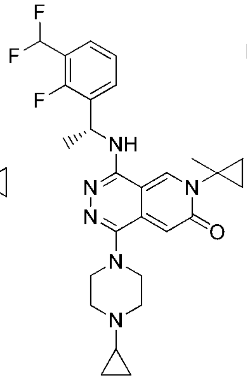
218

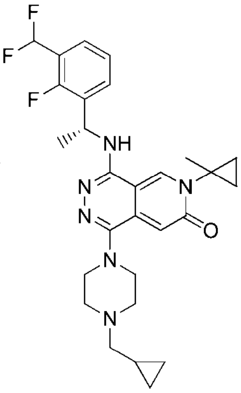
219

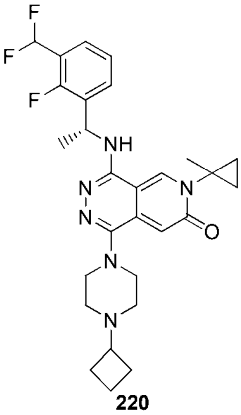
220

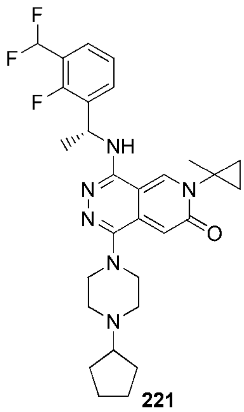
221

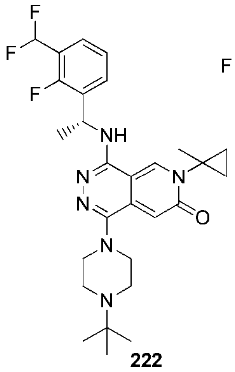
222

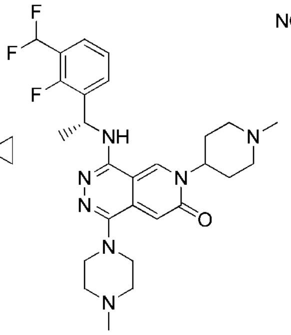
223

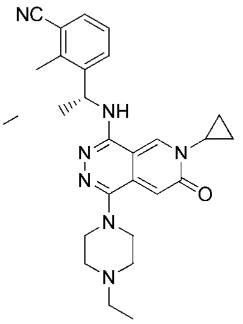
224

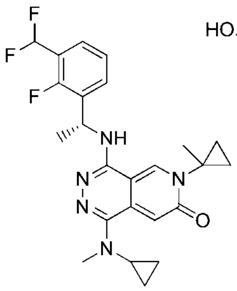
225

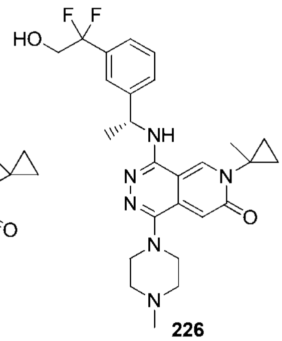
226

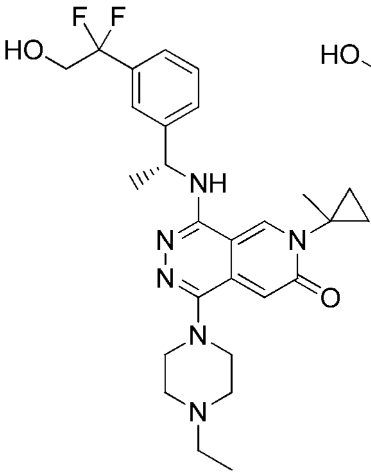
227

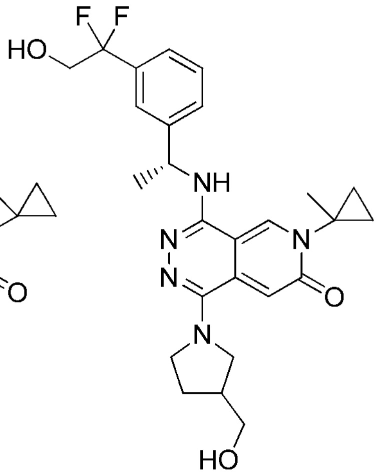
228

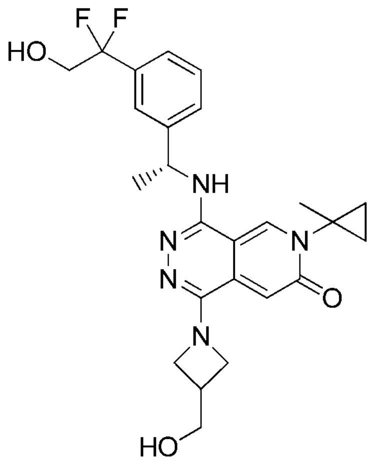
229

.

**16.** A pharmaceutical composition, wherein, the pharmaceutical composition comprises the compound according to any one of claims 1-15, or tautomer, stereoisomer, solvate, metabolite, isotopically labeled compound, pharmaceutically acceptable salt or co-crystal thereof, and a pharmaceutically acceptable excipient or an accessory ingredient.

**17.** Use of the compound according to any one of claims 1-15, or tautomer, stereoisomer, solvate, metabolite, isotopically labeled compound, pharmaceutically acceptable salt or co-crystal thereof, or the pharmaceutical composition according to claim 16 in the manufacture of an SOS1 inhibitor.

**18.** Use of the compound according to any one of claims 1-15, or tautomer, stereoisomer, solvate, metabolite, isotopically labeled compound, pharmaceutically acceptable salt or co-crystal thereof, or the pharmaceutical composition according to claim 16 in the manufacture of a medicament for treating a disease mediated by SOS1.

**19.** The use according to claim 18, wherein, the disease is selected from: cancer, pathogenic skin rash disease.

**20.** The use according to claim 19, wherein, the cancer is selected from: non-small cell lung cancer, lung cancer, pancreatic cancer, ovarian cancer, bladder cancer, prostate cancer, chronic granulocytic leukemia, colorectal cancer, brain cancer, liver cancer, kidney cancer, gastric cancer, and breast cancer;
the pathogenic skin rash disease is selected from: Noonan syndrome, cardiofaciocutaneous syndrome, hereditary gingival fibromatosis type I.

**21.** Use of the compound according to any one of claims 1-15, or tautomer, stereoisomer, solvate, metabolite, isotopically labeled compound, pharmaceutically acceptable salt or co-crystal thereof, or the pharmaceutical composition according to claim 16 in the manufacture of a medicament for treating a disease causing overexpression of an SOS1 protein.

**22.** Use of the compound according to any one of claims 1-15, or tautomer, stereoisomer, solvate, metabolite, isotopically labeled compound, pharmaceutically acceptable salt or co-crystal thereof, or the pharmaceutical composition according to claim 16 in the manufacture of a medicament for treating a disease caused by overexpression of an SOS1 protein.

**INTERNATIONAL SEARCH REPORT**

International application No. **PCT/CN2024/096254**

**A. CLASSIFICATION OF SUBJECT MATTER**

C07D471/04(2006.01)i； C07D237/00(2006.01)i； A61K31/5025(2006.01)i； A61P35/00(2006.01)i

According to International Patent Classification (IPC) or to both national classification and IPC

**B. FIELDS SEARCHED**

Minimum documentation searched (classification system followed by classification symbols)

IPC： C07D，A61K，A61P

Documentation searched other than minimum documentation to the extent that such documents are included in the fields searched

Electronic data base consulted during the international search (name of data base and, where practicable, search terms used)

CNTXT, ENTXTC, WPABS, CNKI, STN(REGISTRY, MARPAT, CAPLUS): 哒嗪, 吡啶酮, 抑制剂, 癌, 肿瘤, pyridazopyridone, SOS1, inhibitor, cancer, tumor, 结构检索, structural search

**C. DOCUMENTS CONSIDERED TO BE RELEVANT**

| Category* | Citation of document, with indication, where appropriate, of the relevant passages | Relevant to claim No. |
|---|---|---|
| PX | WO 2024002318 A1 (SICHUAN HUIYU PHARMACEUTICAL CO., LTD. et al.) 04 January 2024 (2024-01-04)<br>abstract and claims 1-38 | 1-22 |
| PX | WO 2023143147 A1 (SHANGHAI EUREGEN BIOPHARMA CO., LTD.) 03 August 2023 (2023-08-03)<br>abstract and claims 1-13 | 1-22 |
| PX | WO 2023135260 A1 (JAZZ PHARMACEUTICALS IRELAND LIMITED et al.) 20 July 2023 (2023-07-20)<br>claims 1 and 47-57 | 1-22 |
| A | CN 115135315 A (MIRATI THERAPEUTICS, INC.) 30 September 2022 (2022-09-30)<br>abstract and claims 1-88 | 1-22 |
| A | WO 2022271679 A1 (MIRATI THERAPEUTICS, INC.) 29 December 2022 (2022-12-29)<br>entire document | 1-22 |
| PA | WO 2023116902 A1 (BEIJING EARTHWISE TECHNOLOGY CO., LTD.) 29 June 2023 (2023-06-29)<br>entire document | 1-22 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **16 July 2024** | **26 August 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/096254**

**C. DOCUMENTS CONSIDERED TO BE RELEVANT**

| Category* | Citation of document, with indication, where appropriate, of the relevant passages | Relevant to claim No. |
|---|---|---|
| PA | WO 2023125737 A1 (SILEXON AI TECHNOLOGY CO., LTD.) 06 July 2023 (2023-07-06) entire document | 1-22 |

Form PCT/ISA/210 (second sheet) (July 2022)

## INTERNATIONAL SEARCH REPORT
**Information on patent family members**

International application No.
**PCT/CN2024/096254**

| Patent document cited in search report | Publication date (day/month/year) | Patent family member(s) | Publication date (day/month/year) |
|---|---|---|---|
| WO 2024002318 A1 | 04 January 2024 | None | |
| WO 2023143147 A1 | 03 August 2023 | CN 116554166 A | 08 August 2023 |
| WO 2023135260 A1 | 20 July 2023 | None | |
| CN 115135315 A | 30 September 2022 | EP 4076418 A1 | 26 October 2022 |
| | | EP 4076418 A4 | 24 January 2024 |
| | | CA 3161278 A1 | 24 June 2021 |
| | | CO 2022010241 A2 | 21 October 2022 |
| | | TW 202136266 A | 01 October 2021 |
| | | MX 2022007515 A | 19 September 2022 |
| | | KR 20220130126 A | 26 September 2022 |
| | | US 2021188857 A1 | 24 June 2021 |
| | | US 11702418 B2 | 18 July 2023 |
| | | JP 2023507571 A | 24 February 2023 |
| | | IL 294048 A | 01 August 2022 |
| | | BR 112022012106 A2 | 20 September 2022 |
| | | CL 2022001671 A1 | 24 February 2023 |
| | | AU 2020405170 A1 | 30 June 2022 |
| | | US 2023137886 A1 | 04 May 2023 |
| | | WO 2021127429 A1 | 24 June 2021 |
| WO 2022271679 A1 | 29 December 2022 | EP 4358963 A1 | 01 May 2024 |
| WO 2023116902 A1 | 29 June 2023 | TW 202334095 A | 01 September 2023 |
| WO 2023125737 A1 | 06 July 2023 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20190358230 A1
- WO 2019201848 A1
- CN 2023104412 W
- WO 2021127429 A


### Non-patent literature cited in the description


- **BALTANÁS, F.C.** ; **ZARICH, N.** ; **ROJAS-CABAÑEROS, J.M.** ; **SANTOS, E.** *Biochim. Biophys. Acta. Rev. Cancer.*, 2020, vol. 1874, 188445
- **DE CASTRO CARPEÑO, J.** ; **BELDA-INIESTA C.** *Transl Lung Cancer Res.*, 2013, vol. 2 (2), 142-51
- **JEMAL, B. et al.** *CA Cancer J. Clin.*, 2011, vol. 61 (2), 69-90
- **PRIOR, L. et al.** *Cancer Res.*, 2012, vol. 72 (10), 2457-2467
- **LI, L. et al.** *J Exp Clin Cancer Res.*, 2018, vol. 37 (1), 178
- **HUNTER, J.C. et al.** *Mol. Cancer Res.*, 2015, vol. 13 (9), 1325-35
- **KESSLER, D.** ; **GERLACH, D.** ; **KRAUT, N.** ; **MCCONNELL, D.B.** *Curr. Opin. Chem. Biol.*, 2021, vol. 62, 109-118
- **DENAYER et al.** *Genes Chromosomes Cancer*, 2010, vol. 49 (3), 242-52
- Cancer Genome Atlas Research Network. *Nature*, 2014, vol. 511 (7511), 543-50
- **WATANABE et al.** *IUBMB Life*, 2000, vol. 49 (4), 317-20
- **TIMOFEEVA et al.** *Int. J. Oncol.*, 2009, vol. 35 (4), 751-60
- **PIERRE et al.** *Biochem.Pharmacol.*, 2011, vol. 82 (9), 1049-56
- **NICHOLS, R.J. et al.** *Nat Cell Biol.*, 2018, vol. 20 (9), 1064-1073
- **HOFMANN, M.H. et al.** *Cancer Discov*, 2020
- **HILLIG, R.C. et al.** *Proc. Nat. Acad. Sci. U S A*, 2019, vol. 116 (7), 2551-2560